# EUROPEAN PATENT APPLICATION

(11) **EP 4 273 946 A1**
(43) Date of publication of application: **08.11.2023**
(21) Application number: 21915736.9
(22) Date of filing: 28.12.2021
(51) Int. Cl.: H01L 51/00, H01L 51/50

(54) **ORGANIC LIGHT-EMITTING DEVICE COMPRISING NOVEL ORGANIC COMPOUND IN EMISSION LAYER**

(30) Priority: 29.12.2020 KR 20200186330
(71) Applicant: SFC Co., Ltd., Cheongju-si, Chungcheongbuk-do 28122 (KR)
(72) Inventor: LEE, Se-Jin, Cheongju-si Chungcheongbuk-do 28122 (KR); PARK, Seok-Bae, Cheongju-si Chungcheongbuk-do 28122 (KR); KIM, Si-In, Cheongju-si Chungcheongbuk-do 28122 (KR); KIM, Hee-Dae, Cheongju-si Chungcheongbuk-do 28122 (KR); CHOI, Yeong-Tae, Cheongju-si Chungcheongbuk-do 28122 (KR); KIM, Ji-Yung, Cheongju-si Chungcheongbuk-do 28122 (KR); KIM, Kyung-Tae, Cheongju-si Chungcheongbuk-do 28122 (KR); KIM, Myeong-Jun, Cheongju-si Chungcheongbuk-do 28122 (KR); KIM, Kyeong-Hyeon, Cheongju-si Chungcheongbuk-do 28122 (KR); LEE, Seung-Soo, Cheongju-si Chungcheongbuk-do 28122 (KR); LEE, Tae-Gyun, Cheongju-si Chungcheongbuk-do 28122 (KR); KIM, Joon-Ho, Cheongju-si Chungcheongbuk-do 28122 (KR)
(74) Representative: Epping - Hermann - Fischer
(86) International application number: PCT/KR2021/019980
(87) International publication number: WO 2022/145938

(57) **Abstract**

The present invention relates to an organic light-emitting device comprising an organic compound represented by [chemical formula A], and, more particularly, to an organic light-emitting device comprising two emission layers between a first electrode and a second electrode and using the organic compound represented by [chemical formula A] in at least one of the two emission layers, [chemical formula A] being the same as that described in the detailed description of the invention.

## Description

### Technical Field

The present disclosure relates to an organic light-emitting diode containing a novel compound in a light-emitting layer thereof and, more specifically, to an organic light-emitting diode including two light-emitting layers a first electrode and a second electrode, wherein at least one of the two light-emitting layers contains a novel organic compound therein, whereby the organic light-emitting diodes exhibits excellent diode characteristics including high luminous efficiency, low driving voltage, and high longevity.

### Background Art

Organic light-emitting diodes (OLEDs), based on self-luminescence, enjoy the advantage of having a wide viewing angle and being able to be made thinner and lighter than liquid crystal displays (LCDs). In addition, an OLED display exhibits a very fast response time. Accordingly, OLEDs find applications in the illumination field as well as the full-color display field.

In general, the term "organic light-emitting phenomenon" refers to a phenomenon in which electrical energy is converted to light energy by means of an organic material. An OLED using the organic light-emitting phenomenon has a structure usually comprising an anode, a cathode, and an organic material layer interposed therebetween. In this regard, the organic material layer may be, for the most part, of a multilayer structure consisting of different materials, for example, a hole injecting layer, a hole transport layer, a light-emitting layer, an electron transport layer, and an electron injecting layer, in order to improve the efficiency and stability of the organic light-emitting diode (OLED). In the organic light-emitting diode having such a structure, when a voltage is applied between the two electrodes, a hole injected from the anode migrates to the organic layer while an electron is released from the cathode and moves toward the organic layer. In the luminescent zone, the hole and the electron recombine to produce an exciton. When the exciton returns to the ground state from the excited state, the molecule of the organic layer emits light. Such an organic light-emitting diode is known to have characteristics such as self-luminescence, high luminance, high efficiency, low driving voltage, a wide viewing angle, high contrast, and high-speed response.

Materials used as organic layers in OLEDs may be divided into luminescent materials and charge transport materials, for example, a hole injection material, a hole transport material, an electron injection material, and an electron transport material. As for the luminescent materials, there are two main families of OLED: those based on small molecules and those employing polymers. The light-emitting mechanism forms the basis for classification of the luminescent materials as fluorescent or phosphorescent materials, which use excitons in singlet and triplet states, respectively.

Meanwhile, when a single material is employed as the luminescent material, intermolecular actions cause the wavelength of maximum luminescence to shift toward a longer wavelength, decreasing color purity or attenuating light with consequent reduction in the efficiency of the diode. In this regard, a host-dopant system may be used as a luminescent material so as to increase the color purity and the light emission efficiency through energy transfer.

This is based on the principle whereby, when a dopant is smaller in energy band gap than a host accounting for the light-emitting layer, the addition of a small amount of the dopant to the host generates excitons from the light-emitting layer so that the excitons are transported to the dopant, emitting light at high efficiency. Here, light of desired wavelengths can be obtained depending on the kind of dopant because the wavelength of the host moves to the wavelength range of the dopant.

For use as host compounds in a light-emitting layer, heterocyclic compounds have been recently studied. With regard to related art, reference may be made to Korean Patent No. 10-2016-0089693 A (July 28, 2016), which discloses a compound structured to have a dibenzofuran ring moiety bonded to an anthracene ring, and an organic light-emitting diode including same. Also, Korean patent No. 10-0874749 (December 29, 2008) introduces a blue light-emitting compound in which an arylamine is coupled to a pyrene ring, and an organic light-emitting diode including same. In addition, Korean Patent No. 10-2017-0055743 (May 22, 2017) discloses a compound in which an aryl substituent or a heteroaryl substituent is bonded to a fused fluorene ring bearing a heteroatom such as oxygen, nitrogen, sulfur, etc., and an organic light-emitting diode including same.

Despites a variety of types of compounds prepared for use in light emitting layers in organic light emitting diodes including the related arts, there is still a continuing need to develop an organic light-emitting diode that can be stably driven at a lower voltage with high efficiency and longevity.

### Disclosure

### Technical Problem

Therefore, an aspect of the present disclosure is to provide an organic light-emitting diode (OLED) employing a novel organic compound as a host material therein and exhibiting characteristics including high luminous efficiency, low-voltage driving, and high longevity.

### Technical Solution

In order to accomplish the purpose, the present disclosure provides an organic light-emitting diode, including: a first electrode; a second electrode facing the first electrode, and a first light-emitting layer including a first host and a first dopant and a second light-emitting layer including a second host and a second dopant, sequentially disposed between the first electrode and the second electrode, wherein at least one of the first host and the second host include at least one compound represented by the following Chemical Formula A: 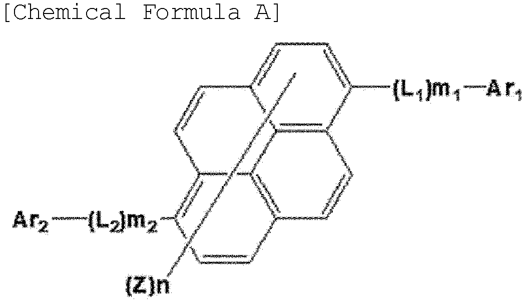 wherein,
linkers L₁ and L₂, which are same or different, are each independently selected from a single bond, a substituted or unsubstituted arylene of 6 to 30 carbon atoms, and a substituted or unsubstituted heteroarylene of 2 to 30 carbon atoms;
m₁ and m₂, which are same or different, are each independently an integer of 1 to 2, wherein when m₁ and m₂ are 2, the corresponding L₁'s are same or different and the L₂'s are same or different;
Ar₁ and Ar₂, which are same or different, are each independently any one selected from a substituted or unsubstituted aryl of 6 to 50 carbon atoms, a substituted or unsubstituted heteroaryl of 2 to 50 carbon atoms, a substituted or unsubstituted alkyl of 1 to 30 carbon atoms, a substituted or unsubstituted cycloalkyl of 3 to 30 carbon atoms, a substituted or unsubstituted alkylsilyl of 1 to 30 carbon atoms, and a substituted or unsubstituted arylsilyl of 6 to 30 carbon atoms;
Z is any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
n is an integer of 0 to 8, wherein when n is 2 or higher, the corresponding Z's are same or different;
the aromatic carbons of the pyrene ring moiety to which the substituent Z or Ar-(L)m is not bonded each have a hydrogen or deuterium atom bonded thereto,
wherein the term 'substituted' in the expression "a substituted or unsubstituted" means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

### Advantageous Effects

Structured to have two light-emitting layers at least one of which employs the organic compound represented by Chemical Formula A as a host material, the organic light-emitting diode can be driven at a lower voltage with improved luminous efficiency and longevity, compared to conventional organic light-emitting diodes.

### Description of Drawings

Figure is a schematic diagram of an OLED according to some embodiments of the present disclosure.

### Best Mode for Invention

Hereinafter, exemplary embodiments which can be easily implemented by those skilled in the art will be described with reference to the accompanying drawing. In each drawing of the present disclosure, sizes or scales of components may be enlarged or reduced from their actual sizes or scales for better illustration, and known components may not be depicted therein to clearly show features of the present disclosure. Therefore, the present disclosure is not limited to the drawings. When describing the principle of the embodiments of the present disclosure in detail, details of well-known functions and features may be omitted to avoid unnecessarily obscuring the presented embodiments.

In drawings, for convenience of description, sizes of components may be exaggerated for clarity. For example, since sizes and thicknesses of components in drawings are arbitrarily shown for convenience of description, the sizes and thicknesses are not limited thereto. Furthermore, throughout the description, the terms "on" and "over" are used to refer to the relative positioning, and mean not only that one component or layer is directly disposed on another component or layer but also that one component or layer is indirectly disposed on another component or layer with a further component or layer being interposed therebetween. Also, spatially relative terms, such as "below", "beneath", "lower", and "between" may be used herein for ease of description to refer to the relative positioning.

Throughout the specification, when a portion may "comprise" or "include" a certain constituent element, unless explicitly described to the contrary, it may not be construed to exclude another constituent element but may be construed to further include other constituent elements. Further, throughout the specification, the word "on" means positioning on or below the object portion, but does not essentially mean positioning on the lower side of the object portion based on a gravity direction.

The present disclosure provides an organic light-emitting diode, including: a first electrode; a second electrode facing the first electrode, and a first light-emitting layer including a first host and a first dopant and a second light-emitting layer including a second host and a second dopant, sequentially disposed between the first electrode and the second electrode, wherein at least one of the first host and the second host include at least one compound represented by the following Chemical Formula A: 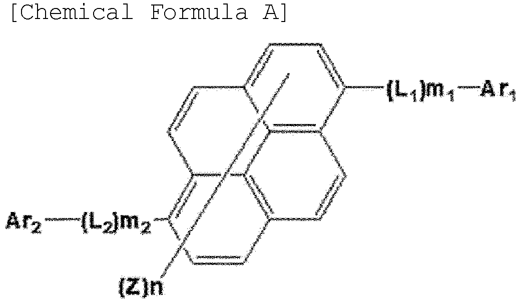 wherein,
linkers L₁ and L₂, which are same or different, are each independently selected from a single bond, a substituted or unsubstituted arylene of 6 to 30 carbon atoms, and a substituted or unsubstituted heteroarylene of 2 to 30 carbon atoms;
m₁ and m₂, which are same or different, are each independently an integer of 1 to 2, wherein when m₁ and m₂ are 2, the corresponding L₁'s are same or different and the L₂'s are same or different;
Ar₁ and Ar₂, which are same or different, are each independently any one selected from a substituted or unsubstituted aryl of 6 to 50 carbon atoms, a substituted or unsubstituted heteroaryl of 2 to 50 carbon atoms, a substituted or unsubstituted alkyl of 1 to 30 carbon atoms, a substituted or unsubstituted cycloalkyl of 3 to 30 carbon atoms, a substituted or unsubstituted alkylsilyl of 1 to 30 carbon atoms, and a substituted or unsubstituted arylsilyl of 6 to 30 carbon atoms;
Z is any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
n is an integer of 0 to 8, wherein when n is 2 or higher, the corresponding Z's are same or different;
the aromatic carbons of the pyrene ring moiety to which the substituent Z or Ar-(L)m is not bonded each have a hydrogen or deuterium atom bonded thereto,
wherein the term 'substituted' in the expression "a substituted or unsubstituted" means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

The expression indicating the number of carbon atoms, such as "a substituted or unsubstituted alkyl of 1 to 30 carbon atoms", "a substituted or unsubstituted aryl of 5 to 50 carbon atoms", etc. means the total number of carbon atoms of, for example, the alkyl or aryl radical or moiety alone, exclusive of the number of carbon atoms of substituents attached thereto. For instance, a phenyl group with a butyl at the para position falls within the scope of an aryl of 6 carbon atoms, even though it is substituted with a butyl radical of 4 carbon atoms.

As used herein, the term "aryl" means an organic radical derived from an aromatic hydrocarbon by removing one hydrogen that is bonded to the aromatic hydrocarbon. Further, the aromatic system may include a fused ring that is formed by adjacent substituents on the aryl radical.

Concrete examples of the aryl include phenyl, o-biphenyl, m-biphenyl, p-biphenyl, o-terphenyl, m-terphenyl, p-terphenyl, naphthyl, anthryl, phenanthryl, pyrenyl, indenyl, fluorenyl, tetrahydronaphthyl, perylenyl, chrysenyl, naphthacenyl, and fluoranthenyl at least one hydrogen atom of which may be substituted by a deuterium atom, a halogen atom, a hydroxy, a nitro, a cyano, a silyl, an amino (-NH₂, -NH(R), -N(R') (R") wherein R' and R" are each independently an alkyl of 1 to 10 carbon atoms, in this case, called "alkylamino"), an amidino, a hydrazine, a hydrazone, a carboxyl, a sulfonic acid, a phosphoric acid, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 6 to 24 carbon atoms, a heteroaryl of 2 to 24 carbon atoms, or a heteroarylalkyl of 2 to 24 carbon atoms.

The substituent heteroaryl used in the compound of the present disclosure refers to a cyclic aromatic system of 2 to 24 carbon atoms bearing as ring members one to three heteroatoms selected from among N, O, P, Si, S, Ge, Se, and Te. In the aromatic system, two or more rings may be fused. One or more hydrogen atoms on the heteroaryl may be substituted by the same substituents as on the aryl.

In addition, the term "heteroaromatic ring", as used herein, refers to an aromatic hydrocarbon ring bearing as aromatic ring members 1 to 3 heteroatoms selected particularly from N, O, P, Si, S, Ge, Se, and Te.

As used herein, the term "alkyl" refers to an alkane missing one hydrogen atom and includes linear or branched structures. Examples of the alkyl substituent useful in the present disclosure include methyl, ethyl, propyl, isopropyl, isobutyl, sec-butyl, tert-butyl, pentyl, iso-amyl, and hexyl. At least one hydrogen atom of the alkyl may be substituted by the same substituent as in the aryl.

The term "cyclo" as used in substituents of the present disclosure, such as cycloalkyl, cycloalkoxy, etc., refers to a structure responsible for a mono- or polycyclic ring of saturated hydrocarbons such as alkyl, alkoxy, etc. Concrete examples of cycloalkyl radicals include cyclopropyl, cyclobutyl, cyclopentyl, cyclohexyl, methylcyclopentyl, methylcyclohexyl, ethylcyclopentyl, ethylcyclohexyl, adamantyl, dicyclopentadienyl, decahydronaphthyl, norbornyl, bornyl, and isobornyl. One or more hydrogen atoms on the cycloalkyl may be substituted by the same substituents as on the aryl and it can be applied to cycloalkoxy, as well.

The term "alkoxy" as used in the compounds of the present disclosure refers to an alkyl or cycloalkyl singularly bonded to oxygen. Concrete examples of the alkoxy include methoxy, ethoxy, propoxy, isobutoxy, sec-butoxy, pentoxy, iso-amyloxy, hexyloxy, cyclobutyloxy, cyclopentyloxy, adamantyloxy, dicyclopentyloxy, bornyloxy, and isobornyloxy. One or more hydrogen atoms on the alkoxy may be substituted by the same substituents as on the aryl.

Concrete examples of the arylalkyl used in the compounds of the present disclosure include phenylmethyl(benzyl), phenylethyl, phenylpropyl, naphthylmethyl, and naphthylethyl. One or more hydrogen atoms on the arylalkyl may be substituted by the same substituents as on the aryl.

Concrete examples of the silyl radicals used in the compounds of the present disclosure include trimethylsilyl, triethylsilyl, triphenylsilyl, trimethoxysilyl, dimethoxyphenylsilyl, diphenylmethylsilyl, diphenylvinlysilyl, methylcyclobutylsilyl, and dimethyl furylsilyl. One or more hydrogen atoms on the silyl may be substituted by the same substituents as on the aryl.

As used herein, the term "alkenyl" refers to an unsaturated hydrocarbon group that contains a carbon-carbon double bond between two carbon atoms and the team "alkynyl" refers to an unsaturated hydrocarbon group that contains a carbon-carbon triple bond between two carbon atoms.

As used herein, the term "alkylene" refers to an organic aliphatic radical regarded as derived from a linear or branched saturated hydrocarbon alkane by removal of two hydrogen atoms from different carbon atoms. Concrete examples of the alkylene include methylene, ethylene, propylene, isopropylene, isobutylene, sec-butylene, tert-butylene, pentylene, iso-amylene, hexylene, and so on. One or more hydrogen atoms on the alkylene may be substituted by the same substituents as on the aryl.

Furthermore, as used herein, the term "diarylamino" refers to an amine radical having two identical or different aryl groups bonded to the nitrogen atom thereof, the term "diheteroarylamino" refers to an amine radical having two identical or different heteroaryl groups bonded to the nitrogen atom thereof, and the term "aryl(heteroaryl)amino" refers to an amine radical having an aryl group and a heteroaryl group both bonded to the nitrogen atom thereof.

As more particular examples accounting for the term "substituted" in the expression "substituted or unsubstituted" used for compounds of Chemical Formula A, the compounds may be substituted by at least one substituents selected from the group consisting of a deuterium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 2 to 18 carbon atoms, an alkoxy of 1 to 12 carbon atoms, an alkylamino of 1 to 12 carbon atoms, a diarylamino of 12 to 18 carbon atoms, a diheteroarylamino of 2 to 18 carbon atoms, an aryl(heteroaryl)amino of 7 to 18 carbon atoms, an alkylsilyl of 1 to 12 carbon atoms, an arylsilyl of 6 to 18 carbon atoms, an aryloxy of 6 to 18 carbon atoms, and an arylthionyl of 6 to 18 carbon atoms.

The organic light-emitting diode according to the present disclosure, comprising a first light-emitting layer and a second light-emitting layer, is characterized by including the pyrene derived compound represented by Chemical Formula A as a host for at least one of the first and the second light-emitting layer, wherein the pyrene derivative compound has the substituents Ar₁-(L₁)ₘ₁- and Ar₂-(L₂)ₘ₂- bonded to the pyrene ring at positions 1 and 6, respectively, (see Structural Formula C, below), whereby the organic light-emitting diode can be driven at lower voltages with higher efficiency and longer life span, compared to conventional organic light-emitting diodes: 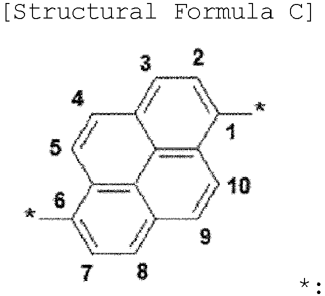 bonding site to linker L₁ or L₂.

In the compound represented by Chemical Formula A according to the present disclosure, the linkers L₁ and L₂ are same or different and may each be independently a substituted or unsubstituted arylene of 6 to 18 carbon atoms. In this regard, m₁ and m₂ are each preferably 1 and the linkers L₁ and L₂ may be same or different and are each independently selected from [Structural Formula 1] and [Structural Formula 2] below: 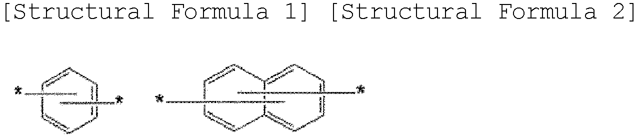
wherein the carbon atoms of the aromatic ring moiety may each have a hydrogen or deuterium atom thereon and
"*-" denotes a bonding site to the pyrene ring moiety, or Ar₁ or Ar₂.

In an embodiment according to the present disclosure, when the linker L₁ is represented by Structural Formula 1, the pyrene ring moiety and the Ar₁ may be at ortho, meta, or para positions on the phenylene moiety of L₁, and preferably at meta positions on the phenylene moiety.

In an embodiment according to the present disclosure, when the linker L₂ is represented by Structural Formula 1, the pyrene ring moiety and the Ar₂ may be at ortho, meta, or para positions on the phenylene moiety of L₂, and preferably at meta positions on the phenylene moiety.

In an embodiment according to the present disclosure, Ar₁ and Ar₂, which are same or different, are each independently a substituted or unsubstituted aryl of 6 to 18 carbon atoms, or a substituted or unsubstituted heteroaryl of 2 to 18 carbon atoms, preferably, Ar₁ and Ar₂, which are same or different, are each independently a substituted or unsubstituted aryl of 10 to 18 carbon atoms.

In an embodiment according to the present disclosure, when Ar₁ and Ar₂ are each a substituted or unsubstituted aryl of 10 to 18 carbon atoms, Ar₁ and Ar₂, which are same or different, are each independently any one selected from 1-naphthyl, 2-naphthyl, 1-fluorene, 2-fluorene, 3-fluorene, 4-fluorene, 1-phenanthrene, 2-phenanthrene, 3-phenanthrene, 4-phenanthrene, and 9-phenanthrene.

In an embodiment according to the present disclosure, when Ar₁ and Ar₂ are same or different and each independently a substituted or unsubstituted aryl of 6 to 18 carbon atoms or a substituted or unsubstituted heteroaryl of 2 to 18 carbon atoms, they may be same or different and are each independently any one selected from [Structural Formula 11] to [Structural Formula 15], below, and preferably an aryl represented by Structural Formula 11: 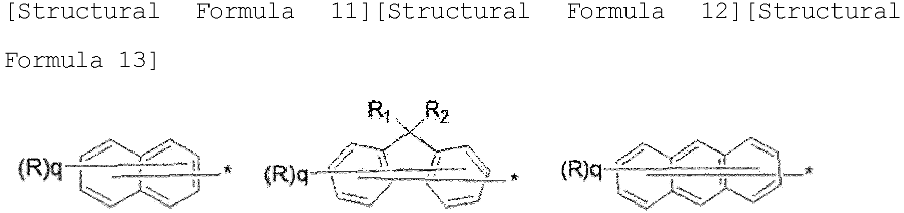 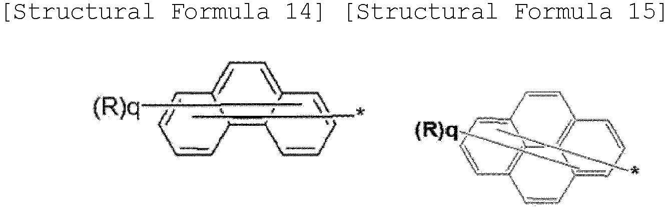
wherein "*-" is a bonding site to the linker L₁ or L₂,
R, R₁, and R₂, which are same or different, are each any one selected from a deuterium atom, a halogen atom, an alkyl of 1 to 12 carbon atoms, a halogenated alkyl of 1 to 24 carbon atoms, a cycloalkyl of 3 to 12 carbon atoms, an aryl of 6 to 18 carbon atoms, an arylalkyl of 7 to 18 carbon atoms, and an alkylaryl of 7 to 18 carbon atoms,
q is an integer of 0 to 9 wherein when q is 2 or higher, the corresponding R's are same or different, and
the carbon atoms of the aromatic ring moieties may each have a hydrogen or deuterium atom when any R is not bonded thereto.

In an embodiment according to the present disclosure, m₁ and m₂ are each 1, the linkers L₁ and L₂ may be same or different and are each independently [Structural Formula 1], and Ar₁ and Ar₂ are same or different and each independently any one selected from [Structural Formula 11] to [Structural Formula 15], below: 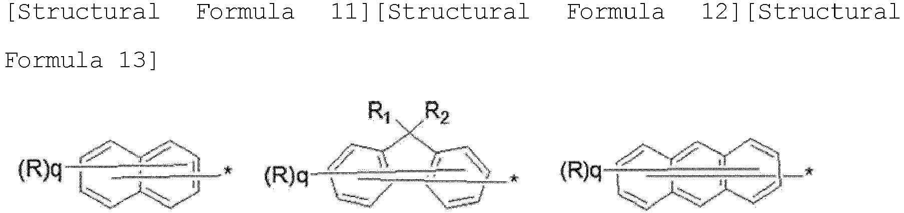 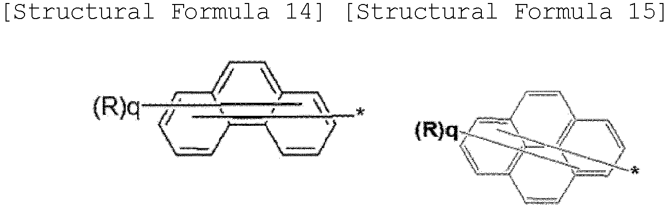 wherein "*-", R, R₁, R₂, and q are each as defined above, and the carbon atoms of the aromatic ring moieties may each have a hydrogen or deuterium atom when any R is not bonded thereto.

In an embodiment according to the present disclosure, Z may be any one selected from a hydrogen atom, a deuterium atom, a halogen atom, a substituted or unsubstituted alkyl of 1 to 12 carbon atoms, a substituted or unsubstituted cycloalkyl of 3 to 12 carbon atoms, and a substituted or unsubstituted aryl of 6 to 18 carbon atoms.

In an embodiment according to the present disclosure, the compound represented by Chemical Formula A may have a structure symmetric about the X-Y axis, except for the substituent Z, as shown in Diagram A, below. That is, the compound represented by Chemical Formula A has a structure in which the substituents Ar₁-(L₁)ₘ₁- and Ar₂-(L₂)ₘ₂- bonded respectively to the pyrene moiety at positions 1 and 6 are structurally identical, and thus is symmetric about the X-Y axis, except for the substituent Z.

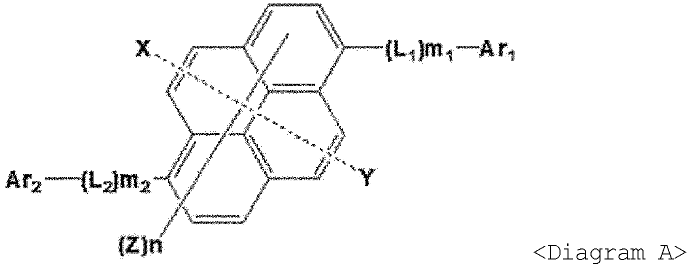

In an embodiment according to the present disclosure, the compound represented by Chemical Formula A may be structurally symmetric about the X-Y axis in Diagram A even when the substituent Z is included. That is, in the compound represented by Chemical Formula A, the substituents Ar₁-(L₁)ₘ₁- and Ar₂-(L₂)ₘ₂- that are bonded respectively to the pyrene moiety at positions 1 and 6 and are structurally identical while the substituent Z is bonded to limited positions of the pyrene moiety or any substituent Z is not bonded to the pyrene moiety, thereby allowing the compound of Chemical Formula A to be symmetric about the X-Y axis.

In addition, the compound represented by Chemical Formula A may be any one selected from [Chemical Formula 1] to [Chemical Formula 180], but is not limited thereto:

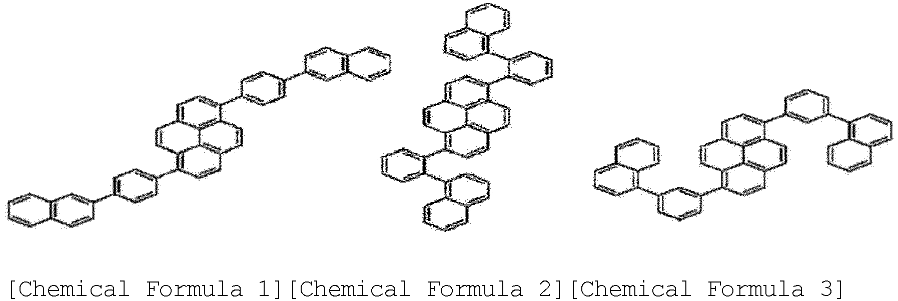

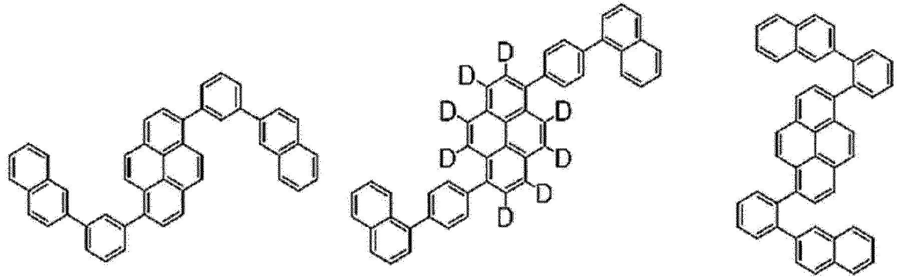

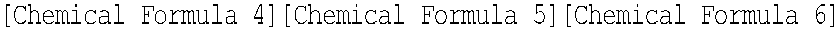

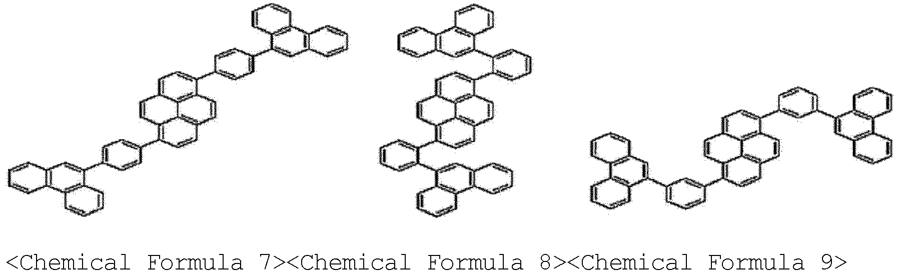

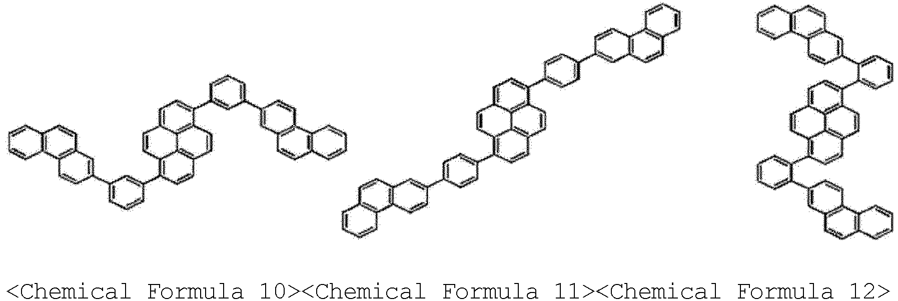

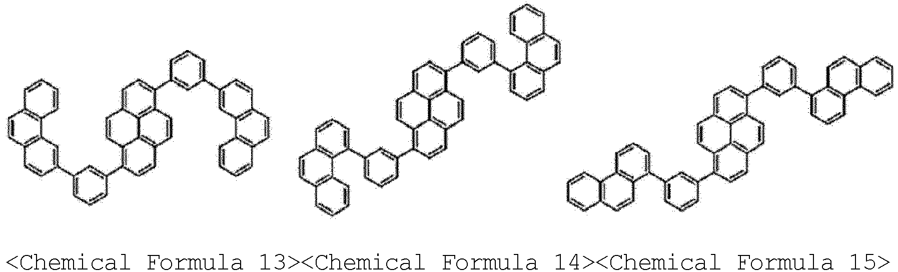

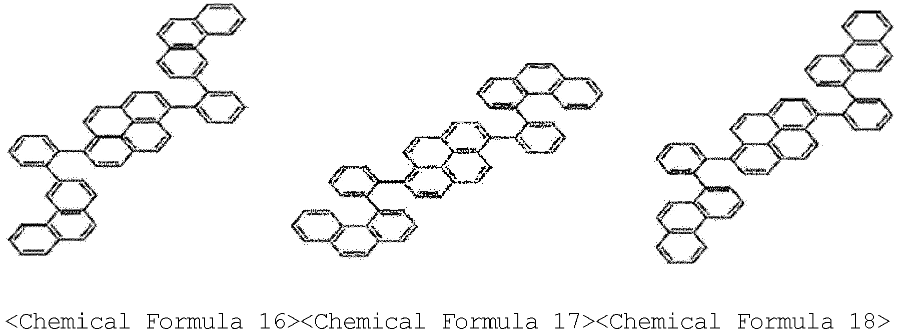

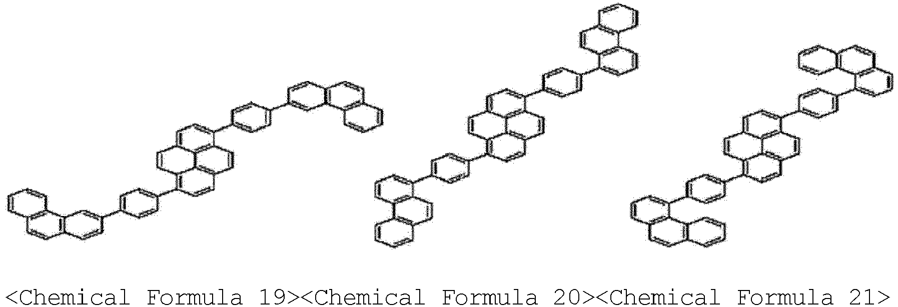

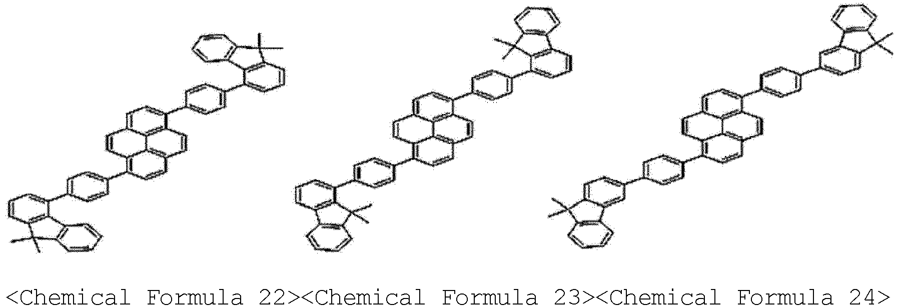

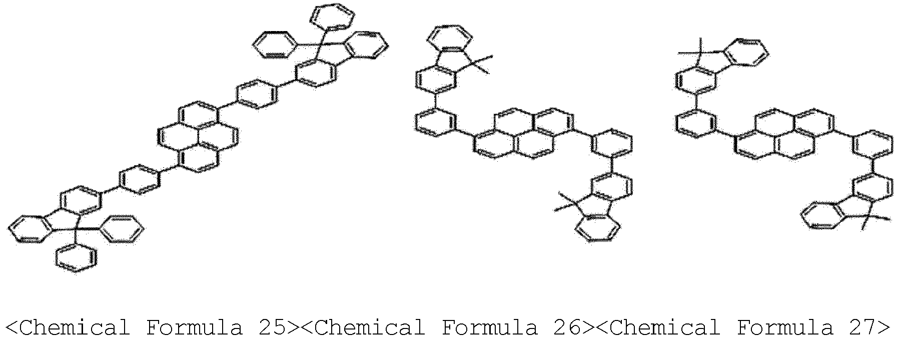

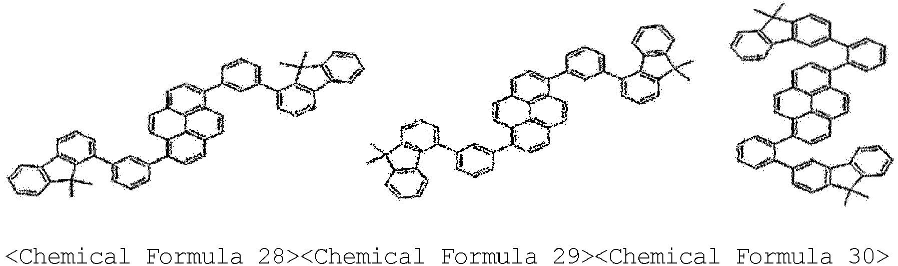

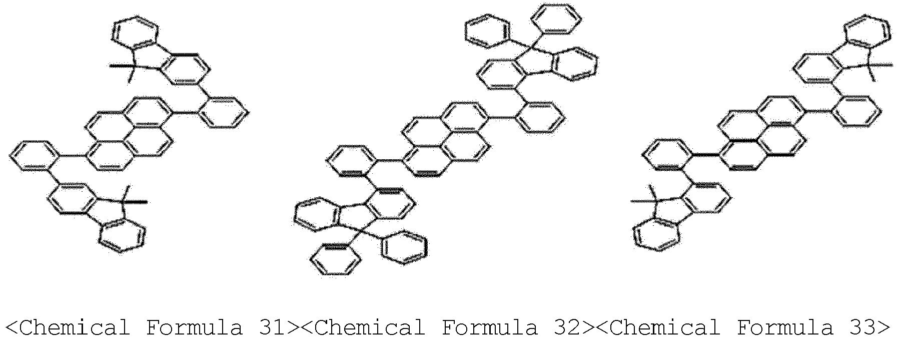

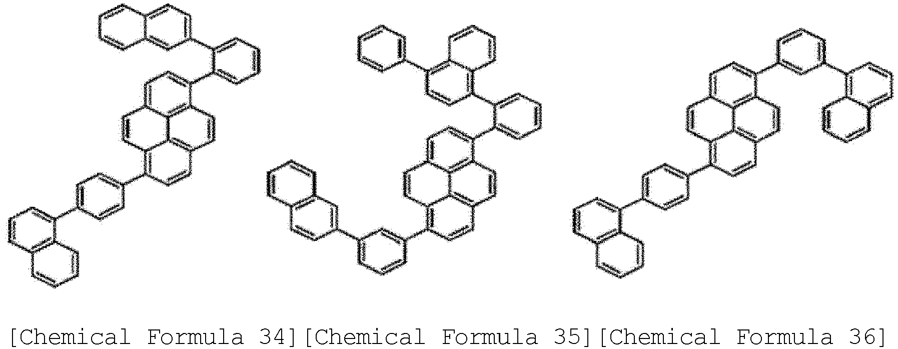

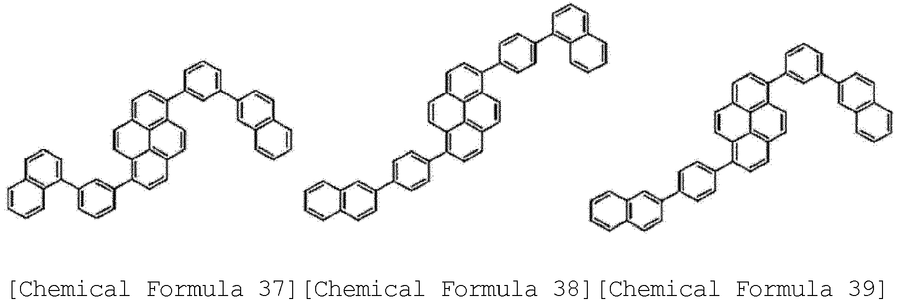

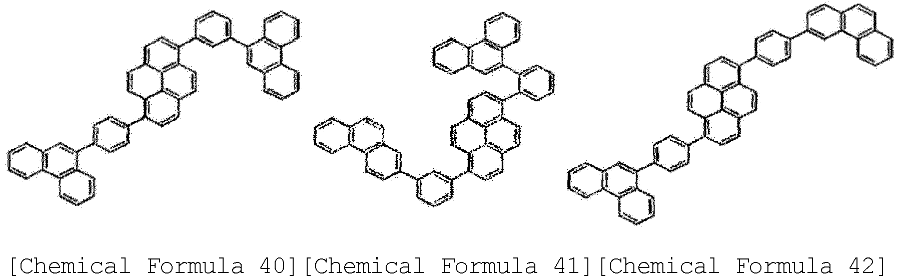

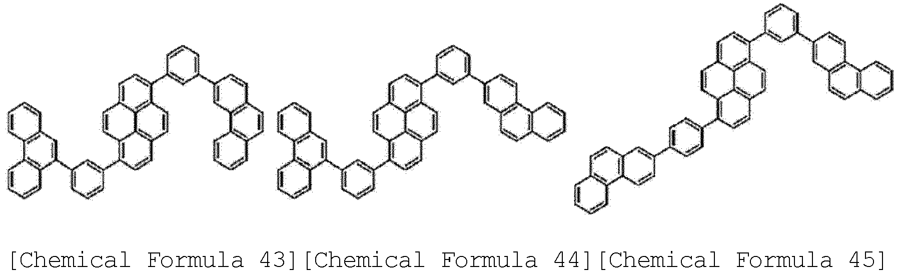

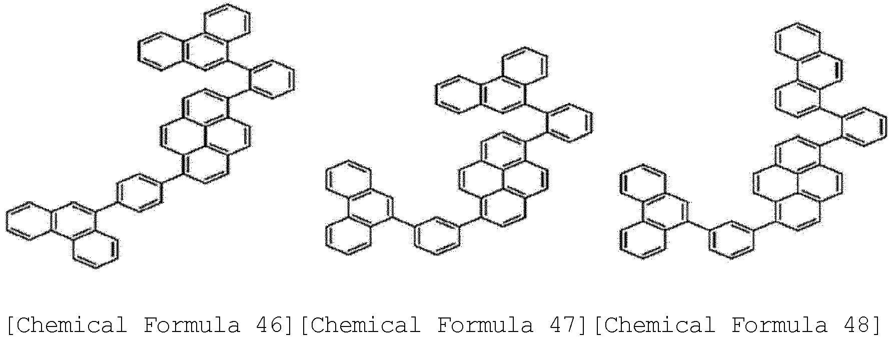

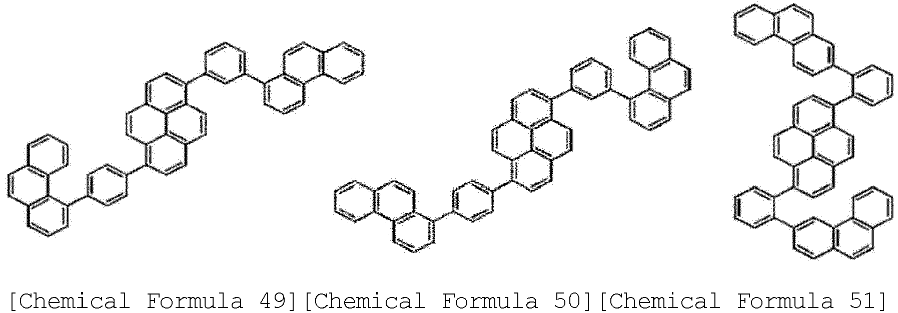

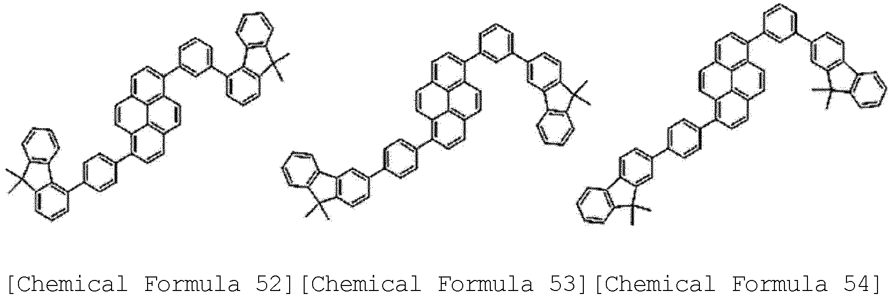

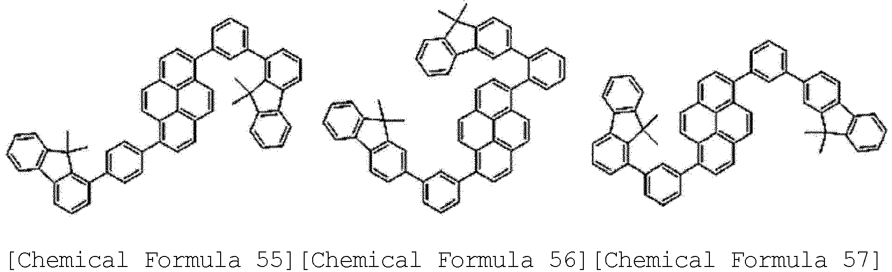

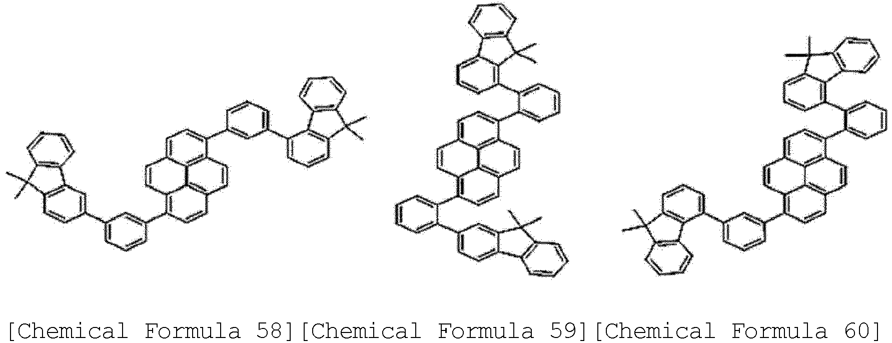

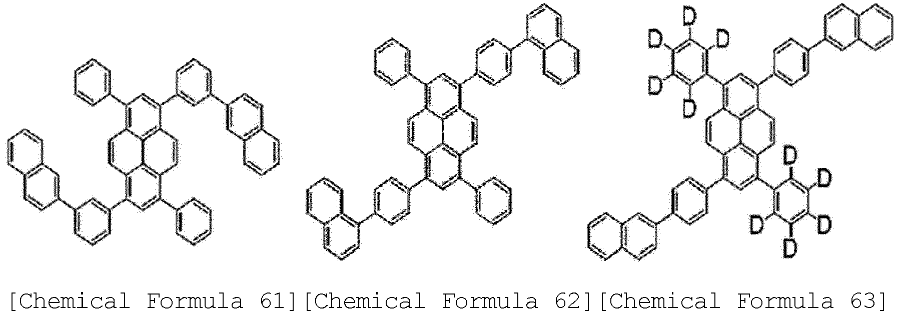

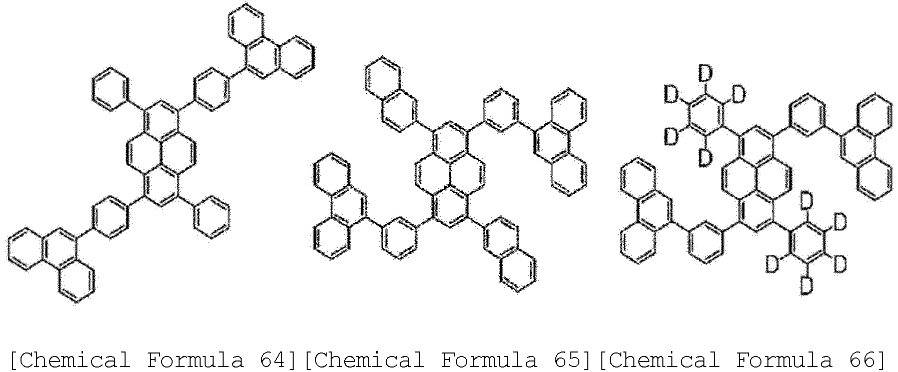

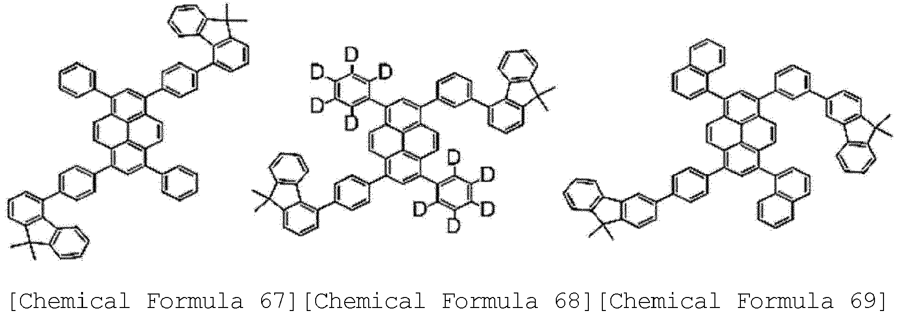

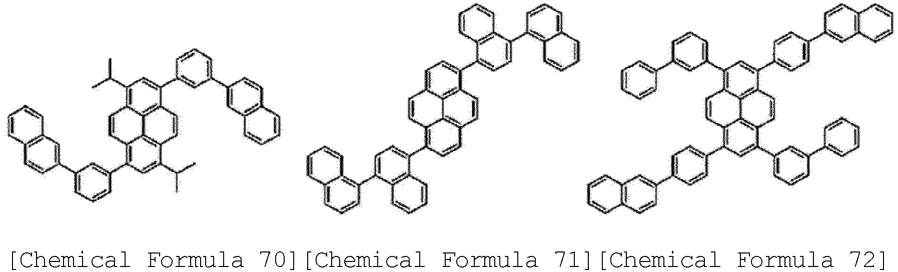

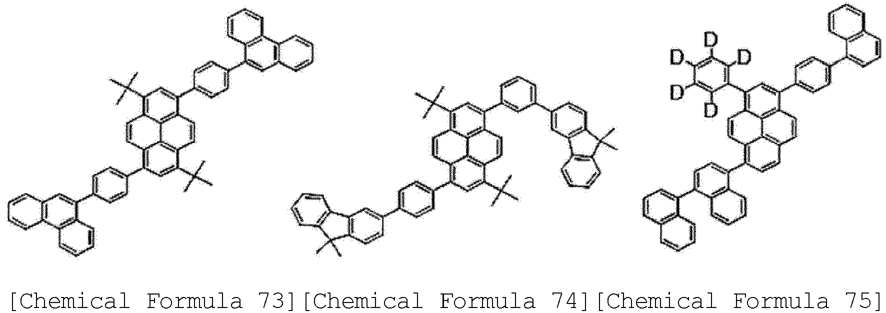

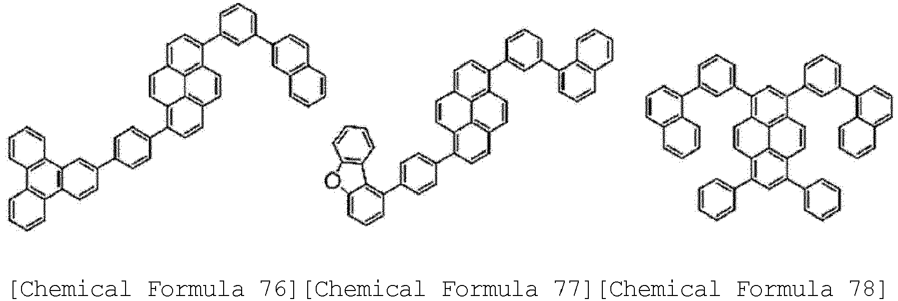

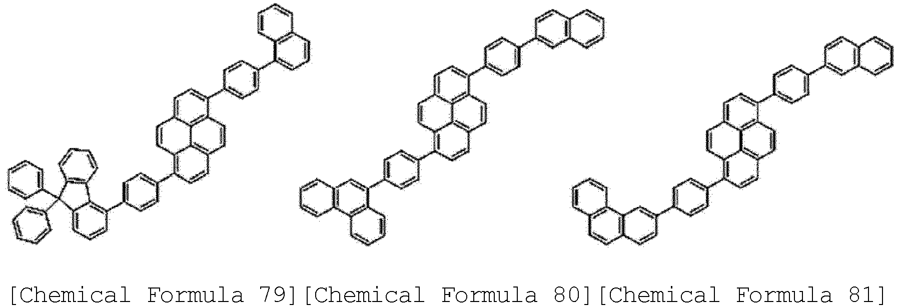

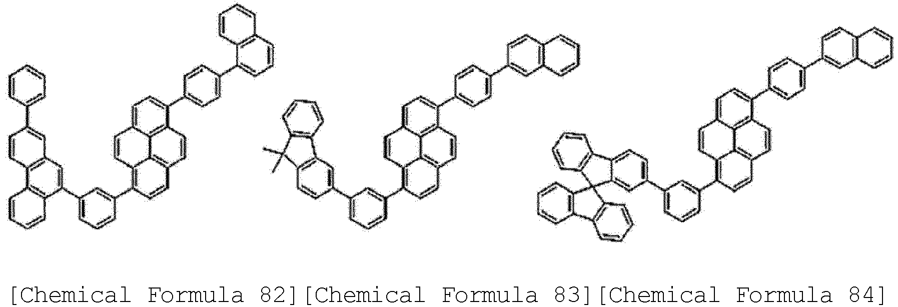

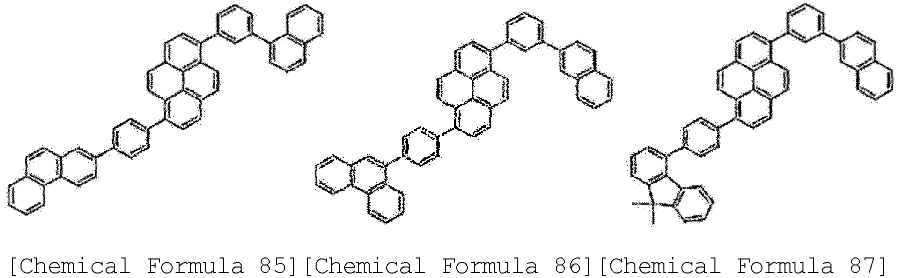

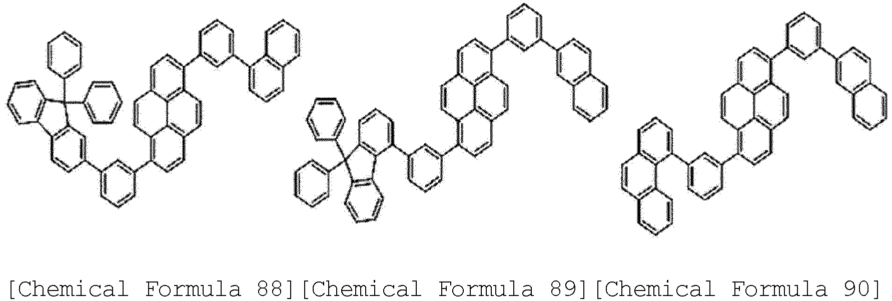

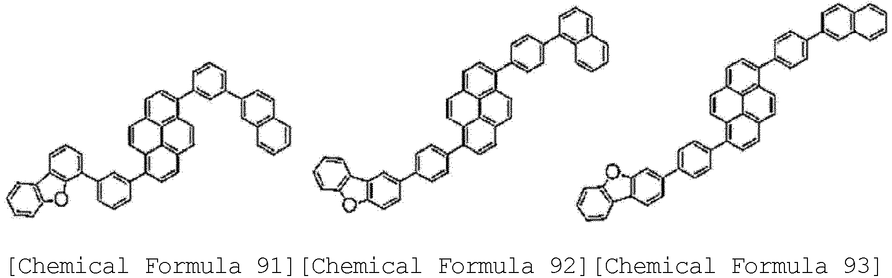

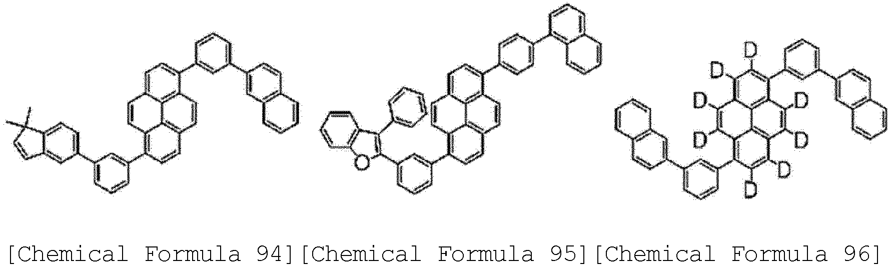

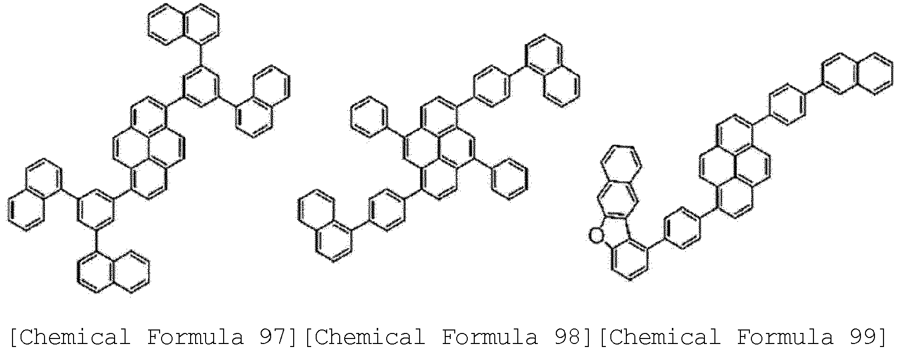

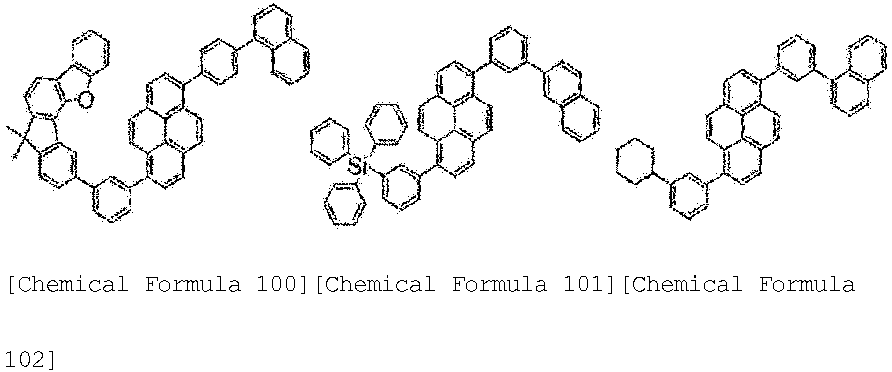

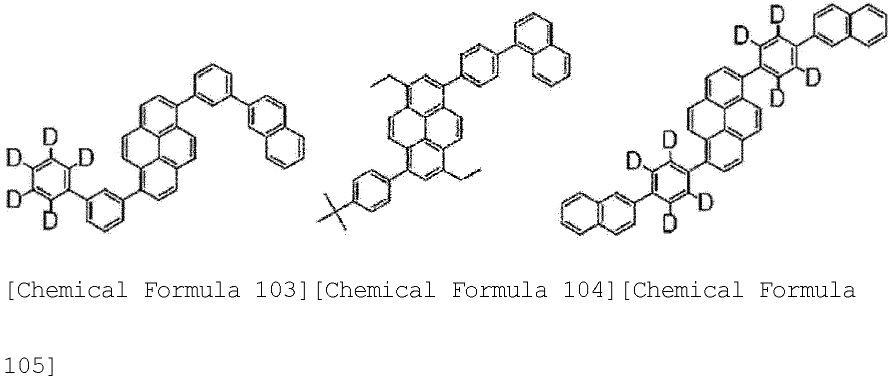

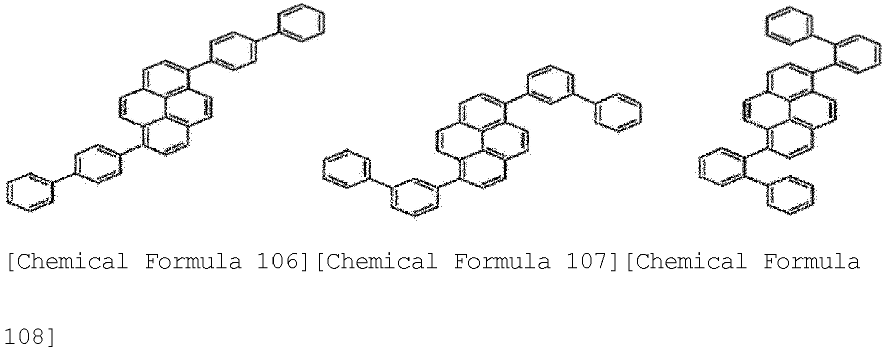

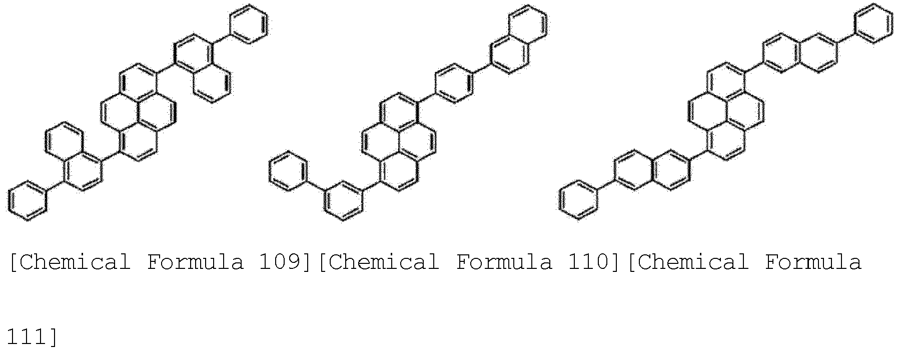

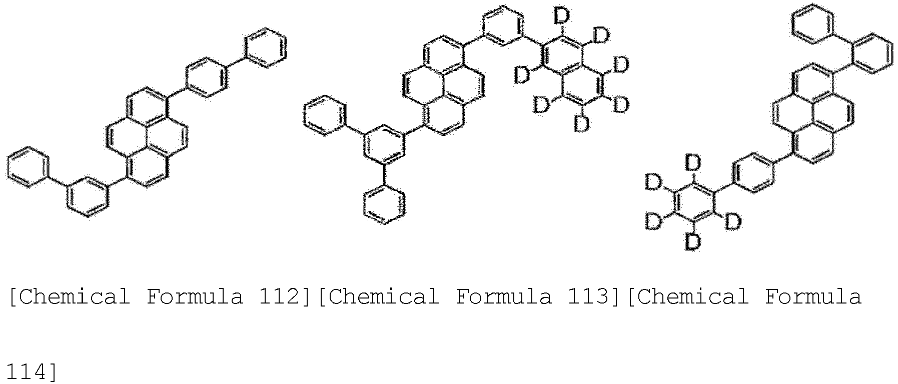

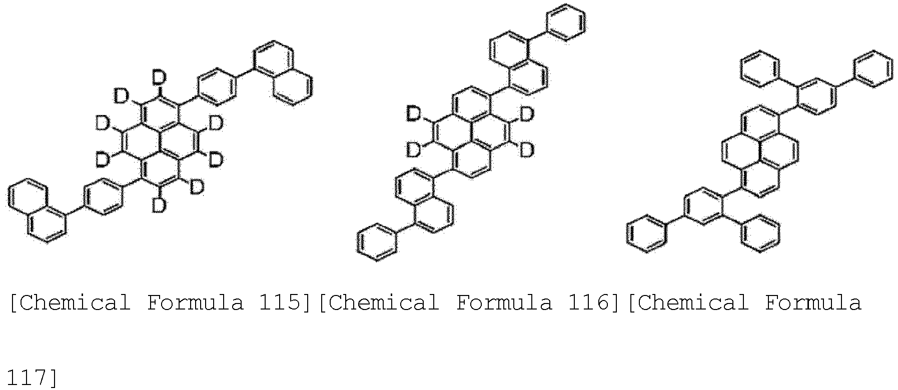

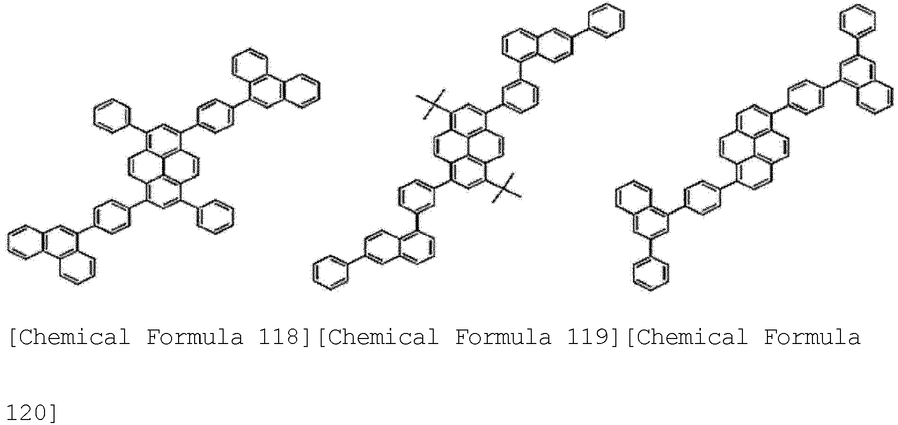

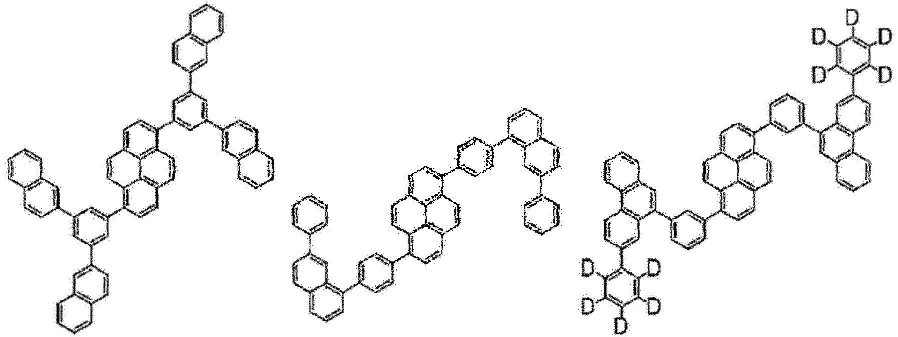

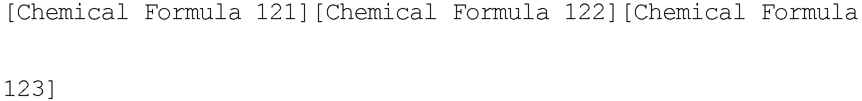

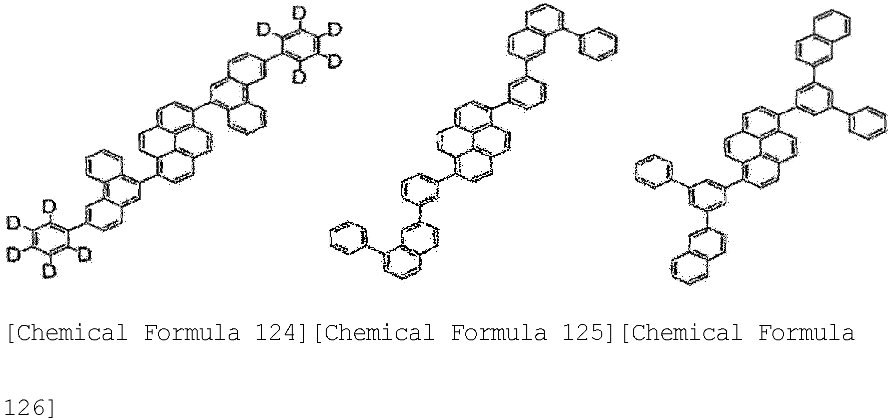

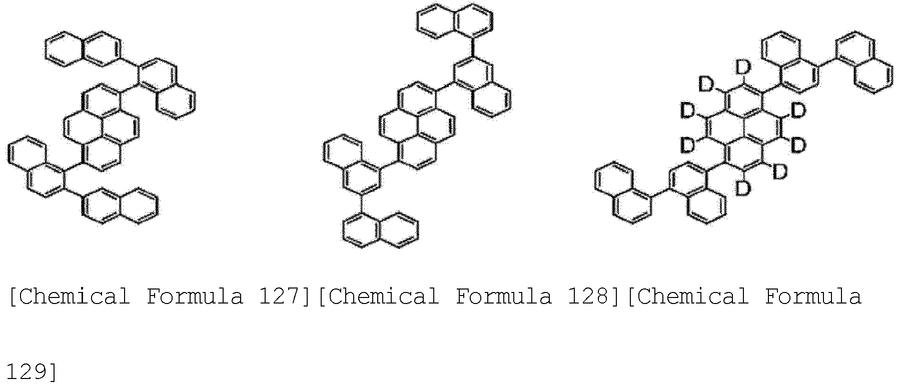

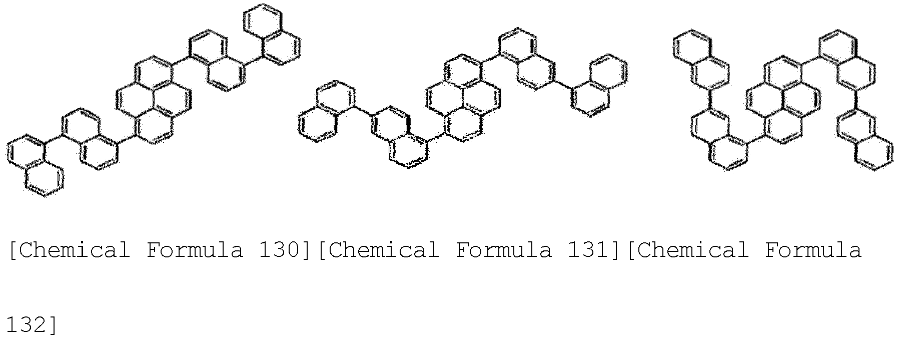

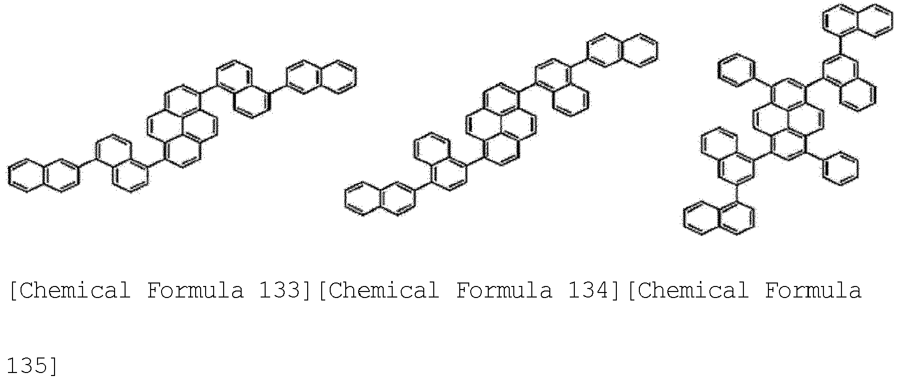

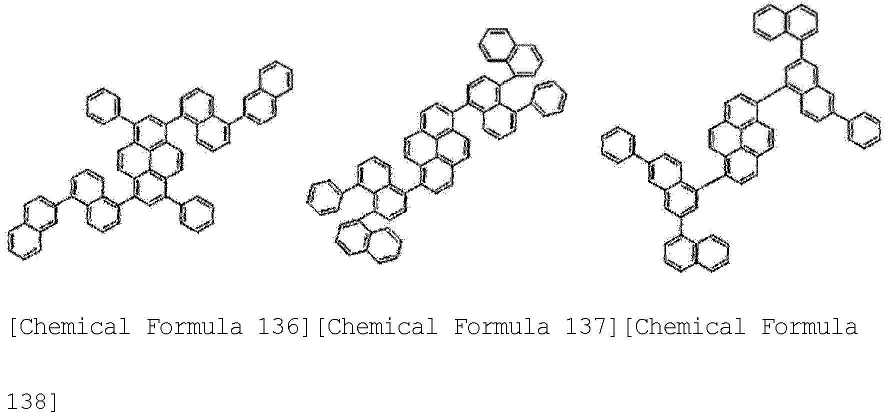

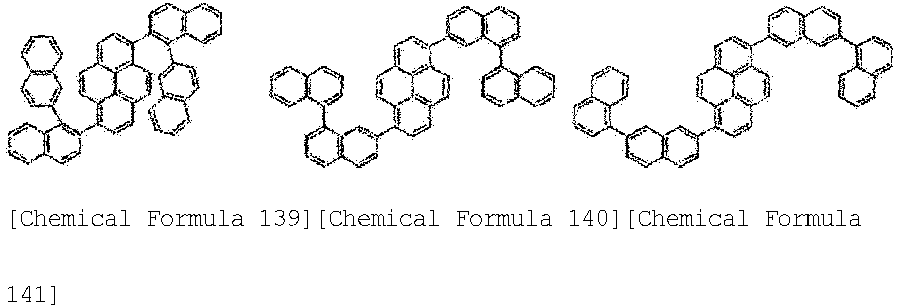

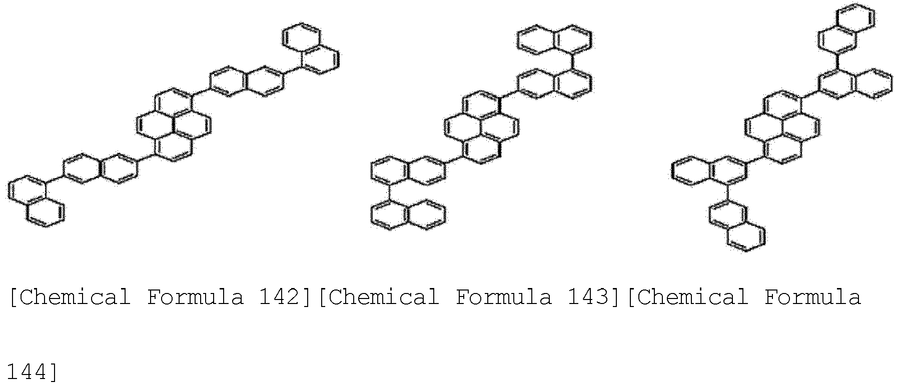

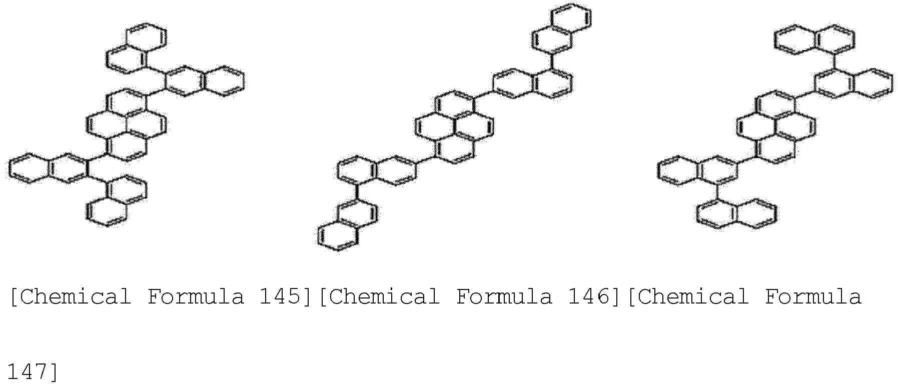

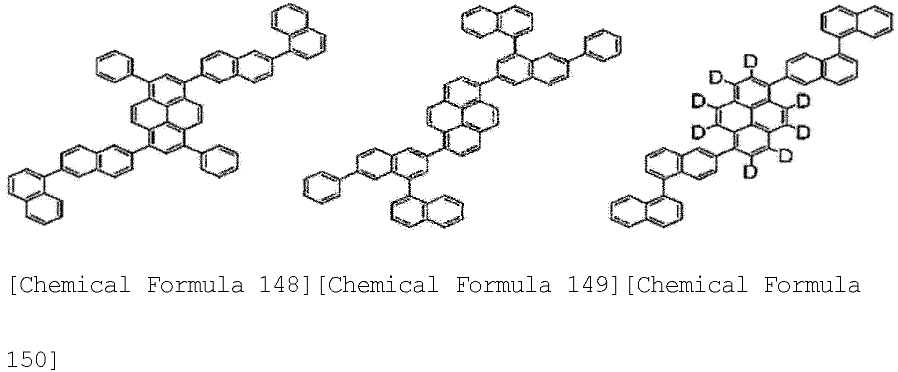

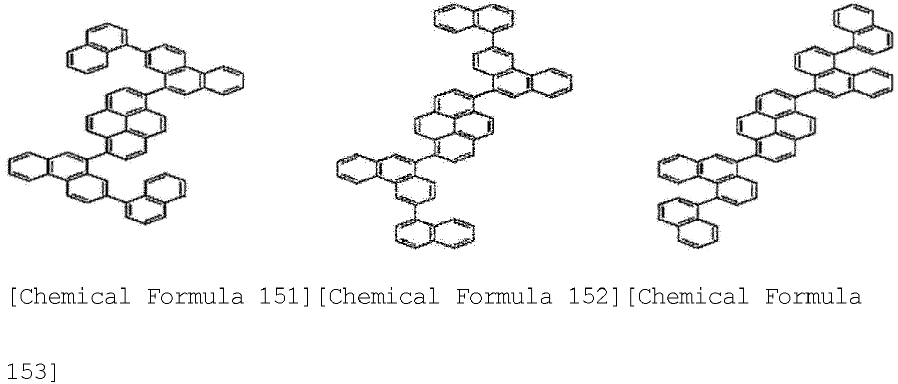

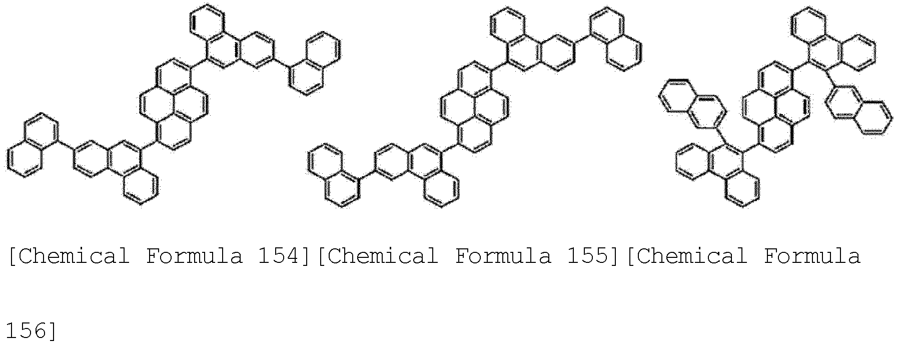

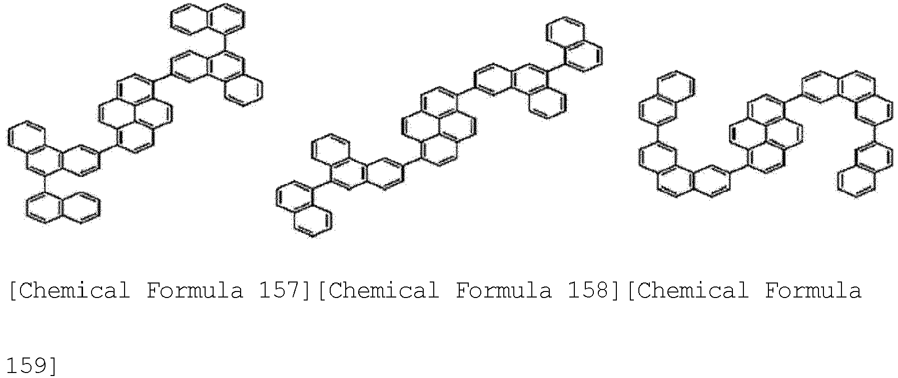

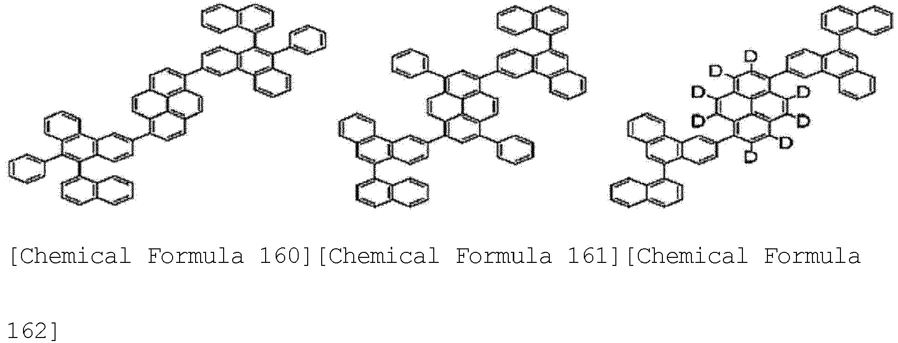

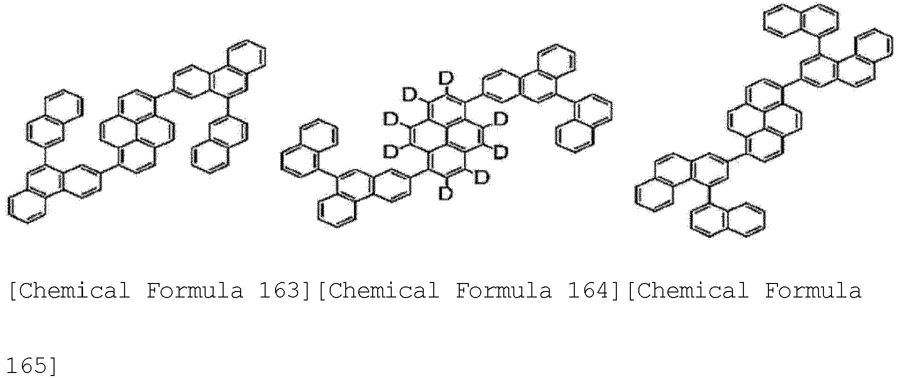

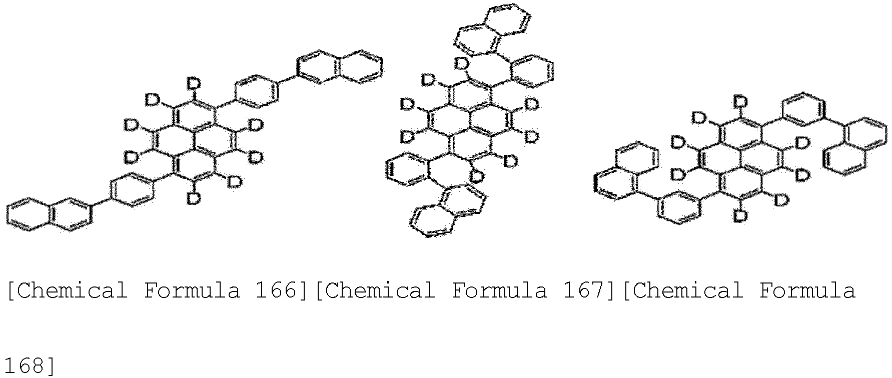

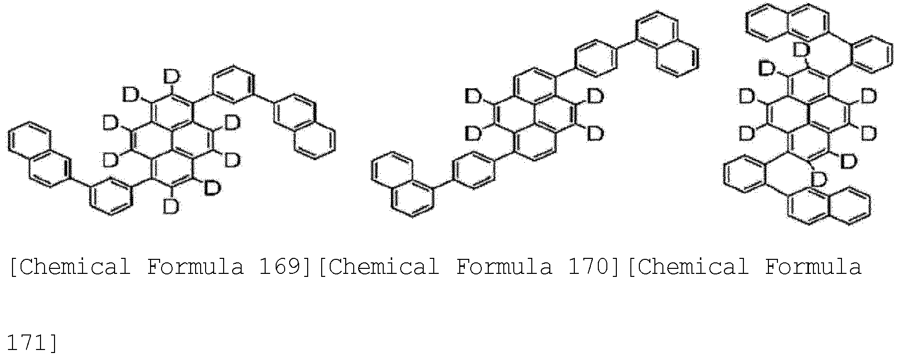

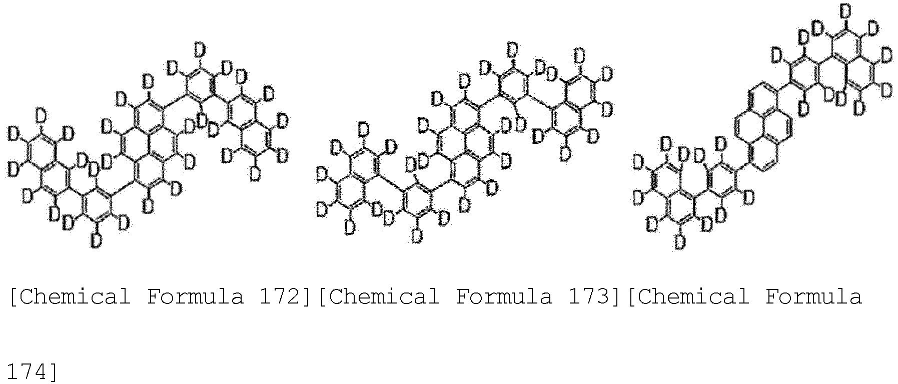

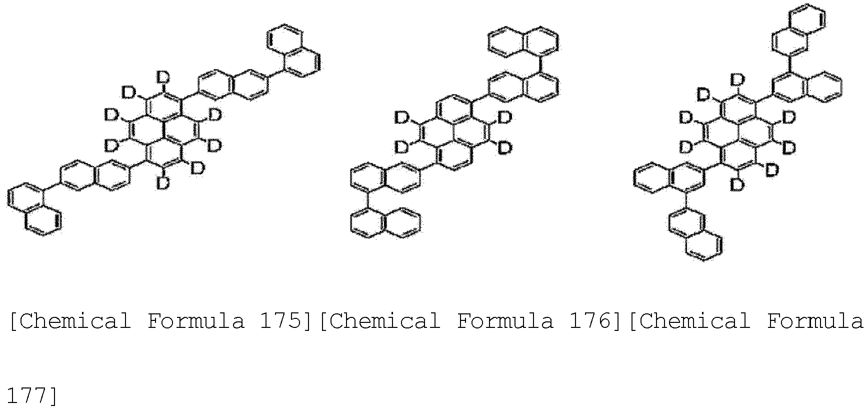

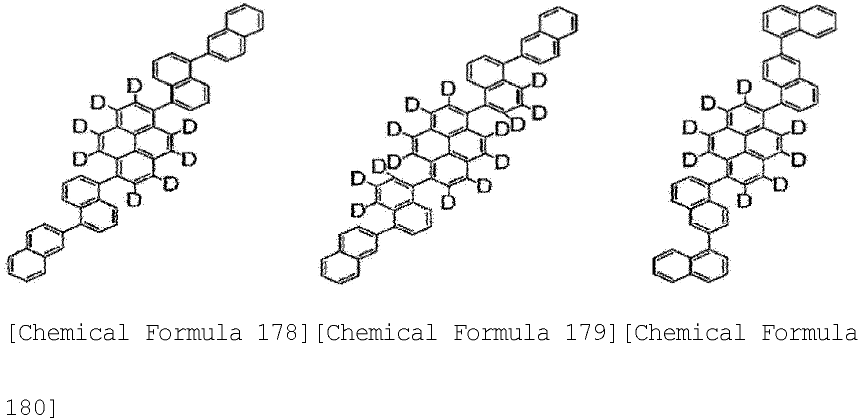

Throughout the description of the present disclosure, the phrase "(an organic layer) includes at least one organic compound" may be construed to mean that " (an organic layer) may include a single organic compound species or two or more different species of organic compounds falling within the scope of the present disclosure".

In this regard, the organic light-emitting diode according to the present disclosure may include at least one of a hole injection layer, a hole transport layer, a functional layer capable of both hole injection and hole transport, a light-emitting layer, an electron transport layer, and an electron injection layer.

In addition, the present disclosure provides an organic light-emitting diode including at least one of a hole transport layer and a hole injection layer between the first electrode and the first light-emitting layer, and at least one of an electron transport and an electron injection layer between the second light-emitting layer and the second electrode and preferably including a hole transport layer and a hole injection layer between the first electrode and the first light-emitting layer, and an electron transport and an electron injection layer between the second light-emitting layer and the second electrode.

Moreover, the organic light-emitting diode according to the present disclosure may include a first dopant in the first light-emitting layer or a second dopant in the second light-emitting layer, wherein at least one of the first and the second dopant is represented by the following Chemical Formulas D1 to D10, and preferably, both the first dopant in the first light-emitting layer and the second dopant in the second light-emitting layer are each independently represented by any one of Chemical Formulas D1 to D10: 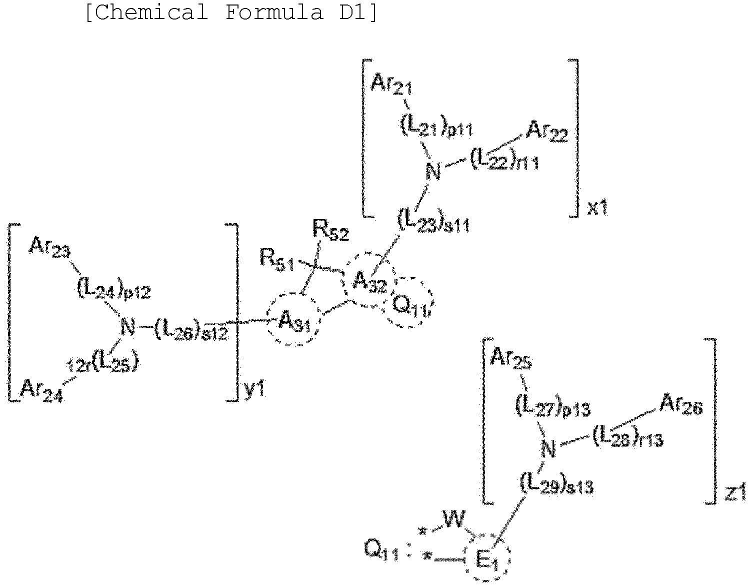 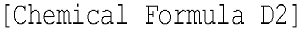 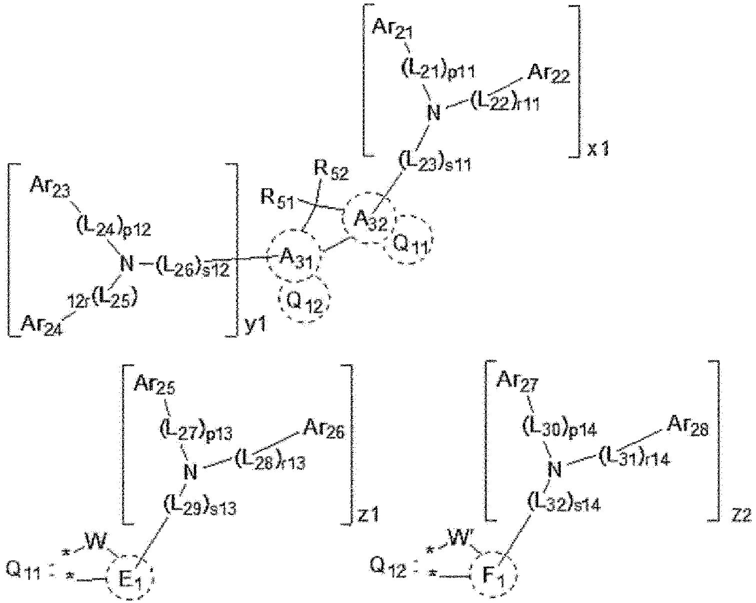 wherein,
A₃₁, A₃₂, E₁, and F₁, which are same or different, are each independently a substituted or unsubstituted aromatic hydrocarbon ring of 6 to 50 carbon atoms, or a substituted or unsubstituted heteroaromatic ring of 2 to 40 carbon atoms,
wherein two adjacent carbon atoms of the aromatic ring A₃₁ and two adjacent carbon atoms of the aromatic ring A₃₂ form a 5-membered fused ring together with a carbon atom to which substituents R₅₁ and R₅₂ are bonded;
linkers L₂₁ to L₃₂, which are same or different, are each independently selected from among a single bond, a substituted or unsubstituted alkylene of 1 to 60 carbon atoms, a substituted or unsubstituted alkenylene of 2 to 60 carbon atoms, a substituted or unsubstituted alkynylene of 2 to 60 carbon atoms, a substituted or unsubstituted cycloalkylene of 3 to 60 carbon atoms, a substituted or unsubstituted heterocycloalkylene of 2 to 60 carbon atoms, a substituted or unsubstituted arylene of 6 to 60 carbon atoms, and a substituted or unsubstituted heteroarylene of 2 to 60 carbon atoms;
W and W', which are same or different, are each independently any one selected from among N-R₅₃, CR₅₄R₅₅, S1R₅₆R₅₇, GeR₅₈R₅₉, O, S, and Se;
R₅₁ to R₅₉, and Ar₂₁ to Ar₂₈, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted alkenyl of 2 to 30 carbon atoms, a substituted or unsubstituted alkynyl of 2 to 20 carbon atoms, a substituted or unsubstituted cycloalkyl of 3 to 30 carbon atoms, a substituted or unsubstituted cycloalkenyl of 5 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a substituted or unsubstituted alkylgermyl of 1 to 30 carbon atoms, a substituted or unsubstituted arylgermyl of 1 to 30 carbon atoms, a cyano, a nitro, and a halogen, wherein R₅₁ and R₅₂ together may form a mono- or polycyclic aliphatic or aromatic ring that may be a heterocyclic ring bearing a heteroatom selected from among N, O, P, Si, S, Ge, Se, and Te as a ring member;
p11 to p14, r11 to r14, and s11 to s14 are each independently an integer of 1 to 3, wherein when any of them is 2 or greater, the corresponding linkers L₂₁ to L₃₂ may be same or different,
x1 is 1, and y1, z1, and z2, which are same or different, are each independently an integer of 0 to 1; and
Ar₂₁ may form a ring with Ar₂₂, Ar₂₃ may form a ring with Ar₂₄, Ar₂₅ may form a ring with Ar₂₆, and Ar₂₇ may form a ring with Ar₂₈,
two adjacent carbon atoms of the A₃₂ ring moiety of Chemical Formula D1 may occupy respective positions of Structural Formula Q₁₁ to form a fused ring, and
two adjacent carbon atoms of the A₃₁ ring moiety of Chemical Formula D2 may occupy respective positions of structural Formula Q₁₂ to form a fused ring, and two adjacent carbon atoms of the A₃₂ ring moiety of Chemical Formula D2 may occupy respective positions of Structural Formula Q₁₁ to form a fused ring, 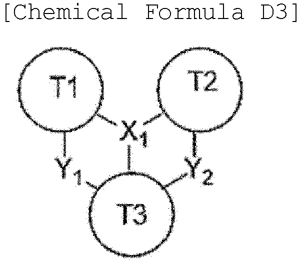 wherein,
X₁ is any one selected from among B, P, and P=O
T1 to T3, which are same or different, are each independently a substituted or unsubstituted aromatic hydrocarbon ring of 6 to 50 carbon atoms, or a substituted or unsubstituted heteroaromatic ring of 2 to 40 carbon atoms;
Y₁ is any one selected from among N-R₆₁, CR₆₂R₆₃, O, S, and SiR₆₄R₆₅;
Y₂ is any one selected from among N-R₆₆, CR₆₇R₆₈, O, S, and SiR₆₉R₇₀;
R₆₁ to R₇₀, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a cyano, and a halogen, and wherein at least one of R₆₁ to R₇₀ may be connected to at least one of T₁ to T₃ to form an additional mono- or polycyclic aliphatic or aromatic ring; 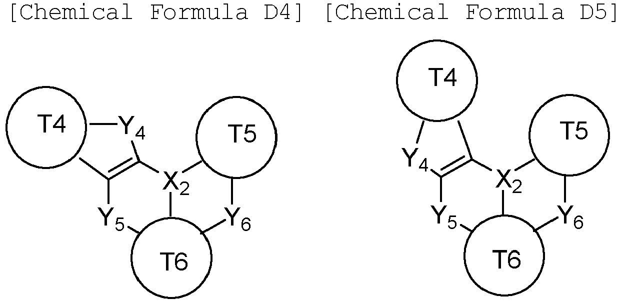 wherein,
X₂ is any one selected from among B, P, and P=O,
T4 to T6 are as defined for T1 to T3 in Chemical Formula D3,
Y₄ is any one selected from among N-R₆₁, CR₆₂R₆₃, O, S, and SiR₆₄R₆₅;
Y₅ is any one selected from among N-R₆₆, CR₆₇R₆₈, O, S, and SiR₆₉R₇₀;
Y₆ is any one selected from among N-R₇₁, CR₇₂R₇₃, O, S, and SiR₇₄R₇₅; and
R₆₁ to R₇₅ being as defined for R₆₁ to R₇₀ in Chemical Formula D3; 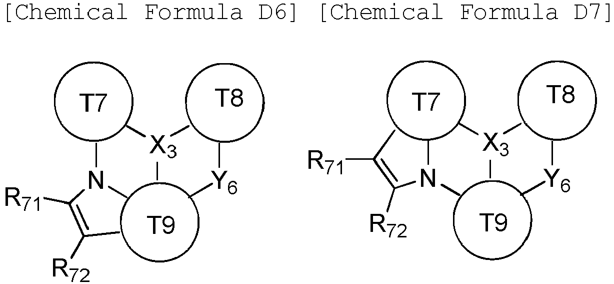
X₃ is any one selected from among B, P, and P=O,
T7 to T9 are defined as for T1 to T3 in Chemical Formula D3,
Y₆ is any one selected from among N-R₆₁, CR₆₂R₆₃, O, S, and SiR₆₄R₆₅;
R₆₁ to R₆₅ and R₇₁ to R₇₂ are each as defined for R₆₁ to R₇₀ in Chemical Formula D3,
wherein at least one of R₇₁ to R₇₂ may be connected to each other to form an additional mono- or polycyclic aliphatic or aromatic ring; 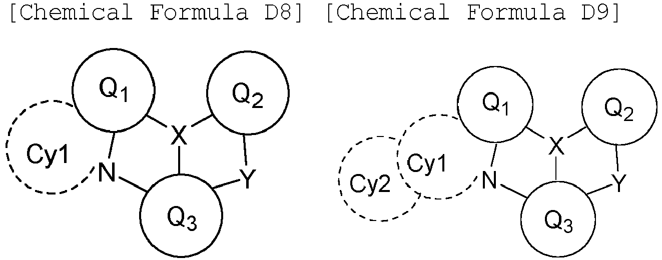 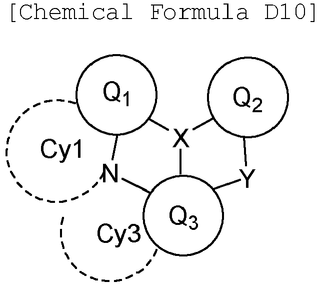 wherein,
X is any one selected from among B, P, and P=O,
Q₁ to Q₃ are each as defined for T1 to T3 in Chemical Formula D3,
Y is any one selected from among N-R₃, CR₄R₅, O, S, and Se,
R₃ to R₅ are each as defined for R₆₁ to R₇₀ in Chemical Formula D3,
R₃ to R₅ may each be connected to the Q₂ or Q₃ ring moiety to form an additional mono- or polycyclic aliphatic or aromatic ring,
R₄ and R₅ may be connected to each other to form an additional mono- or polycyclic aliphatic or aromatic ring,
the ring formed by Cy1 is a substituted or unsubstituted alkylene of 1 to 10 carbon atoms, except for the nitrogen (N) atom, the aromatic carbon atom of Q₁ to which the nitrogen (N) atom is connected, and the aromatic carbon atom of Q₁ to which Cy1 is to bond,
"Cy2" in Chemical Formula D9 forms a saturated hydrocarbon ring added to Cy1 wherein the ring formed by Cy2 is a substituted or unsubstituted alkylene of 1 to 10 carbon atoms, except for the carbon atoms included in Cy1, and
the ring formed by Cy3 in Chemical Formula D10 is a substituted or unsubstituted alkylene of 1 to 10 carbon atoms, except for the aromatic carbon atom of Q₃ to which Cy3 is to bond, the aromatic carbon atom of Q₃ to which the nitrogen (N) atom is connected, the nitrogen (N) atom, and the carbon atom of Cy1 to which the nitrogen (N) atom is connected,
wherein the term "substituted" in the expression "substituted or unsubstituted" used for compounds of Chemical Formulas D1 to D10 means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms, and more particularly, having at least one substituent selected from the group consisting of a deuterium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 2 to 18 carbon atoms, an alkoxy of 1 to 12 carbon atoms, an alkylamino of 1 to 12 carbon atoms, a diarylamino of 12 to 18 carbon atoms, a diheteroarylamino of 2 to 18 carbon atoms, an aryl(heteroaryl)amino of 7 to 18 carbon atoms, an alkylsilyl of 1 to 12 carbon atoms, an arylsilyl of 6 to 18 carbon atoms, an aryloxy of 6 to 18 carbon atoms, and an arylthionyl of 6 to 18 carbon atoms.

Among the dopant compounds according to the present disclosure, the boron compounds represented by Chemical Formulas D3 to D10 may have, on the aromatic hydrocarbon rings or heteroaromatic rings of T1 to T9 or on the aromatic hydrocarbon rings or heteroaromatic rings of Q₁ to Q₃, a substituent selected from a deuterium atom, an alkyl of 1 to 24 carbon atoms, an aryl of 6 to 24 carbon atoms, an alkylamino of 1 to 24 carbon atoms, and an arylamino of 6 to 24 carbon atoms, wherein the alkyl radicals or the aryl radicals in the alkylamino of 1 to 24 carbon atoms and the arylamino of 6 to 24 carbon atoms on the rings may be linked to each other, and particularly a substituent selected from an alkyl of 1 to 12 carbon atoms, an aryl of 6 to 18 carbon atoms, an alkylamino of 1 to 12 carbon atoms, and an arylamino of 6 to 18 carbon atoms wherein the alkyl radicals or aryl radicals in the alkylamino of 1 to 12 carbon atoms and the arylamino of 6 to 18 carbon atoms on the rings may be linked to each other.

Concrete examples of the dopant compounds of Chemical Formulas D1 and D2 used in the light-emitting layer include the compounds of the following Chemical Formulas <d 1> to <d 239>:

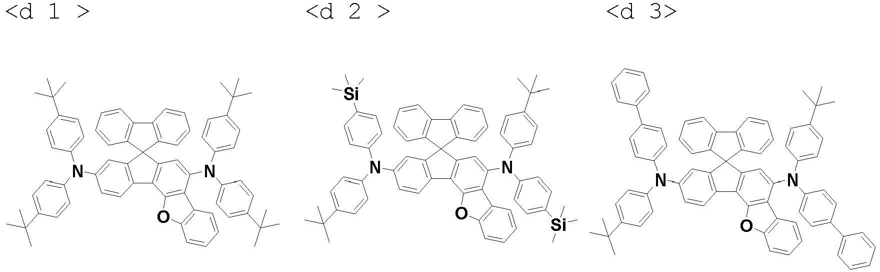

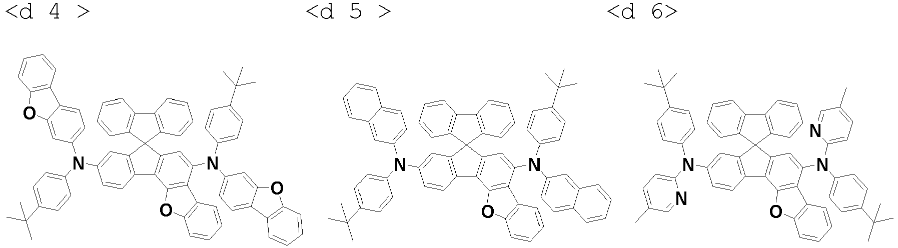

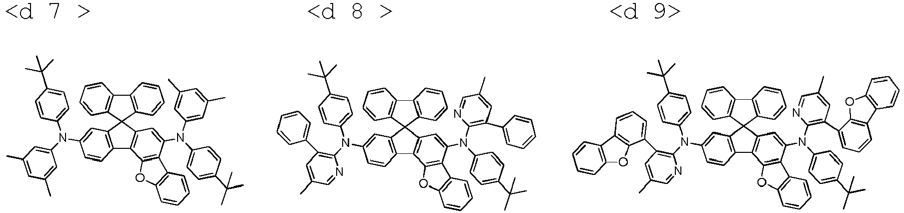

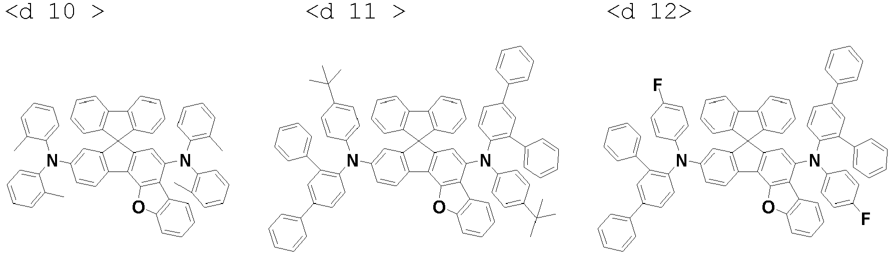

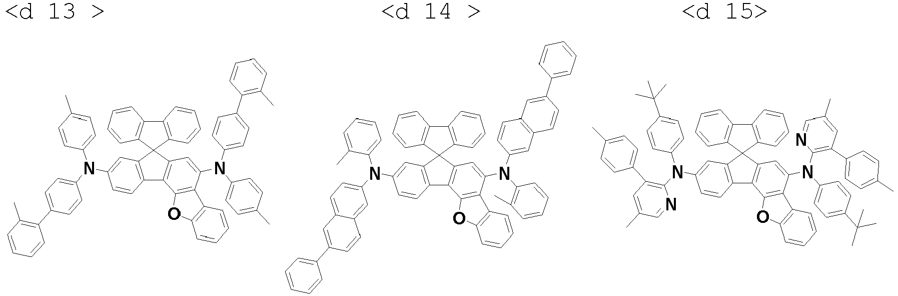

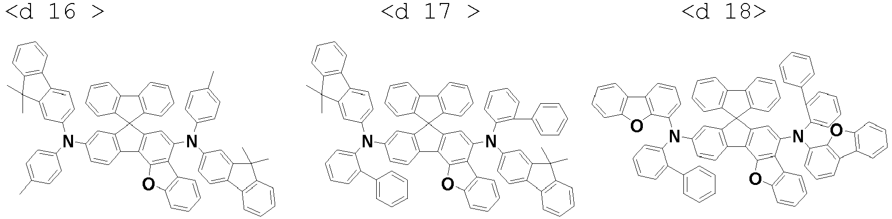

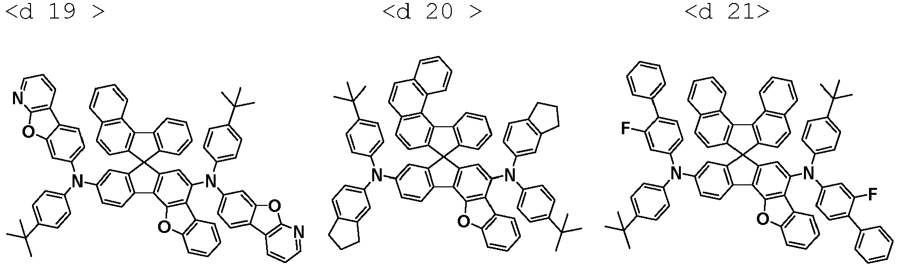

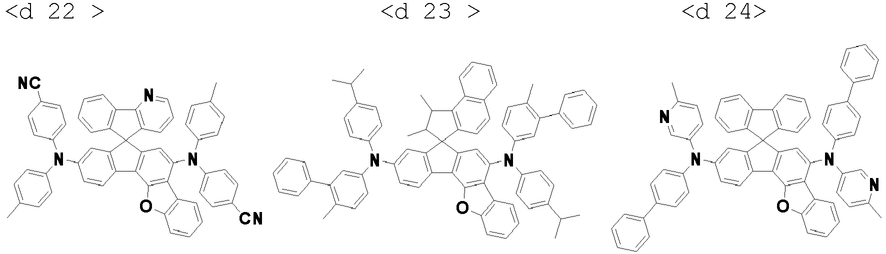

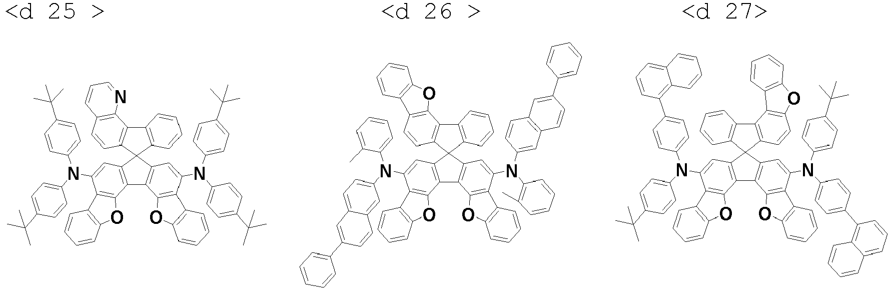

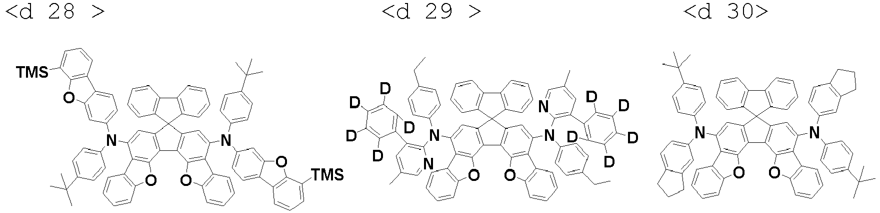

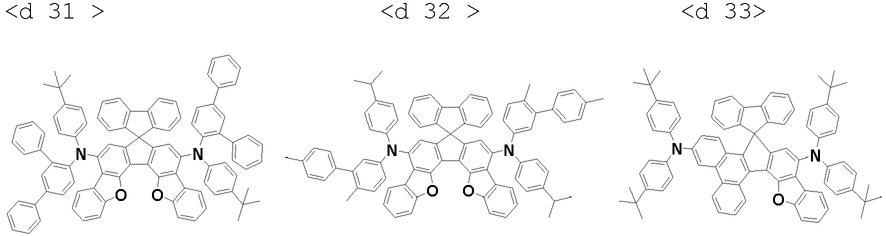

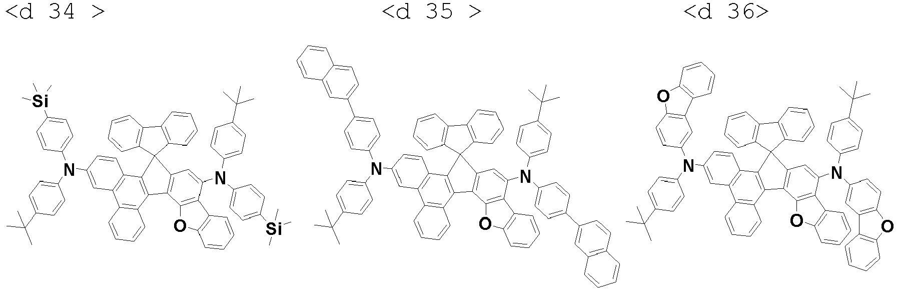

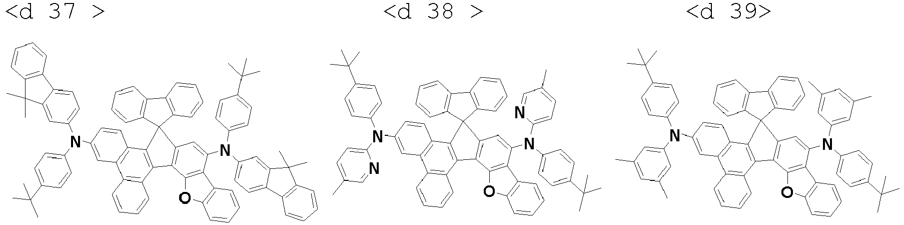

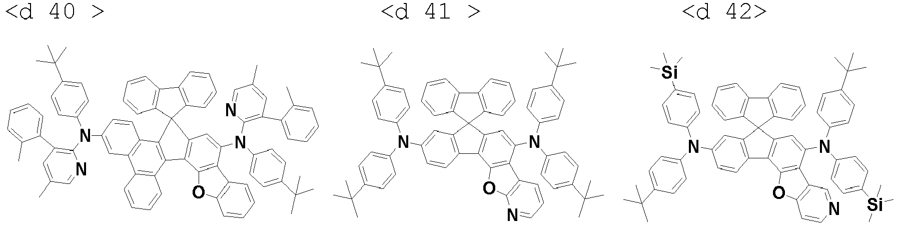

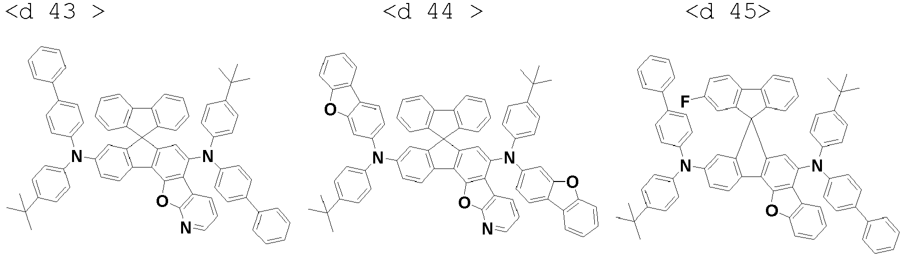

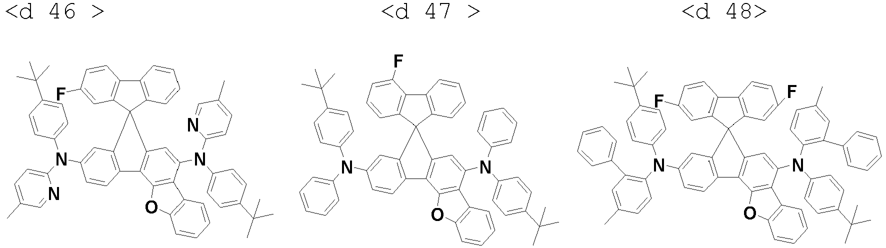

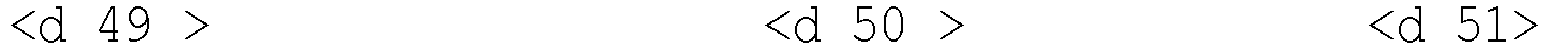

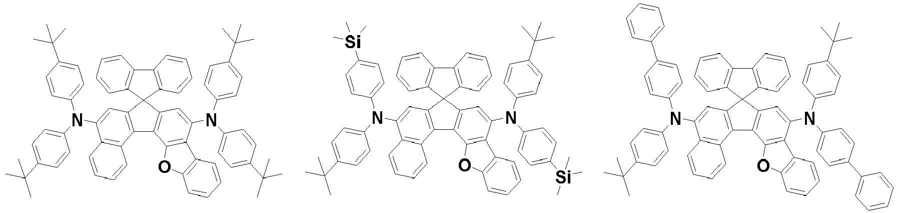

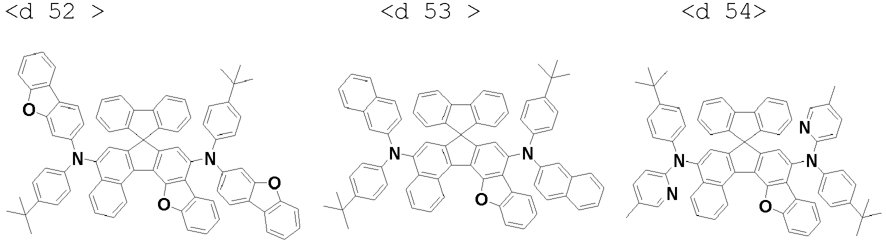

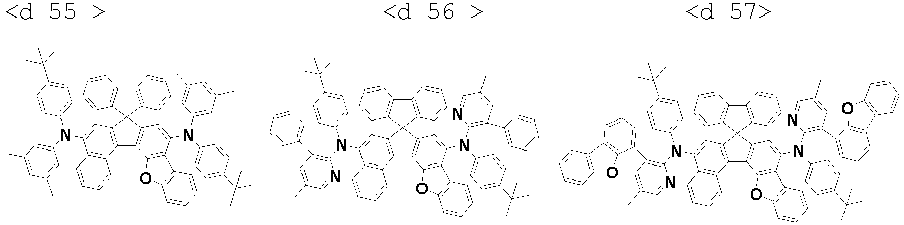

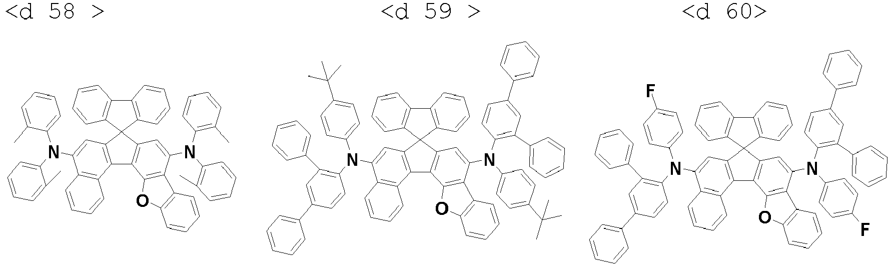

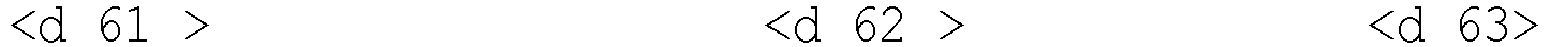

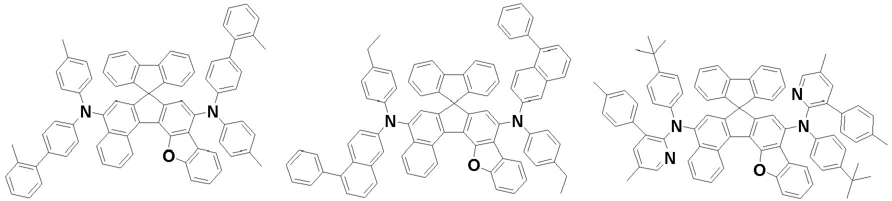

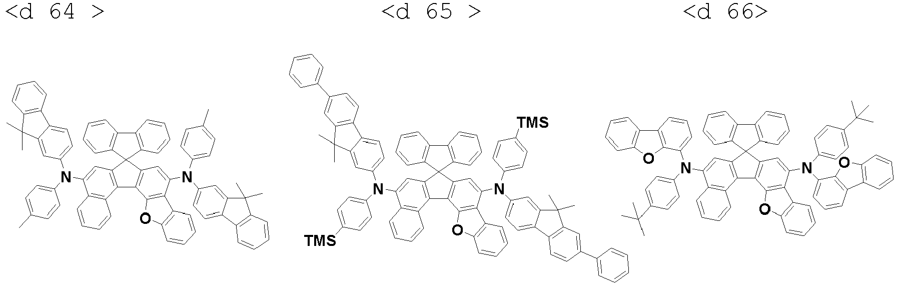

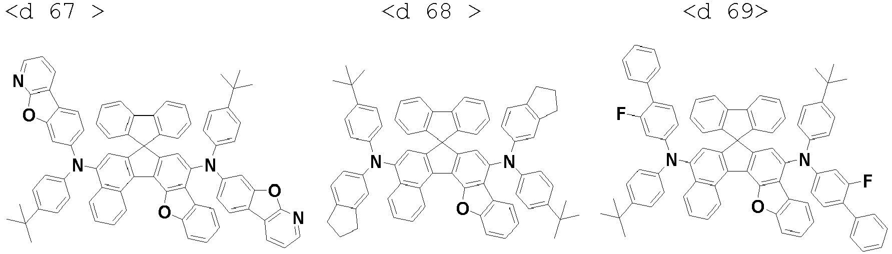

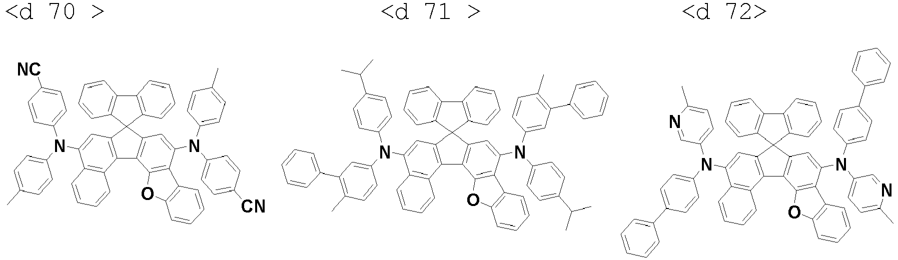

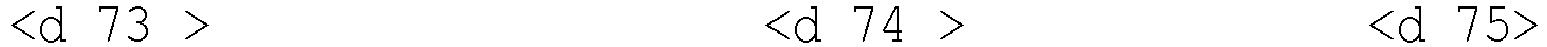

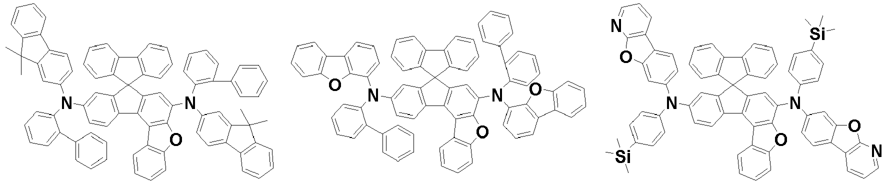

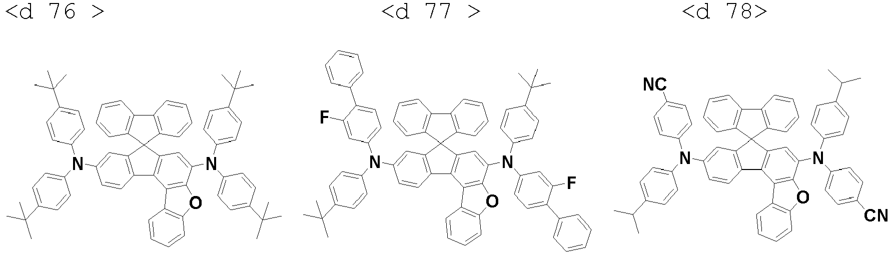

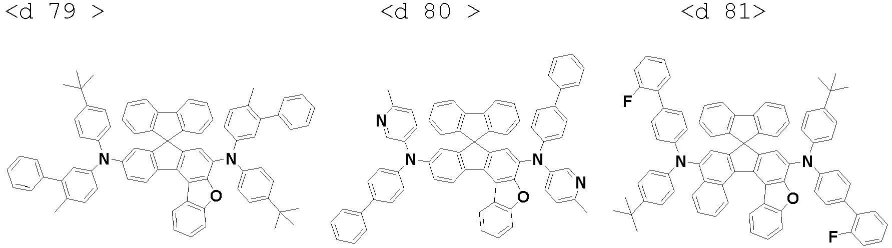

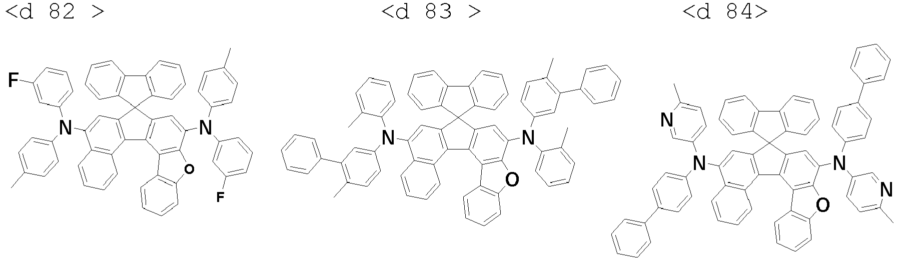

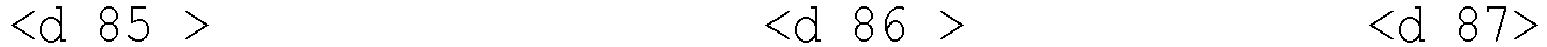

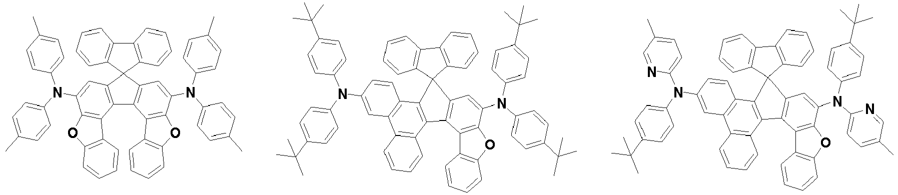

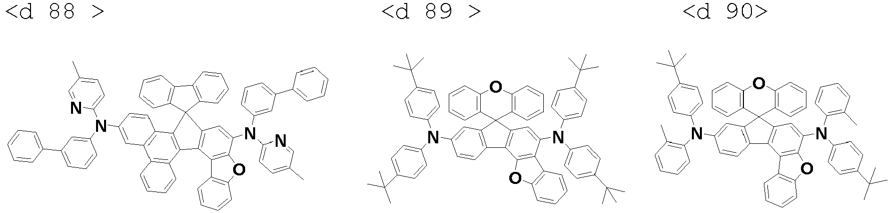

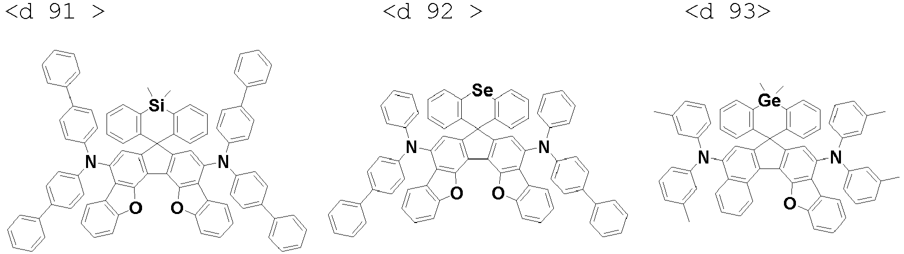

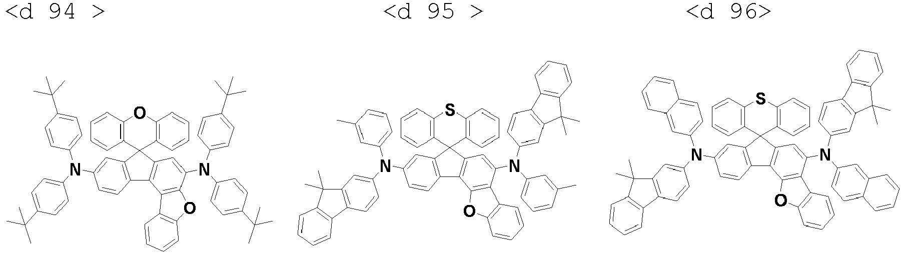

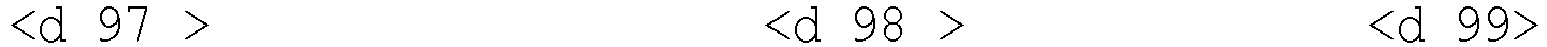

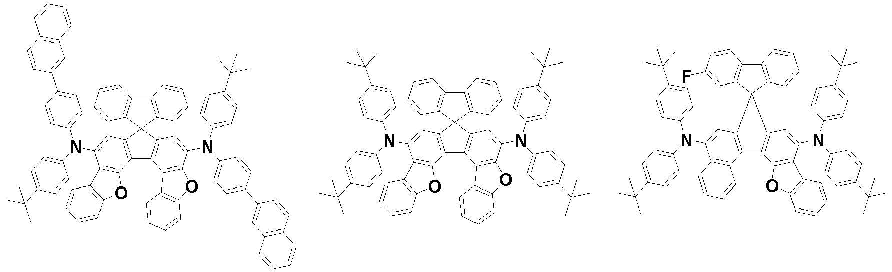

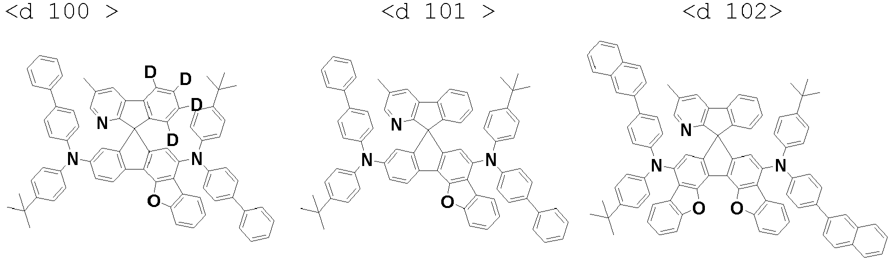

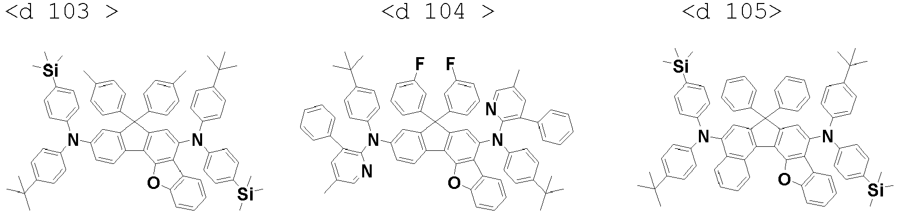

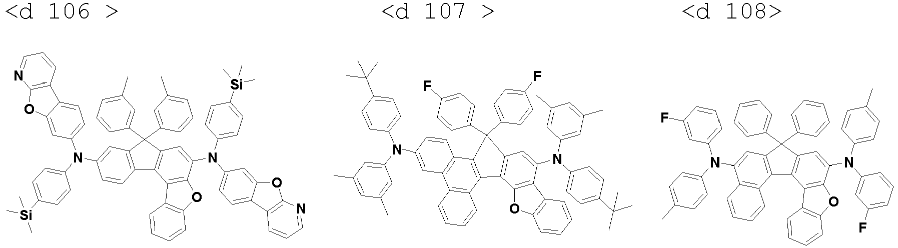

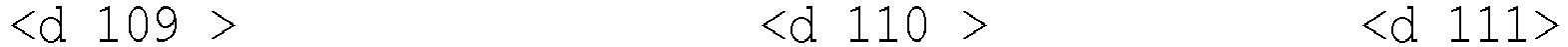

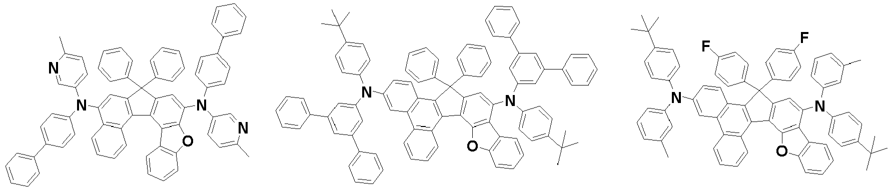

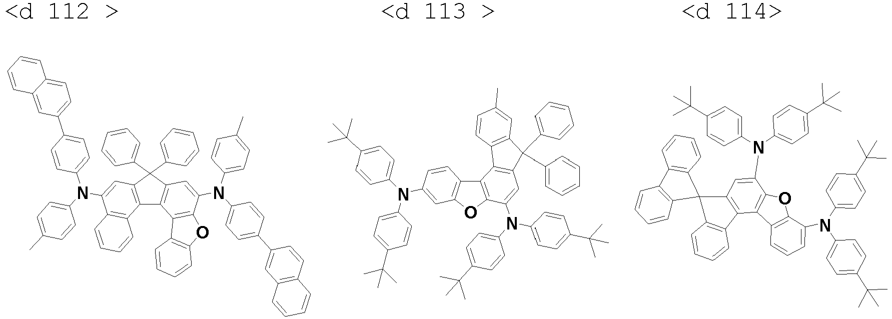

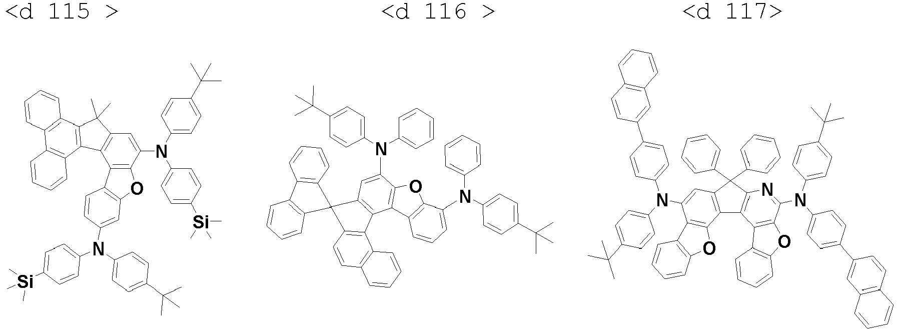

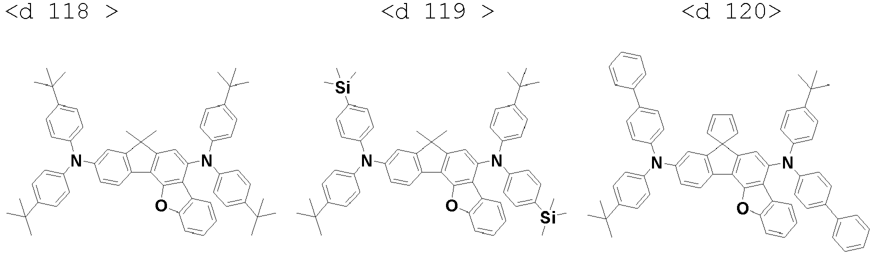

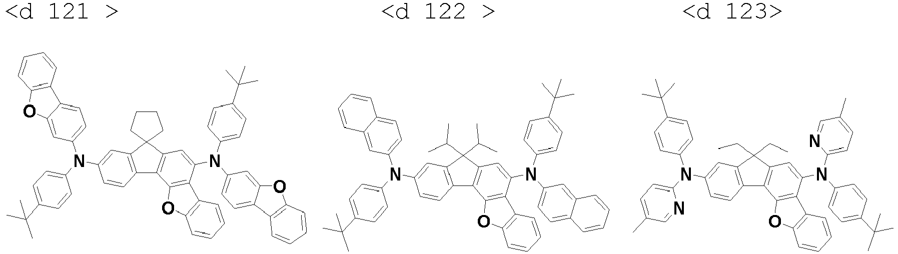

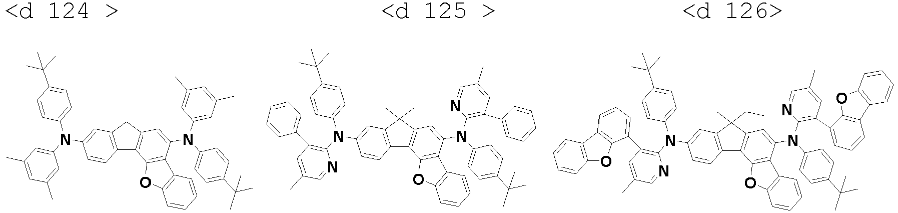

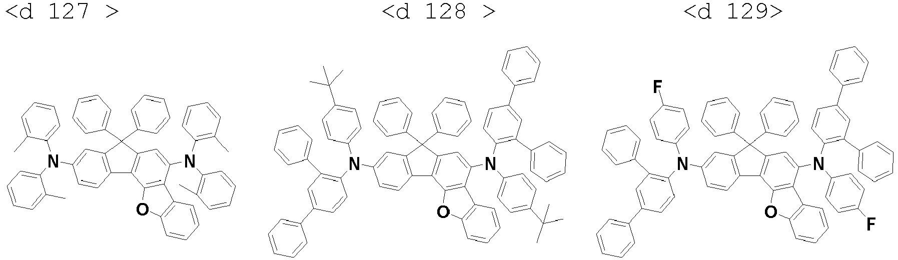

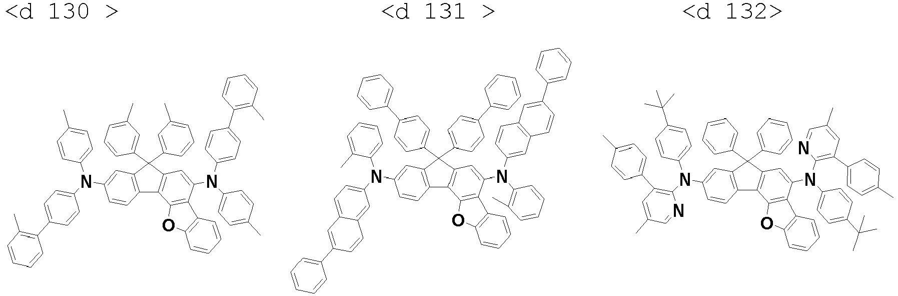

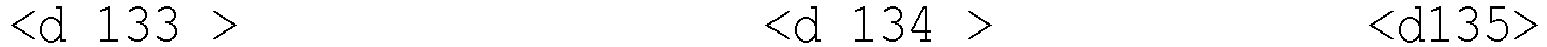

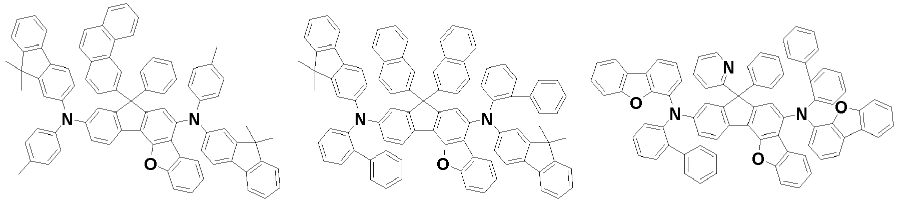

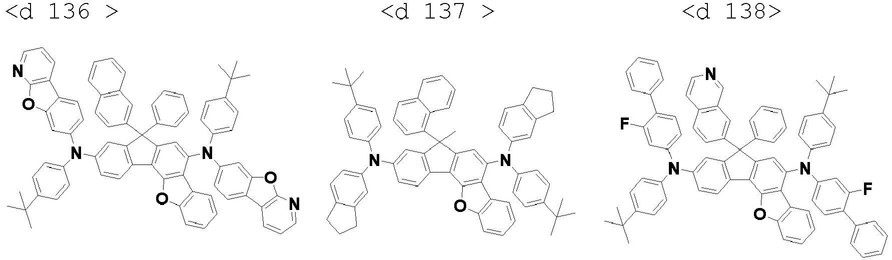

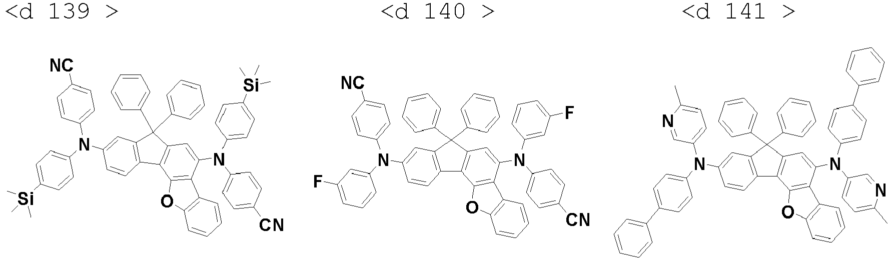

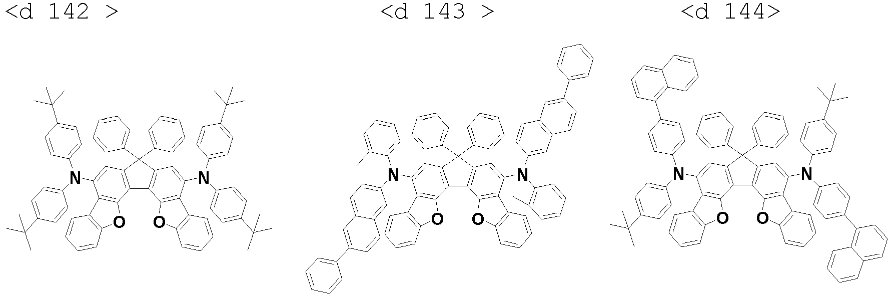

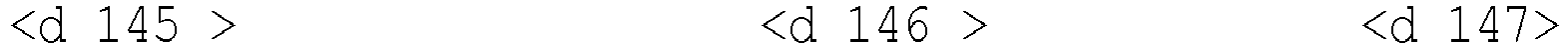

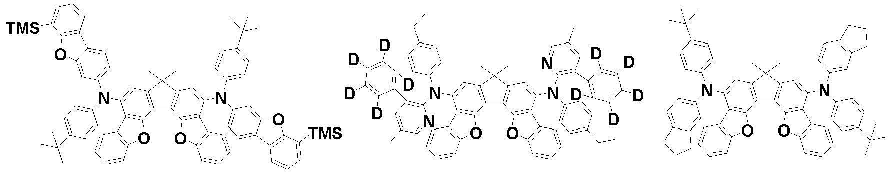

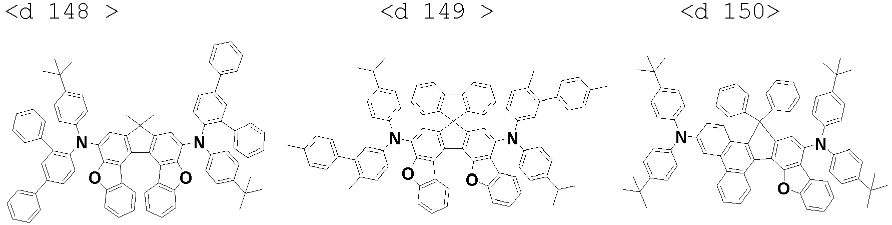

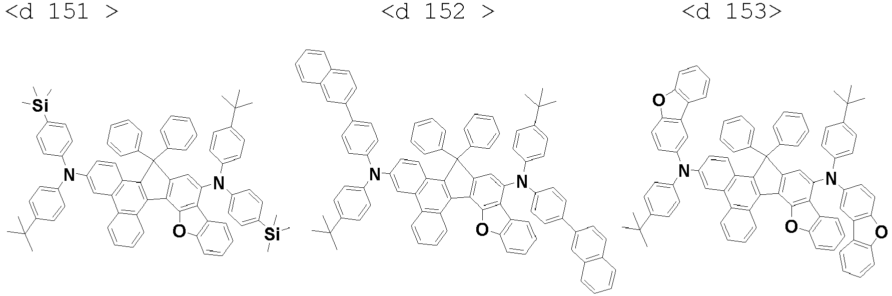

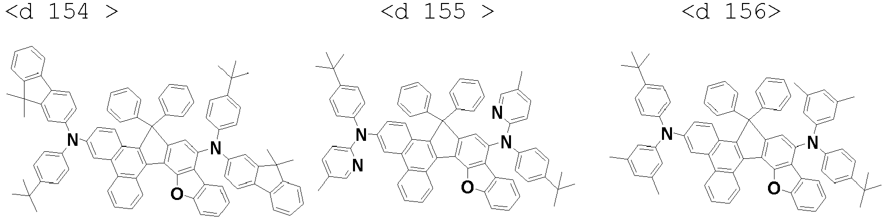

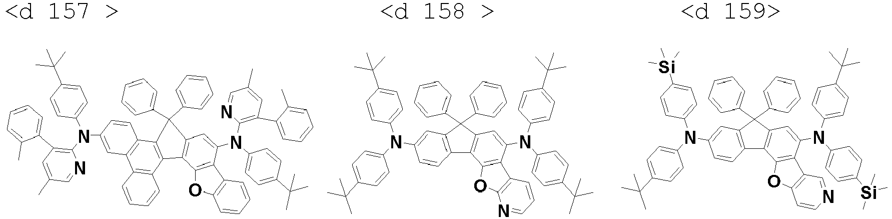

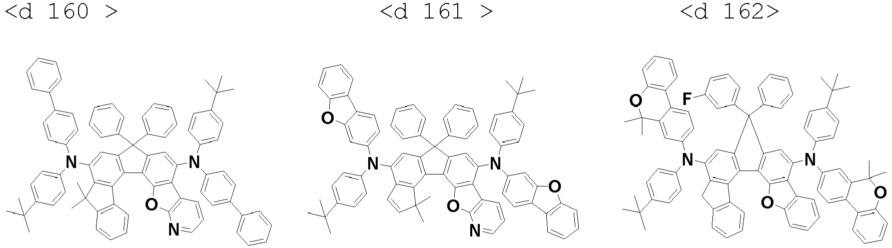

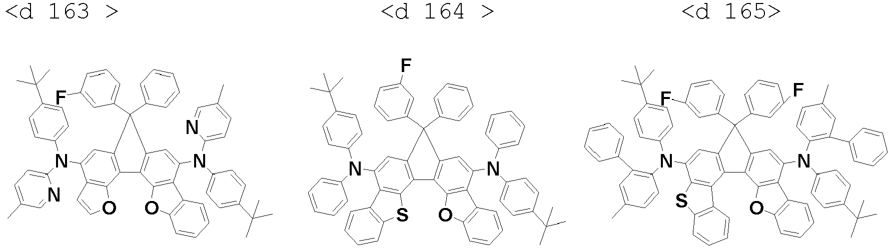

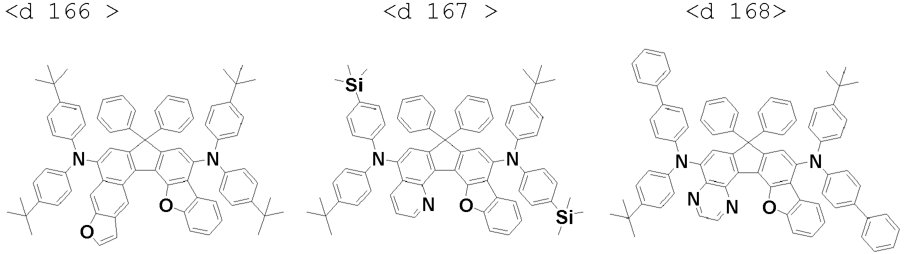

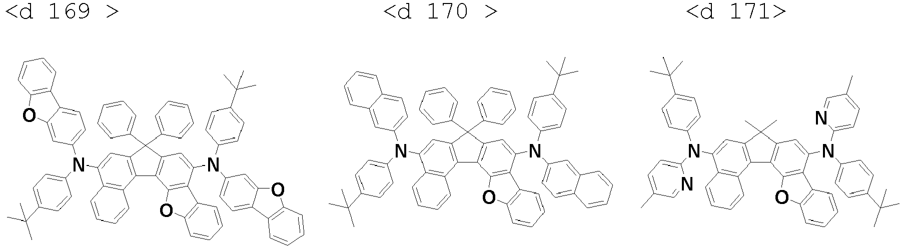

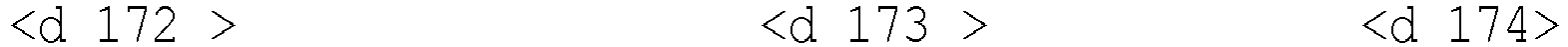

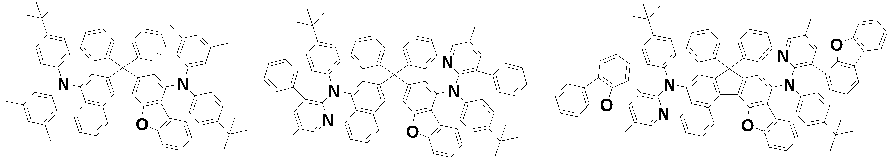

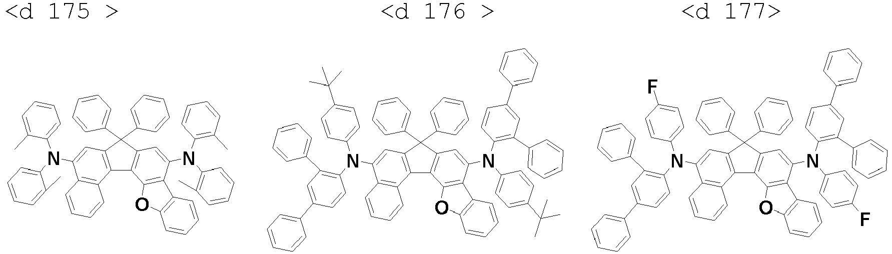

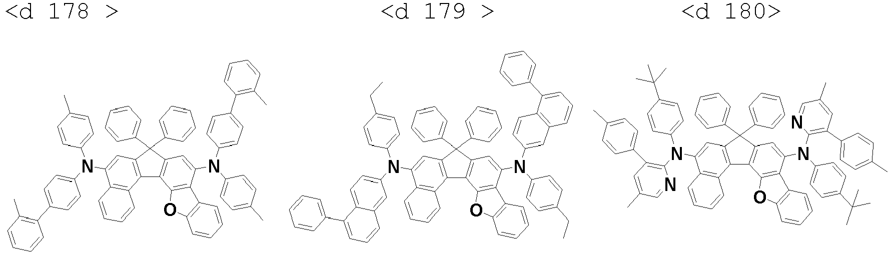

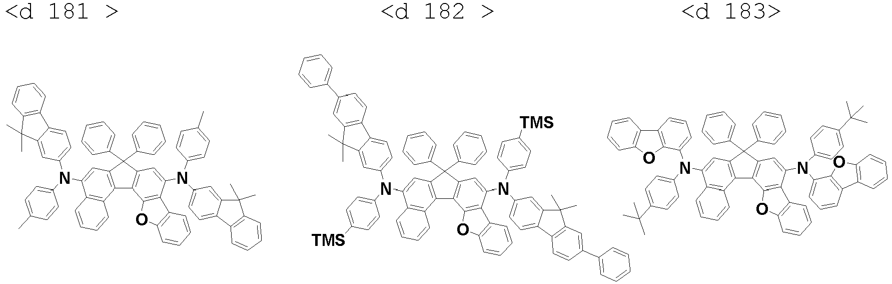

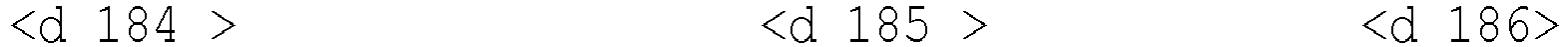

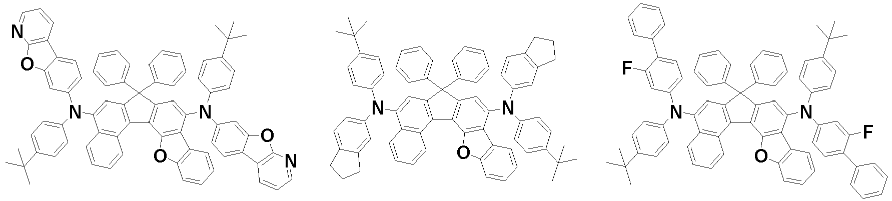

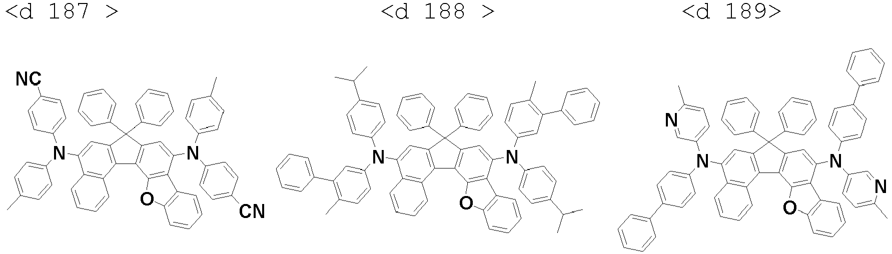

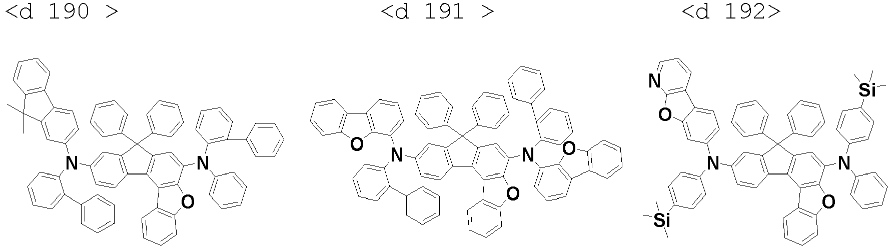

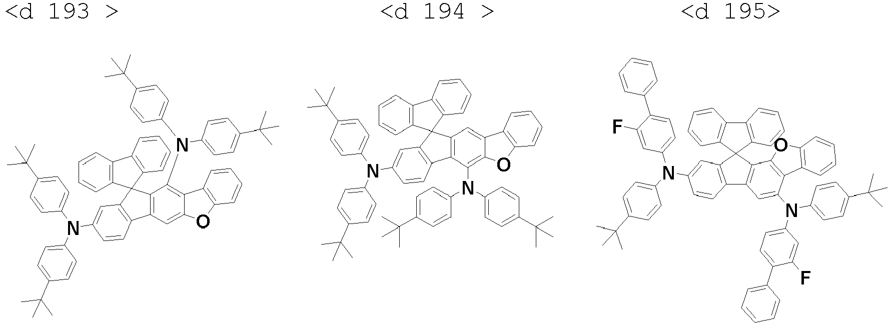

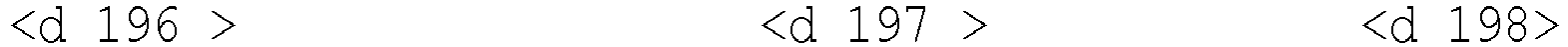

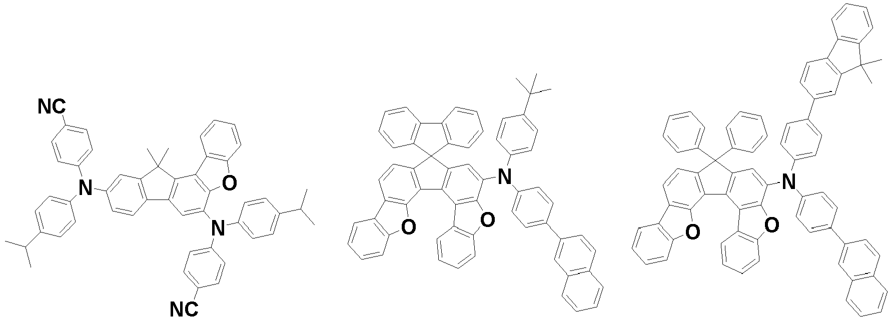

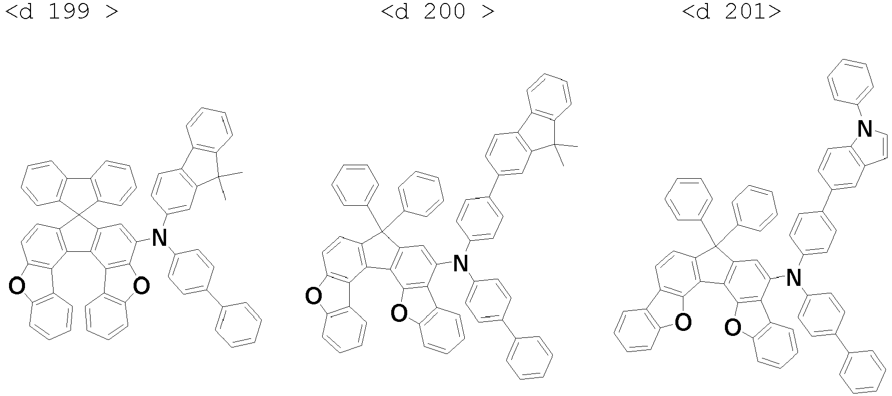

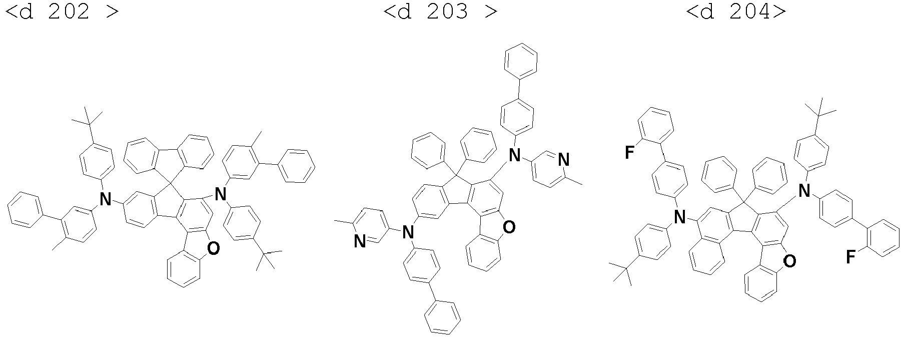

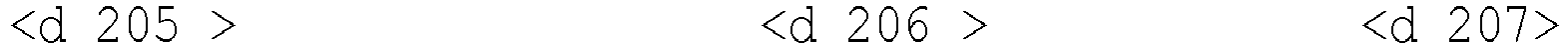

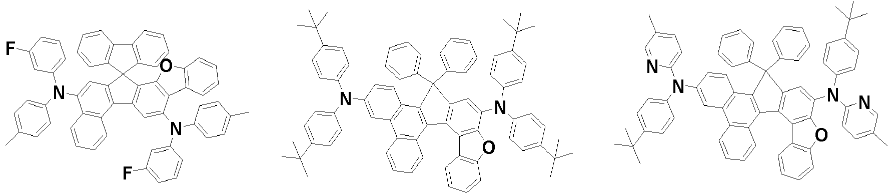

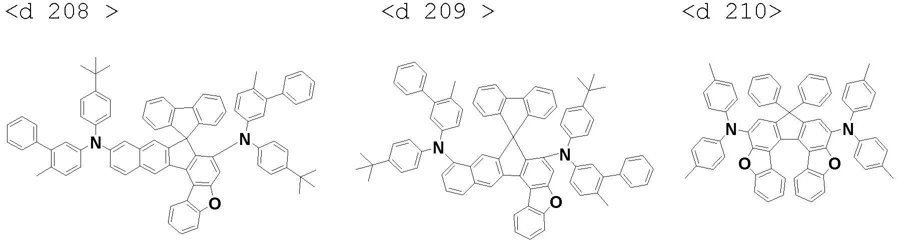

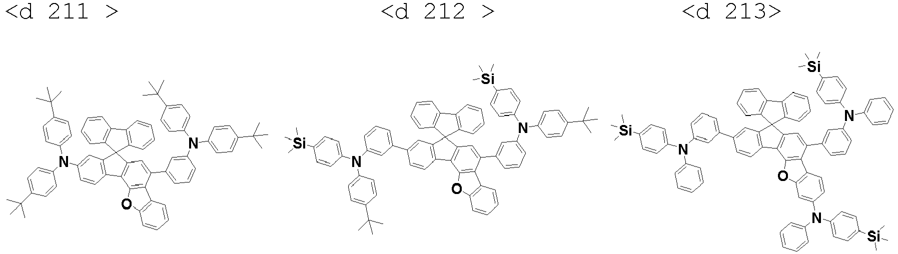

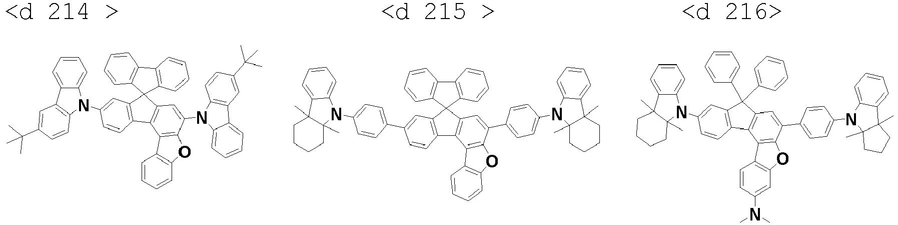

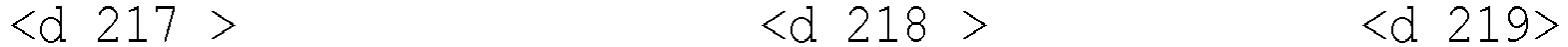

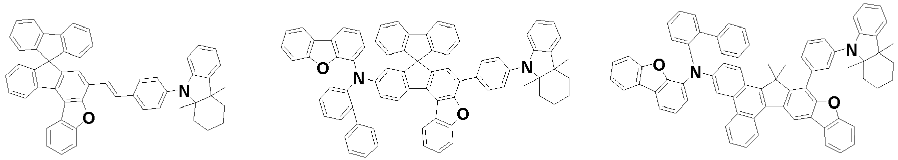

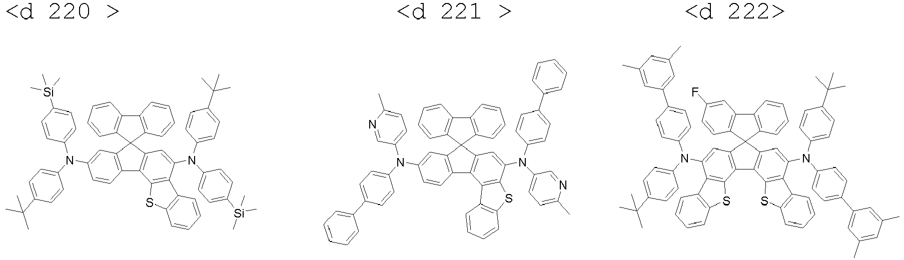

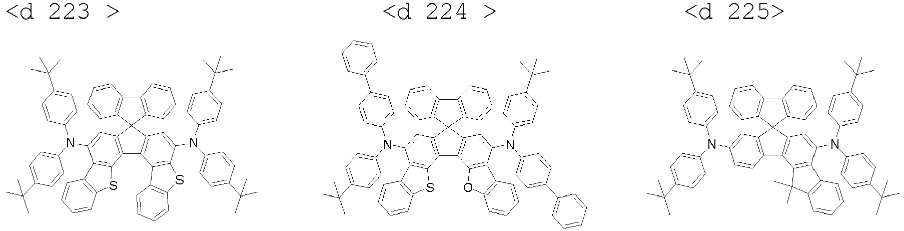

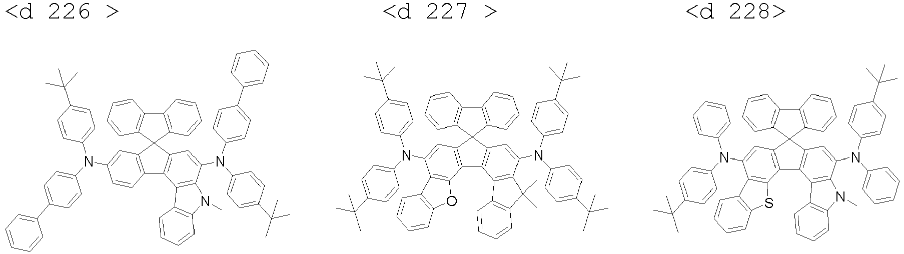

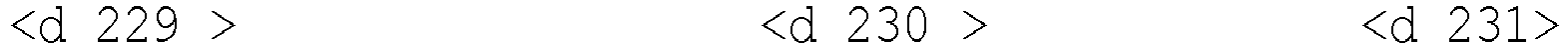

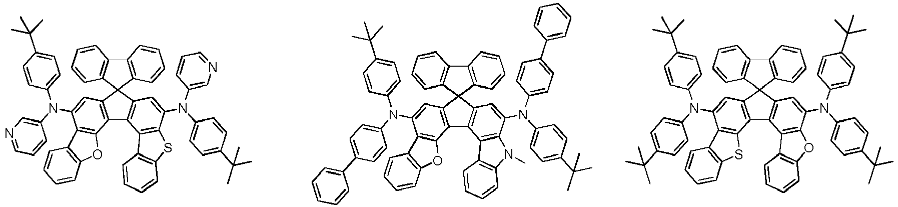

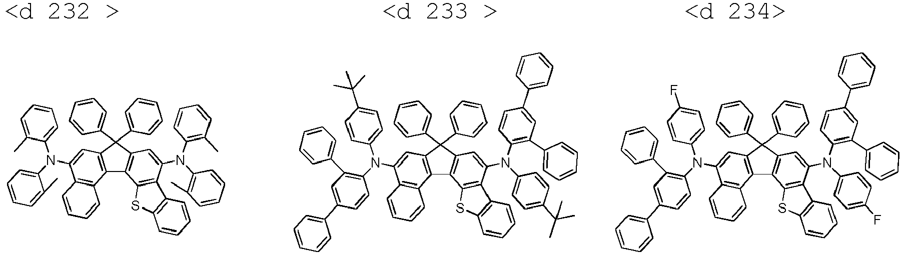

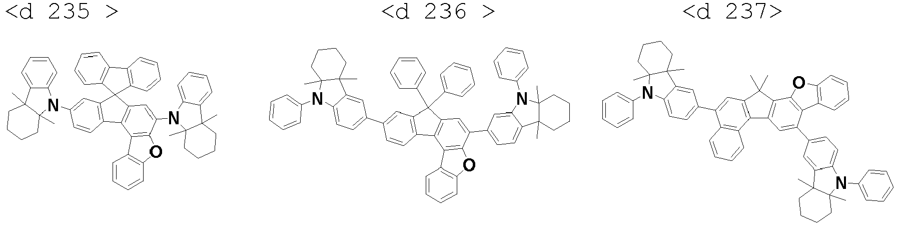

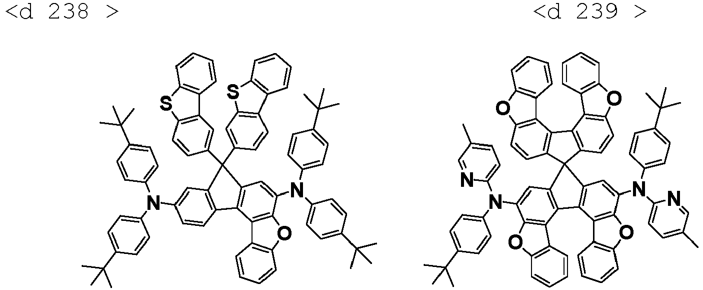

In addition, among the dopant compounds in the light-emitting layer, the compound represented by any one of [Chemical Formula D3] may be a compound represented by any one selected from <D 101> to <D 130>, below:

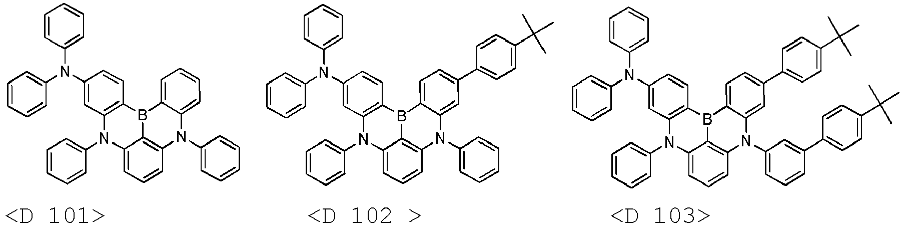

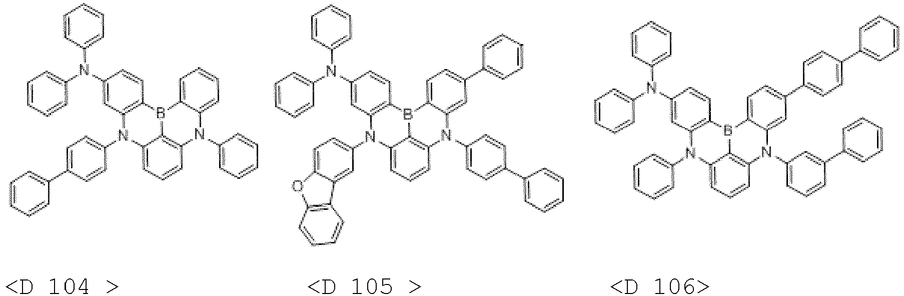

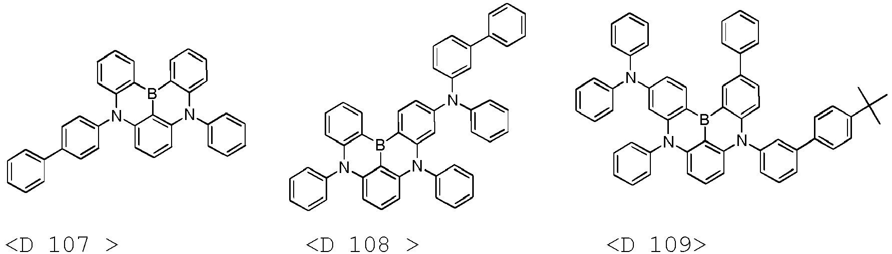

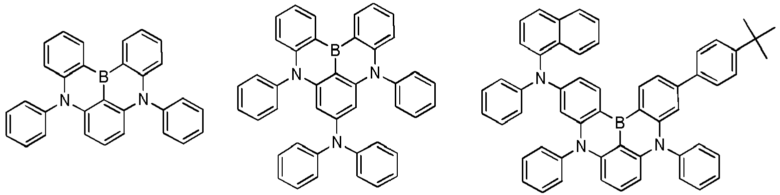

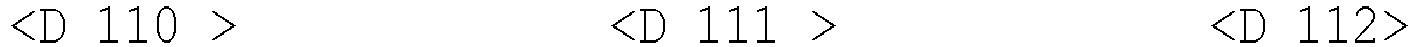

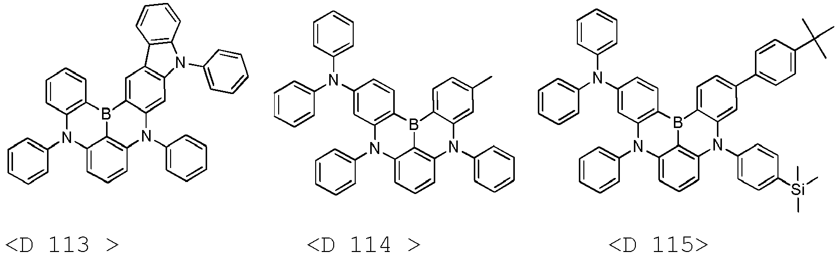

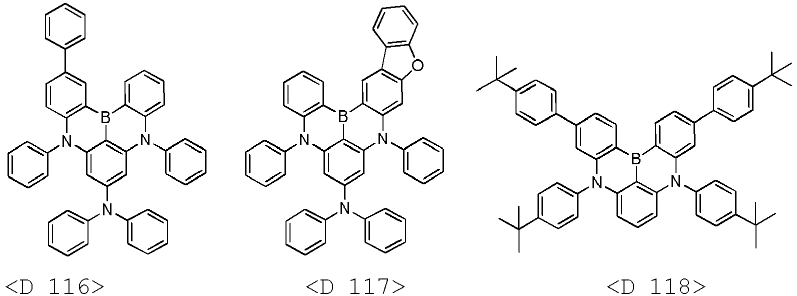

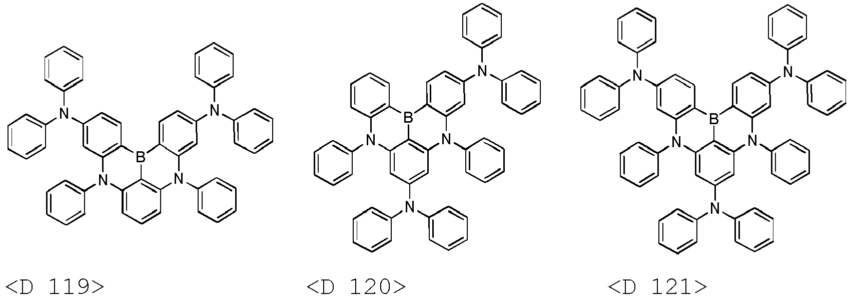

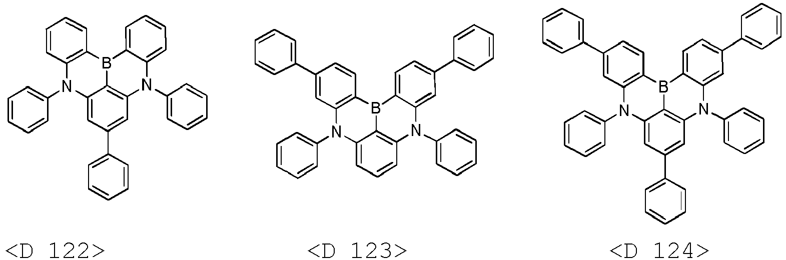

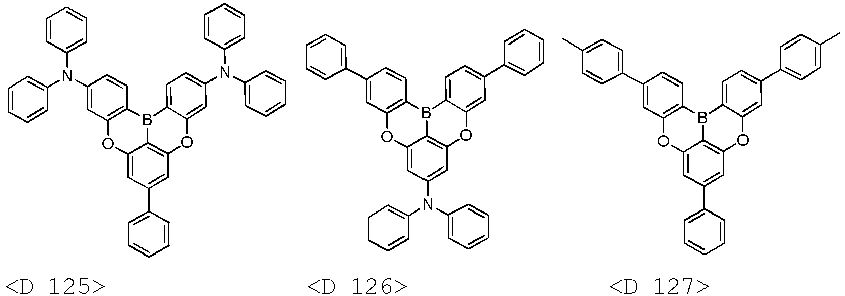

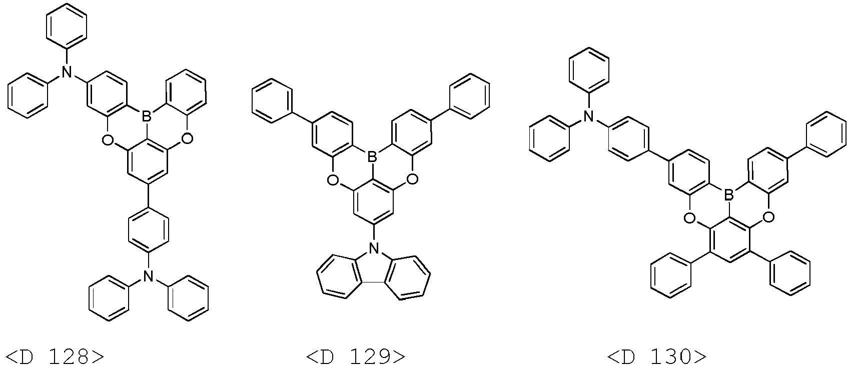

In addition, among the dopant compounds in the light-emitting layer, the compound represented by any one of [Chemical Formula D4], [Chemical Formula D5], and [Chemical Formula D8] to [Chemical Formula D10] may be a compound represented by any one selected from [D 201] to [D 476], below:

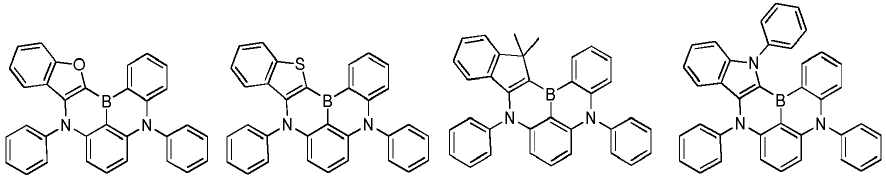

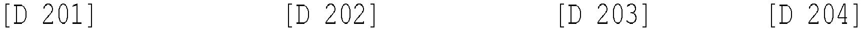

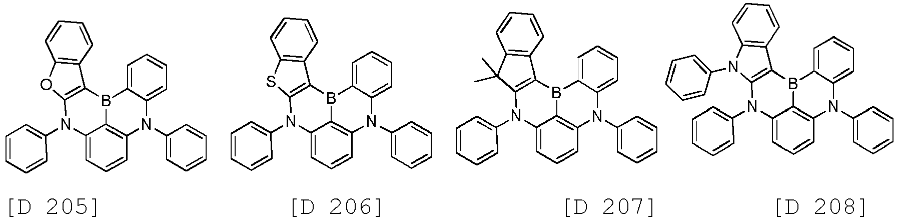

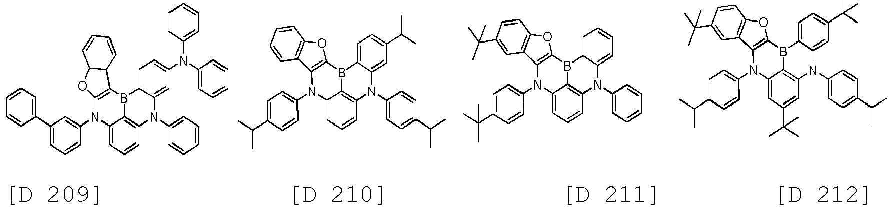

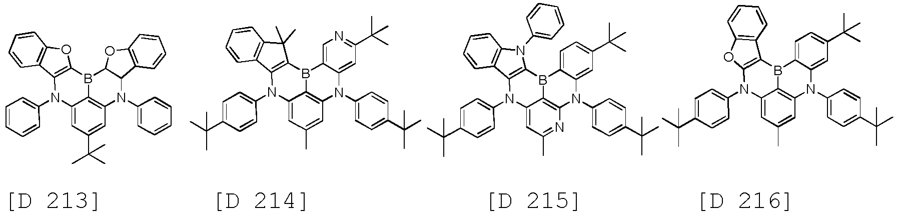

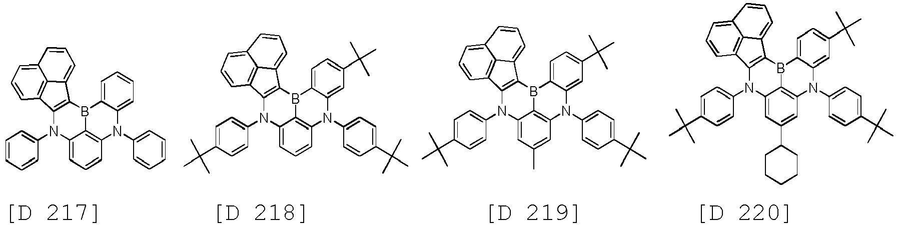

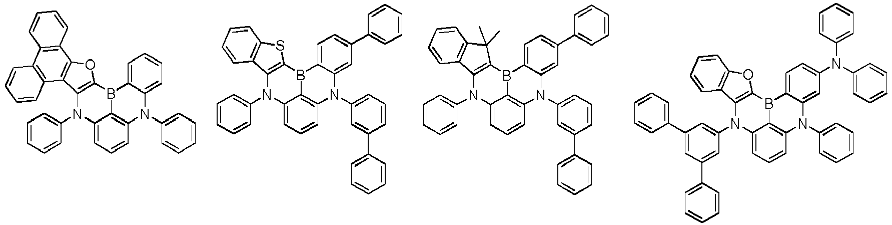

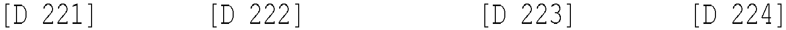

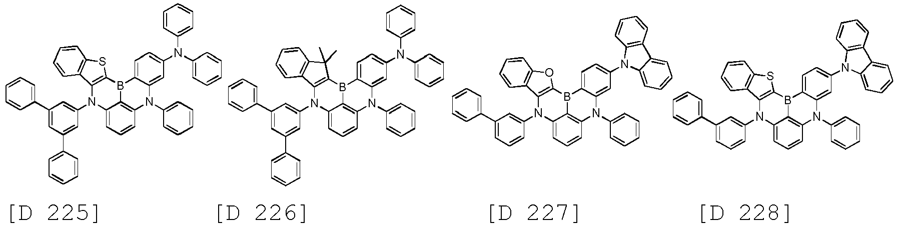

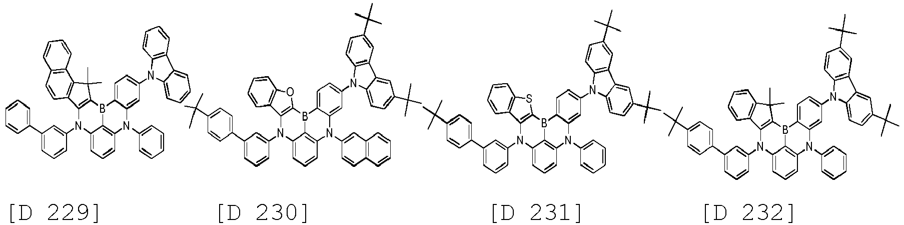

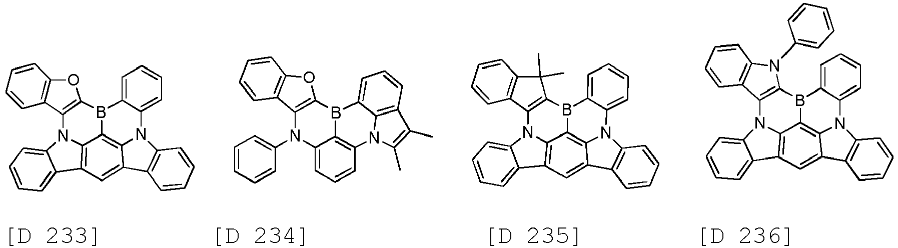

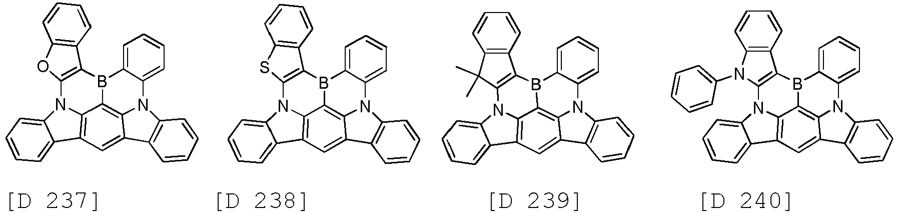

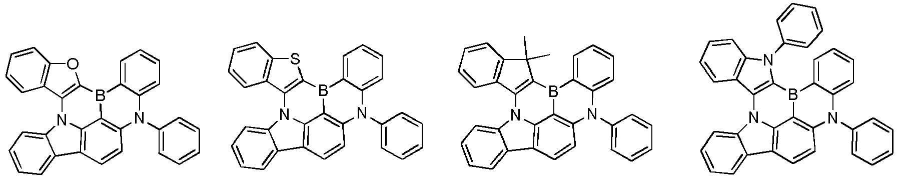

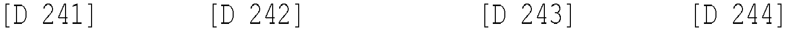

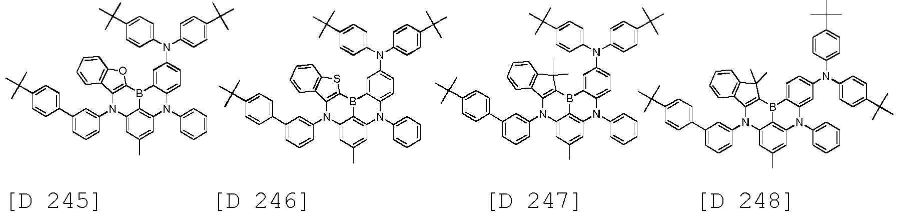

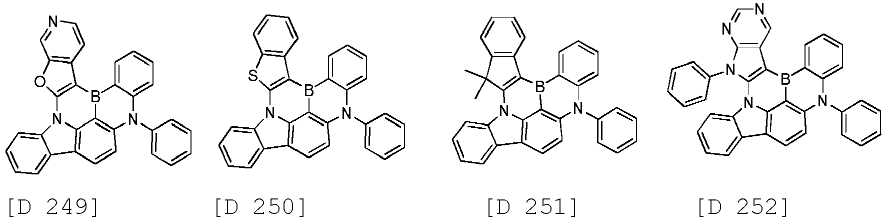

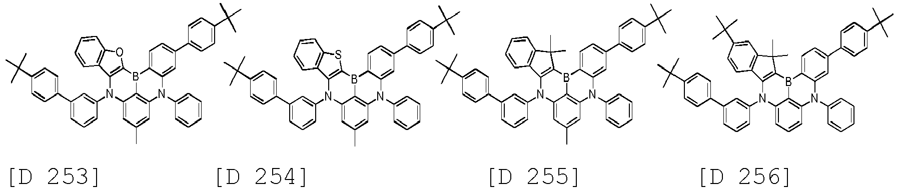

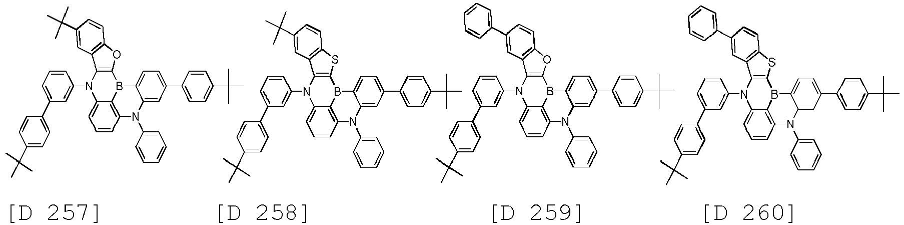

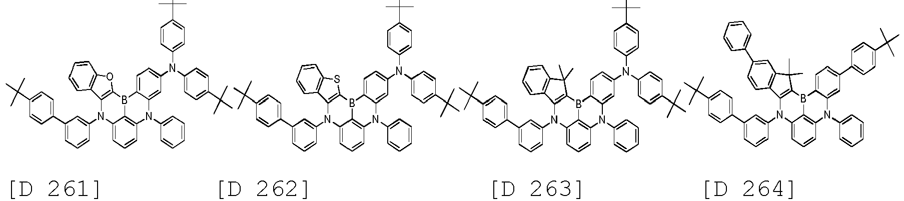

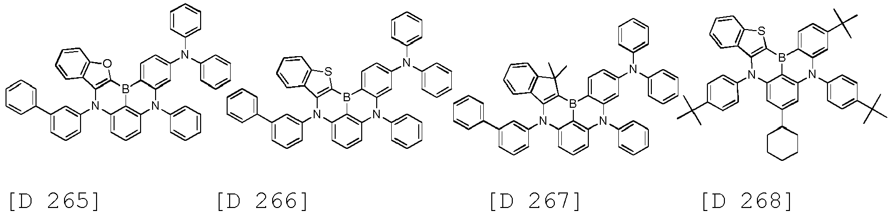

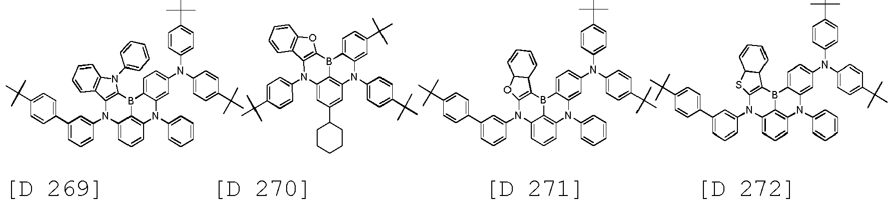

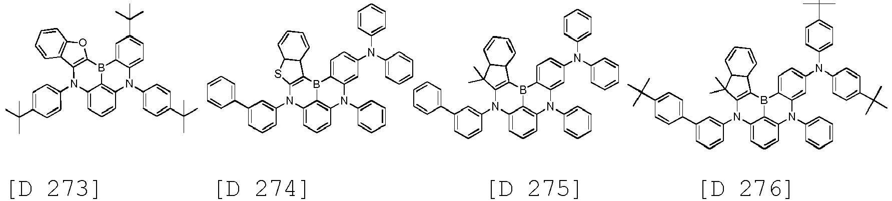

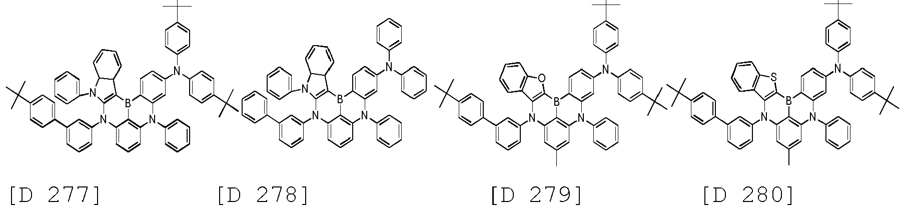

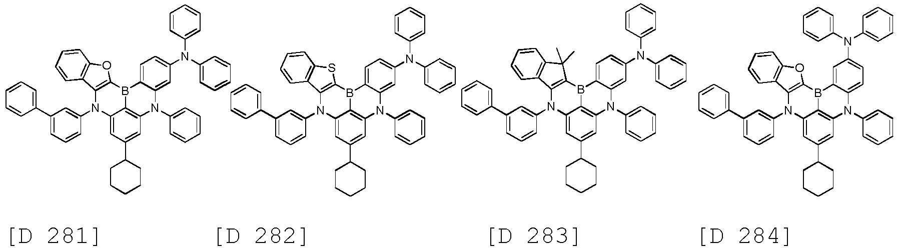

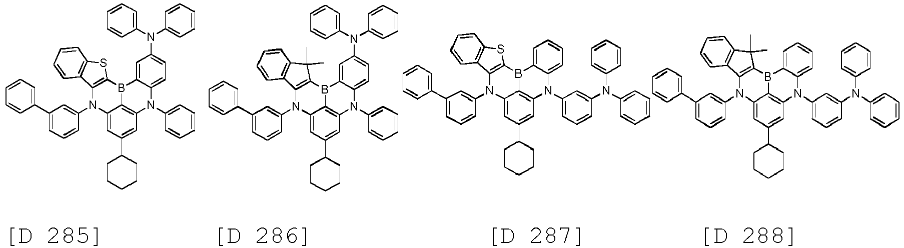

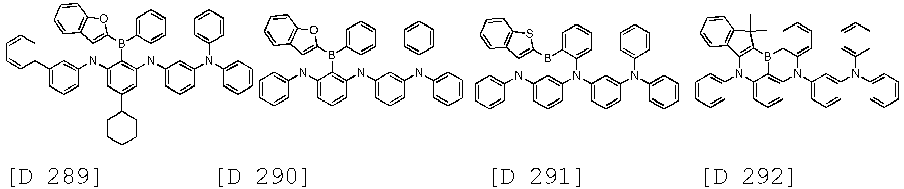

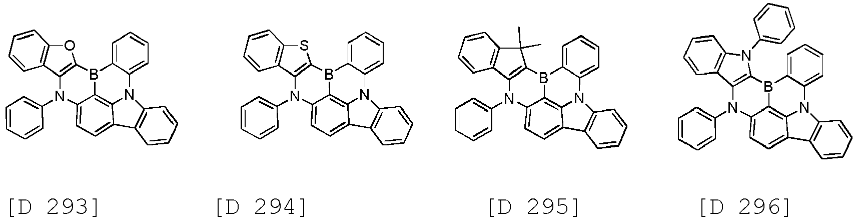

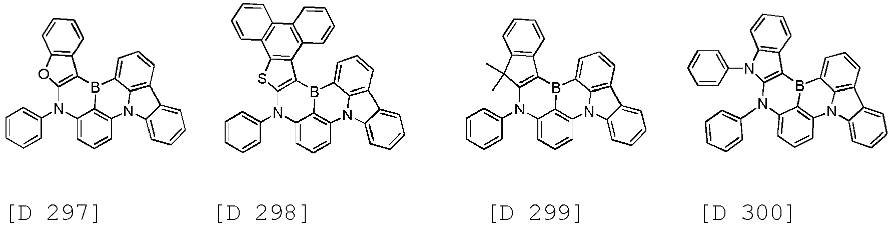

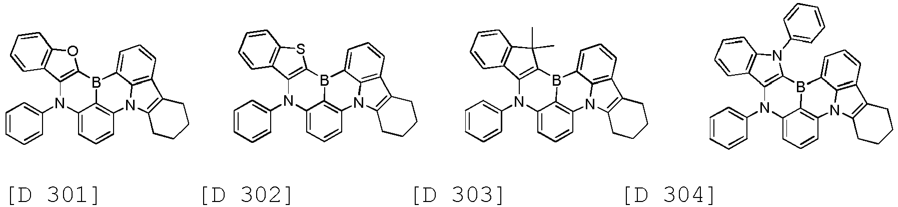

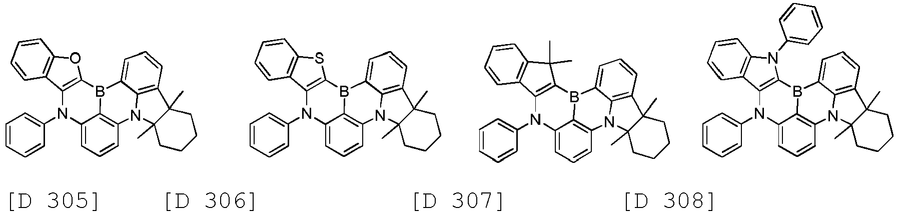

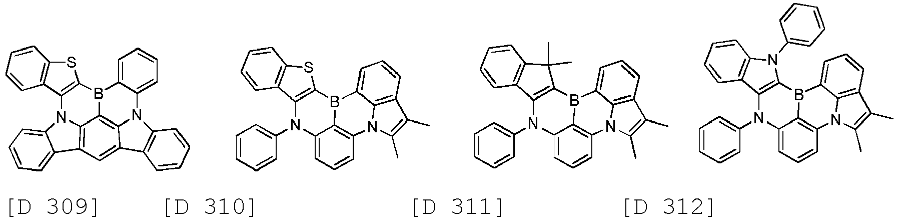

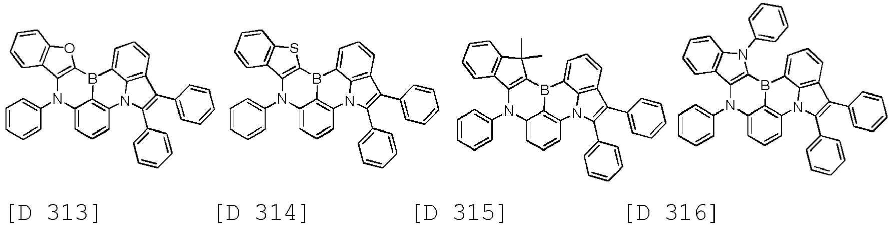

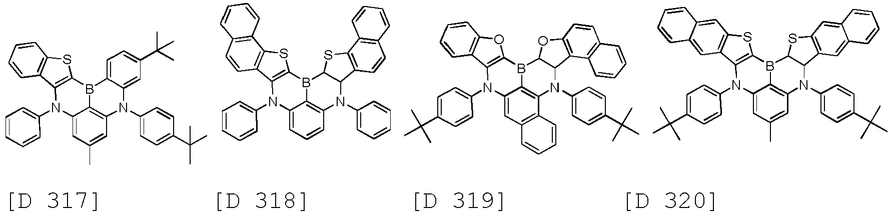

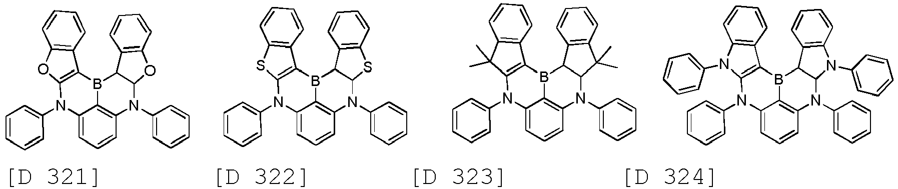

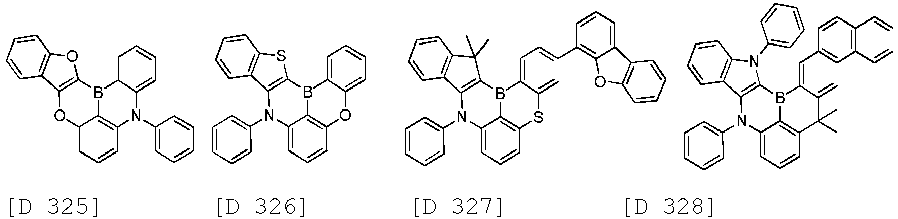

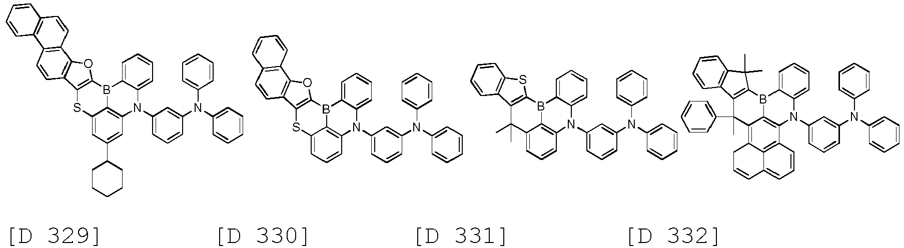

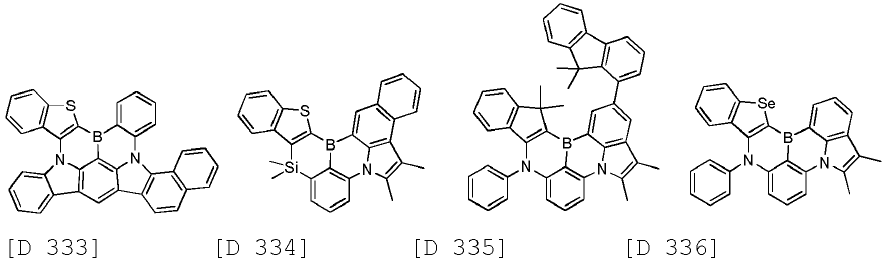

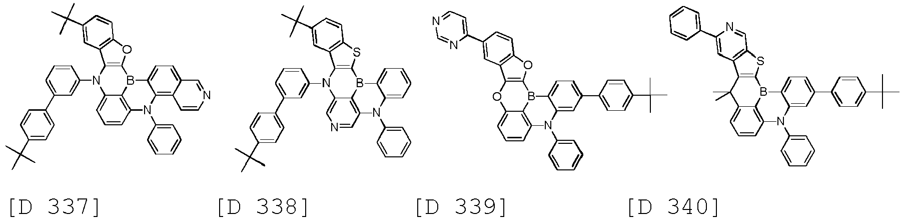

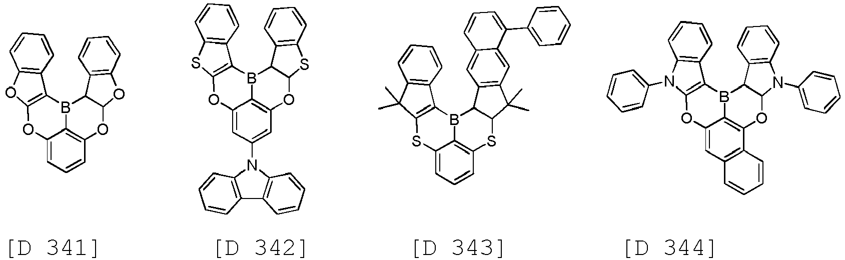

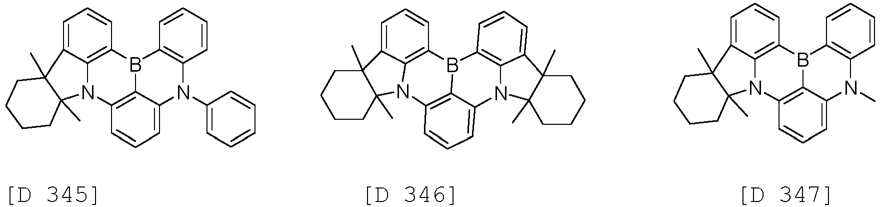

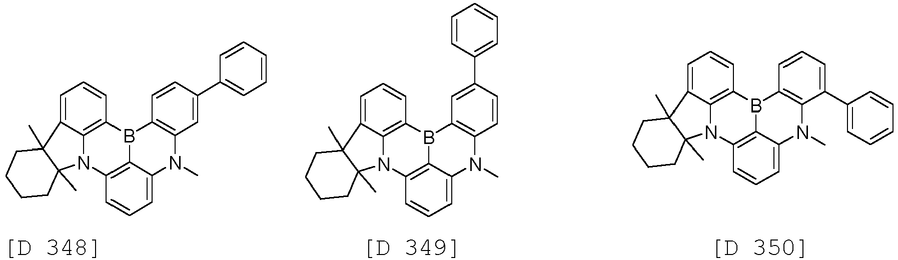

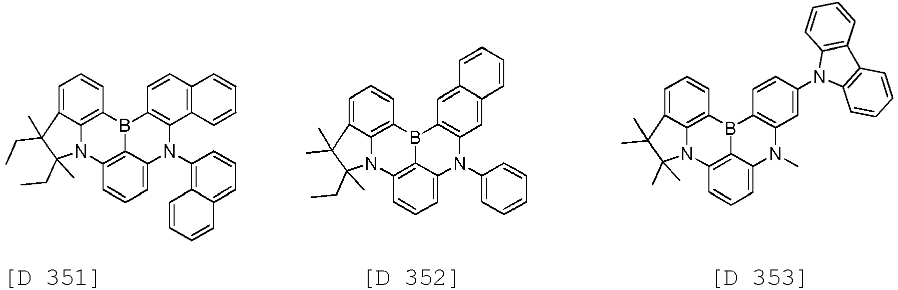

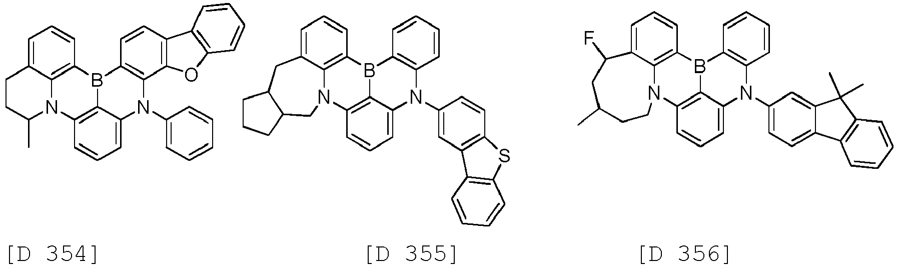

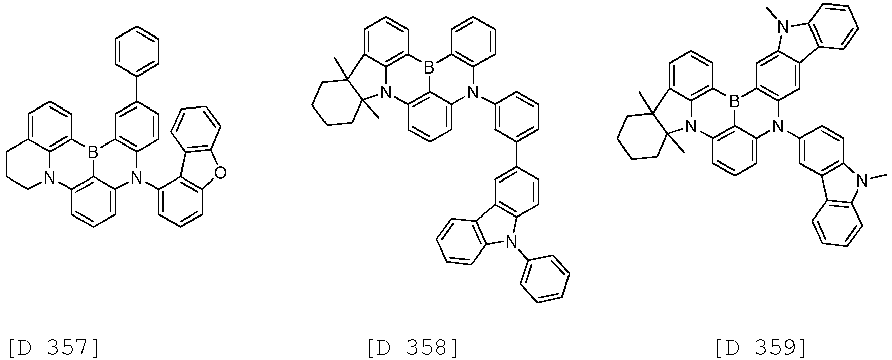

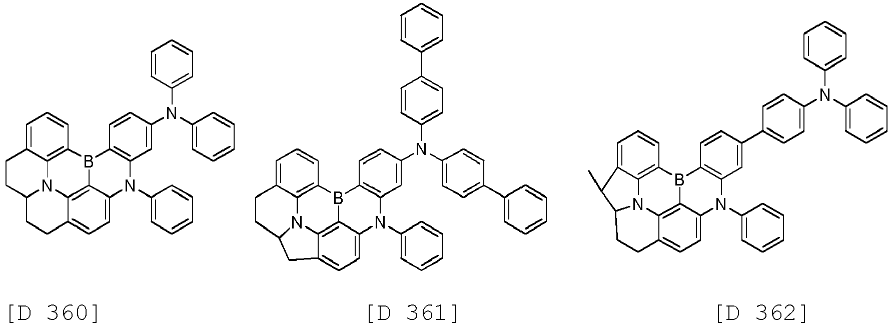

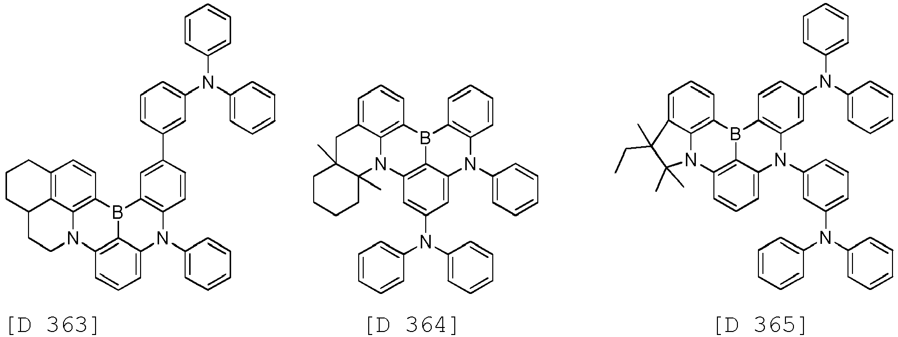

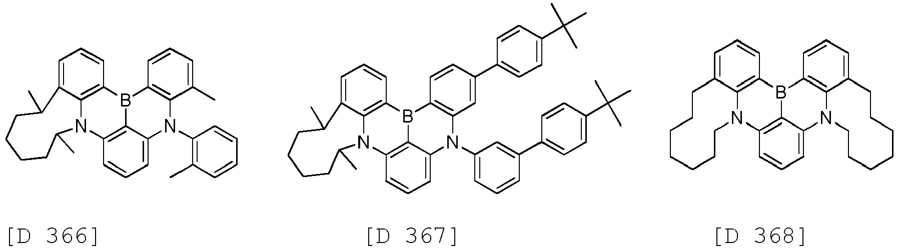

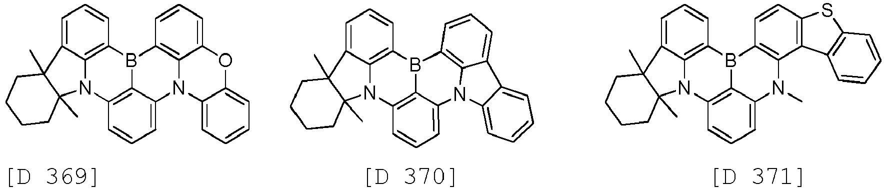

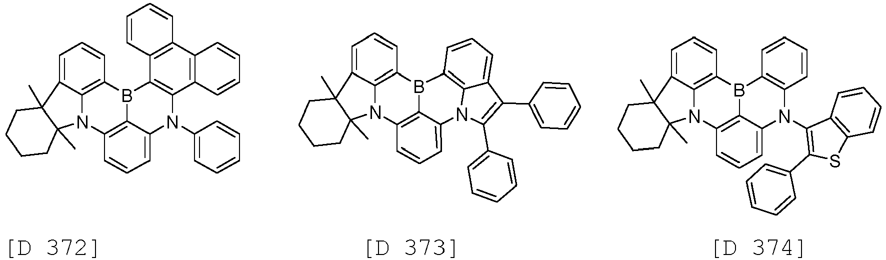

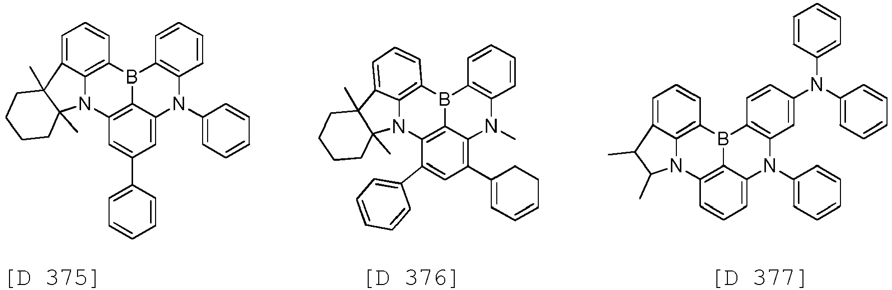

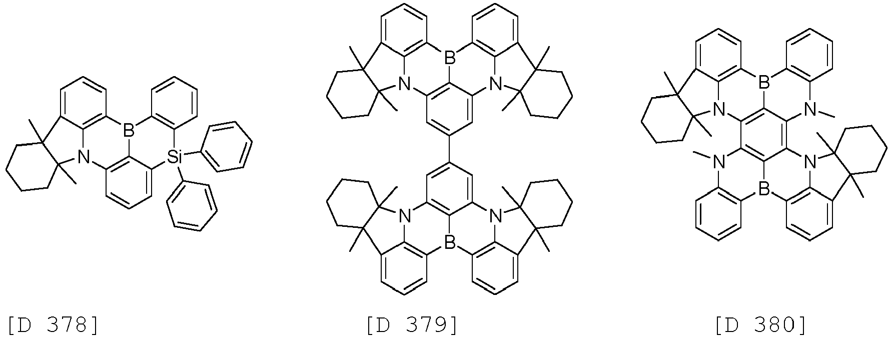

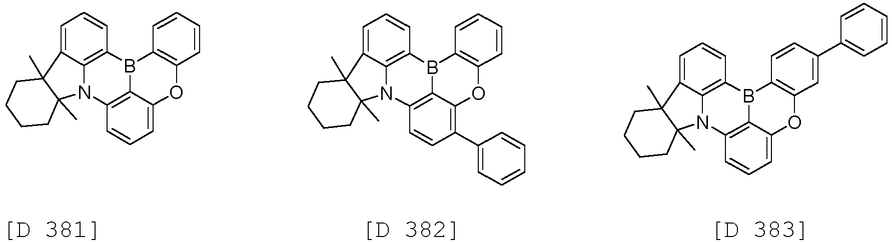

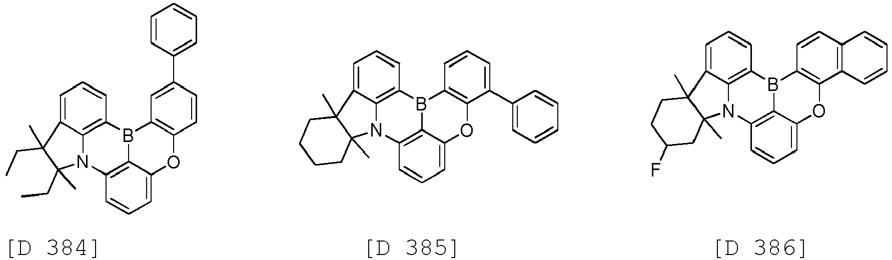

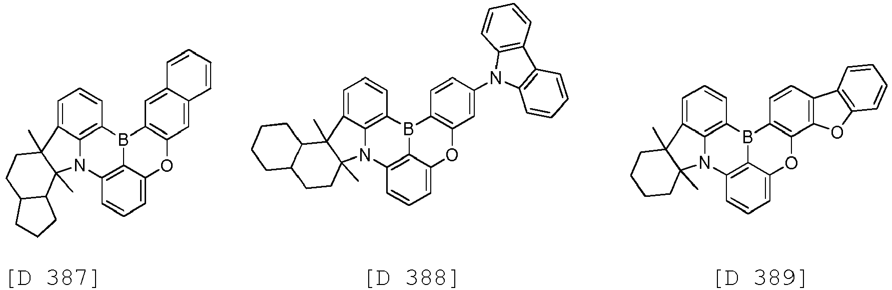

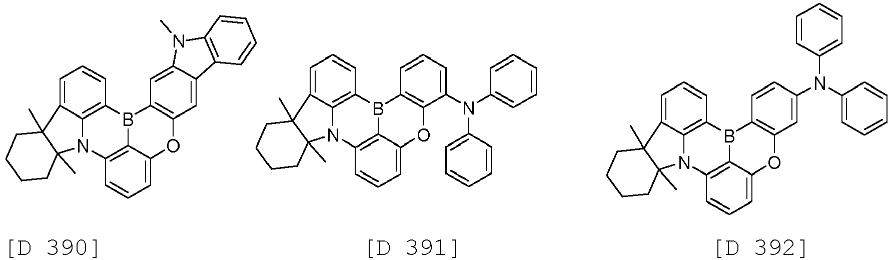

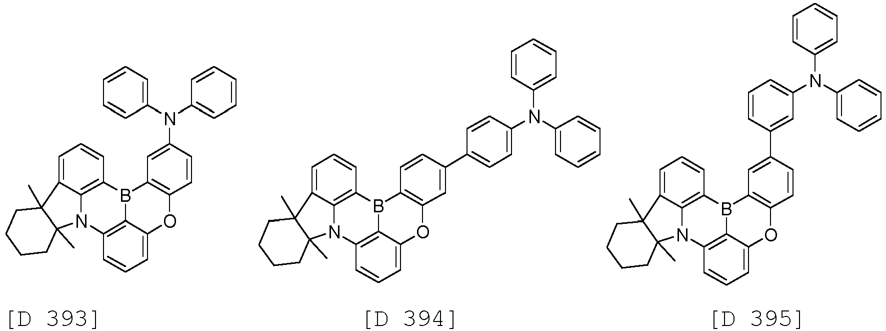

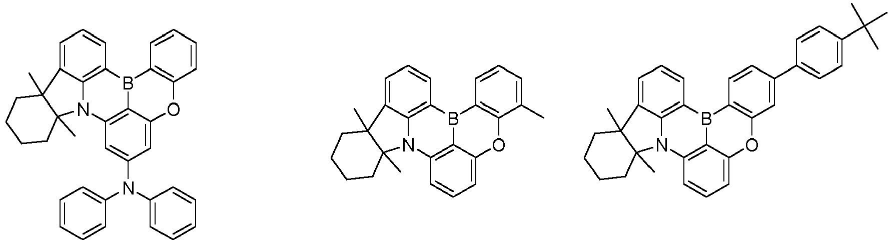

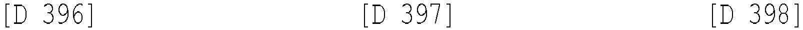

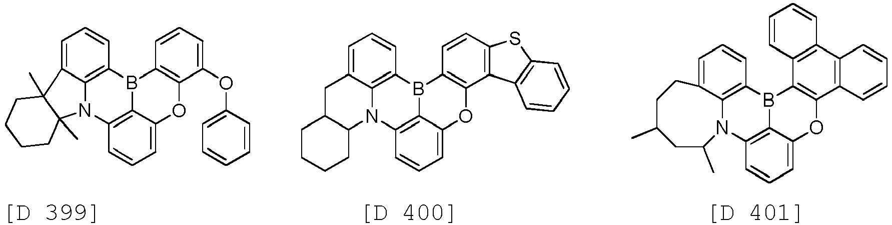

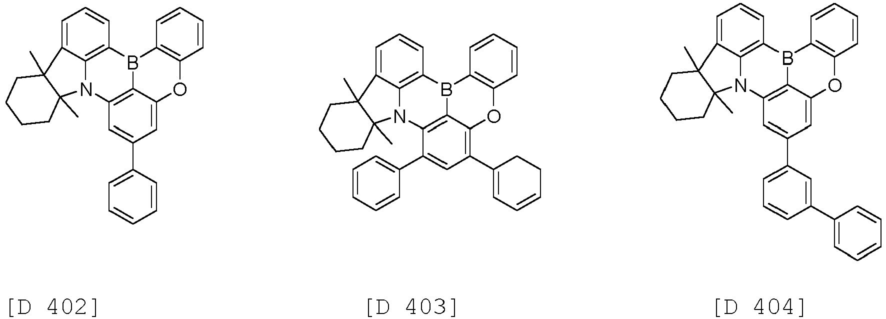

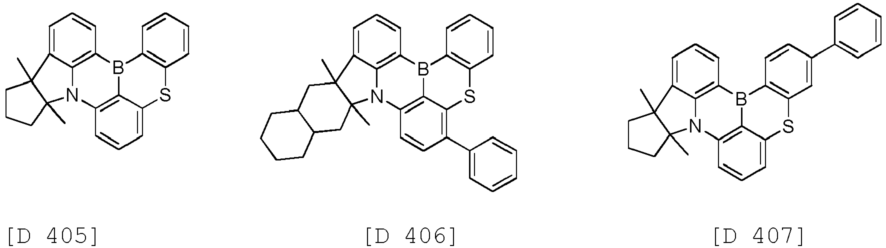

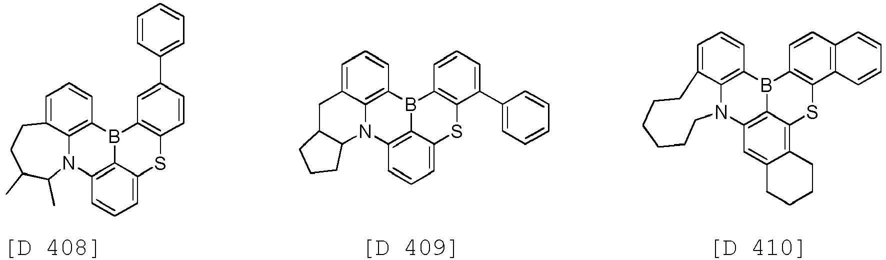

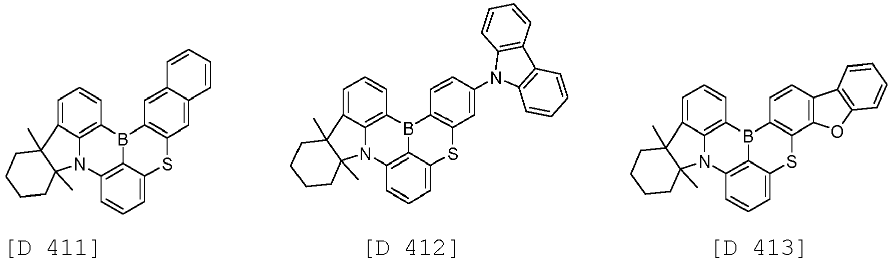

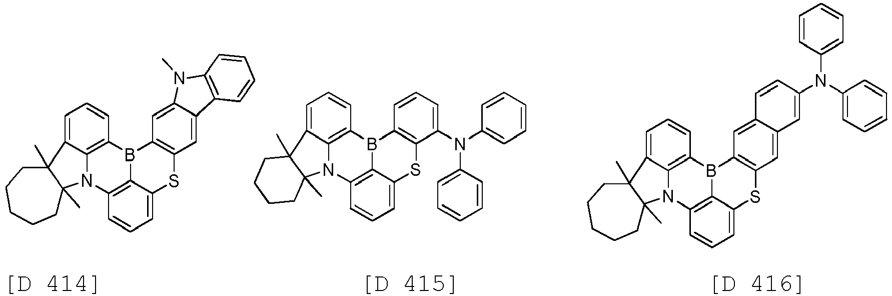

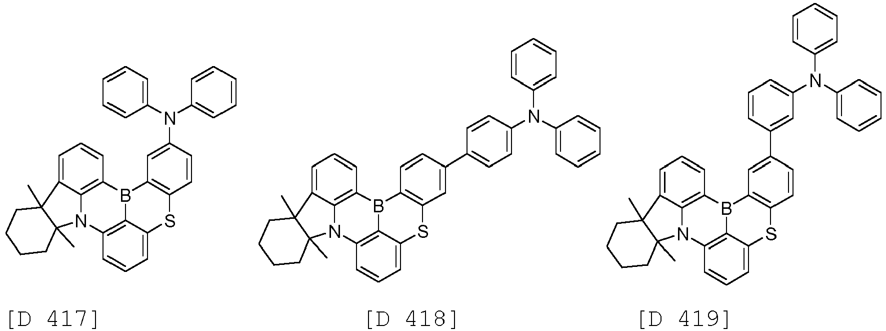

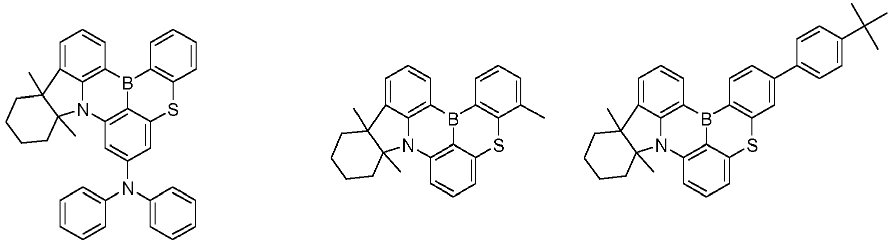

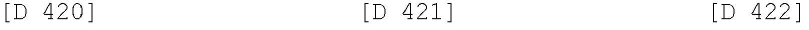

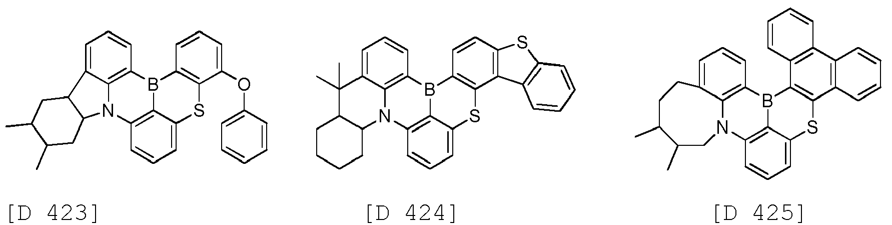

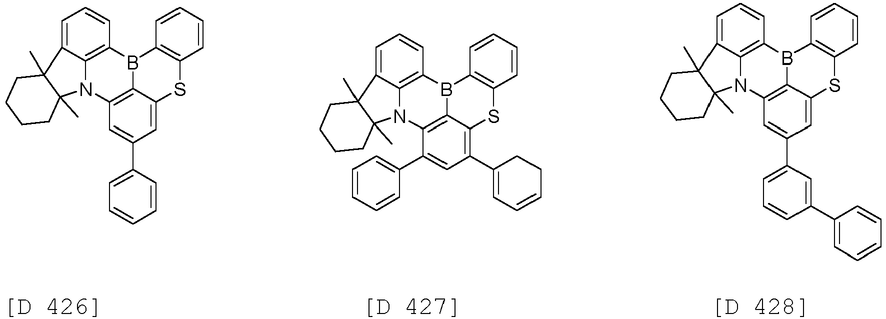

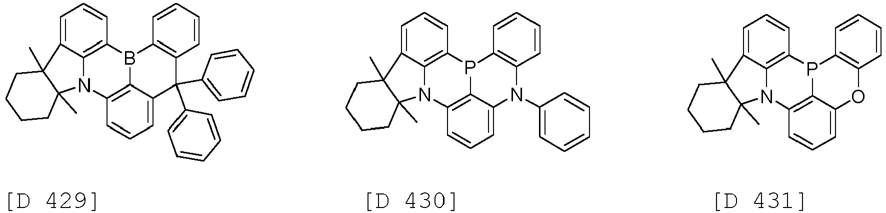

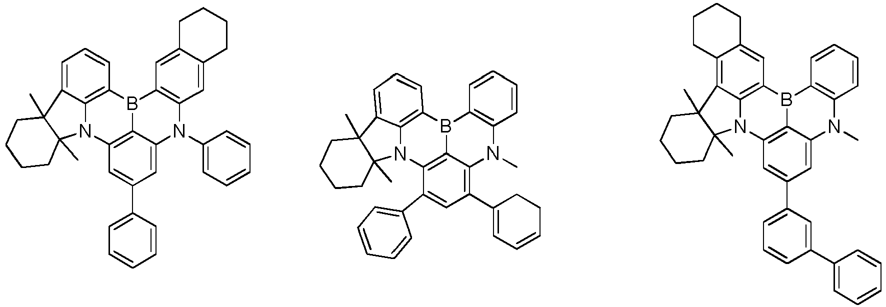

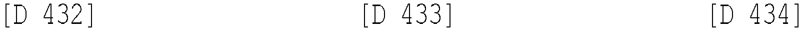

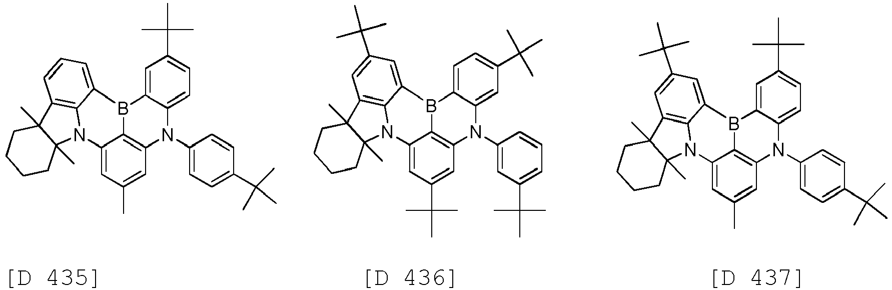

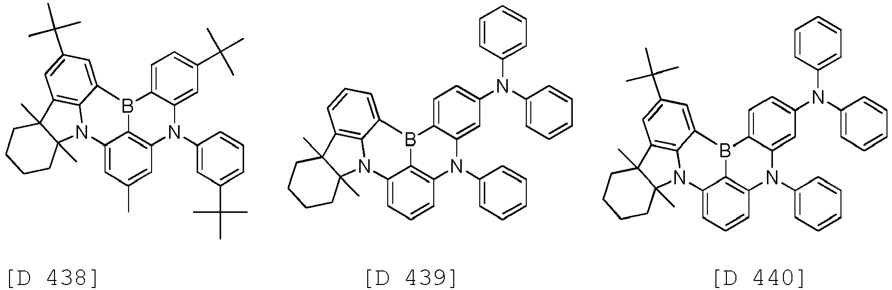

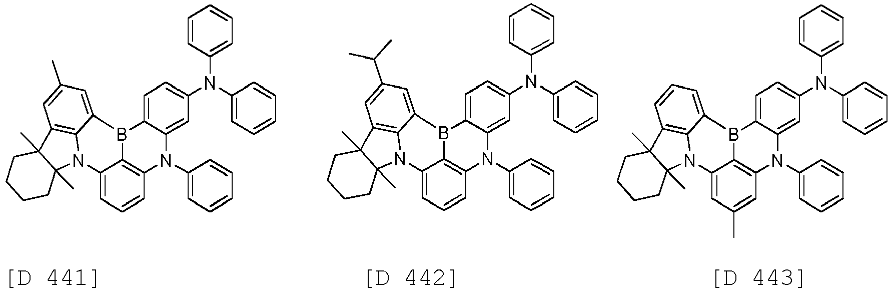

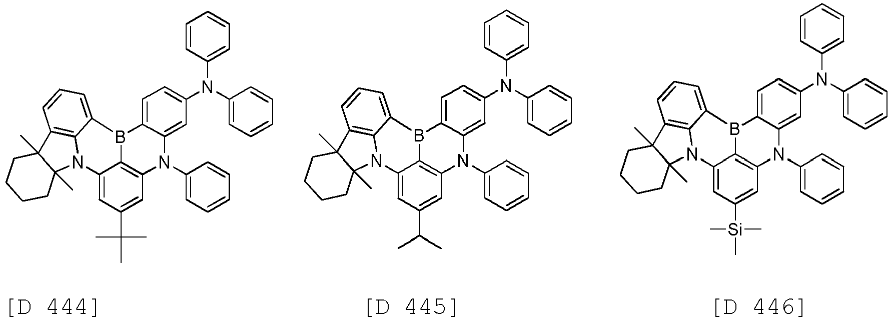

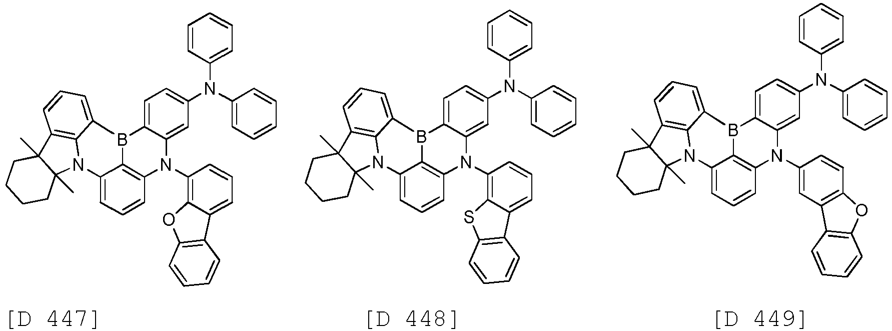

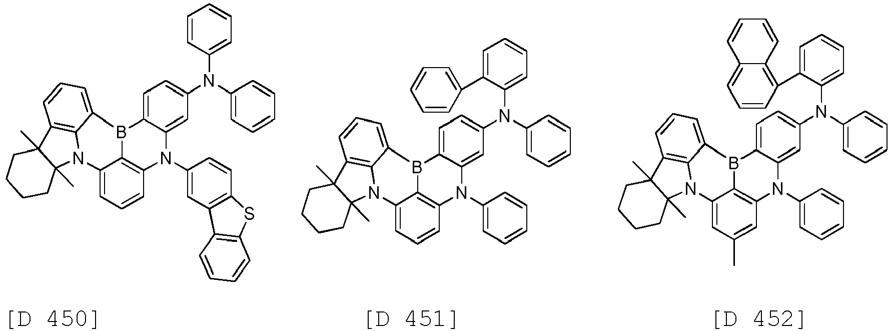

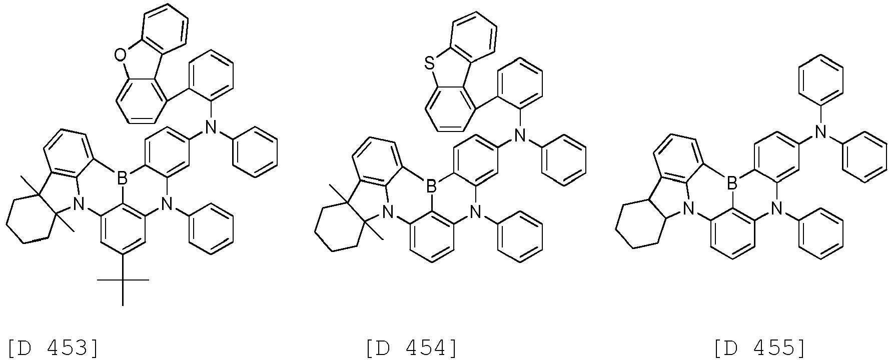

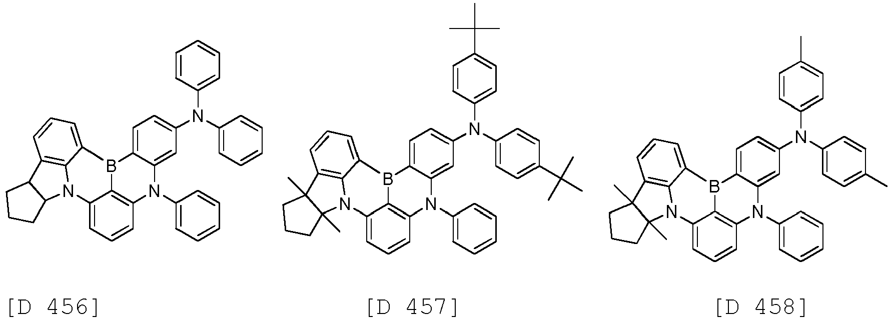

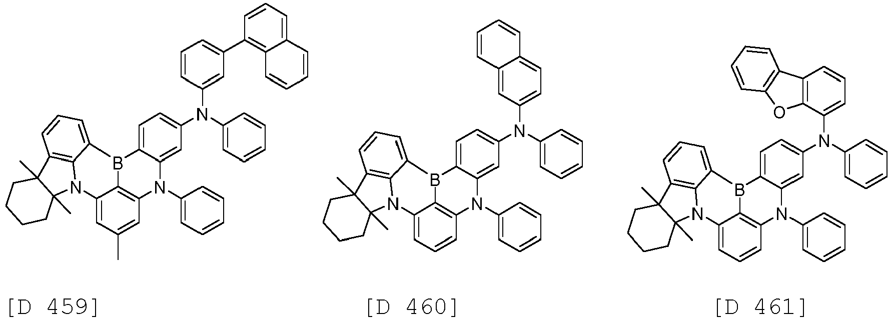

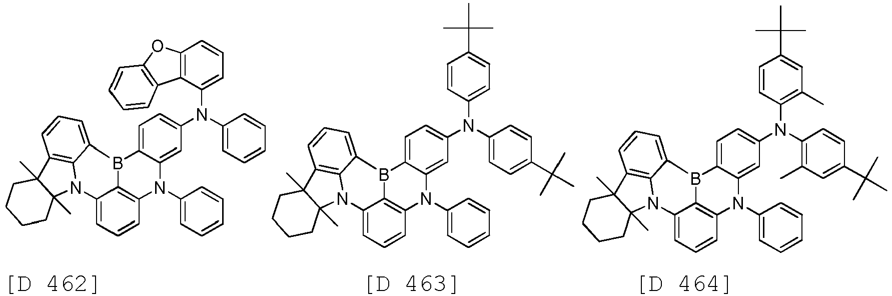

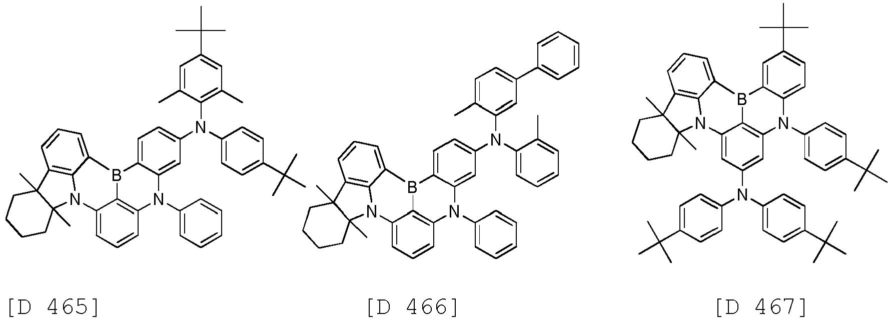

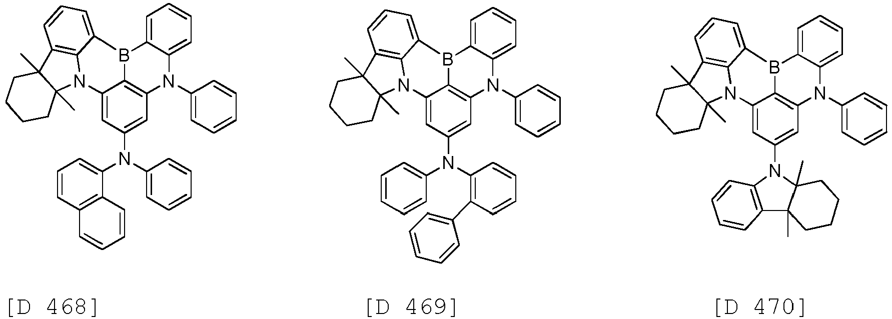

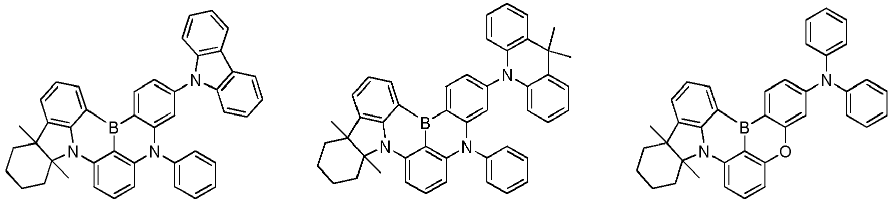

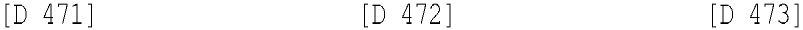

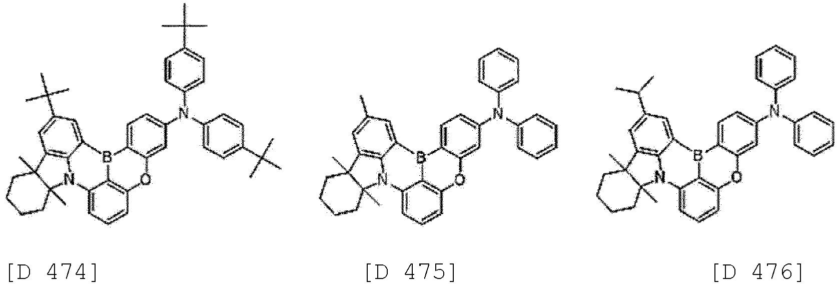

Among the dopant compounds useful in the light-emitting layer according to the present disclosure, examples of the compound represented by Chemical Formula D6 or D7 include the following <D 501> to <D 587>:

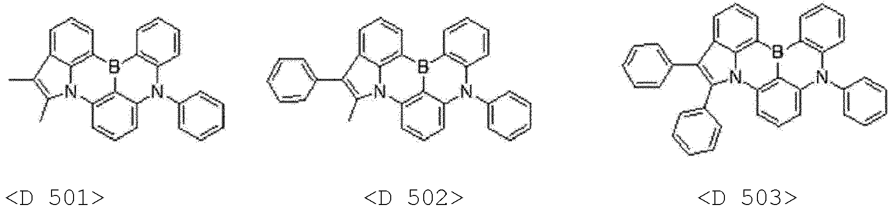

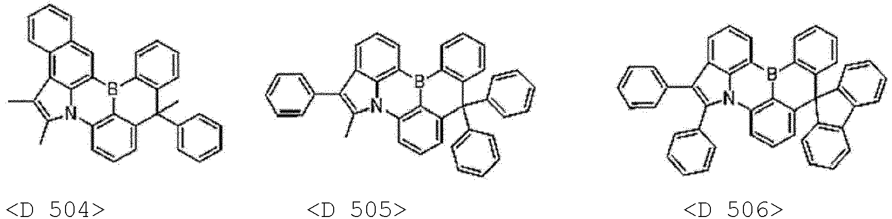

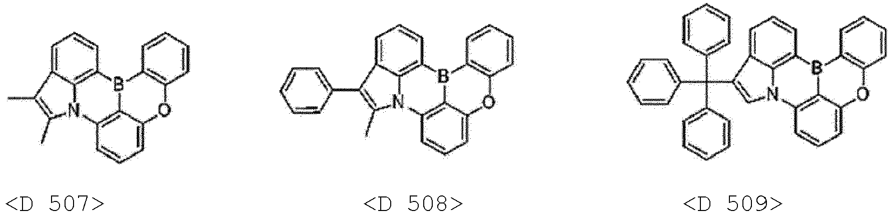

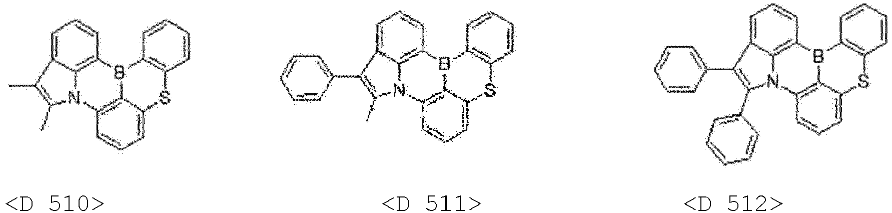

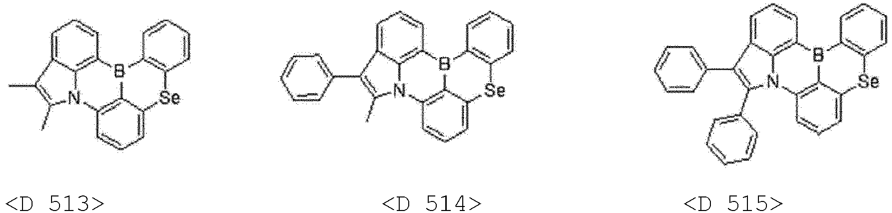

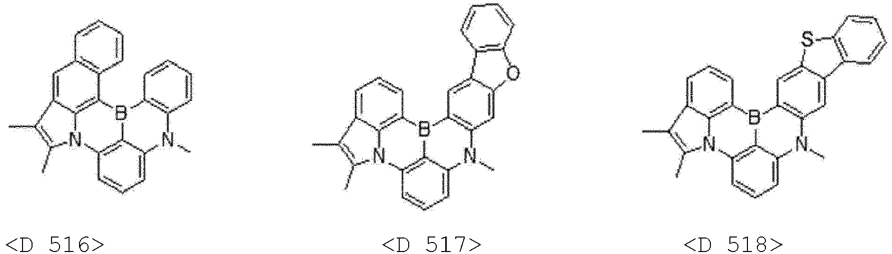

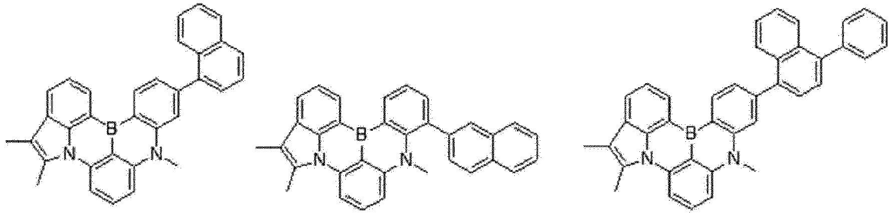

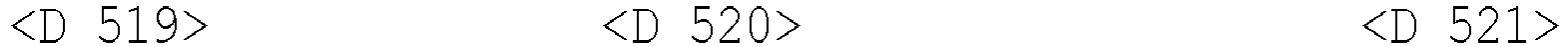

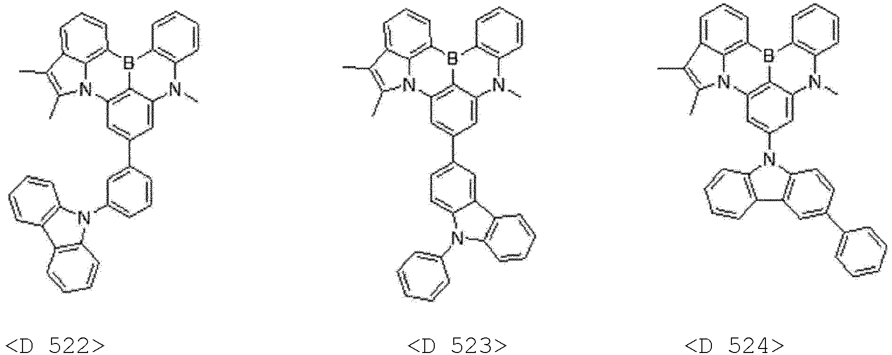

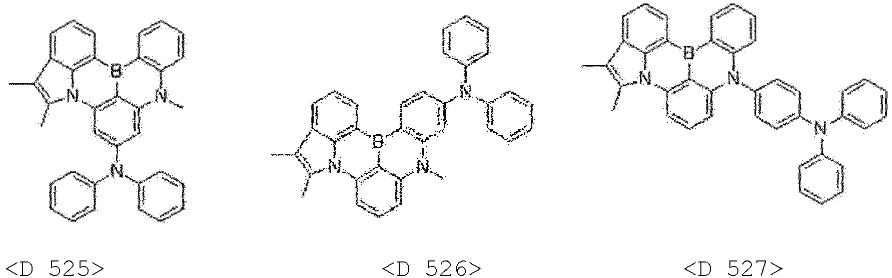

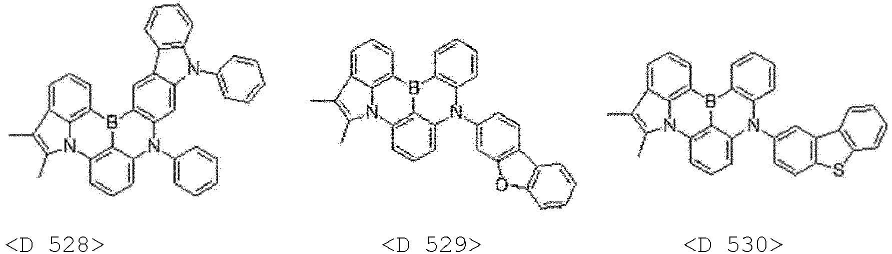

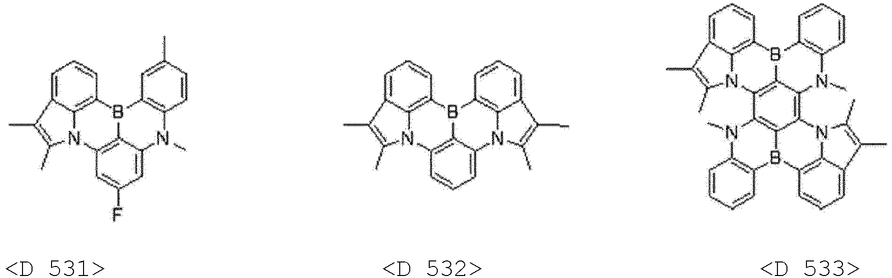

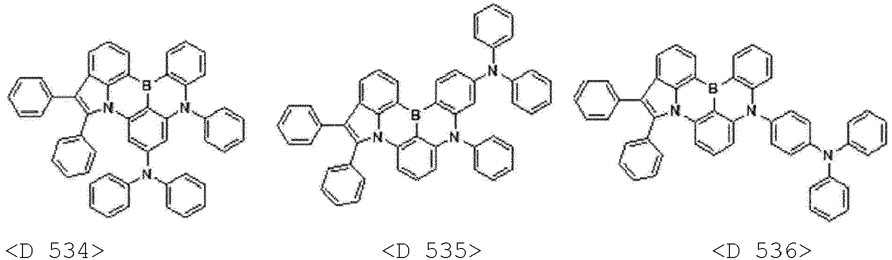

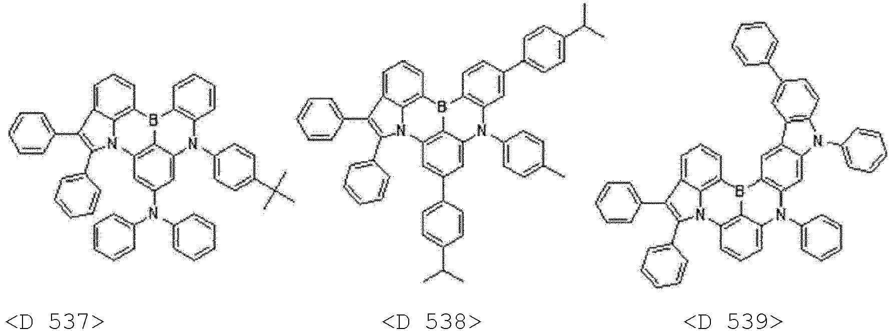

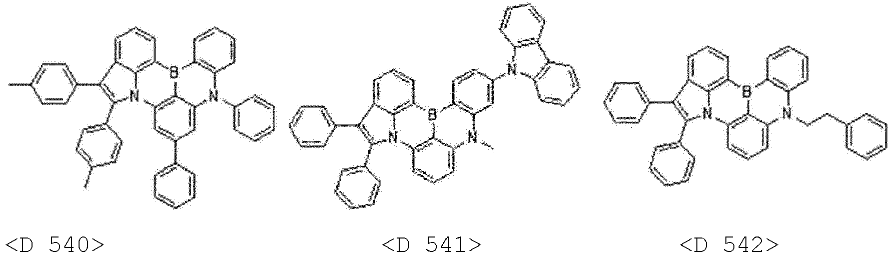

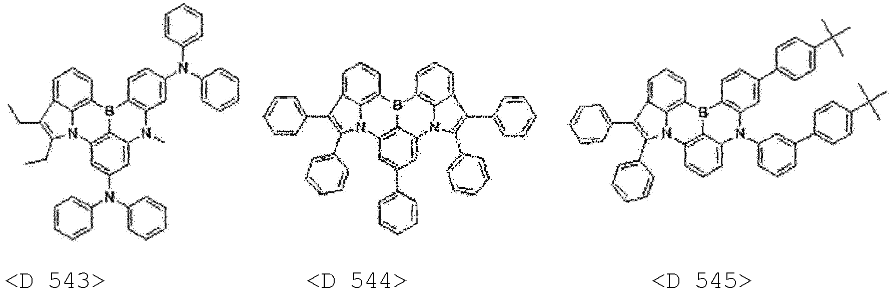

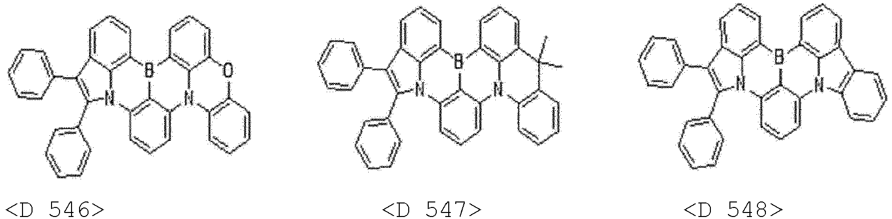

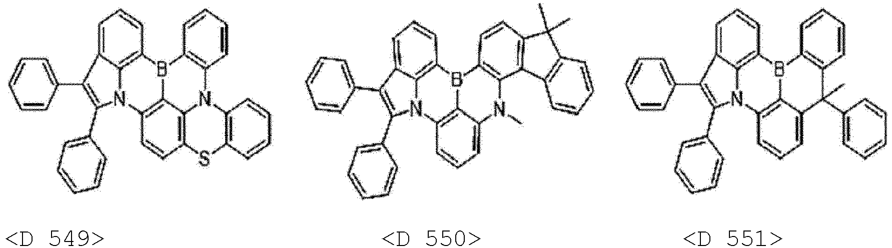

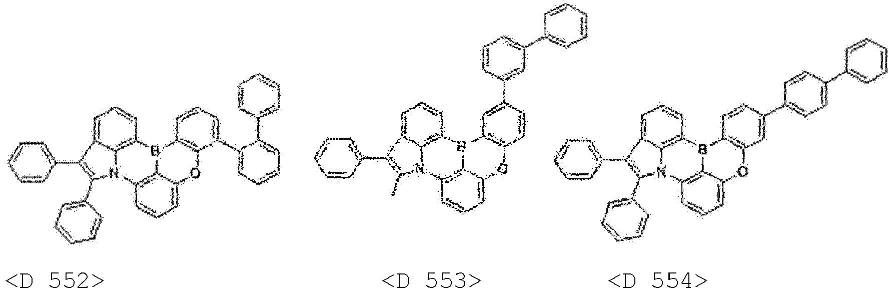

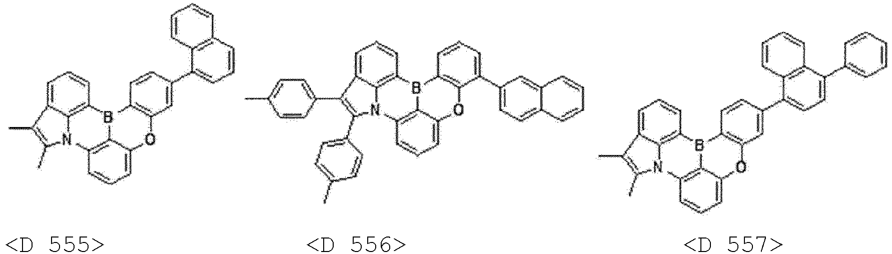

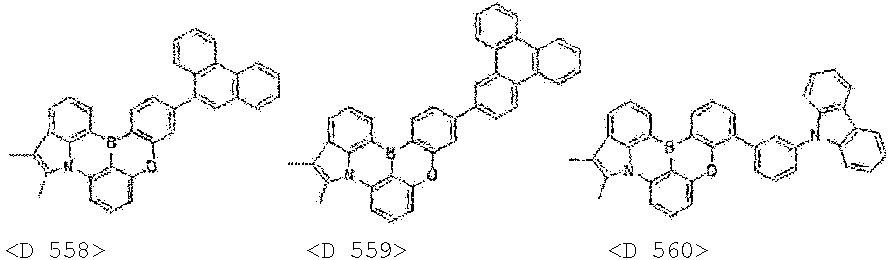

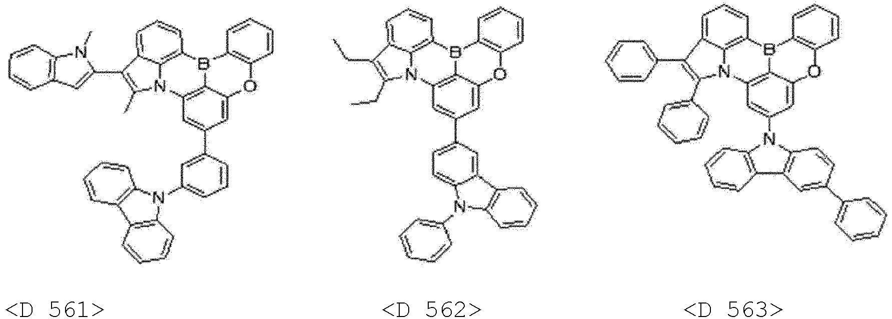

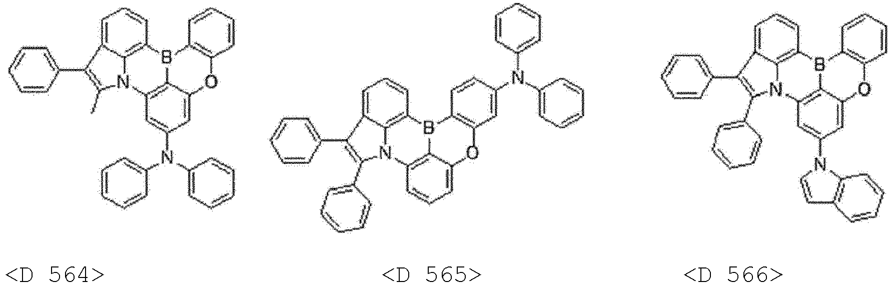

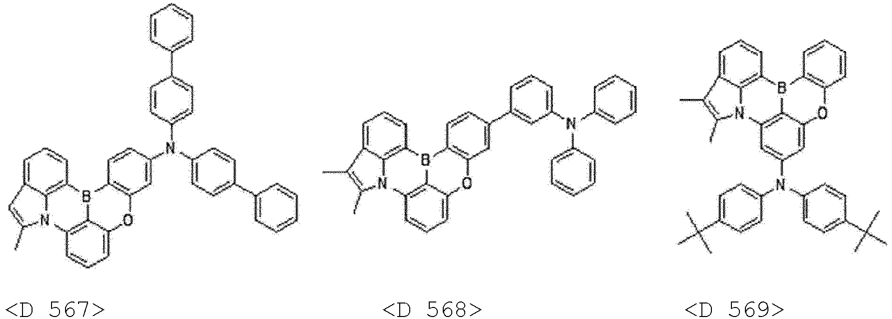

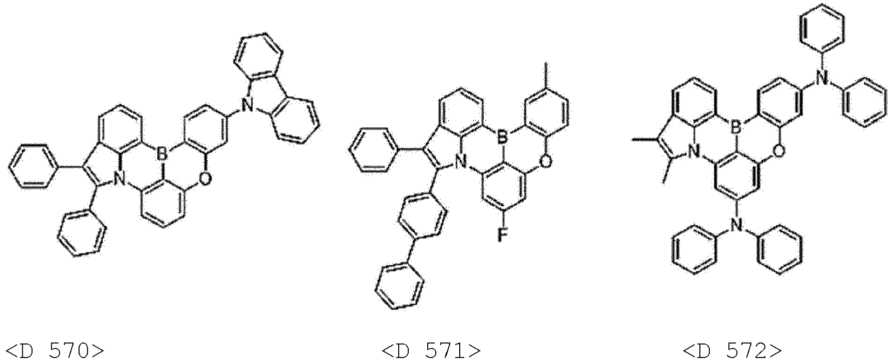

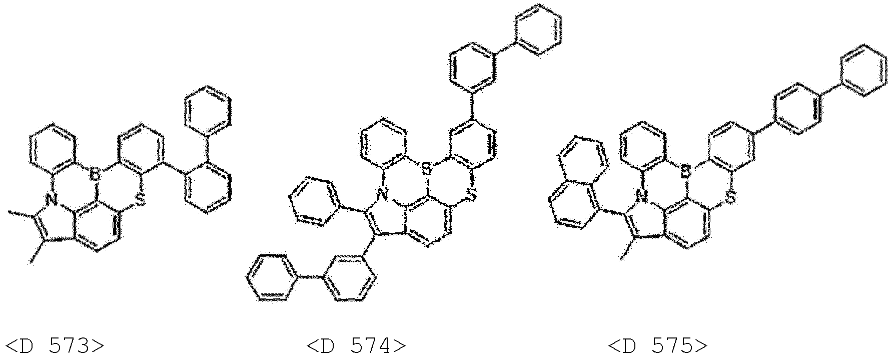

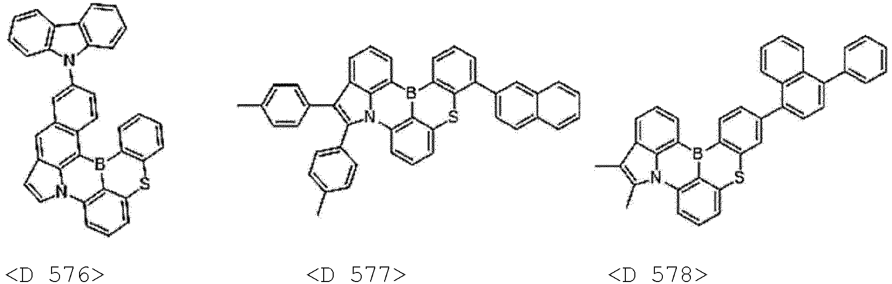

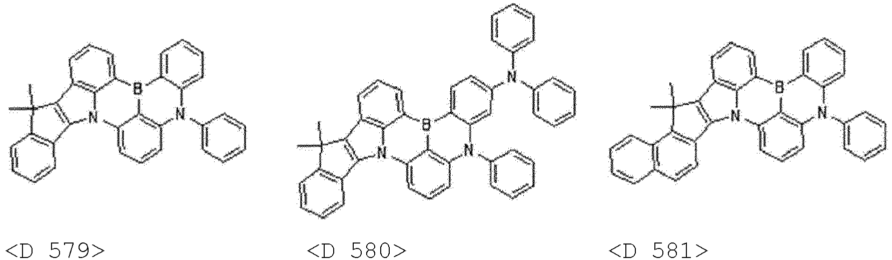

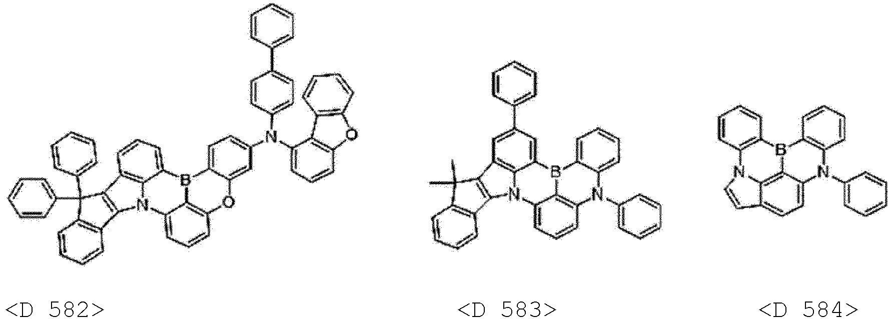

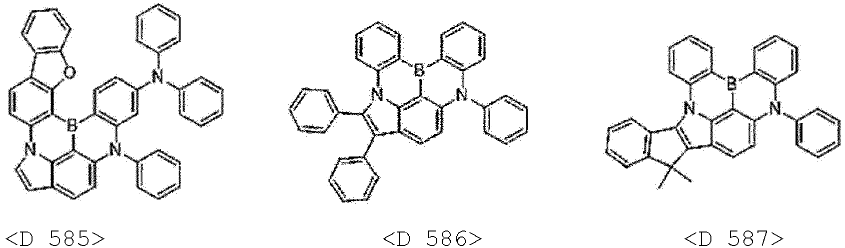

The content of the dopant in the light-emitting layer may range from about 0.01 to 20 parts by weight, based on 100 parts by weight of the host, but is not limited thereto.

In addition to the above-mentioned dopants and hosts, the light-emitting layer may further include various hosts and dopant materials.

In a particular embodiment according to the present disclosure, the first light-emitting layer may include at least one of the compounds represented by Chemical Formula A.

Here, when the first light-emitting layer in the organic light-emitting diode according to the present disclosure includes the compound represented by Chemical Formula A, the second light-emitting layer may employ an anthracene derivative represented by the following Chemical Formula E as a host, whereby more improved efficiency, low-voltage driving, and longevity can be imparted to the diode. 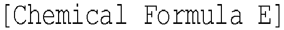 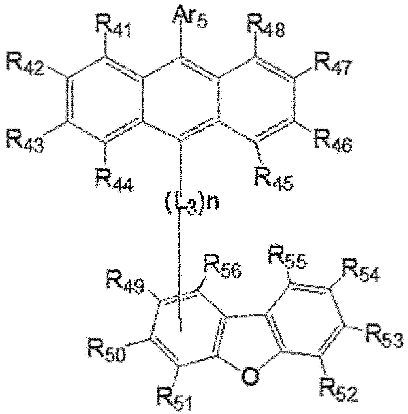 wherein
R₄₁ to R₅₆, which are same or different, are each independently any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a nitro, a cyano, and a halogen;
Ar₅ is a substituted or unsubstituted aryl of 6 to 50 carbon atoms or a substituted or unsubstituted heteroaryl of 2 to 50 carbon atoms;
L₃ is a single bond, a substituted or unsubstituted arylene of 6 to 20 carbon atoms, or a substituted or unsubstituted heteroarylene of 2 to 20 carbon atoms, and
n is an integer of 1 to 2 wherein when n is 2, the L₁'s are same or different,
wherein the team "substituted" in the expression "substituted or unsubstituted" used for compounds of Chemical Formula E means having at least one substituent selected from the group consisting of a deuterium atom, a cyano, a halogen, a hydroxyl, a nitro, an alkyl of 1 to 24 carbon atoms, a halogenated alkyl of 1 to 24 carbon atoms, alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

In addition, the anthracene derivative represented by Chemical formula E, used in the second light-emitting layer, may bear at least one deuterium atoms. In this regard, at least one of the substituents R₄₁ to R₅₆ and Ar₅ may be a deuterated aryl of 6 to 18 carbon atoms.

In addition, in a particular embodiment according to the present disclosure, when the second light-emitting layer contains an anthracene derivative represented by Chemical Formula E as a host, the anthracene derivative represented by Chemical Formula E may preferably be the anthracene derivative represented by Chemical Formula E-1 or E-2: 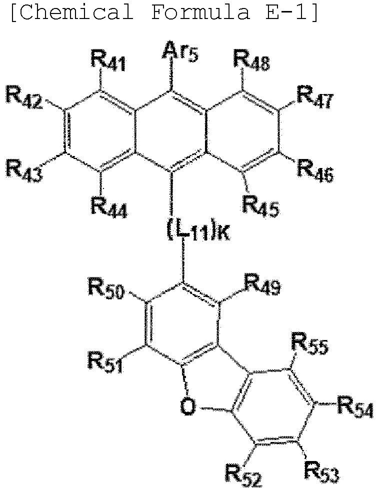 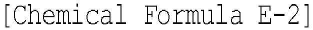 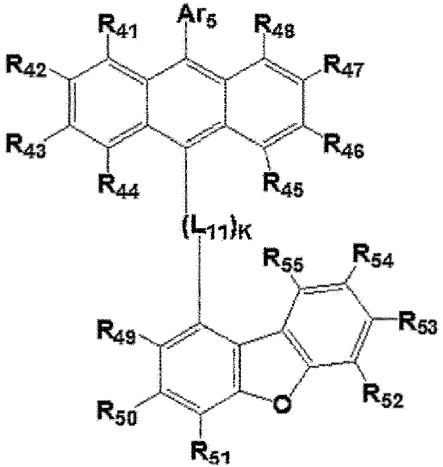 wherein,
R₄₁ to R₄₈, R₄₉ to R₅₅ which are same or different, are each independently any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
Ar₅ is a substituted or unsubstituted aryl of 6 to 50 carbon atoms or a substituted or unsubstituted heteroaryl of 2 to 50 carbon atoms;
L₁₁ is any one selected from among a single bond, a substituted or unsubstituted arylene of 6 to 20 carbon atoms, and a substituted or unsubstituted heteroarylene of 2 to 20 carbon atoms; and
k is an integer of 1 to 2 wherein when k is 2, the L₁₁'s are same or different,
wherein the team "substituted" in the expression "substituted or unsubstituted" used for compounds of Chemical Formulas E-1 and E-2 means having at least one substituent selected from the group consisting of a deuterium atom, a cyano, a halogen, a hydroxyl, a nitro, an alkyl of 1 to 24 carbon atoms, a halogenated alkyl of 1 to 24 carbon atoms, alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

As more particular examples accounting for the term "substituted" in the expression "substituted or unsubstituted" used for compounds of Chemical Formulas E, E-1, and E-2, the compounds may be substituted by at least one substituent selected from the group consisting of a deuterium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 2 to 18 carbon atoms, an alkoxy of 1 to 12 carbon atoms, an alkylamino of 1 to 12 carbon atoms, a diarylamino of 12 to 18 carbon atoms, a heteroarylamino of 2 to 18 carbon atoms, an aryl(heteroaryl)amino of 7 to 18 carbon atoms, an alkylsilyl of 1 to 12 carbon atoms, an arylsilyl of 6 to 18 carbon atoms, an aryloxy of 6 to 18 carbon atoms, and an arylthionyl of 6 to 18 carbon atoms.

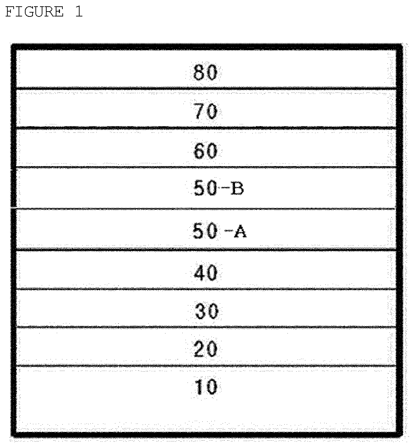

Here, the compound represented by Chemical Formula E-1 or E-2 is characterized by the structure in which position 1 or 2 of one phenyl ring or position 1' or 2' of the other phenyl ring in the dibenzofuran moiety shown in the following Diagram 1 may be connected to position 9 of the anthracenyl moiety or to the linker L₁₁.

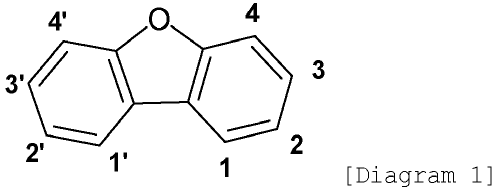

In the present disclosure, the substituent Ar5 on the anthracene derivative represented by any one of Chemical Formulas E, E-1, and E-2 may be a substituent represented by the following Structural Formula C-1: 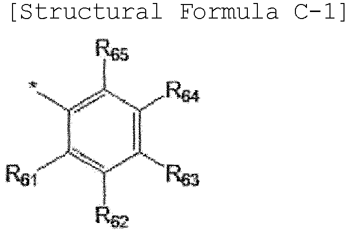 wherein,
R₆₁ to R₆₅, which are same or different, are each independently any one selected from among a hydrogen atom, a deuterium atom, a substituted or unsubstituted alkyl of 1 to 20 carbon atoms, a substituted or unsubstituted aryl of 6 to 50 carbon atoms, a substituted or unsubstituted arylalkyl of 7 to 50 carbon atoms, a substituted or unsubstituted cycloalkyl of 3 to 30 carbon atoms, a substituted or unsubstituted alkylsilyl of 1 to 30 carbon atoms, a substituted or unsubstituted arylsilyl of 6 to 30 carbon atoms, and a halogen, and
"-*" denotes a bonding site to position 10 of the anthracenyl moiety in Chemical Formula E, E-1, or E-2.

In a particular embodiment of the organic light-emitting diode according to the present disclosure, the linker L₁₁ in Chemical Formula E-1 or E-2 may be a single bond or a substituted or unsubstituted arylene of 6 to 14 carbon atoms, and k is an integer of 1 to 2 wherein when k is 2, the L₁₁'s are same or different.

For use in the organic light-emitting diode according to the present disclosure, the anthracene derivative represented by Chemical Formula E may be any one selected from among the following <Compound 201> to <Compound 473>:

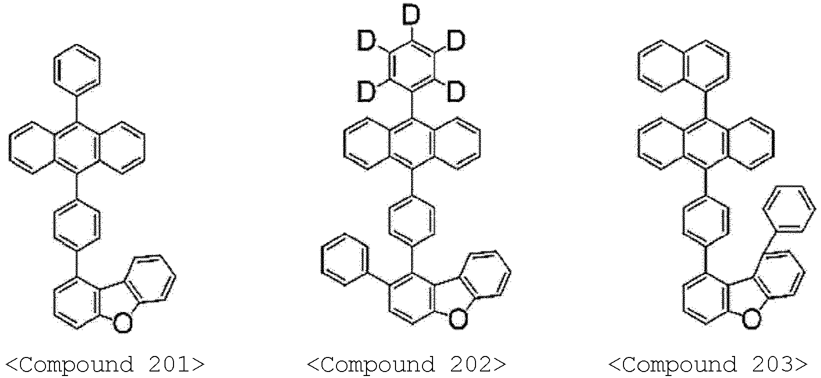

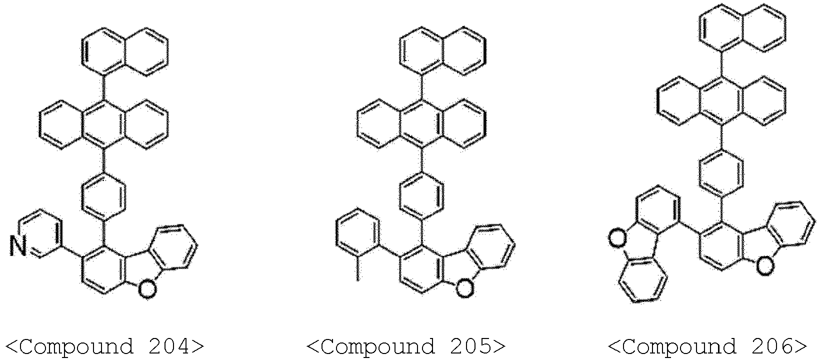

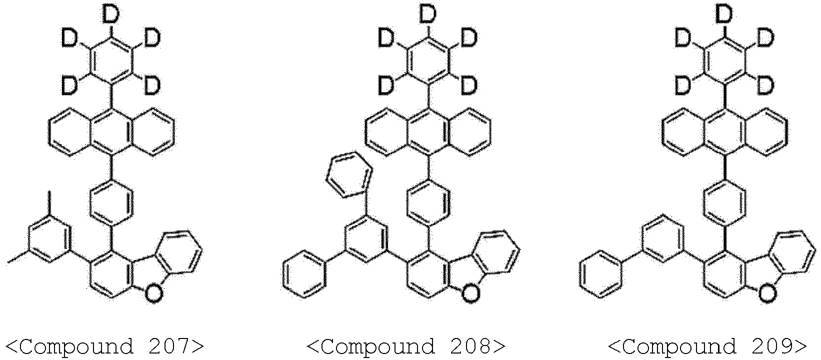

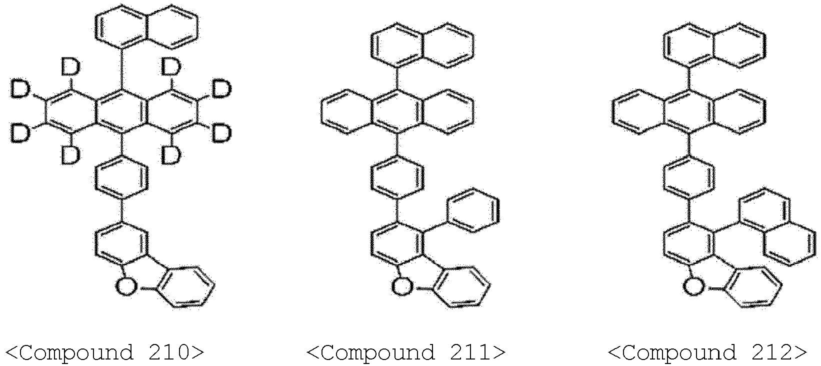

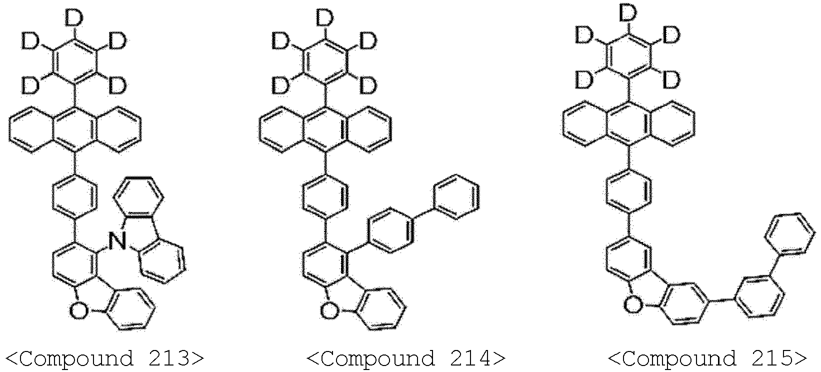

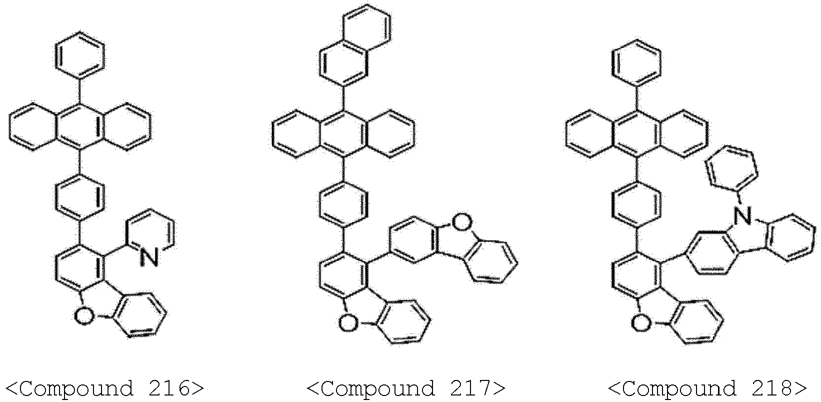

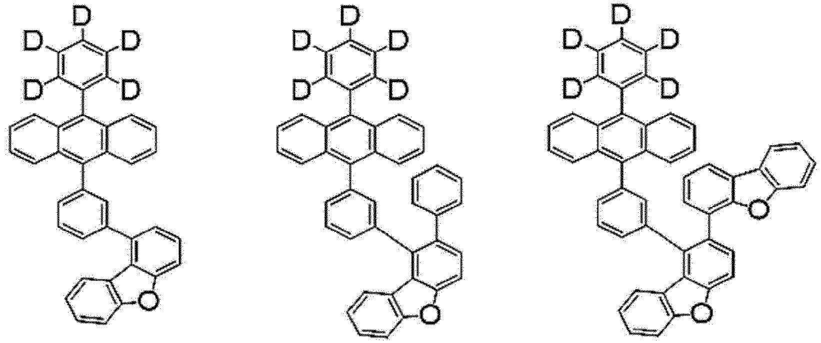

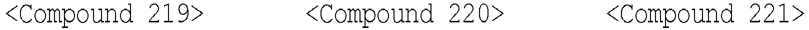

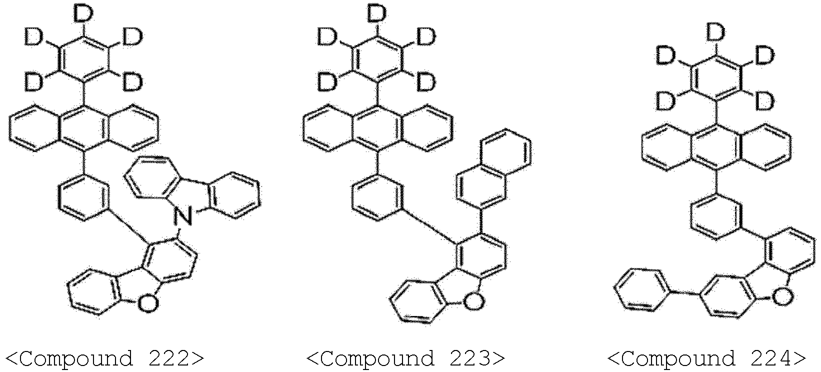

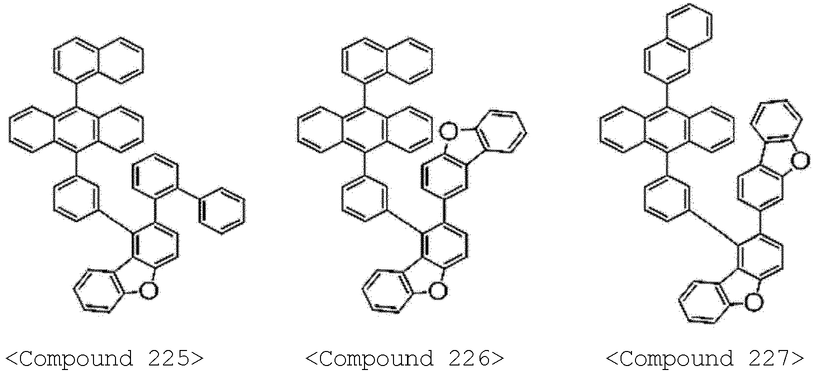

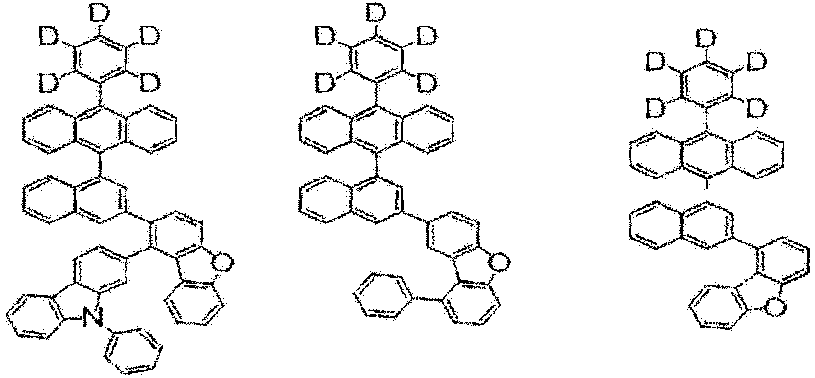

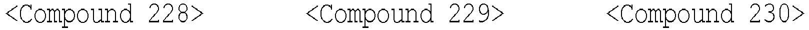

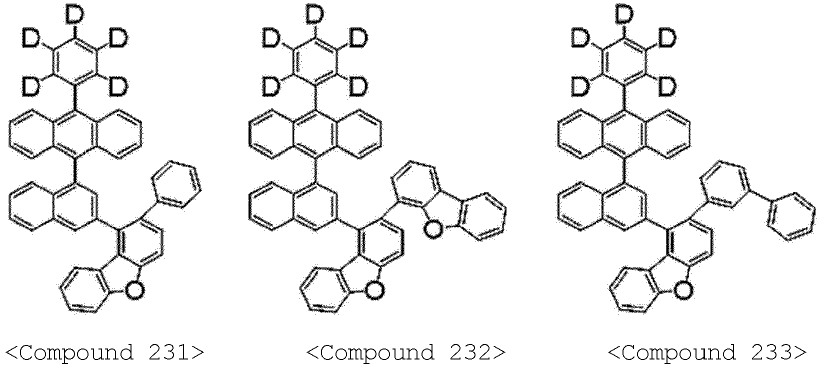

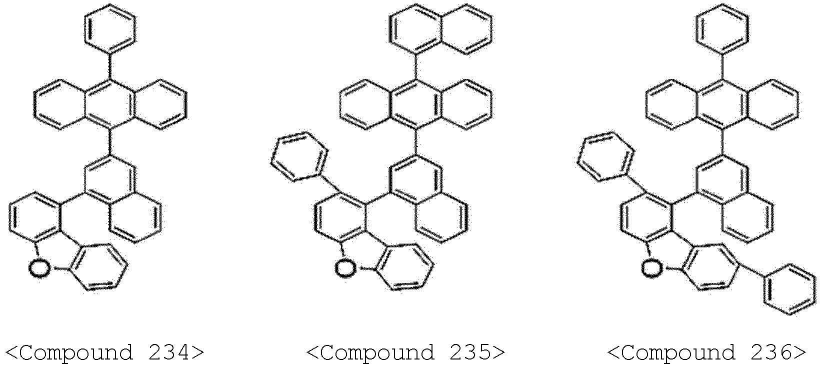

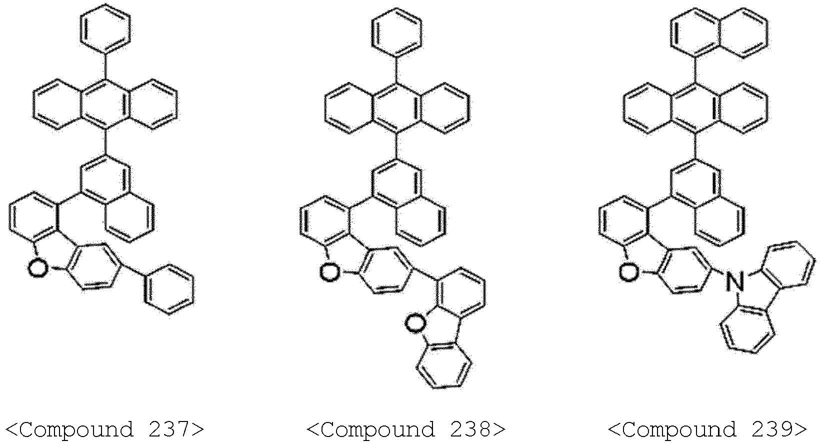

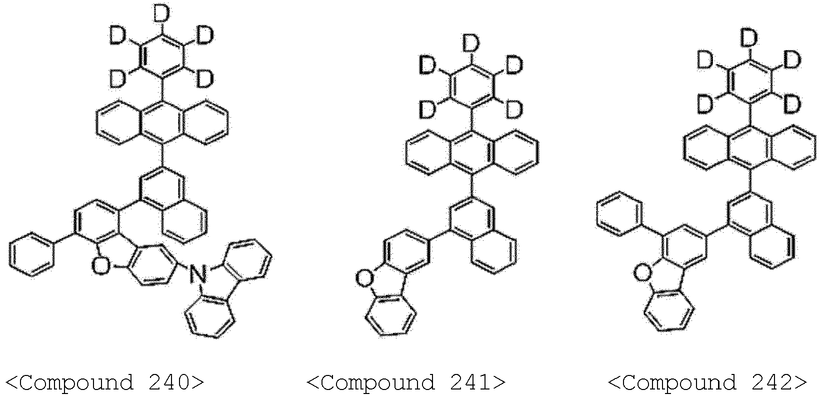

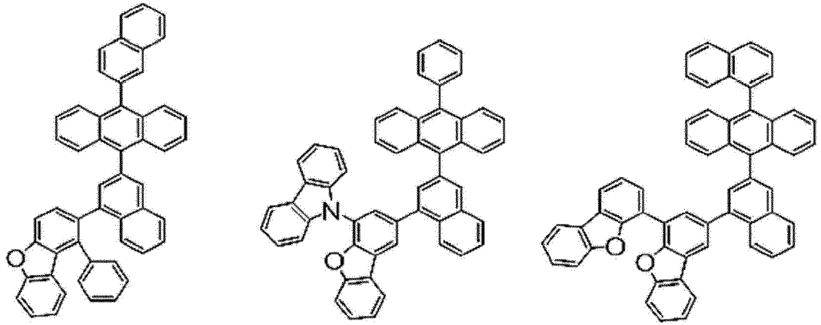

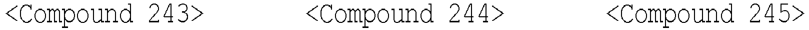

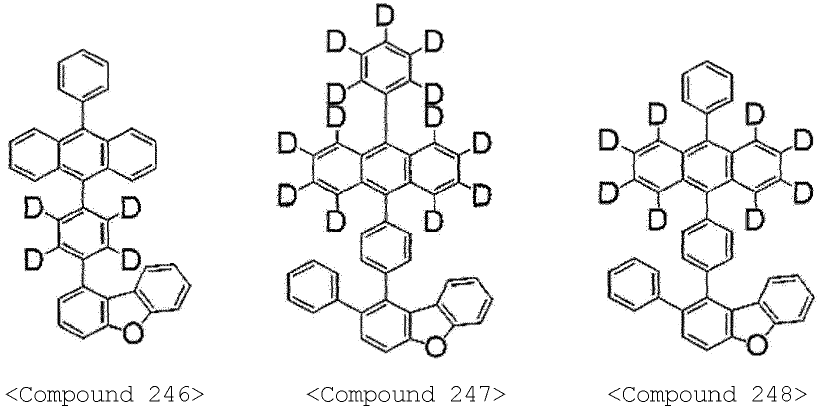

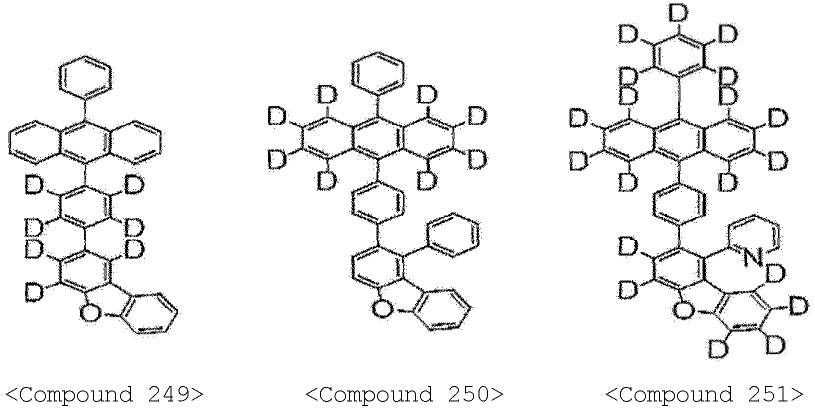

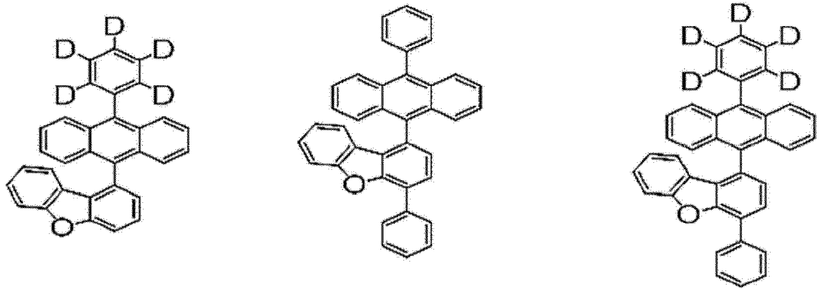

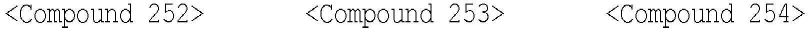

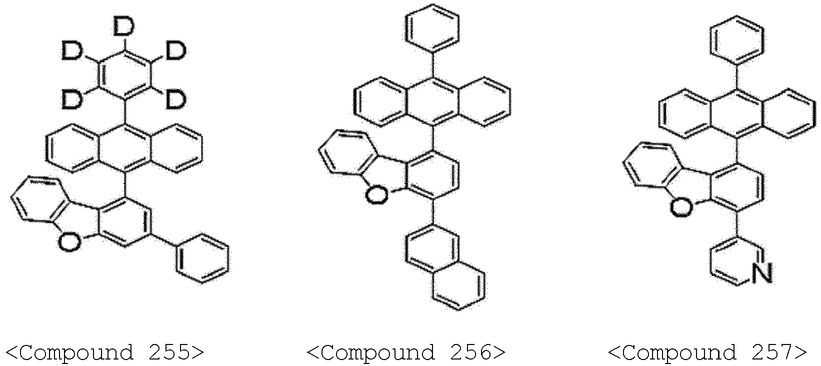

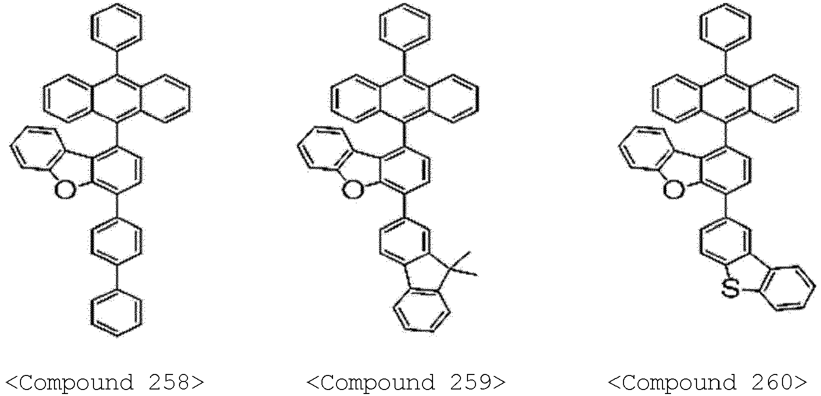

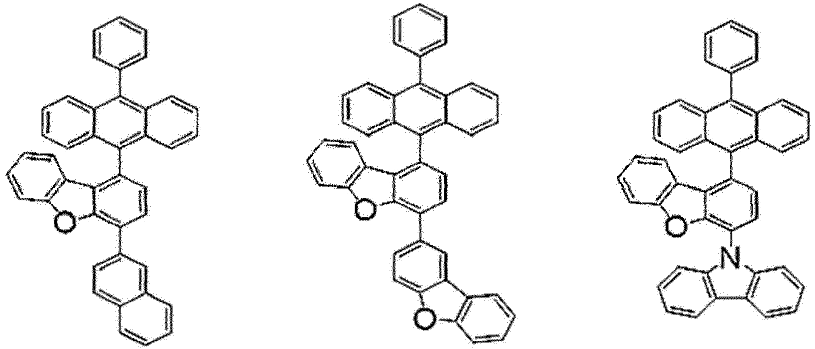

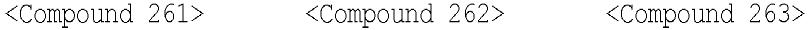

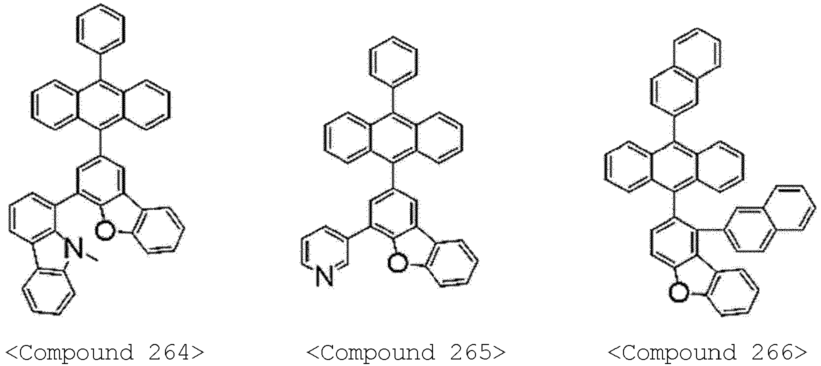

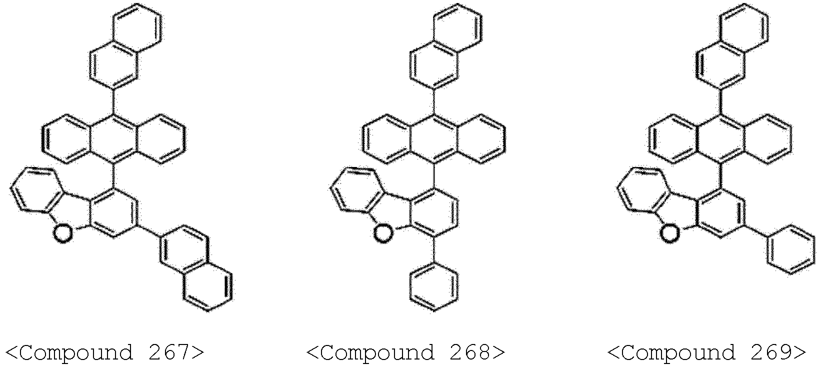

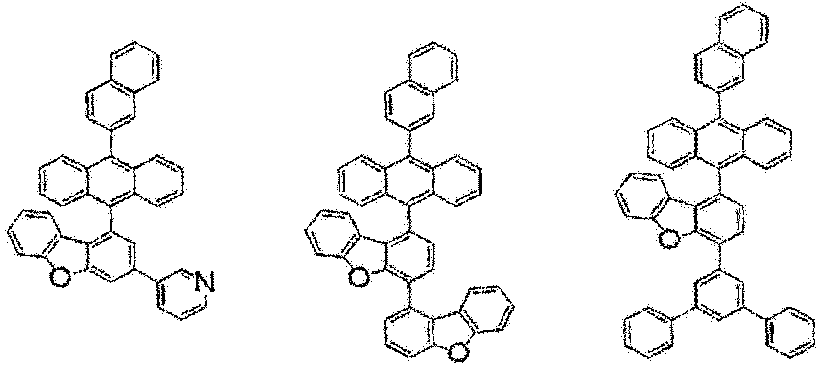

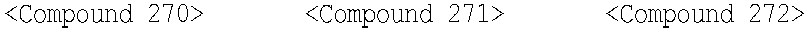

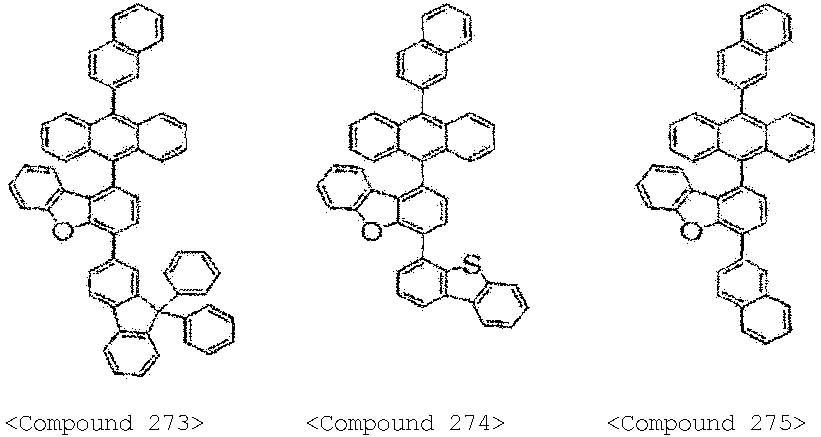

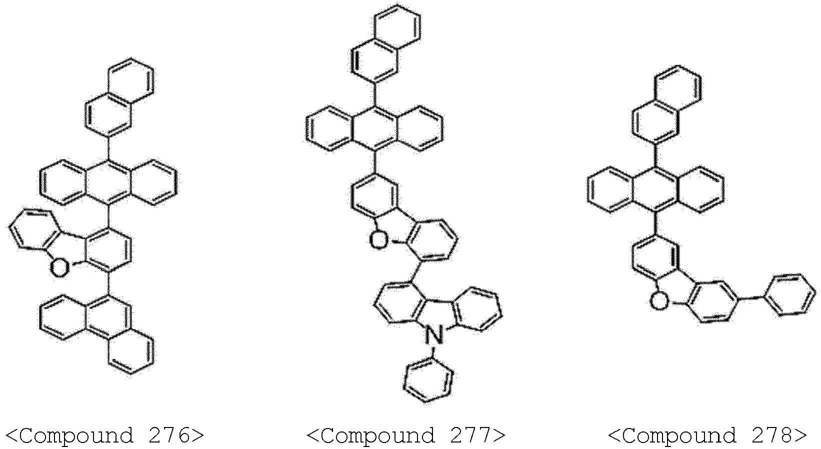

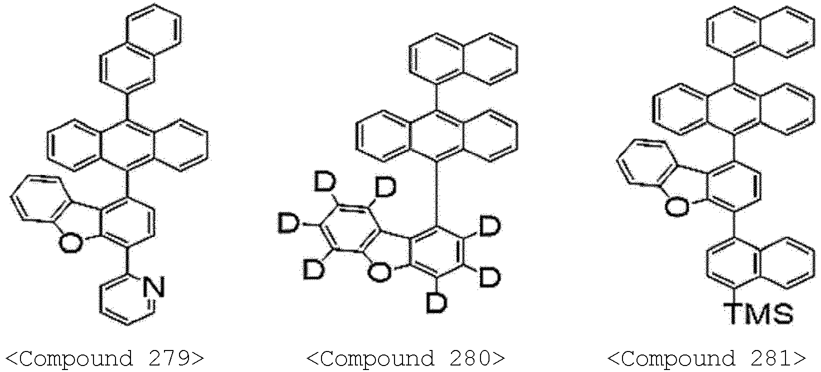

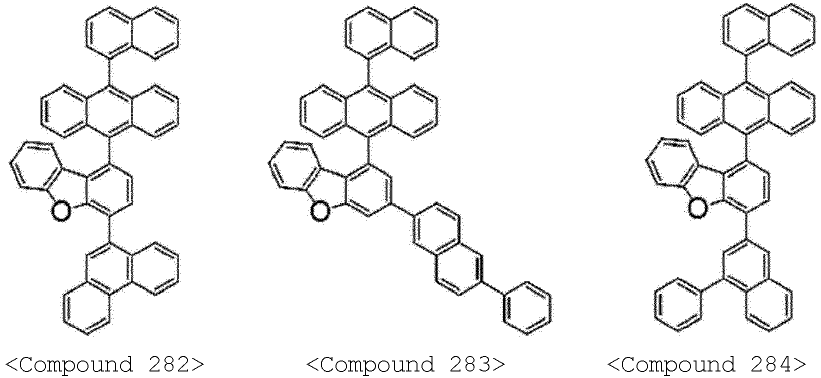

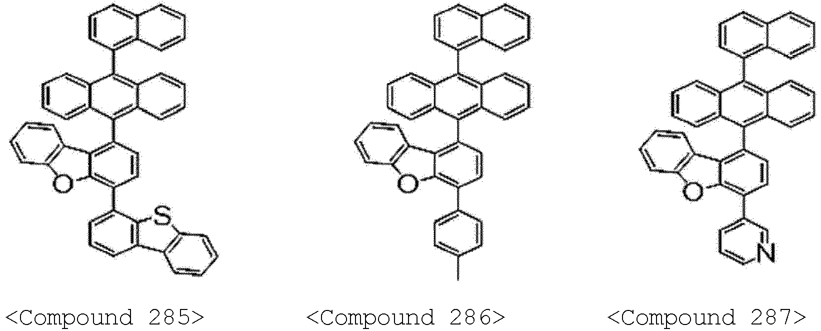

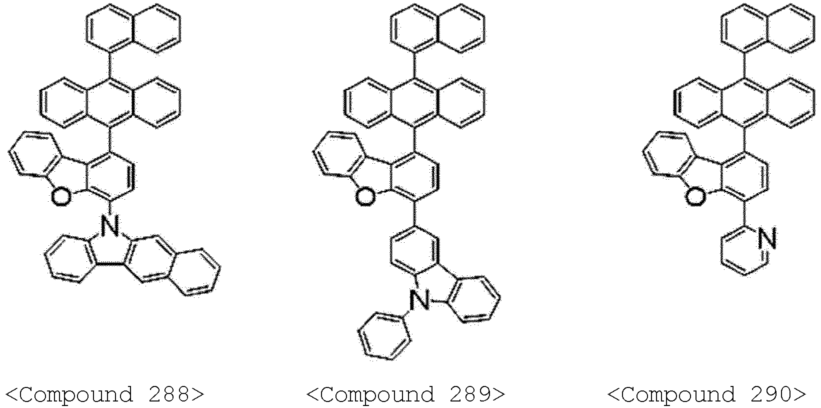

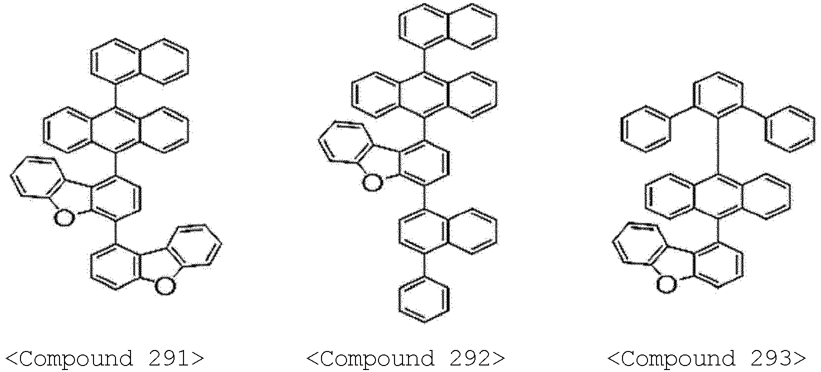

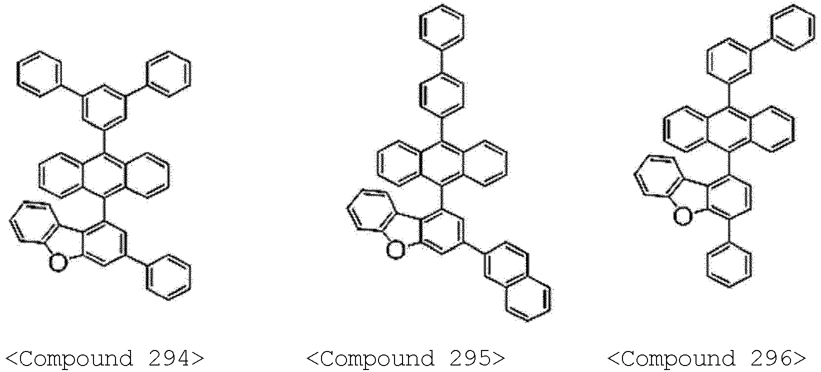

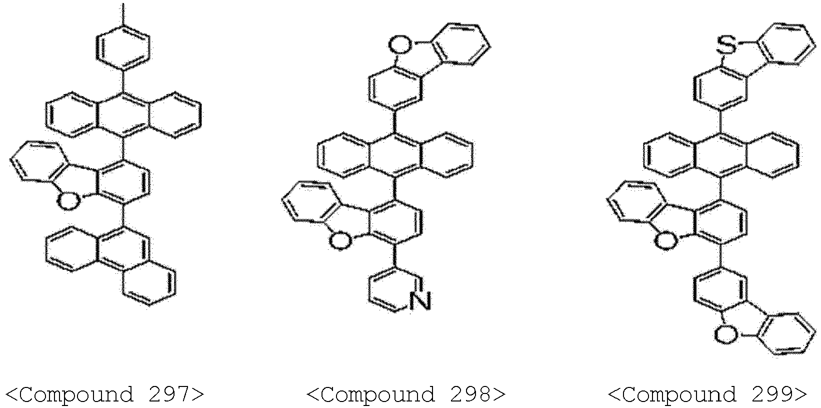

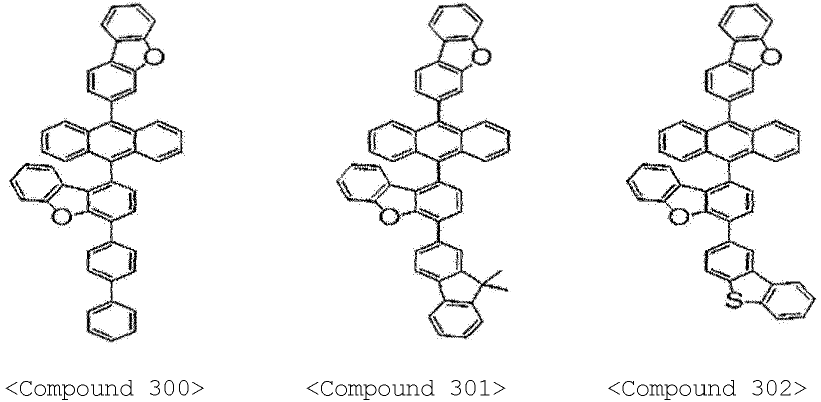

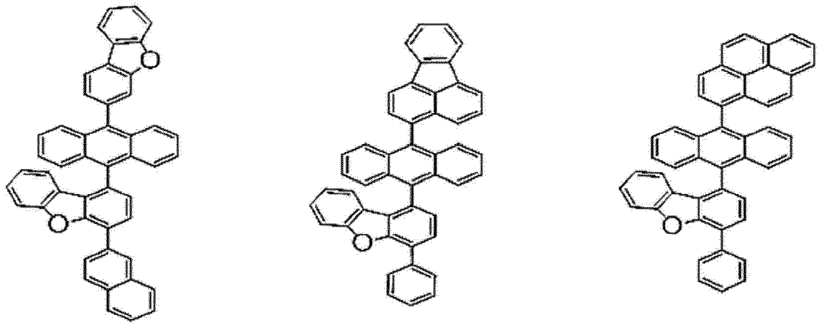

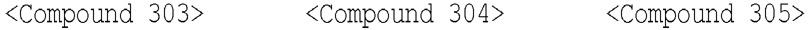

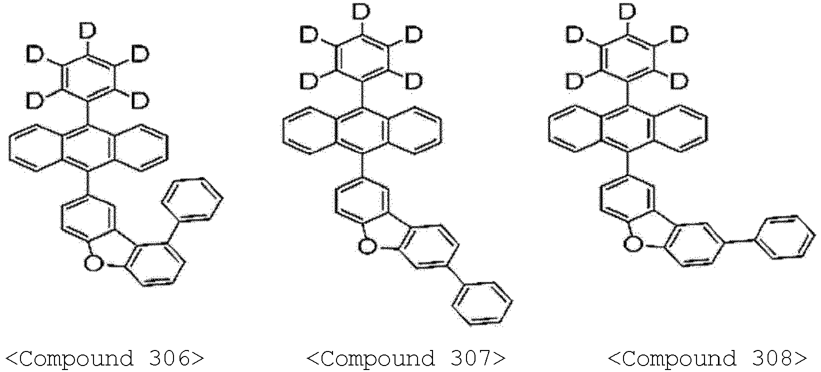

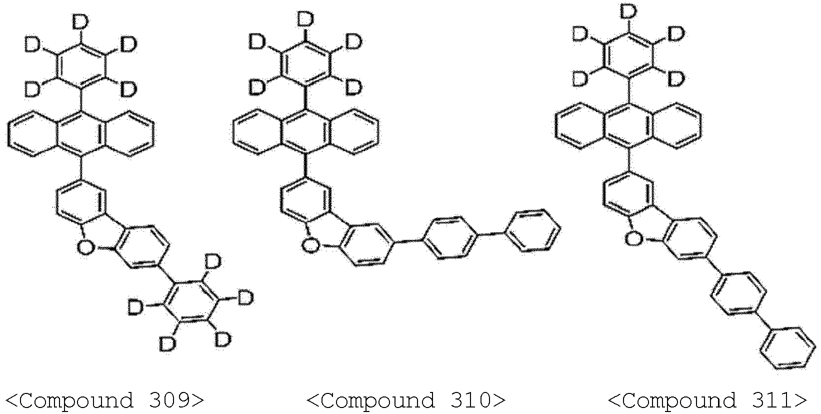

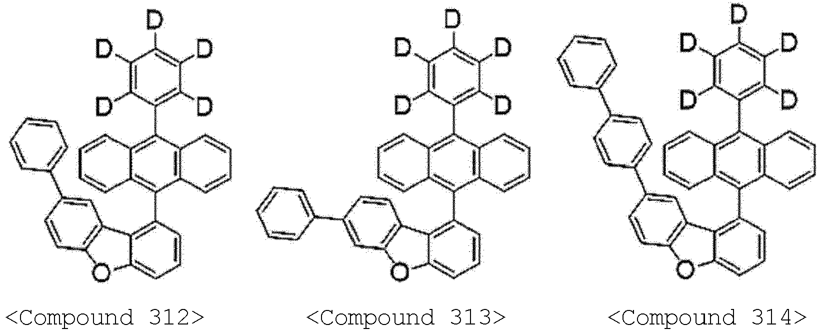

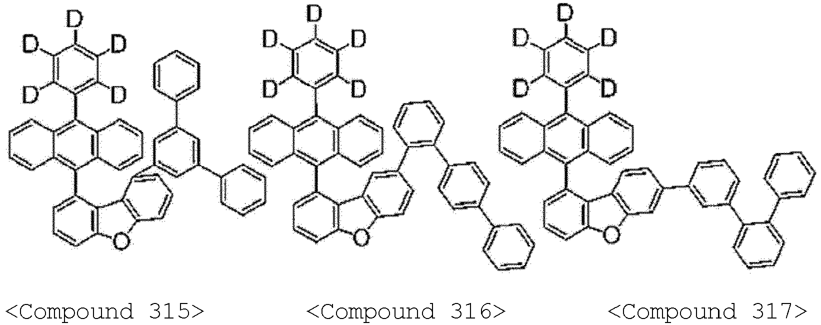

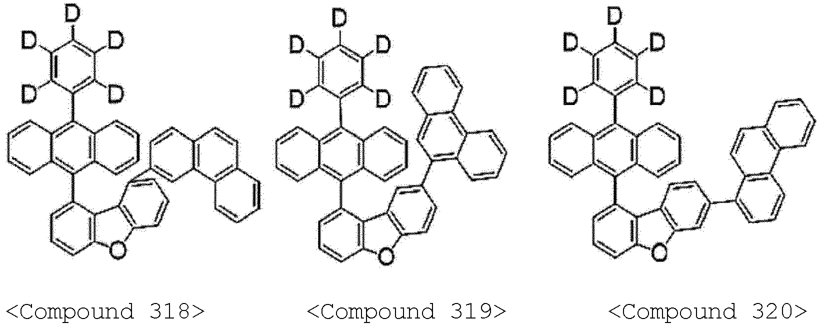

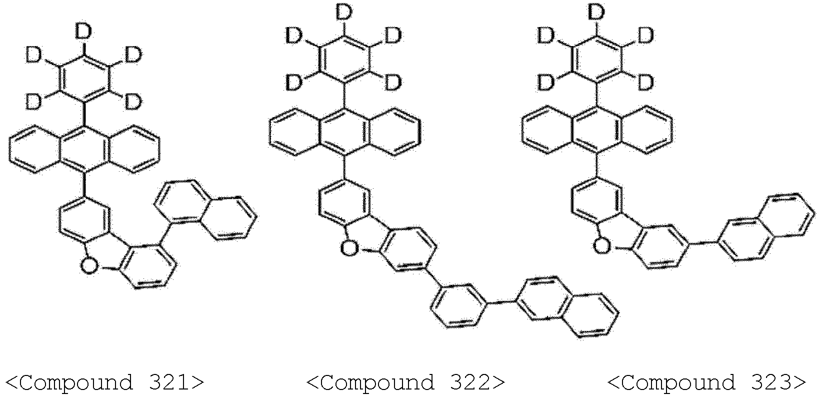

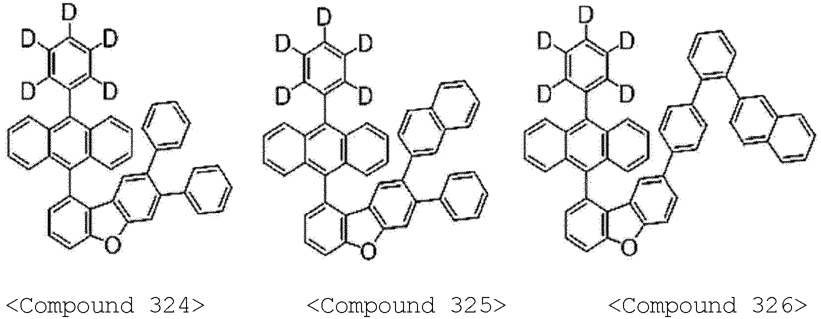

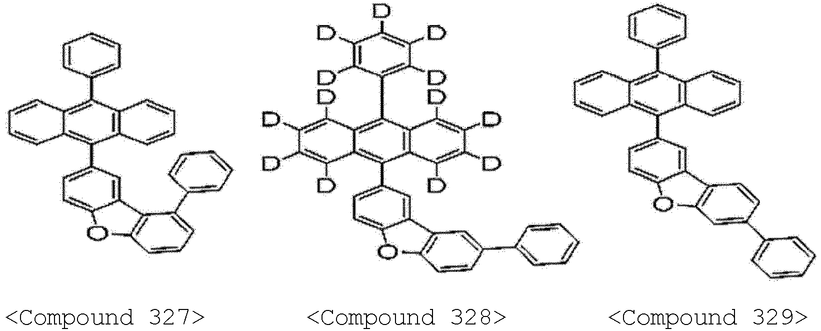

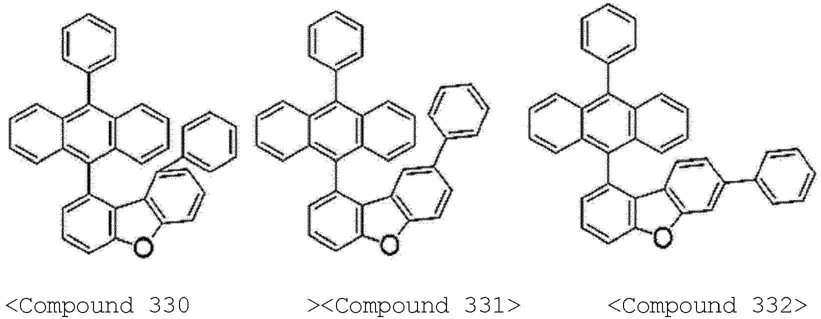

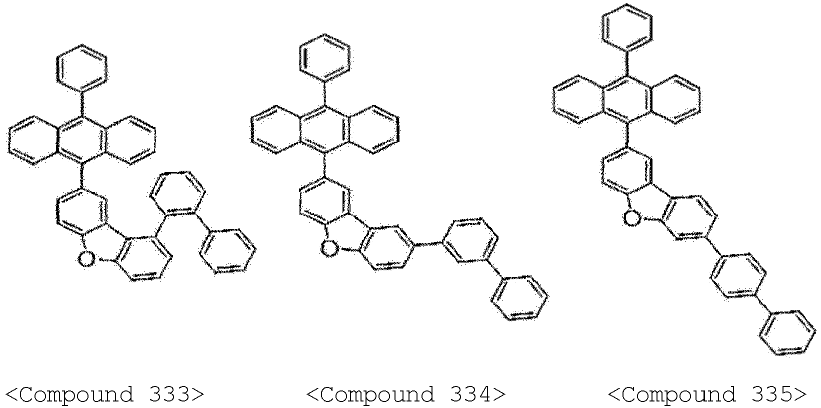

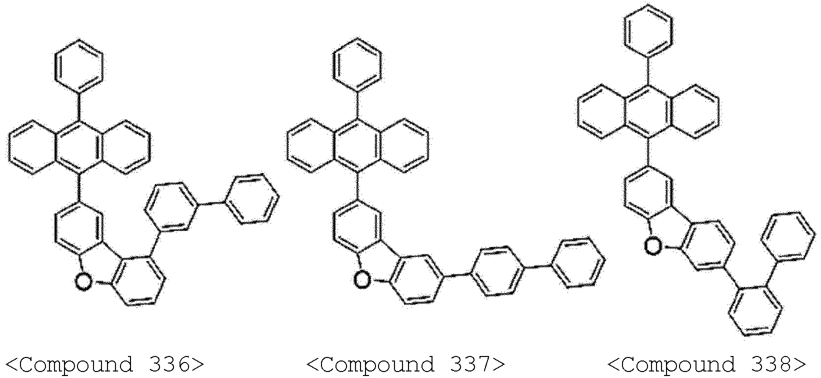

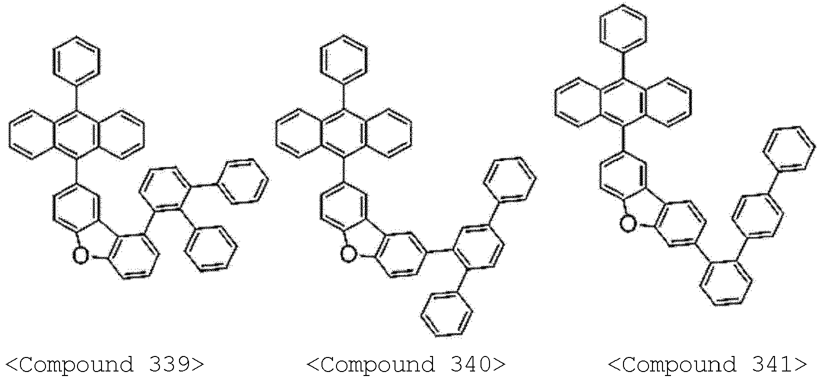

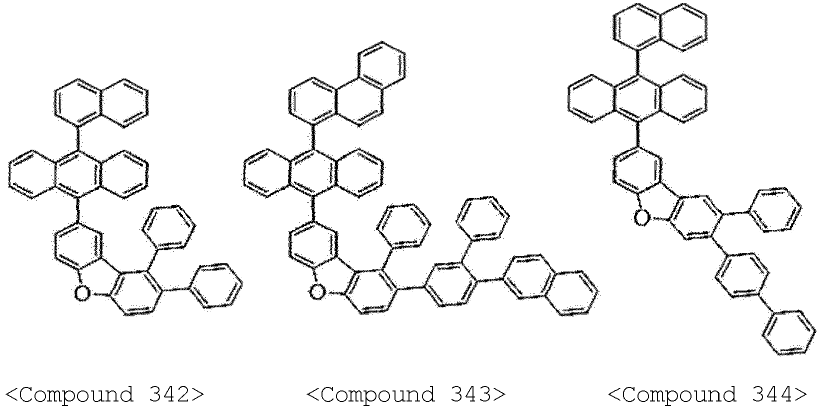

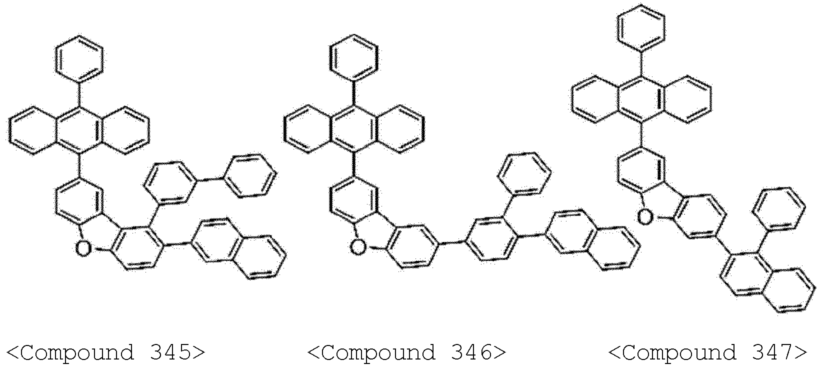

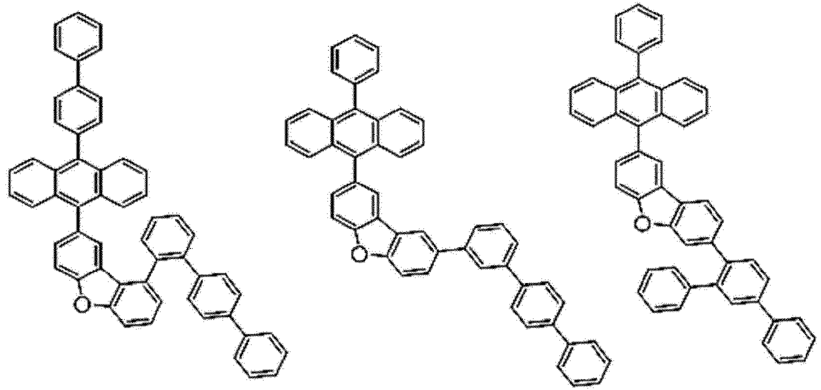

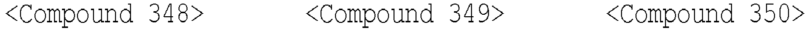

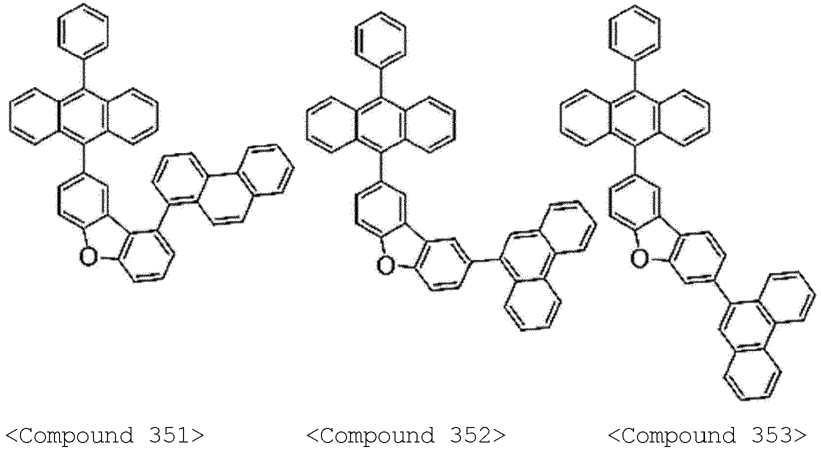

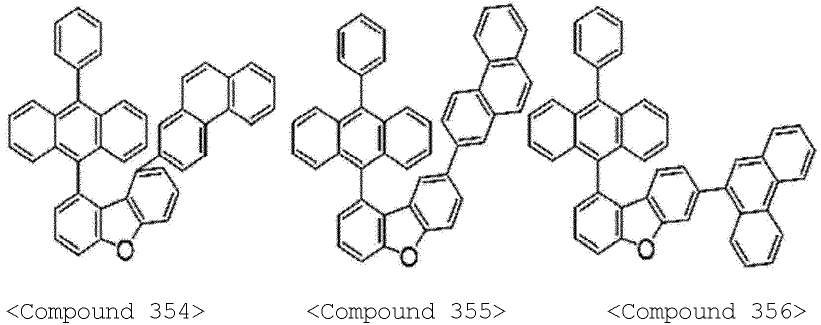

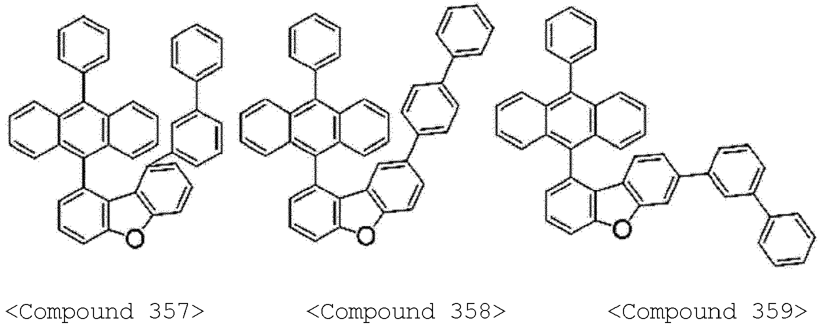

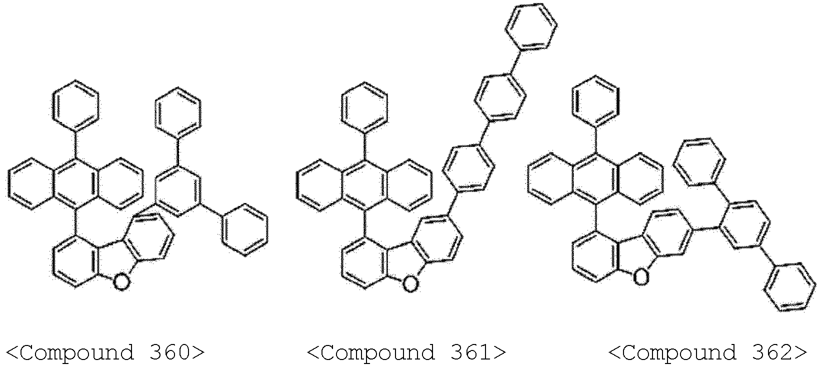

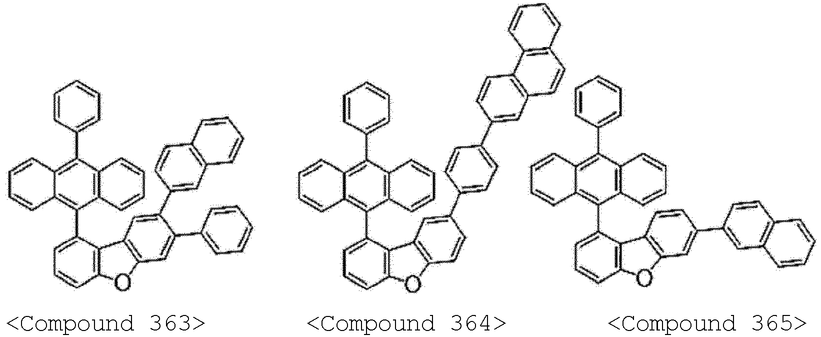

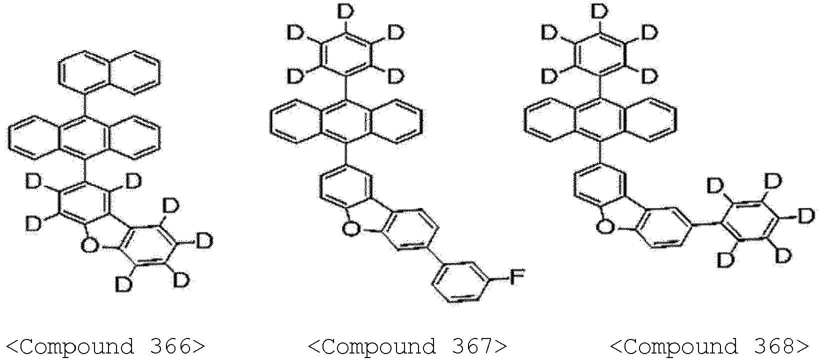

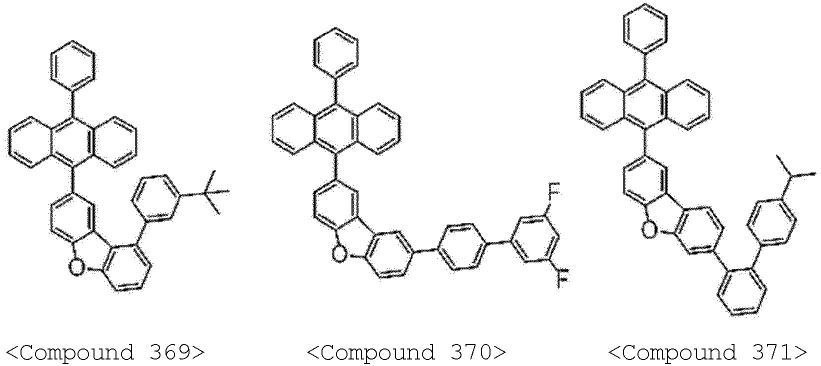

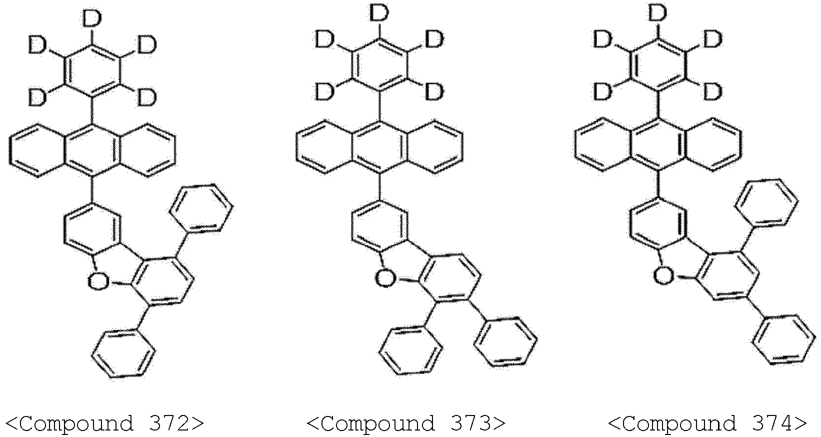

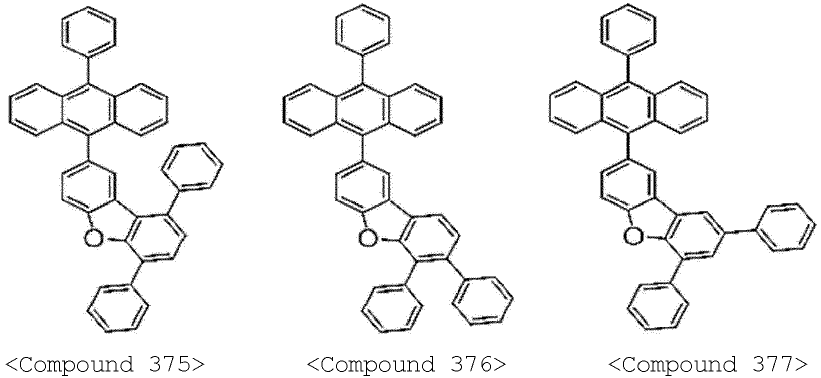

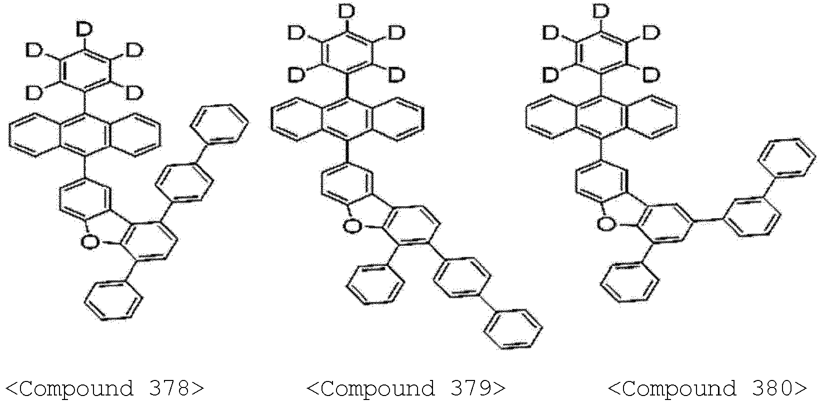

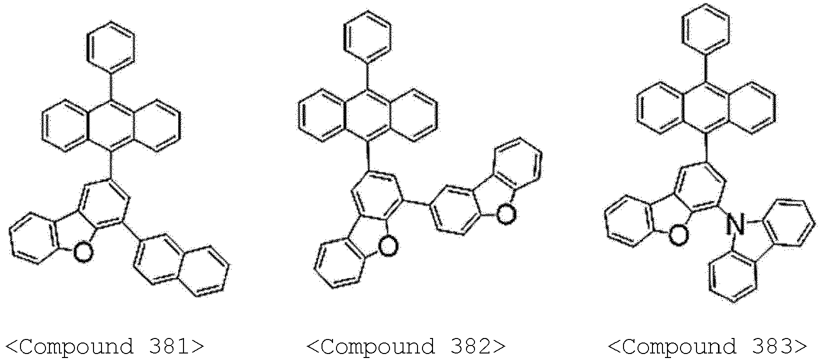

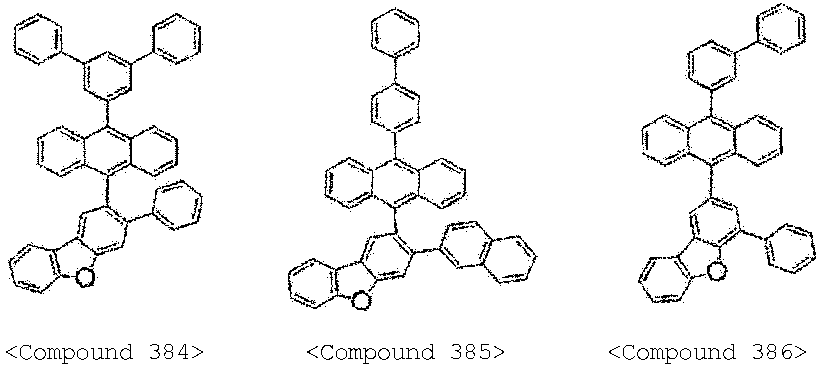

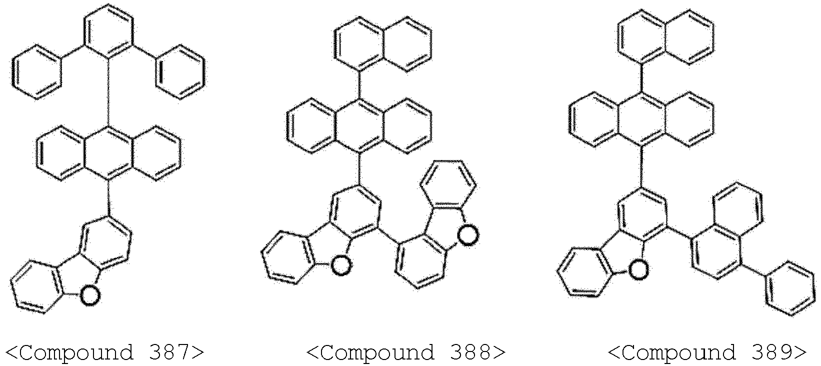

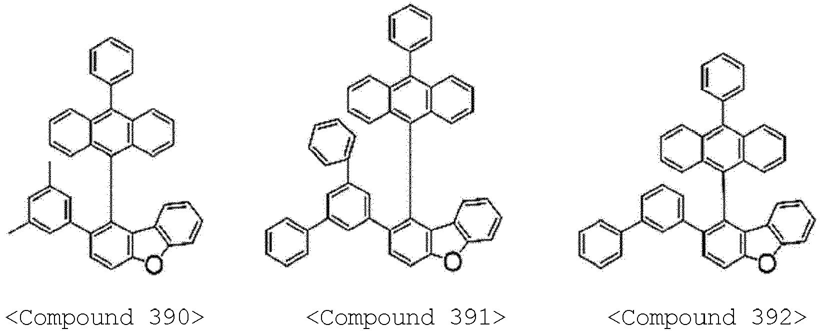

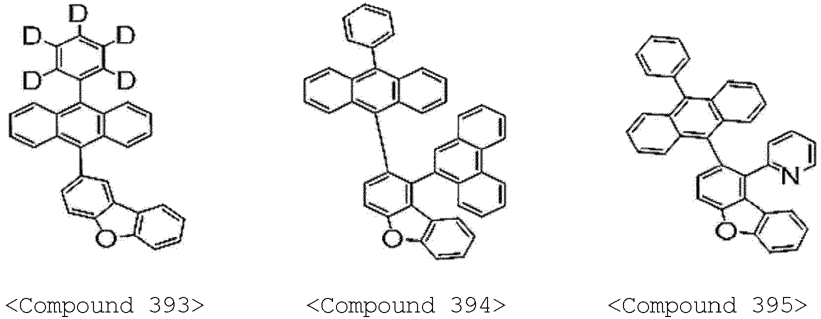

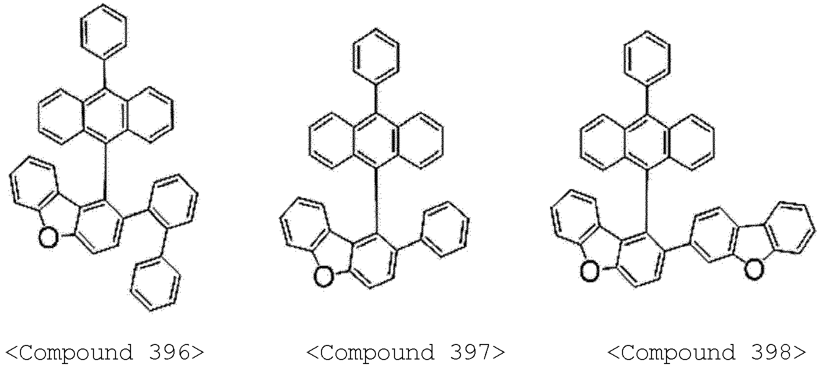

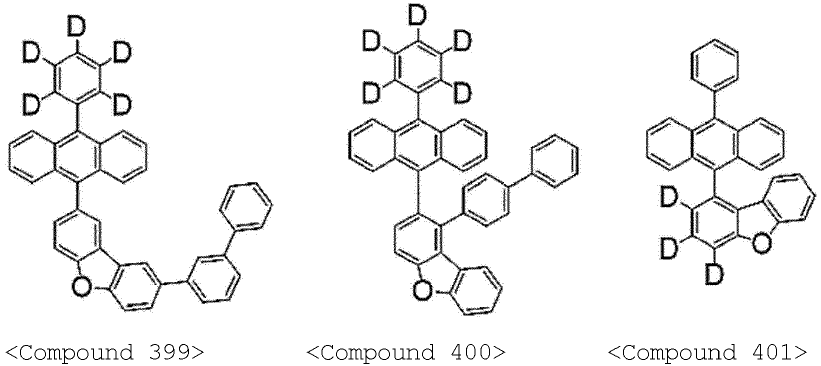

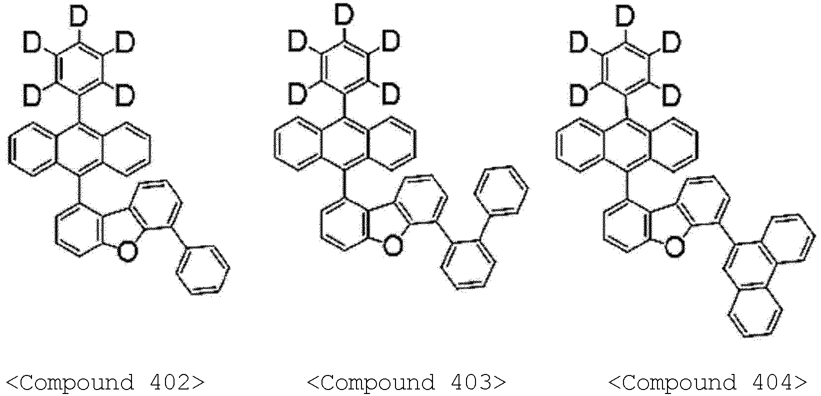

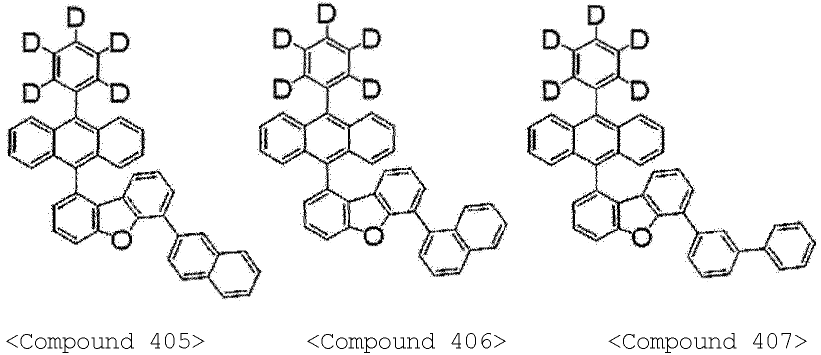

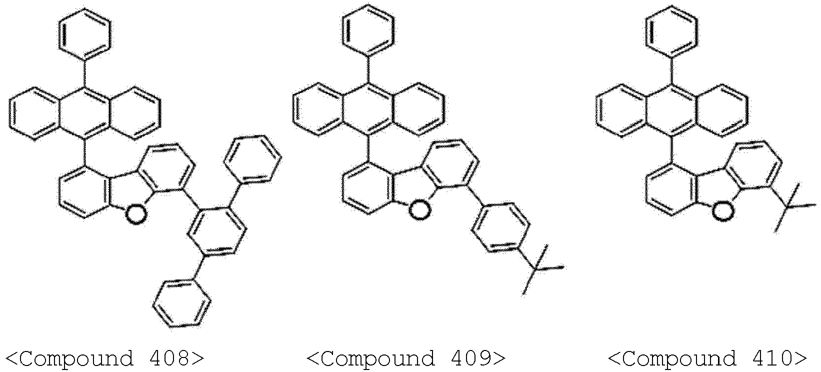

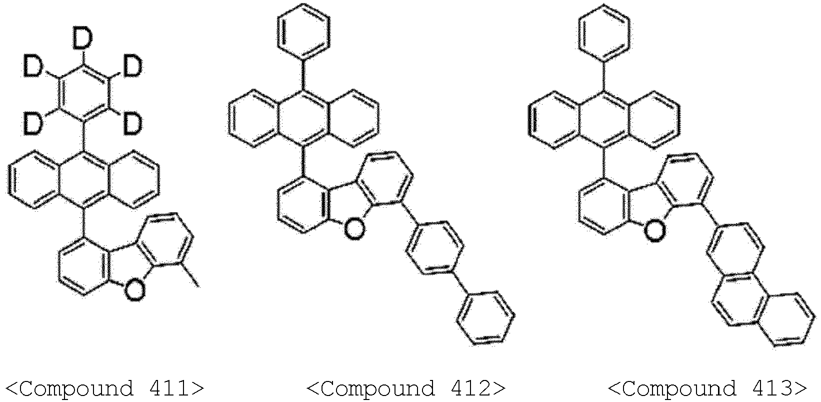

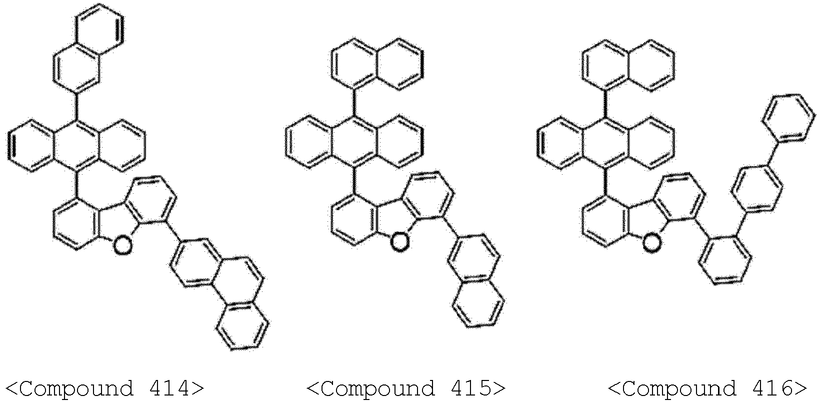

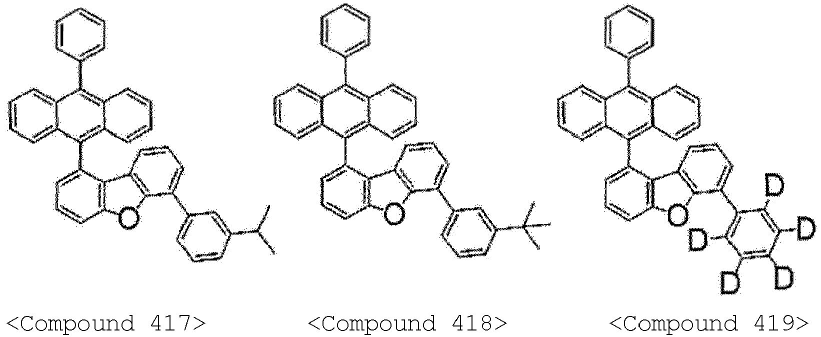

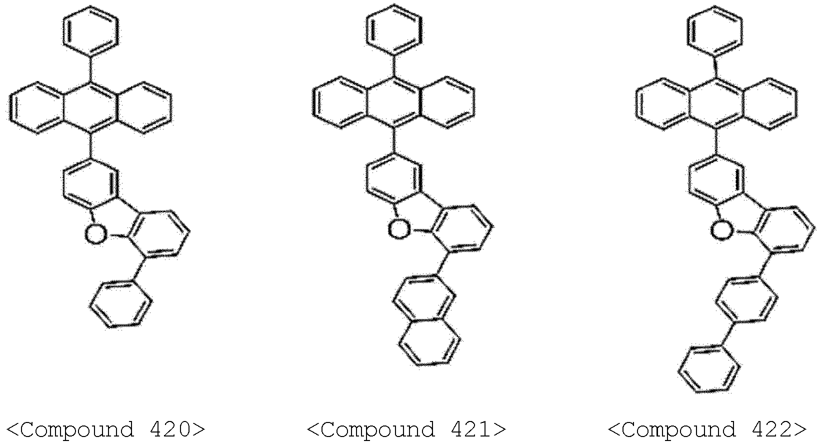

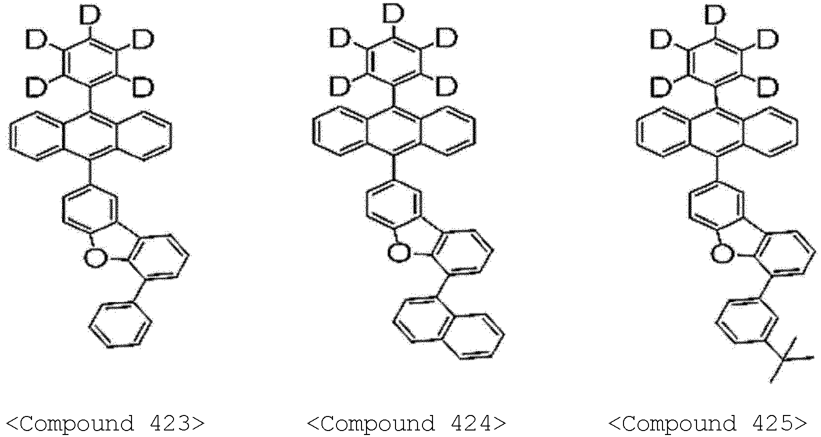

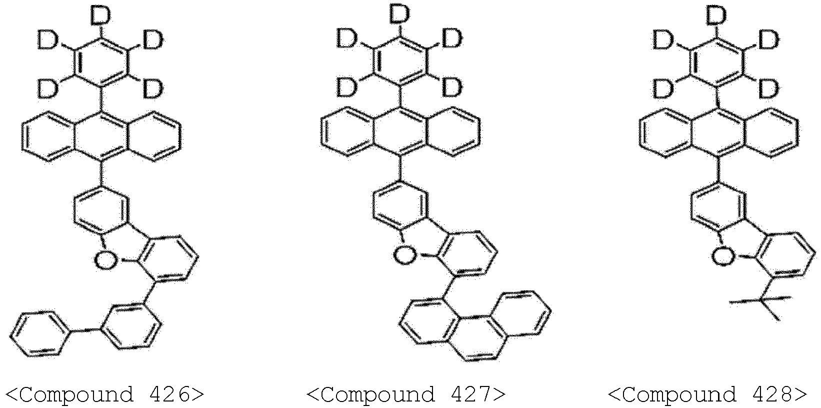

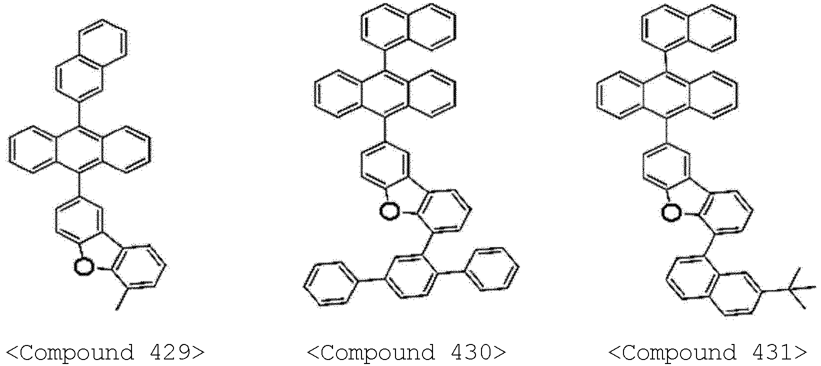

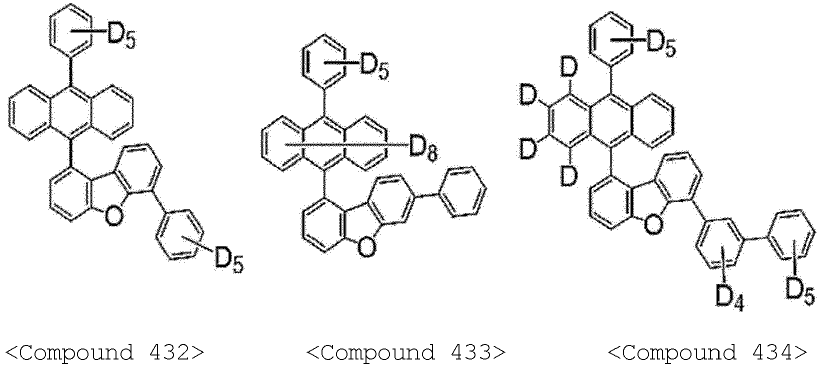

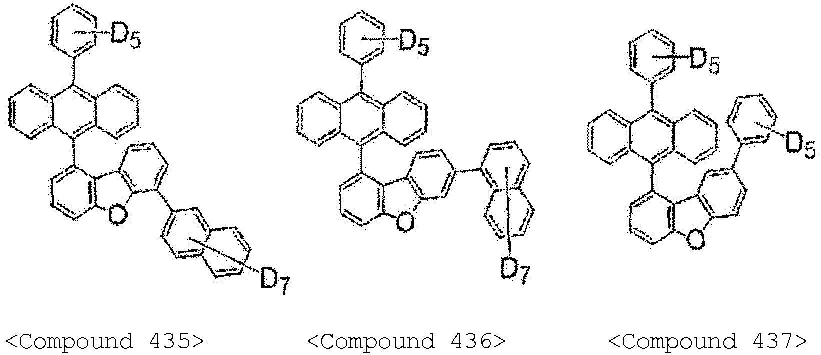

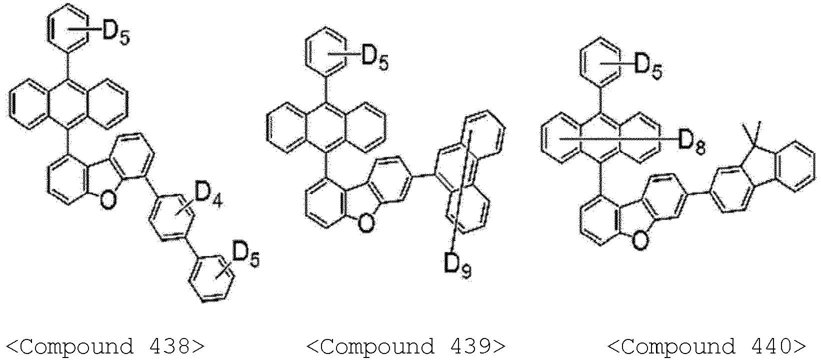

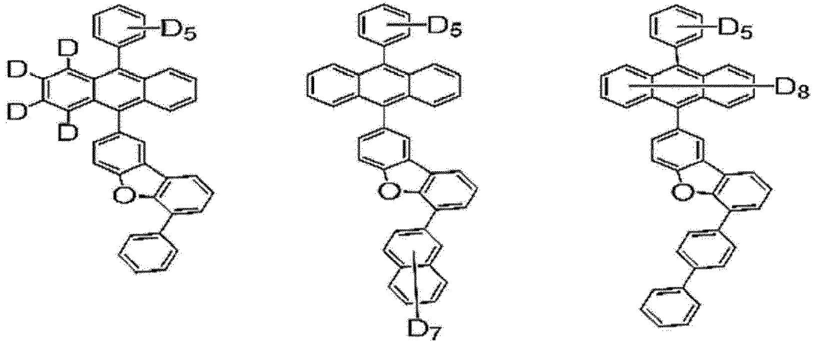

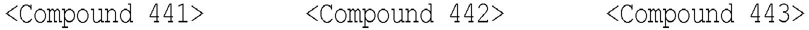

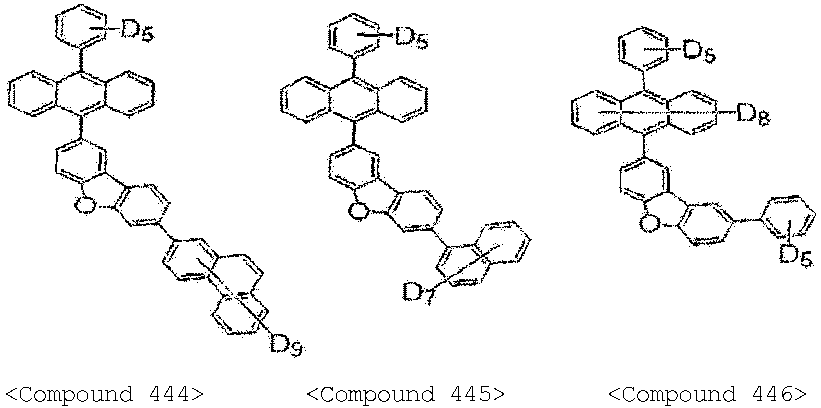

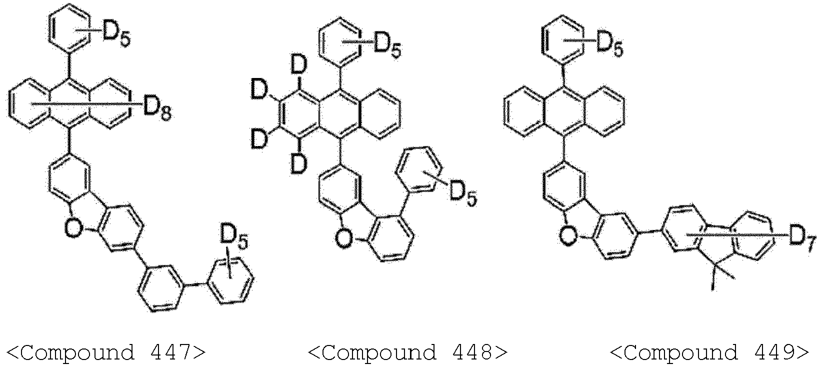

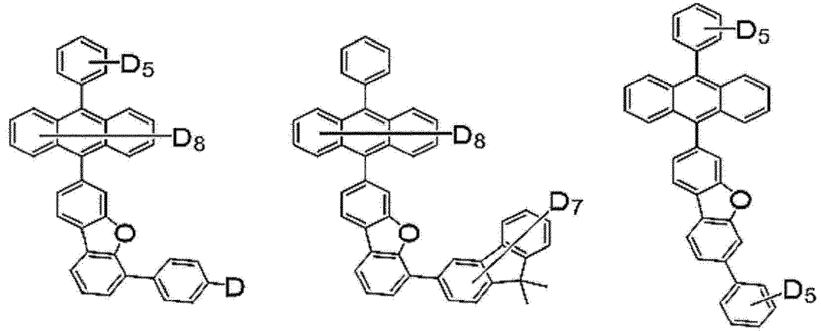

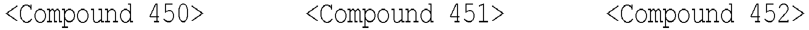

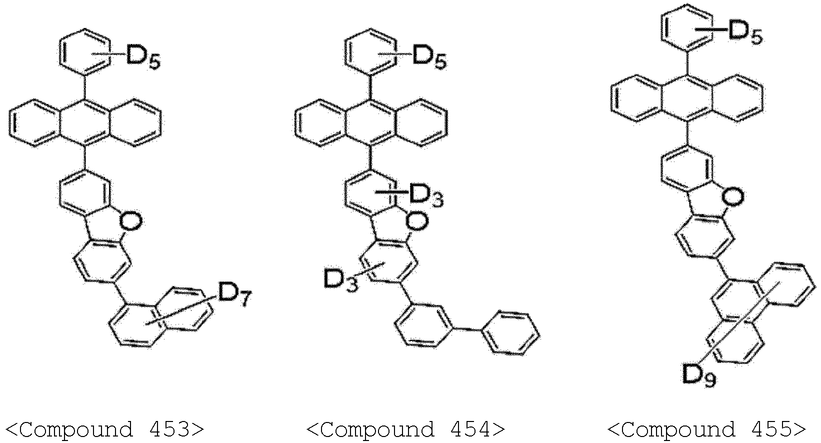

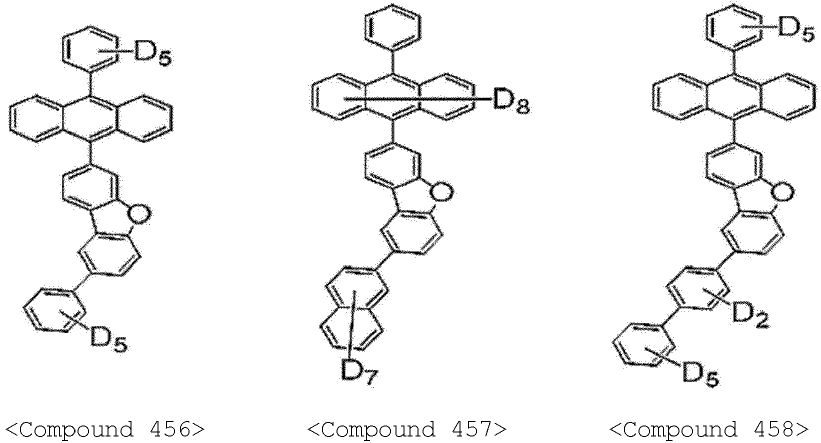

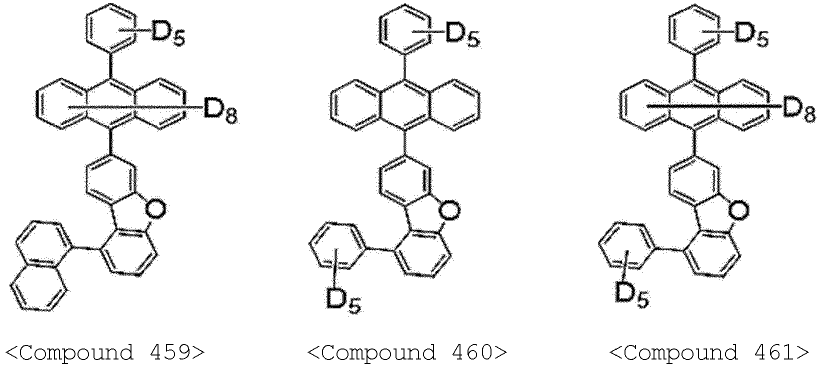

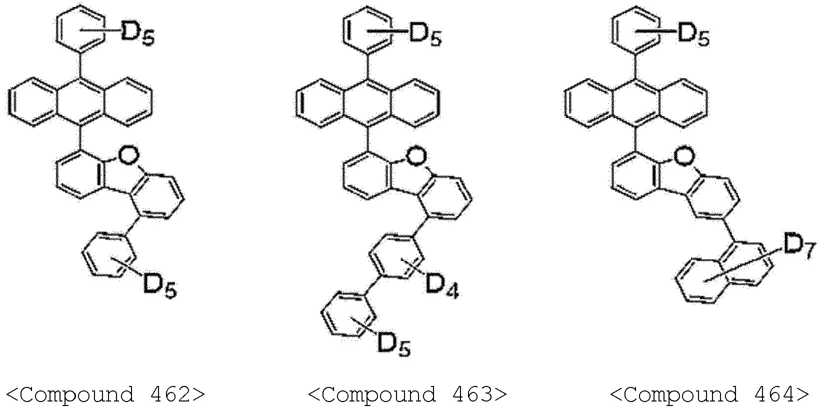

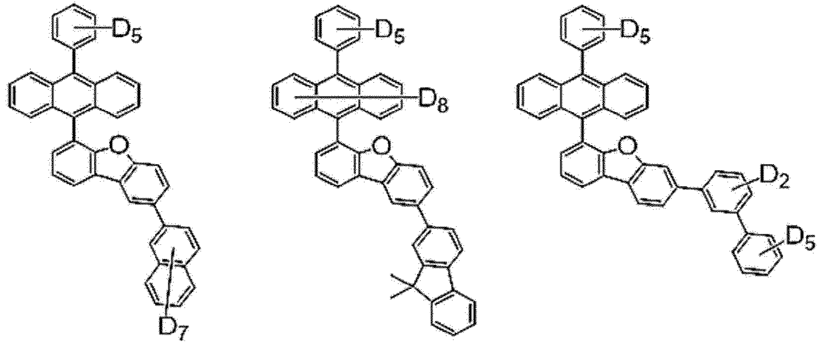

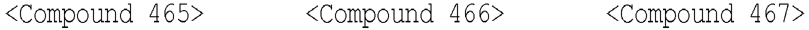

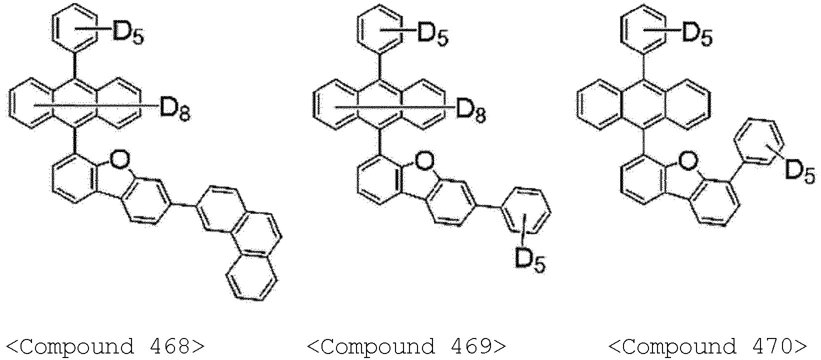

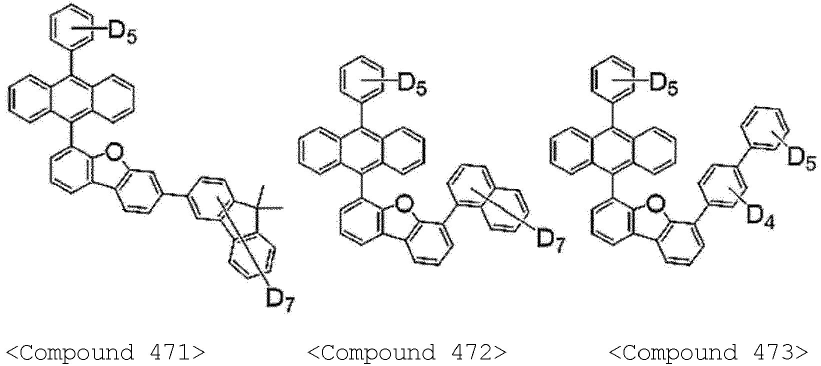

Below, the organic light-emitting diode of the present disclosure will be explained with reference to the drawing.

Figure is a schematic cross-sectional view of the structure of an organic light-emitting diode according to an embodiment of the present disclosure.

As shown in Figure, the organic light-emitting diode according to an embodiment of the present disclosure comprises: an anode (20); a first emission part including a hole transport layer (40), a first light-emitting layer (50-A) and second light-emitting layer (50-B), each containing a host and a dopant, an electron transport layer (60), and a cathode (80) in that order, wherein the anode and the cathode serve as a first electrode and a second electrode, respectively, with the interposition of the hole transport layer between the anode and the light-emitting layer, and the electron transport layer between the light-emitting layer and the cathode.

Furthermore, the organic light-emitting diode according to an embodiment of the present disclosure may comprise a hole injection layer (30) between the anode (20) and the hole transport layer (40), and an electron injection layer (70) between the electron transport layer (60) and the cathode (80) .

Reference is made to Figure with regard to the organic light emitting diode of the present disclosure and the fabrication method therefor.

First, a substrate (10) is coated with an anode electrode material to form an anode (20). So long as it is used in a typical organic electroluminescence (EL) device, any substrate may be used as the substrate (10). Preferable is an organic substrate or transparent plastic substrate that exhibits excellent transparency, surface smoothness, ease of handling, and waterproofness. As the anode electrode material, indium tin oxide (ITO), indium zinc oxide (IZO), tin oxide (SnO₂), or zinc oxide (ZnO), which are transparent and superior in terms of conductivity, may be used.

A hole injection layer material is applied on the anode (20) by thermal deposition in a vacuum or by spin coating to form a hole injection layer (30). Subsequently, thermal deposition in a vacuum or by spin coating may also be conducted to form a hole transport layer (40) with a hole transport layer material on the hole injection layer (30).

So long as it is typically used in the art, any material may be selected for the hole injection layer without particular limitations thereto. Examples include, but are not limited to, 2-TNATA [4,4',4"-tris(2-naphthylphenyl-phenylamino)-triphenylamine], NPD [N,N'-di(1-naphthyl)-N,N'-diphenylbenzidine)], TPD [N,N'-diphenyl-N,N'-bis(3-methylphenyl)-1,1'-biphenyl-4,4'-diamine], and DNTPD [N,N'-diphenyl-N,N'-bis-[4-(phenyl-m-tolyl-amino)-phenyl]-biphenyl-4,4'-diamine].

Any material that is typically used in the art may be selected for the hole transport layer without particular limitations thereto. Examples include, but are not limited to, N,N'-bis(3-methylphenyl)-N,N'-diphenyl-[1,1-biphenyl]-4,4'-diamine (TPD) and N,N'-di(naphthalen-1-yl)-N,N'-diphenylbenzidine (a-NPD).

In an embodiment of the present disclosure, an electron blocking layer may be additionally disposed on the hole transport layer. Functioning to prevent the electrons injected from the electron injection layer from entering the hole transport layer from the light-emitting layer, the electron blocking layer is adapted to increase the life span and luminous efficiency of the diode. The electron blocking layer may be formed at a suitable position between the light emitting layer and the hole injection layer. Particularly, the electron blocking layer may be formed between the light emitting layer and the hole transport layer.

Next, the first light-emitting layer (50-A) and the second light-emitting layer (50-B) may be sequentially deposited on the hole transport layer (40) or the electron blocking layer by deposition in a vacuum or by spin coating.

Herein, each of the first and the second light-emitting layer may contain a host and a dopant and the materials are as described above.

That is, the light-emitting layer includes a first light-emitting layer (50-A) and a second light-emitting layer (50-B) that may be formed by independent deposition or coating processes using the same or different host and dopant materials.

In a more particular embodiment, the first light-emitting layer may contain at least one of the compounds represented by Chemical Formula A as a fluorescent host and the second light-emitting layer may contain at least one of the anthracene derivatives represented by Chemical formula E as a fluorescent host while the same or different compounds selected from Chemical Formulas D1 to D10 may be used as respective fluorescent dopants in the first light-emitting layer and the second light-emitting layer.

In the present disclosure, the host materials available in the first light-emitting layer and the second light-emitting layer are different in lowest unoccupied molecular orbital (LUMO) and highest occupied molecule orbital (HOMO). That is, the host material (BH1) used in the first light-emitting layer is lower in LUMO and higher in HOMO than that (BH2) used in the second light-emitting layer, so that the host material (BH1) used in the first light-emitting layer is structured to allow for easier injection of holes and/or electrons than the host material (BH2) used in the second light-emitting layer.

In some embodiments of the present disclosure, each of the light-emitting layers particularly ranges in thickness from 50 to 2,000 Å.

In addition, as for the deposition thickness ratio between the first light-emitting layer (containing the compound represented by Chemical Formula A) and the second light-emitting layer (containing an anthracene represented by Chemical Formula E), the thickness of the first light-emitting layer accounts preferably for 10% to 70% and more preferably for 20% to 50% of the total thickness of the first and the second light-emitting layer.

Meanwhile, the electron transport layer (60) is applied on the light-emitting layer by deposition in a vacuum and spin coating.

A material for use in the electron transport layer functions to stably carry the electrons injected from the electron injection electrode (cathode), and may be an electron transport material known in the art. Examples of the electron transport material known in the art include quinoline derivatives, particularly, tris(8-quinolinolate)aluminum (Alq₃), Liq, TAZ, BAlq, beryllium bis(benzoquinolin-10-olate) (Bebq₂), Compound 201A, Compound 202A, BCP, and oxadiazole derivatives such as PBD, BMD, and BND, but are not limited thereto:

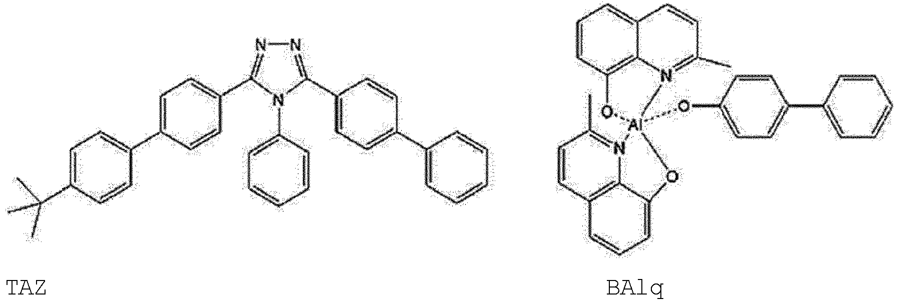

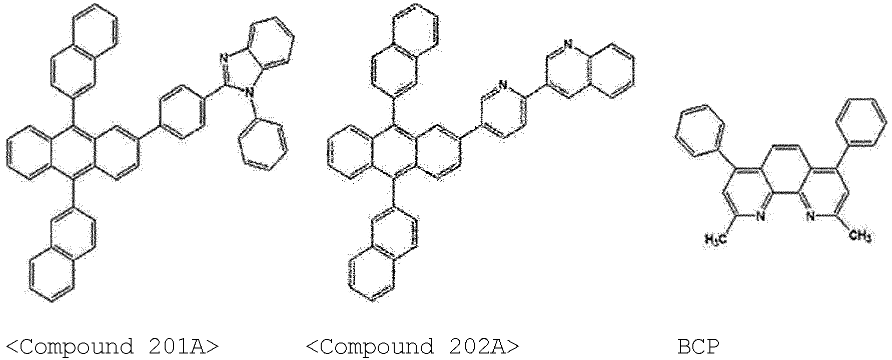

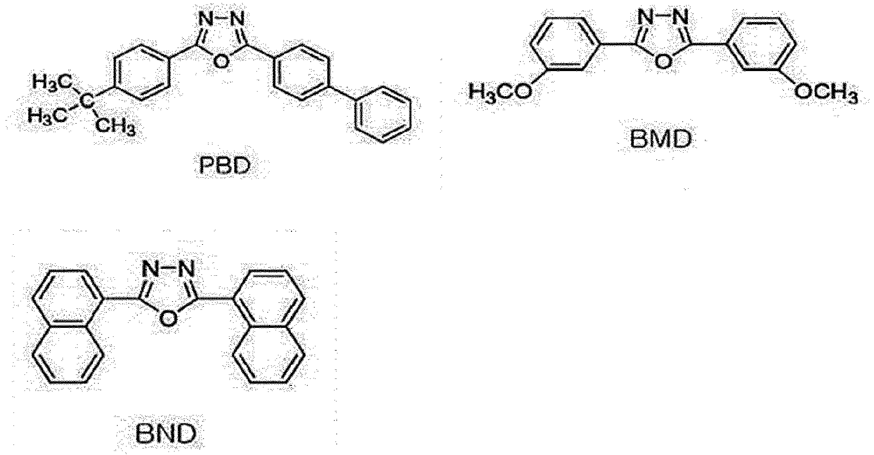

In the organic light emitting diode of the present disclosure, an electron injection layer (EIL) that functions to facilitate electron injection from the cathode may be deposited on the electron transport layer. The material for the EIL is not particularly limited.

Any material that is conventionally used in the art can be available for the electron injection layer without particular limitations. Examples include CsF, NaF, LiF, Li₂O, and BaO. Deposition conditions for the electron injection layer may vary, depending on compounds used, but may be generally selected from condition scopes that are almost the same as for the formation of hole injection layers.

The electron injection layer may range in thickness from about 1 Å to about 100 Å, and particularly from about 3 Å to about 90 Å. Given the thickness range for the electron injection layer, the diode can exhibit satisfactory electron injection properties without actually elevating a driving voltage.

In order to facilitate electron injection, the cathode may be made of a material having a small work function, such as metal or metal alloy such as lithium (Li), magnesium (Mg), calcium (Ca), an alloy aluminum (Al) thereof, aluminum-lithium (Al-Li), magnesium-indium (Mg-In), and magnesium-silver (Mg-Ag). Alternatively, ITO or IZO may be employed to form a transparent cathode for an organic light-emitting diode.

Moreover, the organic light-emitting diode of the present disclosure may further comprise a light-emitting layer containing a blue, green, or red luminescent material that emits radiations in the wavelength range of 380 nm to 800 nm. That is, the light-emitting layer in the present disclosure has a multi-layer structure wherein the blue, green, or red luminescent material may be a fluorescent material or a phosphorescent material.

Furthermore, at least one selected from among the layers may be deposited using a single-molecule deposition process or a solution process.

Here, the deposition process is a process by which a material is vaporized in a vacuum or at a low pressure and deposited to form a layer, and the solution process is a method in which a material is dissolved in a solvent and applied for the formation of a thin film by means of inkjet printing, roll-to-roll coating, screen printing, spray coating, dip coating, spin coating, etc.

Also, the organic light-emitting diode of the present disclosure may be applied to a device selected from among flat display devices, flexible display devices, monochrome or grayscale flat illumination devices, and monochrome or grayscale flexible illumination devices.

A better understanding of the present disclosure may be obtained through the following examples which are set forth to illustrate, but are not to be construed as limiting the present disclosure.

### (EXAMPLES)

### <Preparation of Host Compounds>

### SYNTHESIS EXAMPLE 1. Synthesis of Compound of Chemical Formula 3

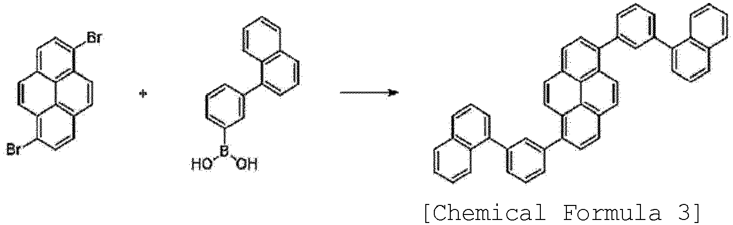

In a round-bottom flask purged with nitrogen, 1,6-dibromopyrene (10 g, 27.7 mmol), 3-(1-naphthyl)phenylboronic acid (14.5 g, 58.3 mmol), tetrakis triphenylphosphine palladium (Pd[PPh₃]₄) (1.3 g, 1.1 mmol), potassium carbonate (15.4 g, 111.1 mmol), toluene (50 ml), 1,4-dioxane (50 ml), and water (30 ml) were stirred together for 12 hours under reflux in a nitrogen condition. After completion of the reaction, the reaction mixture was cooled to room temperature and then subjected to extraction with ethyl acetate and water. The organic layer was concentrated in a vacuum and purification by column chromatography afforded [Chemical Formula 3] (9.7 g, yield 58%).
MS(MALDI-TOF) : m/z 606.23[M]⁺

### SYNTHESIS EXAMPLE 2. Synthesis of Compound of Chemical Formula 4

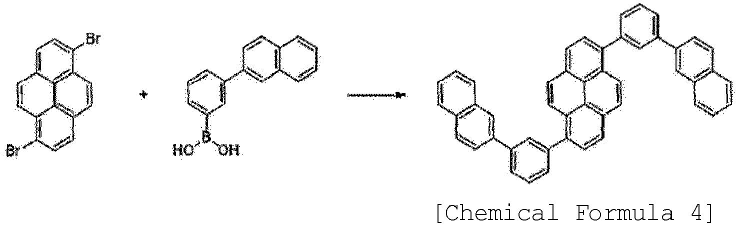

The same procedure as in Synthesis Example 1 was carried out, with the exception of using 3-(2-naphthyl)phenylboronic acid instead of 3-(1-naphthyl)phenylboronic acid, to afford [Chemical Formula 4] (yield 55%).
MS(MALDI-TOF) : m/z 606.23[M]⁺

### SYNTHESIS EXAMPLE 3. Synthesis of Compound of Chemical Formula 9

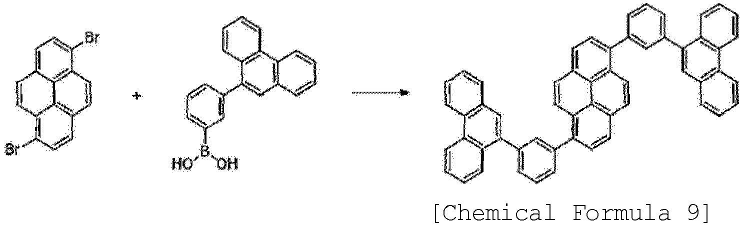

The same procedure as in Synthesis Example 1 was carried out, with the exception of using 3-(phenanthren-9-yl)phenylboronic acid instead of 3-(1-naphthyl)phenylboronic acid, to afford [Chemical Formula 9] (yield 54%).
MS(MALDI-TOF) : m/z 706.27 [M]⁺

### SYNTHESIS EXAMPLE 4. Synthesis of Compound of Chemical Formula 26

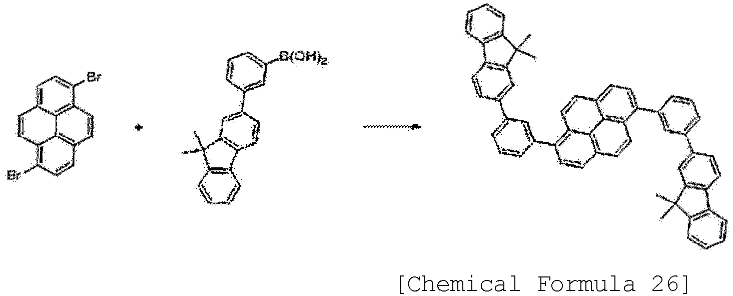

The same procedure as in Synthesis Example 1 was carried out, with the exception of using [3-(9,9-dimethyl-9H-fluoren-2-yl)phenylboronic acid instead of 3-(1-naphthyl)phenylboronic acid, to afford [Chemical Formula 26] (yield 55%).
MS(MALDI-TOF) : m/z 738.33 [M] ⁺

### SYNTHESIS EXAMPLE 5. Synthesis of Compound of Chemical Formula 62

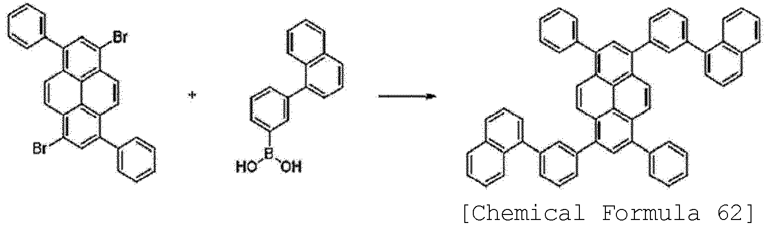

The same procedure as in Synthesis Example 1 was carried out, with the exception of using 1,6-dibromo-3,8-diphenylpyrene instead of 1,6-dibromopyrene, to afford [Chemical Formula 62] (yield 58%).
MS(MALDI-TOF) : m/z 758.30 [M]⁺

### SYNTHESIS EXAMPLE 6. Synthesis of Compound of Chemical Formula 1

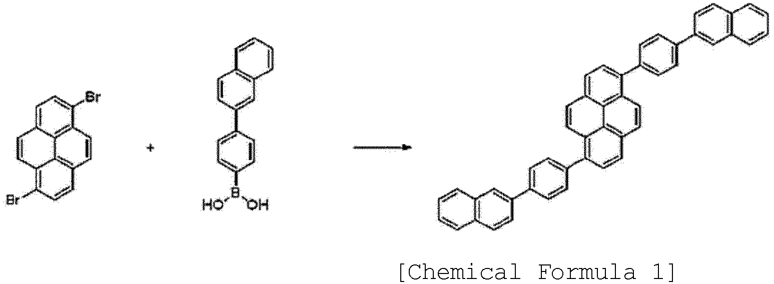

The same procedure as in Synthesis Example 1 was carried out, with the exception of using 4-(2-naphthyl)phenylboronic acid instead of 3-(1-naphthyl)phenylboronic acid, to afford [Chemical Formula 1] (yield 55%).
MS(MALDI-TOF) : m/z 606.23 [M]⁺

### SYNTHESIS EXAMPLE 7. Synthesis of Compound of Chemical Formula 6

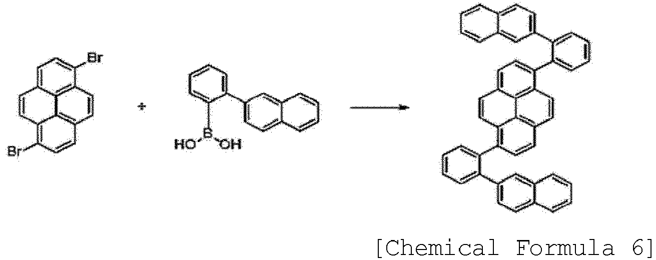

The same procedure as in Synthesis Example 1 was carried out, with the exception of using 2-(naphthalen-2-yl)phenylboronic acid instead of 3-(1-naphthyl)phenylboronic acid, to afford [Chemical Formula 6] (yield 58%).
MS(MALDI-TOF) : m/z 606.23 [M] ⁺

### <Preparation of Dopant Compounds>

### SYNTHESIS EXAMPLE 8. Synthesis of D 202

### Synthesis Example 8-1. Synthesis of <8-a>

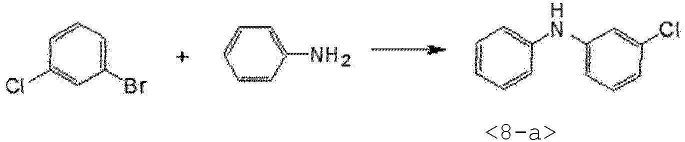

In a 100-mL reactor, 1-bromo-3-chlorobenzene (3.1 g, 16 mmol), aniline (5.8 g, 16 mmol), palladium acetate (0.1 g, 1 mmol), sodium tert-butoxide (3 g, 32 mmol), bis(diphenylphosphino)-1,1'-binaphthyl (0.2 g, 1 mmol), and toluene (45 mL) were stirred together under reflux for 24 hours. After completion of the reaction, filtration was conducted and the filtrate was concentrated and purified by column chromatography to afford <8-a> (5.2 g, yield 82%).

### Synthesis Example 8-2. Synthesis of <8-b>

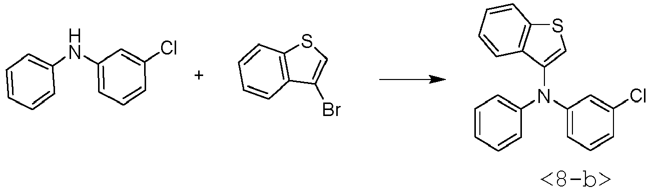

In a 250-mL reactor, <8-a> (20 g, 98 mmol), 3-bromobenzothiophene (20.9 g, 98 mmol), palladium acetate (0.5 g, 2 mmol), sodium tert-butoxide (18.9 g, 196 mmol), tri-tert-butyl phosphine (0.8 g, 4 mmol), and toluene (200 mL) were stirred together under reflux for 5 hours. After completion of the reaction, filtration was conducted and the filtrate was concentrated and purified by column chromatography to afford <8-b> (24.7 g, yield 75%).

### Synthesis Example 8-3. Synthesis of <8-c>

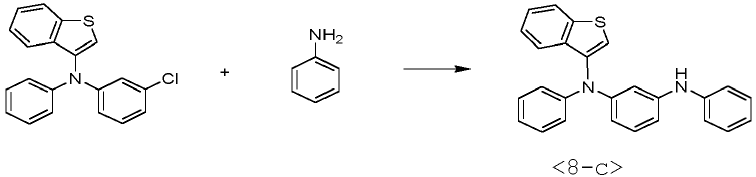

In a 100-mL reactor, <8-b> (5.4 g, 16 mmol), aniline (5.8 g, 16 mmol), palladium acetate (0.1 g, 1 mmol), sodium tert-butoxide (3 g, 32 mmol), bis(diphenylphosphino)-1,1'-binaphthyl (0.2 g, 1 mmol), and toluene (45 mL) were stirred together under reflux for 24 hours. After completion of the reaction, filtration was conducted and the filtrate was concentrated and purified by column chromatography to afford <8-c> (4.6 g, yield 73%)

### Synthesis Example 8-4. Synthesis of <8-d>

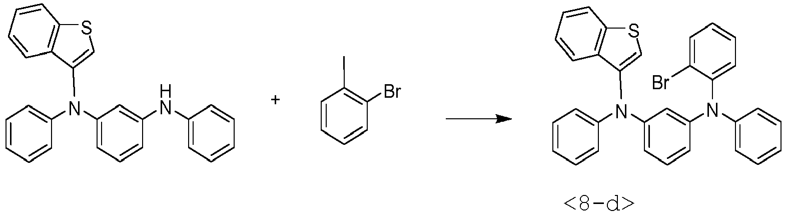

The same procedure as in Synthesis Example 8-2 was carried out, with the exception of using <8-c> and 1-bromo-2-iodobenzene instead of <8-a> and 3-bromobenzothiophene, respectively, to afford <8-d>. (yield 77%)

### Synthesis Example 8-5. Synthesis of <D 202>

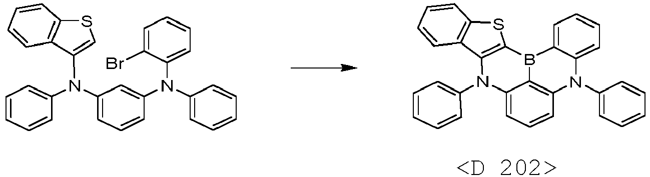

In a 300-mL reactor, <8-d> (12.6 g, 23 mmol) and tert-butyl benzene (120 mL) were added with drops of n-butyl lithium (42.5 mL, 68 mmol) at -78°C. Thereafter, the mixture was stirred at 60°C for 3 hours. Then, nitrogen was blown at 60°C to remove heptane. At -78°C, boron tribromide (11.3 g, 45 mmol) were dropwise added, followed by stirring at room temperature for 1 hour. At 0°C, N,N-diisopropylethylamine (5.9 g, 45 mmol) was dropwise added, followed by stirring at 120°C for 2 hours. After completion of the reaction, an aqueous sodium acetate solution was added. The reaction mixture was stirred before extraction with ethyl acetate. The organic layer thus formed was concentrated and purified by column chromatography to afford <D 202> (1.1 g, yield 10%).
MS (MALDI-TOF) : m/z 476.15 [M⁺]

### SYNTHESIS EXAMPLE 9. Synthesis of [D 265]

### Synthesis Example 9-1. Synthesis of <9-a>

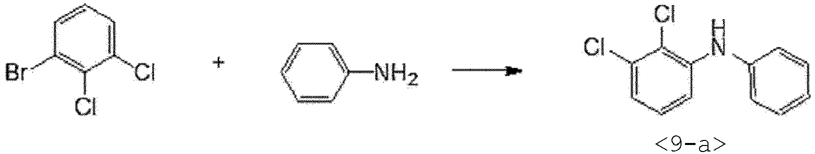

The same procedure as in Synthesis Example 8-1 was carried out, with the exception of using 1-bromo-2,3-dichlorobenzene instead of 1-bromo-3-chlorobenzene, to afford <9-a>. (yield 71%)

### Synthesis Example 9-2. Synthesis of <9-b>

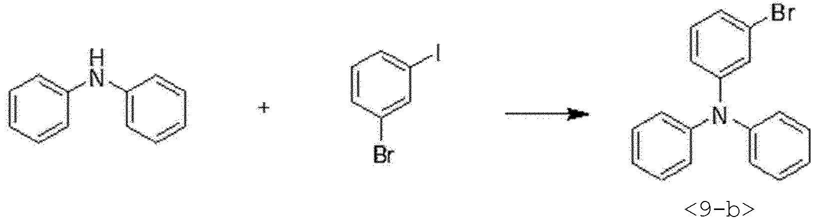

The same procedure as in Synthesis Example 8-2 was carried out, with the exception of using diphenylamine and 1-bromo-3-iodobenzene instead of <8-a> and 1-bromo-3-chlorobenzene, respectively, to afford <9-b>. (yield 77%)

### Synthesis Example 9-3. Synthesis of <9-c>

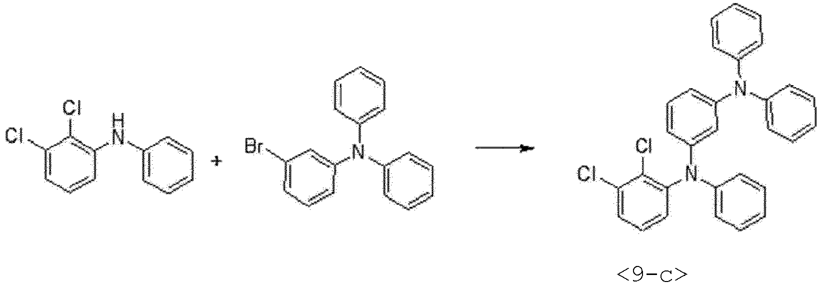

The same procedure as in Synthesis Example 8-2 was carried out, with the exception of using <9-a> and <9-b> instead of <8-a> and 1-bromo-3-chlorobenzene, respectively, to afford <9-c>. (yield 75%)

### Synthesis Example 9-4. Synthesis of <9-d>

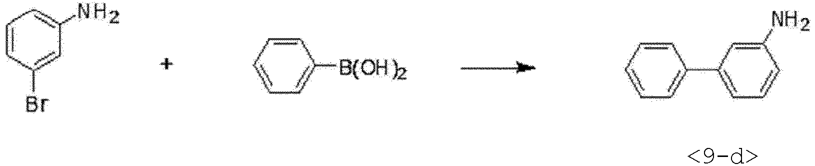

In a 1-L reactor, 3-bromoaniline (30 g, 174 mmol), phenyl boronic acid (25.5 g, 209 mmol), tetrakis(triphenylphosphine) palladium (4 g, 3 mmol), potassium carbonate (48.2 g, 349 mmol), 1,4-dioxane (150 mL), toluene (150 mL), and distilled water (90 mL) were stirred together under reflux for 4 hours. After completion of the reaction, separation was made of layers at room temperature, and the organic layer was concentrated in a vacuum and purified by column chromatography to afford <9-d> (24 g, yield 80%).

### Synthesis Example 9-5. Synthesis of <9-e>

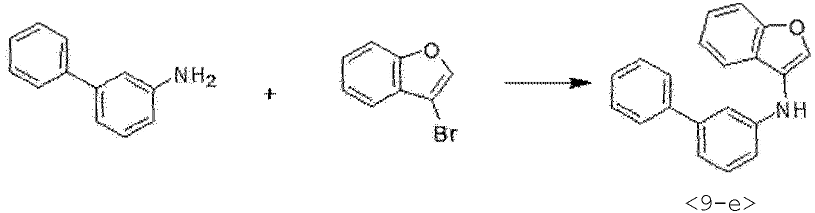

The same procedure as in Synthesis Example 8-1 was carried out, with the exception of using 3-bromobenzofuran and <9-d> instead of 1-bromo-3-chlorobenzene and aniline, respectively, to afford <9-e>. (yield 68%)

### Synthesis Example 9-6. Synthesis of <9-f>

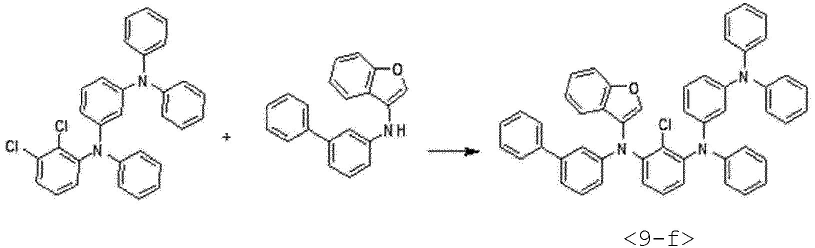

The same procedure as in Synthesis Example 8-2 was carried out, with the exception of using <9-c> and <9-e> instead of <8-a> and 1-bromo-3-chlorobenzene, respectively, to afford <9-f>. (yield 68%)

### Synthesis Example 9-7. Synthesis of <D 265>

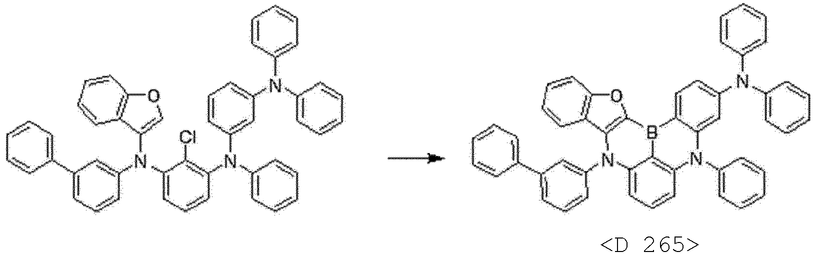

In a 250-mL reactor, a mixture of <9-f> (21 g, 37 mmol) and tert-butylbenzene was dropwise added with tert-butyl lithium (42.4 mL, 74 mmol) at -78°C. Thereafter, the mixture was stirred at 60°C for 3 hours. Then, nitrogen was blown at 60°C to remove pentane. At -78°C, boron tribromide (7.1 mL, 74 mmol) was dropwise added, followed by stirring at room temperature for 1 hour. At 0°C, N,N-diisopropylethylamine (6 g, 74 mmol) was dropwise added, followed by stirring 120°C for 2 hours. After completion of the reaction, an aqueous sodium acetate solution was added at room temperature. The reaction mixture was stirred before extraction with ethyl acetate. The organic layer was concentrated and purified by column chromatography to afford <D 265> (2.0 g, yield 17%).
MS (MALDI-TOF) : m/z 703.28 [M⁺]

### EXAMPLE 10. Synthesis of [D 459]

### Synthesis Example 10-1. Synthesis of <10-a>

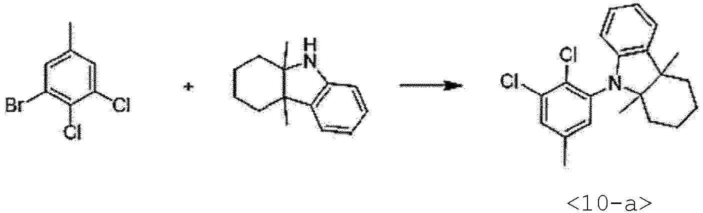

The same procedure as in Synthesis Example 8-2 was carried out, with the exception of using 4a,9a-dimethyl-2,3,4,4a,9,9a-hexahydro-1H-carbazole and 1-bromo-2,3-dichloro-5-methylbenzene instead of <8-a> and 3-bromobenzothiophene, respectively, to afford <10-a>. (yield 32%)

### Synthesis Example 10-2. Synthesis of <10-b>

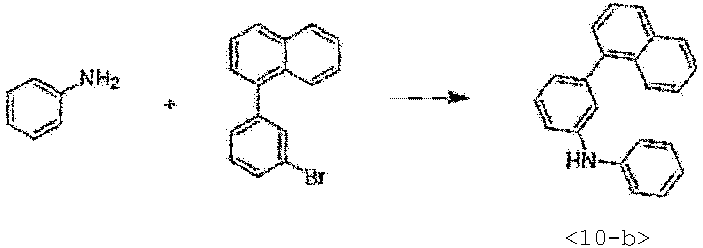

The same procedure as in Synthesis Example 8-1 was carried out, with the exception of using 3-(1-naphthyl)-1-bromobenzene instead of 1-bromo-3-chlorobenzene, to afford <10-b>. (yield 77%)

### Synthesis Example 10-3. Synthesis of <10-c>

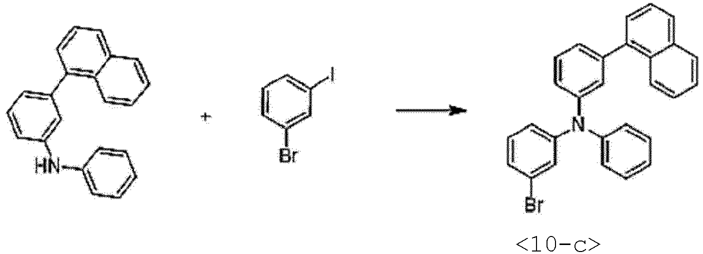

The same procedure as in Synthesis Example 8-2 was carried out, with the exception of using <10-b> and 1-bromo-3-iodobenzene instead of <8-a> and 3-bromobenzothiophene, respectively, to afford <10-c>. (yield 64%)

### Synthesis Example 10-4. Synthesis of <10-d>

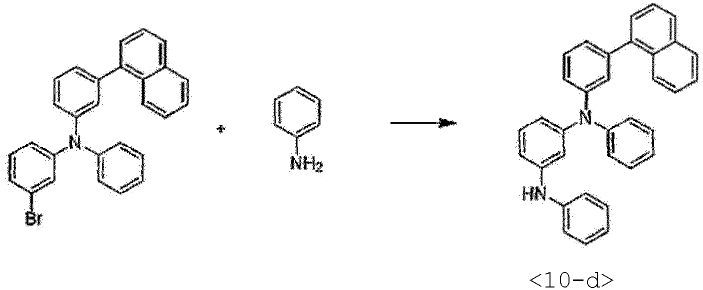

The same procedure as in Synthesis Example 8-1 was carried out, with the exception of using <8-c> instead of 1-bromo-3-chlorobenzene, to afford <10-d>. (yield 75%)

### Synthesis Example 10-5. Synthesis of <10-e>

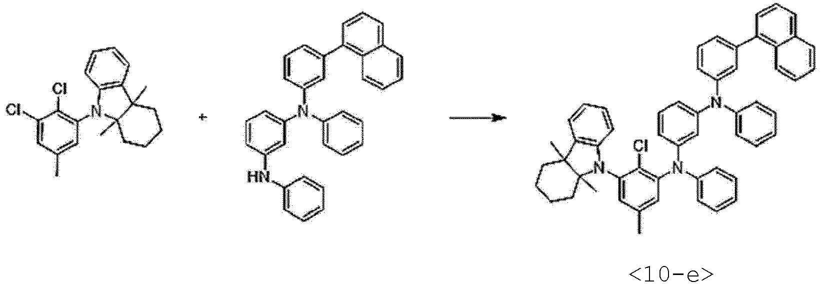

The same procedure as in Synthesis Example 8-2 was carried out, with the exception of using <10-d> and <10-a> instead of <8-a> and 3-bromobenzothiophene, to afford <10-e>. (yield 65%)

### Synthesis Example 10-6. Synthesis of <D 459>

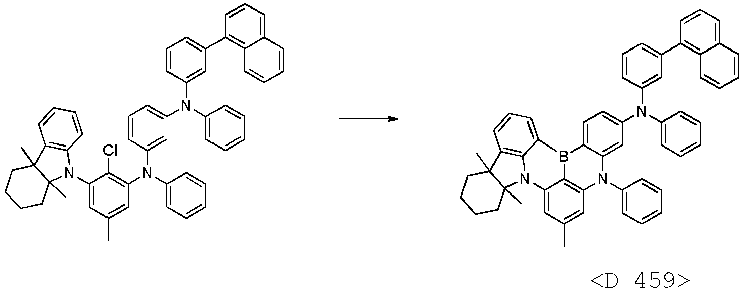

The same procedure as in Synthesis Example 9-7 was carried out, with the exception of using <10-e> instead of <9-f>, to afford <D 459>. (yield 11%)
MS (MALDI-TOF) : m/z 759.38 [M⁺]

### EXAMPLES 1 TO 42: Fabrication of Organic Light-Emitting Diodes Including First and Second Light-Emitting Layer

An ITO glass substrate was patterned to have a translucent area of 2 mm×2 mm and cleansed. The ITO glass was mounted in a vacuum chamber that was then set to have a base pressure of 1×10⁻⁷ torr. On the ITO glass substrate, films were sequentially formed of DNTPD (700 Å) and α-NPD (300 Å). Subsequently, a light-emitting layer was formed by sequentially depositing a first light-emitting layer and a second light-emitting layer. The first light-emitting layer (50 Å) was formed of a mixture of the pyrene compound (compound represented by Chemical Formula A) and the dopant compound (2 wt%) listed in Table 1, below, and the second light-emitting layer (150 Å) was formed of a mixture of the anthracene compound (compound represented by Chemical Formula E) and the dopant compound (2 wt%) listed in Tble 1. Then, [Chemical Formula E-1] and [Chemical Formula E-2] were deposited at a weight ratio of 1:1 to form an electron transport layer (300 Å) on which an electron injection layer of [Chemical Formula E-2] (10 Å) was formed and then covered with an Al layer (1000 Å) to fabricate an organic light-emitting diode. The organic light-emitting diodes thus obtained were measured at 0.4 mA for luminescence properties.

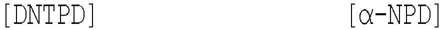

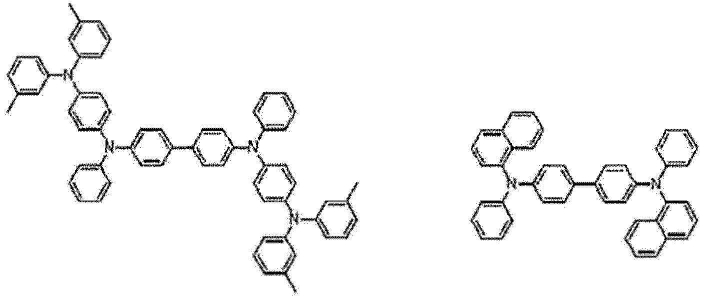

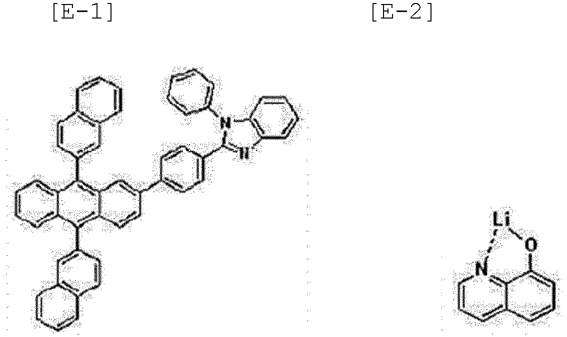

**TABLE 1**

| | 1^{st} Light-Emitting Layer | | 2^{nd} Light-Emitting Layer | | EQE | T97 | V |
|---|---|---|---|---|---|---|---|
| | Host | Dopant | Host | Dopant | | | |
| Ex. 1 | Chemical Formula 3 | D202 | Cpd. 219 | D202 | 13.6 | 438 | 3.6 |
| Ex. 2 | Chemical Formula 3 | D202 | Cpd. 313 | D202 | 13.2 | 426 | 3.5 |
| Ex. 3 | Chemical Formula 4 | D202 | Cpd. 313 | D202 | 13.8 | 450 | 3.6 |
| Ex. 4 | Chemical Formula 4 | D202 | Cpd. 441 | D202 | 13.4 | 442 | 3.5 |
| Ex. 5 | Chemical Formula 9 | D202 | Cpd. 219 | D202 | 13.5 | 435 | 3.6 |
| Ex. 6 | Chemical Formula 9 | D202 | Cpd. 441 | D202 | 13.4 | 424 | 3.5 |
| Ex. 7 | Chemical Formula 26 | D202 | Cpd. 219 | D202 | 13.6 | 444 | 3.6 |
| Ex. 8 | Chemical Formula 26 | D202 | Cpd. 313 | D202 | 13.2 | 432 | 3.6 |
| Ex. 9 | Chemical Formula 62 | D202 | Cpd. 313 | D202 | 13.3 | 429 | 3.5 |
| Ex. 10 | Chemical Formula 62 | D202 | Cpd. 441 | D202 | 13.4 | 440 | 3.6 |
| Ex. 11 | Chemical Formula 3 | D265 | Cpd. 219 | D265 | 12.9 | 430 | 3.6 |
| Ex. 12 | Chemical Formula 3 | D265 | Cpd. 441 | D2 65 | 13.1 | 385 | 3.5 |
| Ex. 13 | Chemical Formula 4 | D265 | Cpd. 219 | D265 | 13.2 | 380 | 3.6 |
| Ex. 14 | Chemical Formula 4 | D265 | Cpd. 313 | D265 | 13.4 | 400 | 3.5 |
| Ex. 15 | Chemical Formula 9 | D265 | Cpd. 313 | D265 | 13.1 | 397 | 3.6 |
| Ex. 16 | Chemical Formula 9 | D265 | Cpd. 441 | D265 | 13.0 | 382 | 3.5 |
| Ex. 17 | Chemical Formula 26 | D265 | Cpd. 219 | D265 | 13.3 | 386 | 3.4 |
| Ex. 18 | Chemical Formula 26 | D265 | Cpd. 441 | D2 65 | 13.2 | 384 | 3.6 |
| Ex. 19 | Chemical Formula 62 | D265 | Cpd. 219 | D265 | 12.8 | 381 | 3.6 |
| Ex. 20 | Chemical Formula 62 | D2 65 | Cpd. 313 | D265 | 12.9 | 395 | 3.5 |
| Ex. 21 | Chemical Formula 3 | D459 | Cpd. 313 | D459 | 13.1 | 416 | 3.6 |
| Ex. 22 | Chemical Formula 3 | D459 | Cpd. 441 | D459 | 13.5 | 400 | 3.5 |
| Ex. 23 | Chemical Formula 4 | D459 | Cpd. 219 | D459 | 13.2 | 393 | 3.6 |
| Ex. 24 | Chemical Formula 4 | D459 | Cpd. 441 | D459 | 13.5 | 420 | 3.5 |
| Ex. 25 | Chemical Formula 9 | D459 | Cpd. 219 | D459 | 13.2 | 405 | 3.6 |
| Ex. 26 | Chemical Formula 9 | D459 | Cpd. 313 | D459 | 13.0 | 418 | 3.5 |
| Ex. 27 | Chemical Formula 26 | D459 | Cpd. 313 | D459 | 13.3 | 414 | 3.6 |
| Ex. 28 | Chemical Formula 26 | D459 | Cpd. 441 | D459 | 13.1 | 404 | 3.6 |
| Ex. 29 | Chemical Formula 62 | D459 | Cpd. 219 | D459 | 13.4 | 412 | 3.5 |
| Ex. 30 | Chemical Formula 62 | D459 | Cpd. 441 | D459 | 13.2 | 410 | 3.6 |
| Ex. 31 | Chemical Formula 1 | D202 | Cpd. 219 | D202 | 13.0 | 384 | 3.6 |
| Ex. 32 | Chemical Formula 1 | D202 | Cpd. 313 | D202 | 12.8 | 380 | 3.6 |
| Ex. 33 | Chemical Formula 6 | D202 | Cpd. 313 | D202 | 13.1 | 381 | 3.6 |
| Ex. 34 | Chemical Formula 6 | D202 | Cpd. 441 | D202 | 12.9 | 381 | 3.6 |
| Ex. 35 | Chemical Formula 1 | D265 | Cpd. 219 | D265 | 13.0 | 382 | 3.6 |
| Ex. 36 | Chemical Formula 1 | D265 | Cpd. 441 | D265 | 12.9 | 384 | 3.6 |
| Ex. 37 | Chemical Formula 6 | D265 | Cpd. 219 | D265 | 12.8 | 375 | 3.6 |
| Ex. 38 | Chemical Formula 6 | D265 | Cpd. 313 | D265 | 13.0 | 370 | 3.6 |
| Ex. 39 | Chemical Formula 1 | D459 | Cpd. 313 | D459 | 12.8 | 380 | 3.6 |
| Ex. 40 | Chemical Formula 1 | D459 | Cpd. 441 | D459 | 12.9 | 375 | 3.6 |
| Ex. 41 | Chemical Formula 6 | D459 | Cpd. 219 | D459 | 13.0 | 377 | 3.6 |
| Ex. 42 | Chemical Formula 6 | D459 | Cpd. 441 | D459 | 12.9 | 380 | 3.6 |

### COMPARATIVE EXAMPLES 1 TO 20

Organic light emitting diodes of Comparative Examples 1 to 20 were fabricated in the same manner as in the Examples, with the exception of using the compounds listed in Table 2, below, instead of the compounds for the first and the second light-emitting layer in the Examples. The luminescence of the organic light-emitting diodes thus obtained was measured at 0.4 mA.

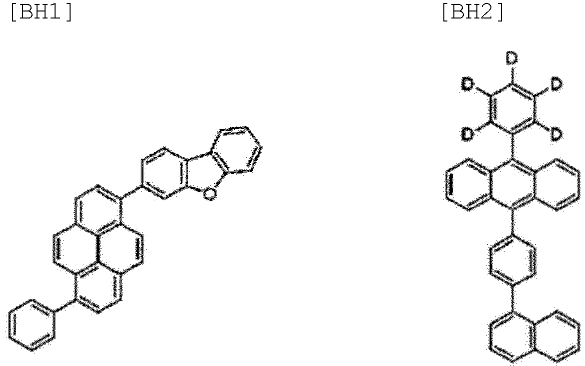

**TABLE 2**

| | 1^{st} Light-Emitting Layer | | 2^{nd} Light-Emitting Layer | | EQE | T97 | V |
|---|---|---|---|---|---|---|---|
| | Host | Dopant | Host | Dopant | | | |
| C. Ex. 1 | Chemical Formula 3 | D202 | Chemical Formula 4 | D202 | 9.2 | 280 | 4.1 |
| C. Ex. 2 | Chemical Formula 9 | D459 | Chemical Formula 26 | D459 | 8.9 | 270 | 4.2 |
| C. Ex. 3 | Chemical Formula 62 | D202 | BH1 | D202 | 8.4 | 260 | 4.0 |
| C. Ex. 4 | Chemical Formula 1 | D2 65 | BH1 | D2 65 | 8.2 | 255 | 3.9 |
| C. Ex. 5 | Chemical Formula 3 | D459 | BH1 | D459 | 7.9 | 265 | 4.0 |
| C. Ex. 6 | BH1 | D202 | BH1 | D202 | 6.5 | 170 | 4.2 |
| C. Ex. 7 | BH1 | D459 | BH1 | D459 | 6.3 | 160 | 4.0 |
| C. Ex. 8 | BH1 | D202 | Cpd. 219 | D202 | 8.1 | 272 | 3.9 |
| C. Ex. 9 | BH1 | D459 | Cpd. 313 | D459 | 7.8 | 260 | 3.8 |
| C. Ex. 10 | Chemical Formula 4 | D202 | BH2 | D202 | 8.8 | 292 | 3.8 |
| C. Ex. 11 | Chemical Formula 9 | D265 | BH2 | D2 65 | 8.5 | 280 | 3.9 |
| C. Ex. 12 | Chemical Formula 6 | D459 | BH2 | D459 | 8.6 | 284 | 3.9 |
| C. Ex. 13 | BH1 | D202 | BH2 | D202 | 6.1 | 182 | 4.0 |
| C. Ex. 14 | BH1 | D459 | BH2 | D459 | 5.8 | 175 | 4.0 |
| C. Ex. 15 | Cpd. 441 | D202 | Cpd. 219 | D202 | 9.9 | 300 | 3.9 |
| C. Ex. 16 | Cpd. 313 | D459 | Cpd. 441 | D459 | 9.5 | 288 | 3.9 |
| C. Ex. 17 | Cpd. 219 | D202 | BH2 | D202 | 8.2 | 284 | 3.8 |
| C. Ex. 18 | Cpd. 313 | D265 | BH2 | D2 65 | 8.0 | 275 | 3.9 |
| C. Ex. 19 | BH2 | D202 | BH2 | D202 | 5.9 | 175 | 4.0 |
| C. Ex. 20 | BH2 | D459 | BH2 | D459 | 5.6 | 170 | 4.0 |

As is understood from data of Tables 1 and 2, the organic light-emitting diodes according to the present disclosure can drive at lower voltages with higher luminous efficiency, compared to those of the Comparative Examples. Thus, the organic light-emitting diodes according to the present disclosure is highly available in the organic light-emitting diode field.

### Industrial Applicability

Compared to conventional compounds, the compounds of the present disclosure allow organic light-emitting diodes to exhibit superiority in terms of luminous efficiency and longevity, thus finding applicability in the organic light-emitting diode field and related industrial fields.

## Claims

1. an organic light-emitting diode, including: a first electrode; a second electrode facing the first electrode, and a first light-emitting layer including a first host and a first dopant and a second light-emitting layer including a second host and a second dopant, sequentially disposed between the first electrode and the second electrode, wherein at least one of the first host and the second host include at least one compound represented by the following Chemical Formula A: 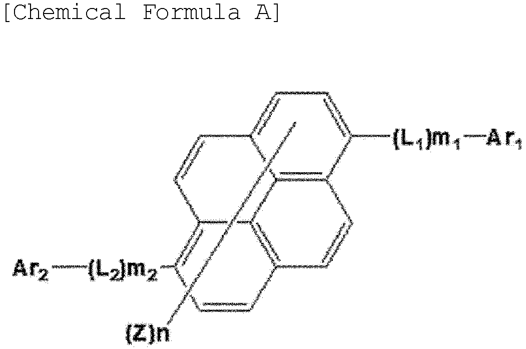 wherein,
linkers L₁ and L₂, which are same or different, are each independently selected from a single bond, a substituted or unsubstituted arylene of 6 to 30 carbon atoms, and a substituted or unsubstituted heteroarylene of 2 to 30 carbon atoms;
m₁ and m₂, which are same or different, are each independently an integer of 1 to 2, wherein when m₁ and m₂ are 2, the corresponding L₁'s are same or different and the L₂'s are same or different;
Ar₁ and Ar₂, which are same or different, are each independently any one selected from a substituted or unsubstituted aryl of 6 to 50 carbon atoms, a substituted or unsubstituted heteroaryl of 2 to 50 carbon atoms, a substituted or unsubstituted alkyl of 1 to 30 carbon atoms, a substituted or unsubstituted cycloalkyl of 3 to 30 carbon atoms, a substituted or unsubstituted alkylsilyl of 1 to 30 carbon atoms, and a substituted or unsubstituted arylsilyl of 6 to 30 carbon atoms;
Z is any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
n is an integer of 0 to 8, wherein when n is 2 or higher, the corresponding Z's are same or different;
the aromatic carbons of the pyrene ring moiety to which the substituent Z or Ar-(L)m is not bonded each have a hydrogen or deuterium atom bonded thereto,
wherein the term 'substituted' in the expression "a substituted or unsubstituted" means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

2. The organic light-emitting diode of claim 1, wherein the linkers L₁ and L₂ are same or different and are each independently a substituted or unsubstituted arylene of 6 to 18 carbon atoms.

3. The organic light-emitting diode of claim 2, wherein m₁ and m₂ are each 1 and the linkers L₁ and L₂ are same or different and are each independently selected from [Structural Formula 1] and [Structural Formula 2] below: 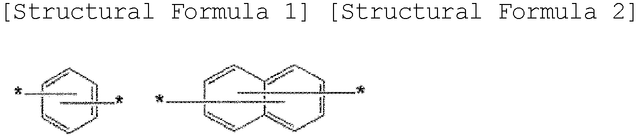
wherein the carbon atoms of the aromatic ring moiety can each have a hydrogen or deuterium atom thereon and
"*-" denotes a bonding site to the pyrene ring moiety, or Ar₁ or Ar₂.

4. The organic light-emitting diode of claim 1, wherein Ar₁ and Ar₂ are same or different and each independently a substituted or unsubstituted aryl of 6 to 18 carbon atoms or a substituted or unsubstituted heteroaryl of 2 to 18 carbon atoms.

5. The organic light-emitting diode of claim 4, wherein Ar₁ and Ar₂ are same or different and each independently any one selected from [Structural Formula 11] to [Structural Formula 15], below: 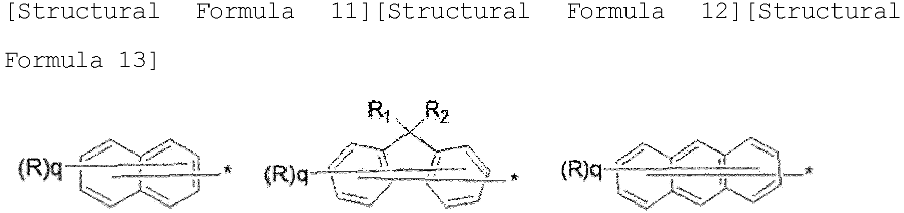 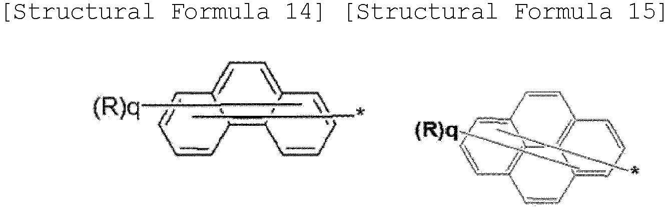
wherein "*-" is a bonding site to the linker L₁ or L₂,
R, R₁, and R₂, which are same or different, are each any one selected from a deuterium atom, a halogen atom, an alkyl of 1 to 12 carbon atoms, a halogenated alkyl of 1 to 24 carbon atoms, a cycloalkyl of 3 to 12 carbon atoms, an aryl of 6 to 18 carbon atoms, an arylalkyl of 7 to 18 carbon atoms, and an alkylaryl of 7 to 18 carbon atoms,
q is an integer of 0 to 9 wherein when q is 2 or higher, the corresponding R's are same or different, and
the carbon atoms of the aromatic ring moieties can each have a hydrogen or deuterium atom when any R is not bonded thereto.

6. The organic light-emitting diode of claim 1, wherein the compound represented by Chemical Formula A has a structure symmetric about the X-Y axis shown in the following structural diagram, except for the substituent Z:

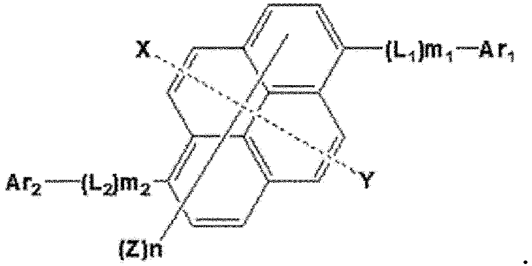

7. The organic light-emitting diode of claim 6, wherein the compound represented by Chemical Formula A has a structure symmetric about the X-Y axis shown in the following structural diagram, including the substituent Z.

8. The organic light-emitting diode of claim 5, wherein Ar₁ and Ar₂ are same or different and each independently an aryl represented by Structural Formula 11.

9. The organic light-emitting diode of claim 3, wherein the linkers L₁ and L₂ are same or different and each independently [Structural Formula 1], and Ar₁ and Ar₂ are same or different and each independently any one selected from [Structural Formula 11] to [Structural Formula 15], below: 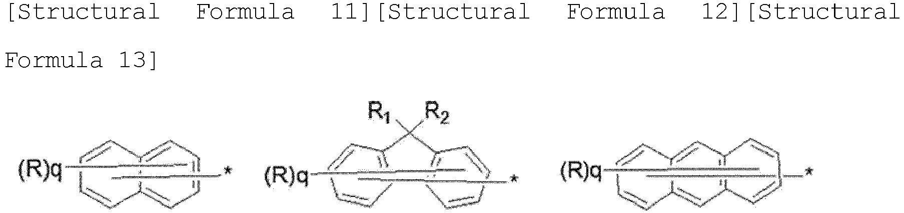 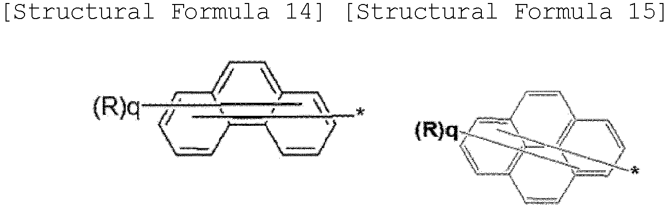
wherein "*-" is a bonding site to the linker L₁ or L₂,
R, R₁, and R₂, which are same or different, are each any one selected from a deuterium atom, a halogen atom, an alkyl of 1 to 12 carbon atoms, a halogenated alkyl of 1 to 24 carbon atoms, a cycloalkyl of 3 to 12 carbon atoms, an aryl of 6 to 18 carbon atoms, an arylalkyl of 7 to 18 carbon atoms, and an alkylaryl of 7 to 18 carbon atoms,
q is an integer of 0 to 9 wherein when q is 2 or higher, the corresponding R's are same or different, and
the carbon atoms of the aromatic ring moieties can each have a hydrogen or deuterium atom when any R is not bonded thereto.

10. The organic light-emitting diode of claim 1, wherein Z is any one selected from a hydrogen atom, a deuterium atom, a halogen atom, a substituted or unsubstituted alkyl of 1 to 12 carbon atoms, a substituted or unsubstituted cycloalkyl of 3 to 12 carbon atoms, and a substituted or unsubstituted aryl of 6 to 18 carbon atoms.

11. The organic light-emitting diode of claim 1, wherein the compound is any one selected from [Chemical Formula 1] to

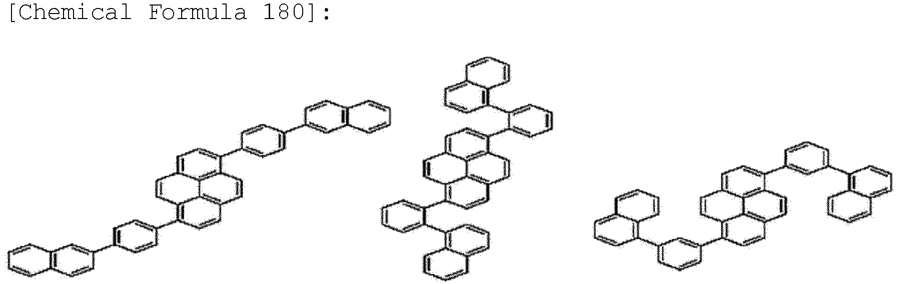

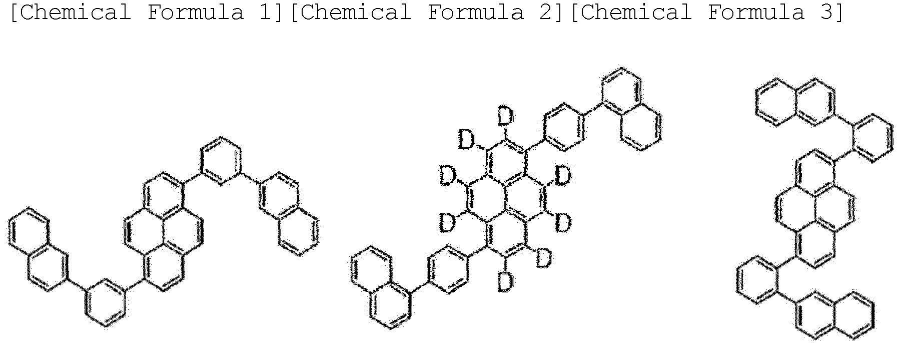

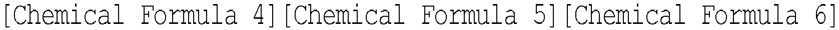

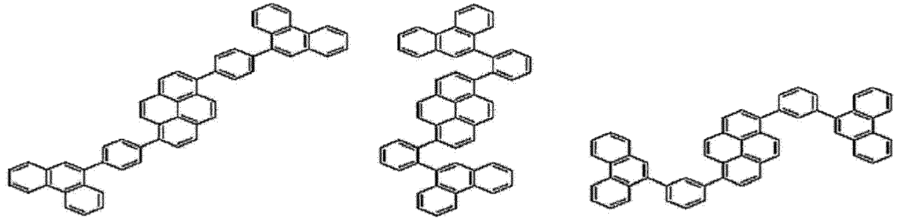

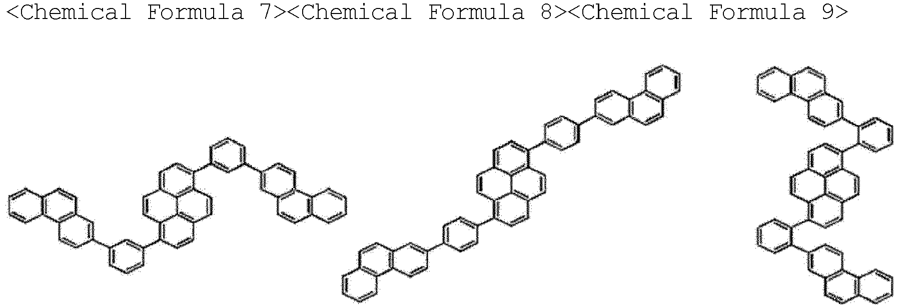

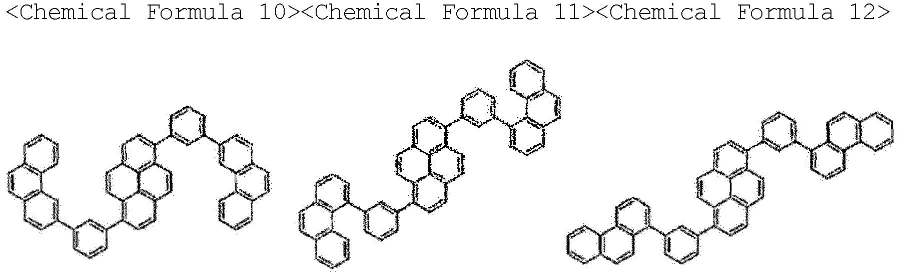

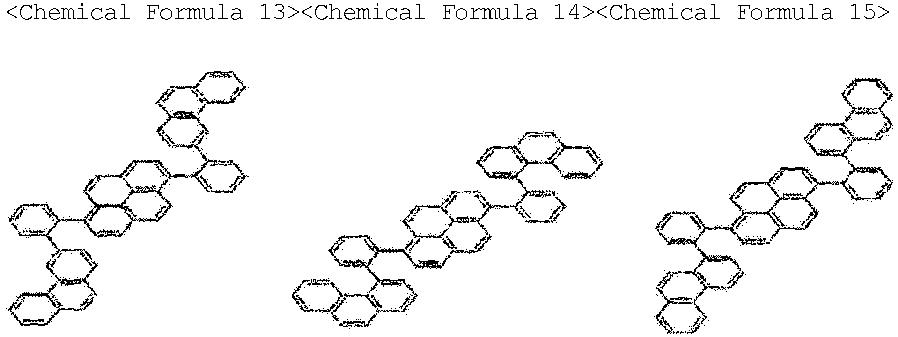

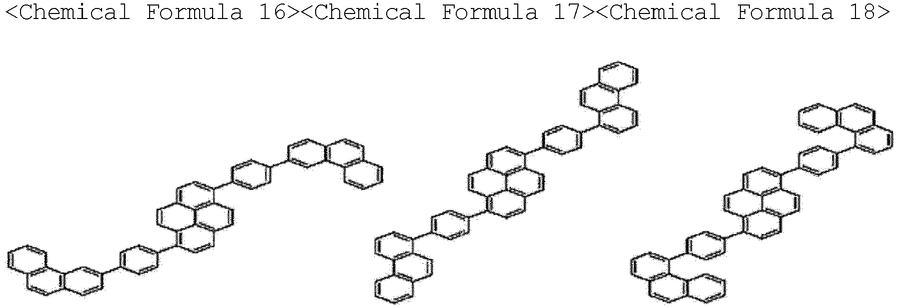

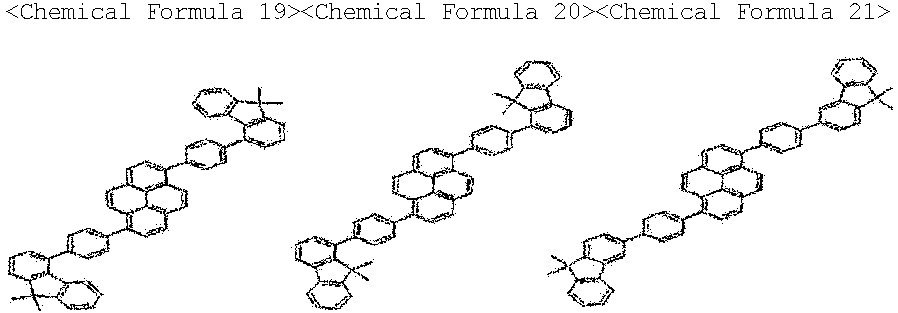

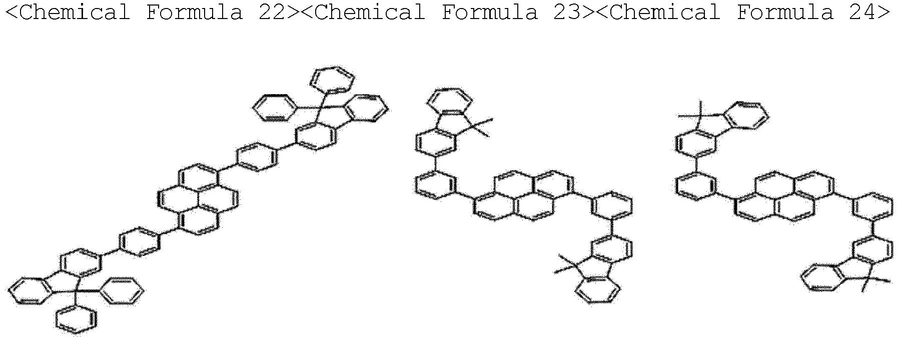

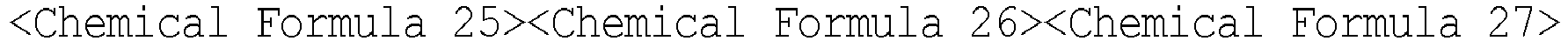

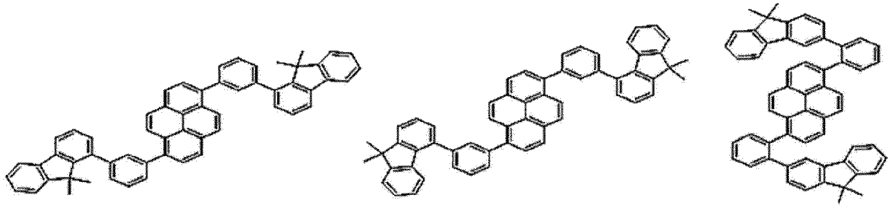

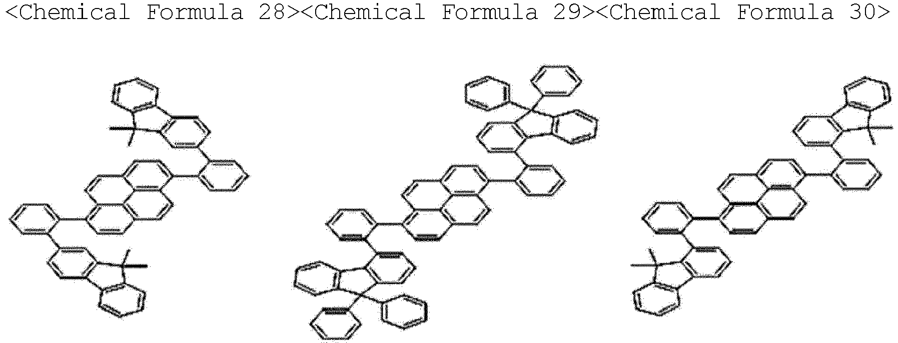

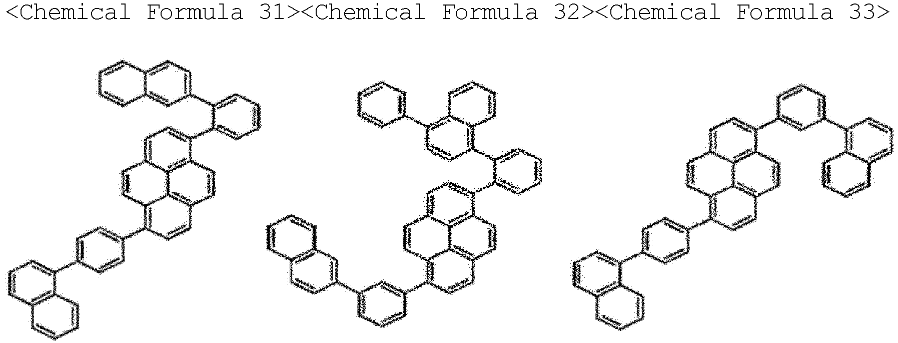

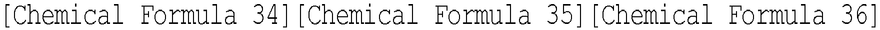

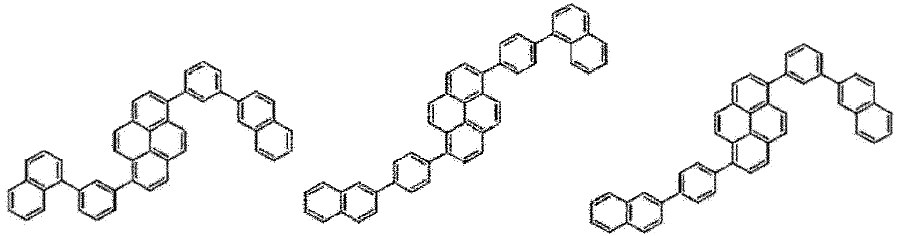

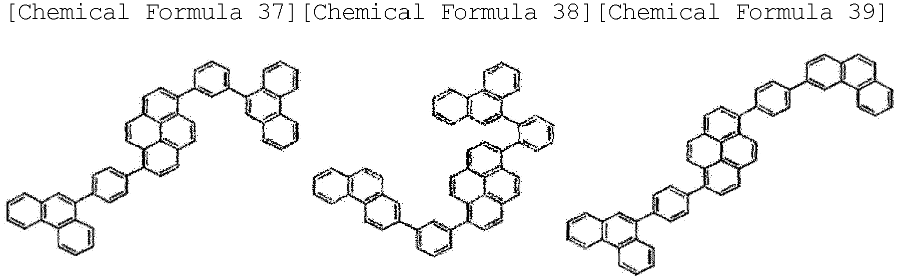

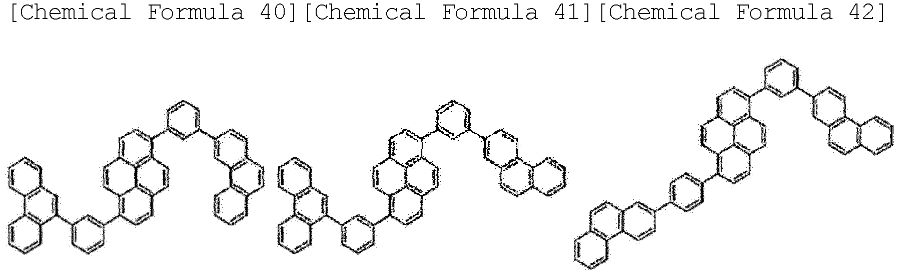

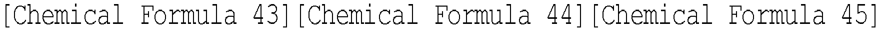

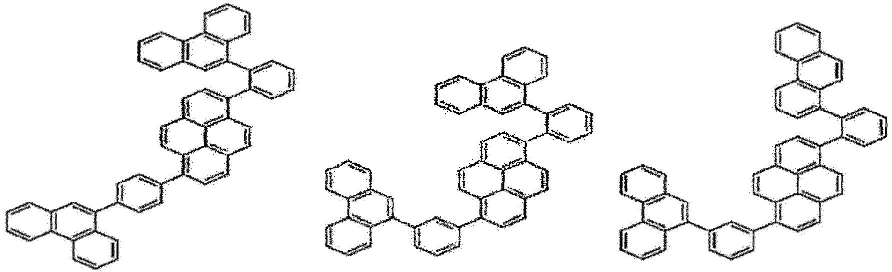

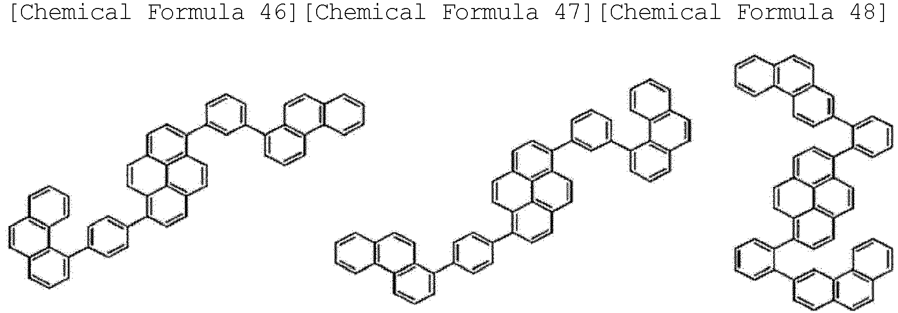

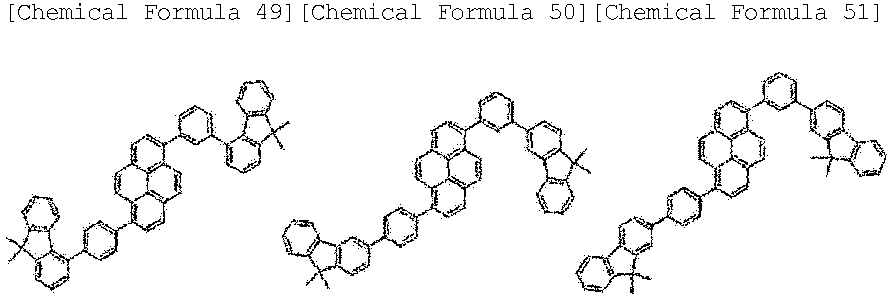

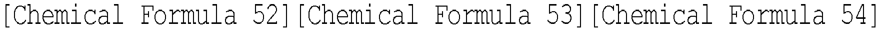

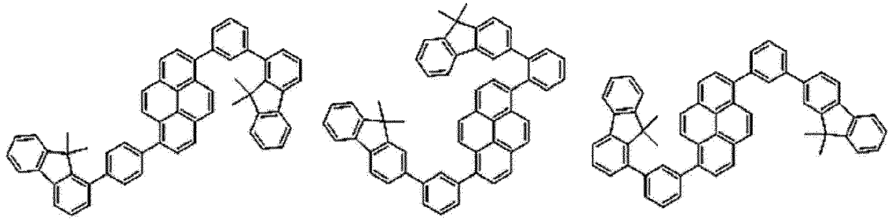

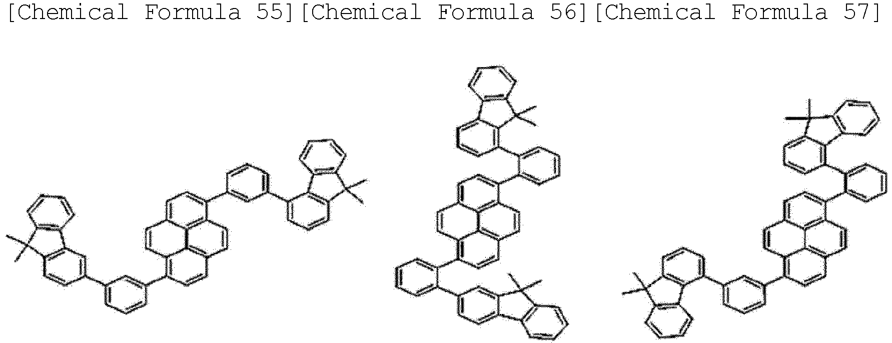

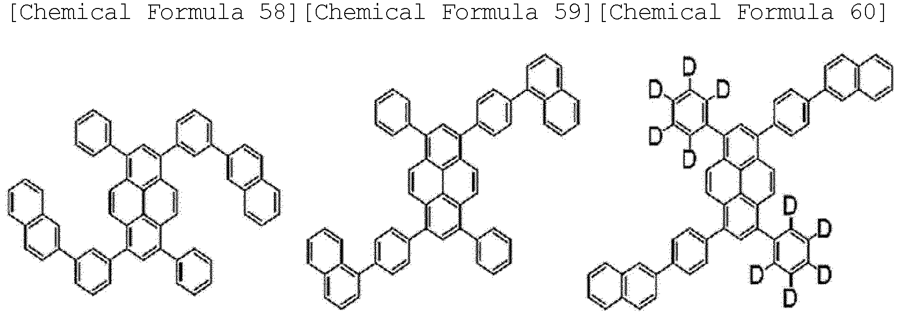

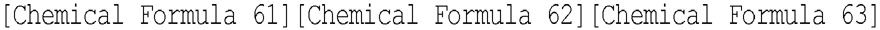

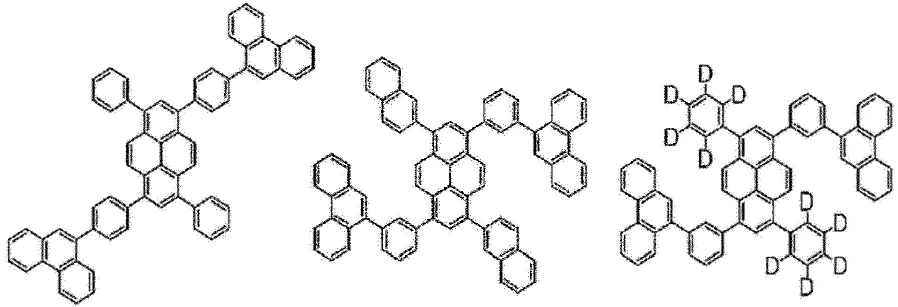

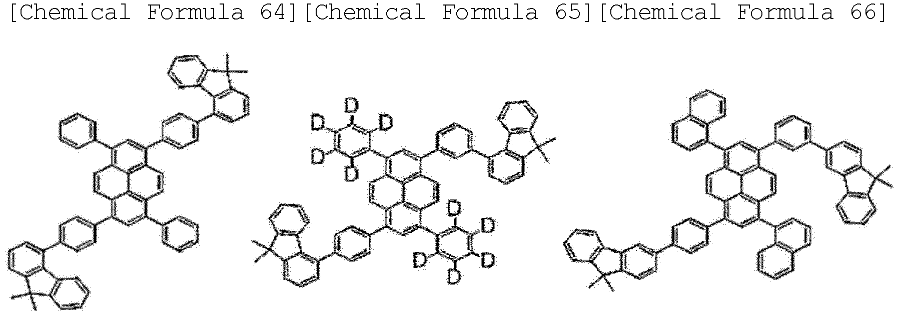

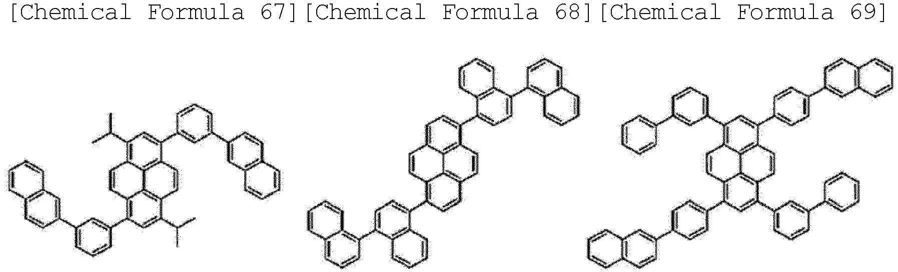

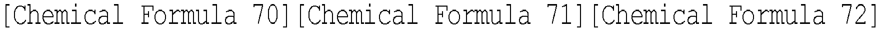

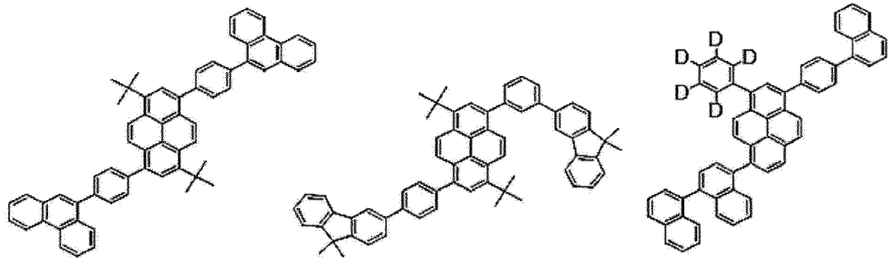

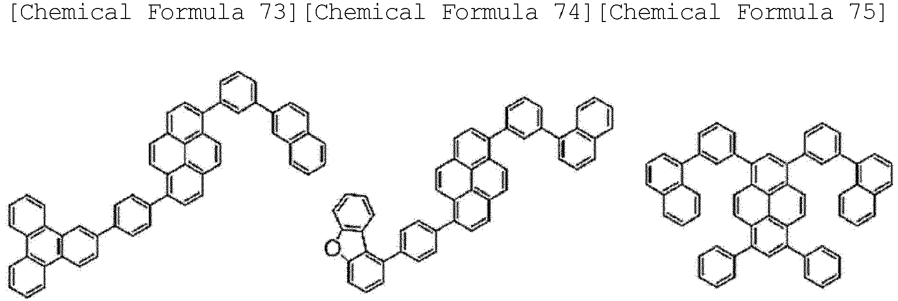

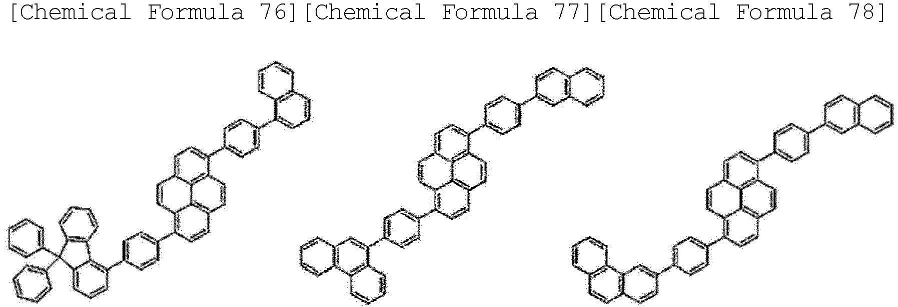

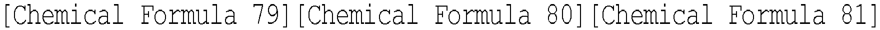

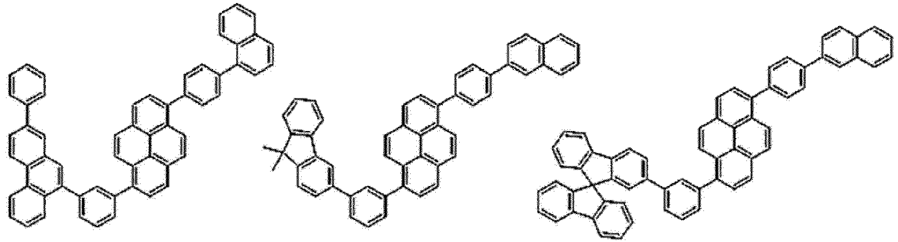

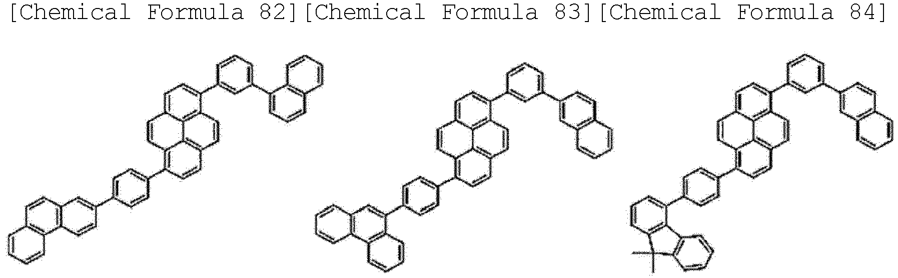

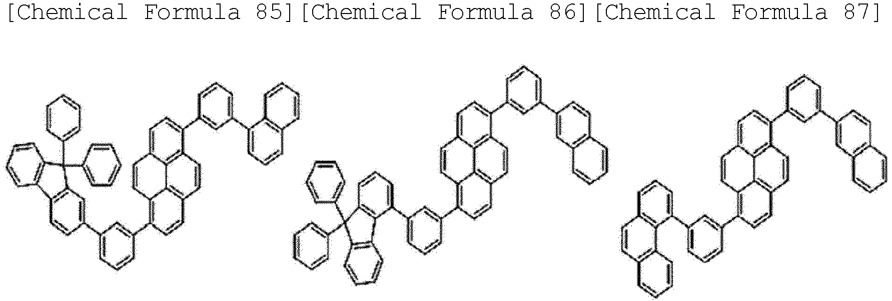

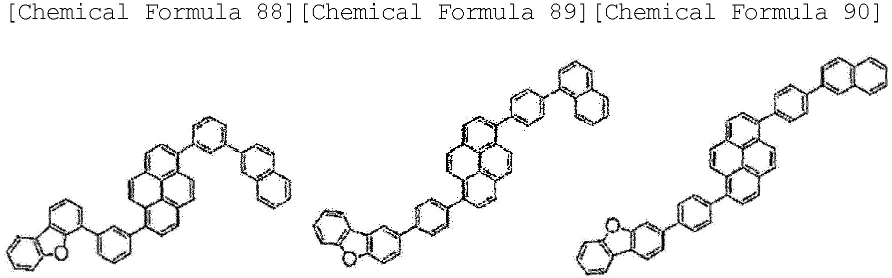

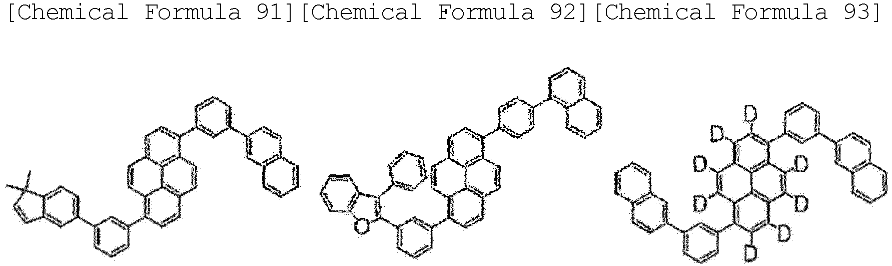

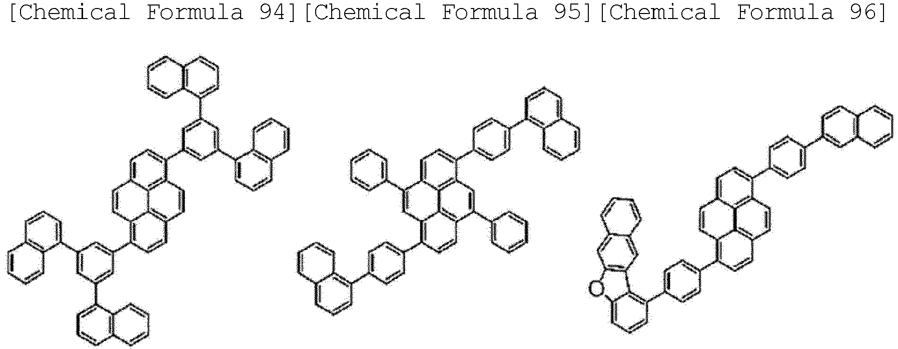

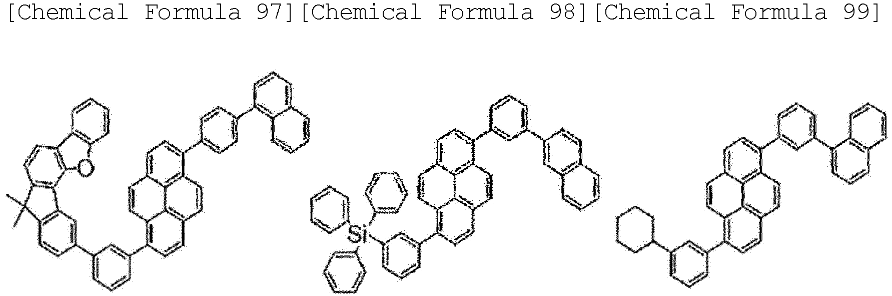

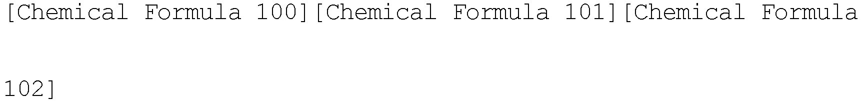

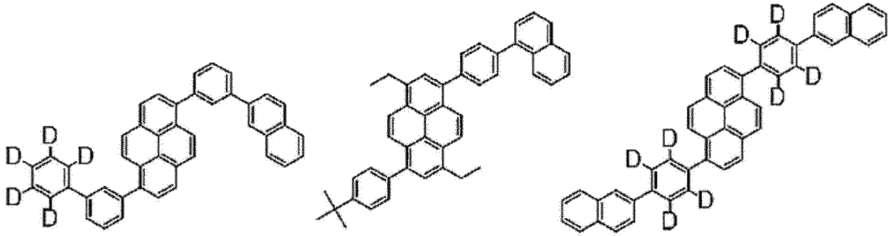

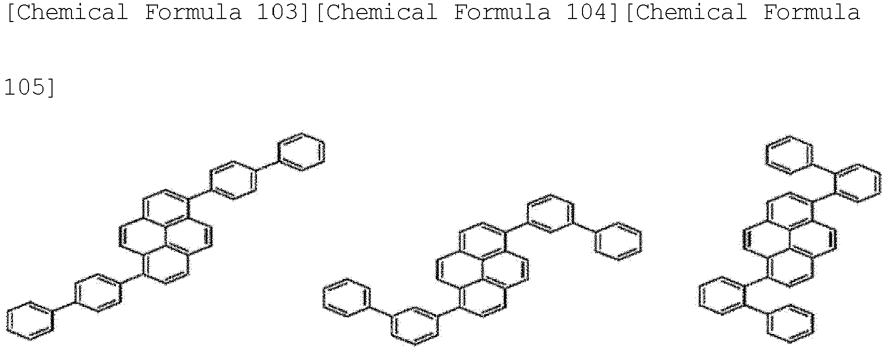

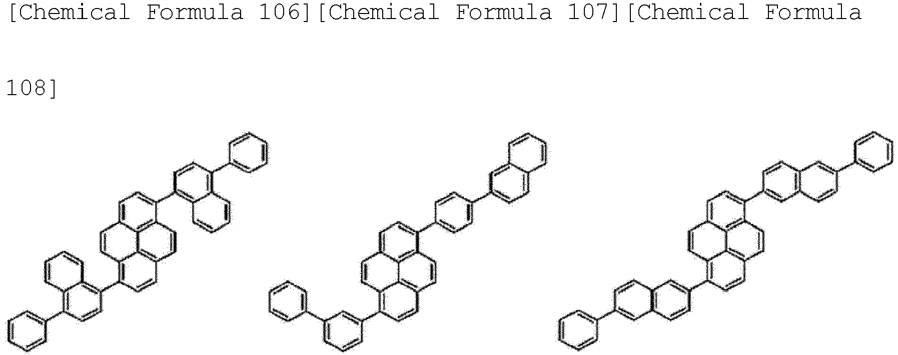

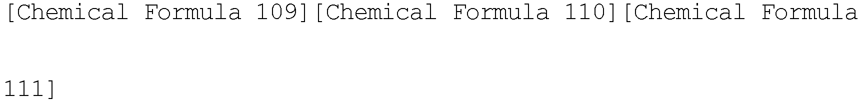

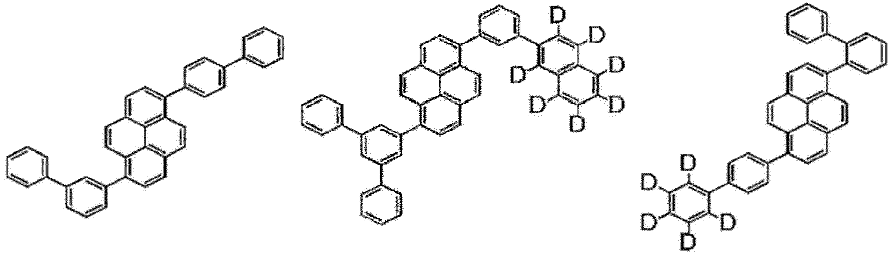

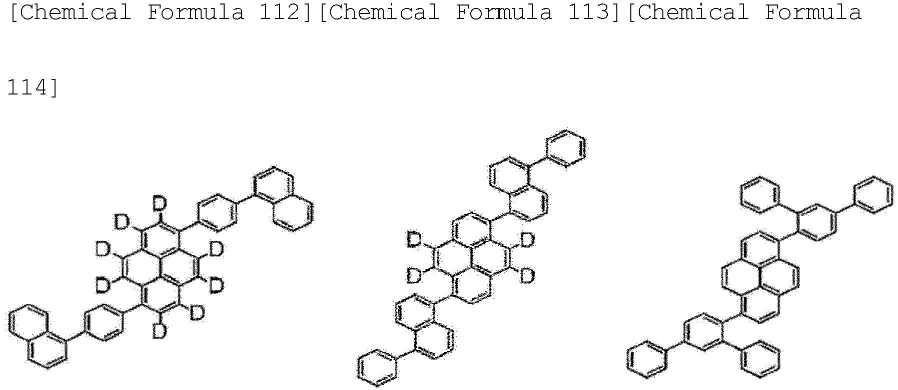

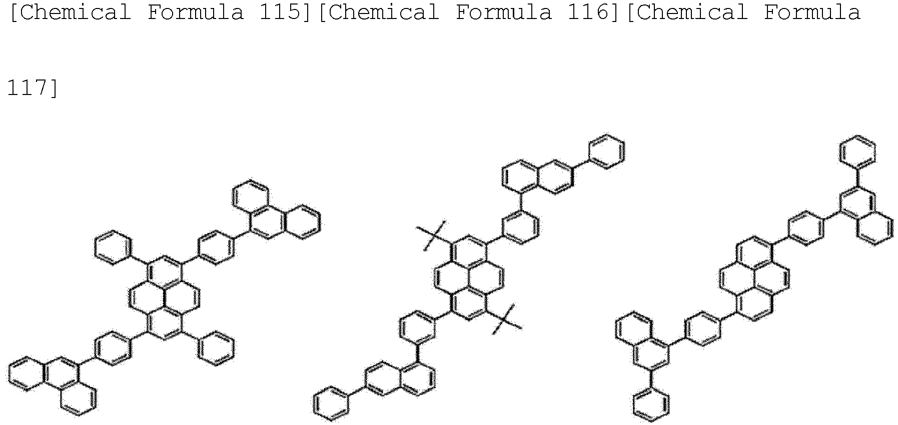

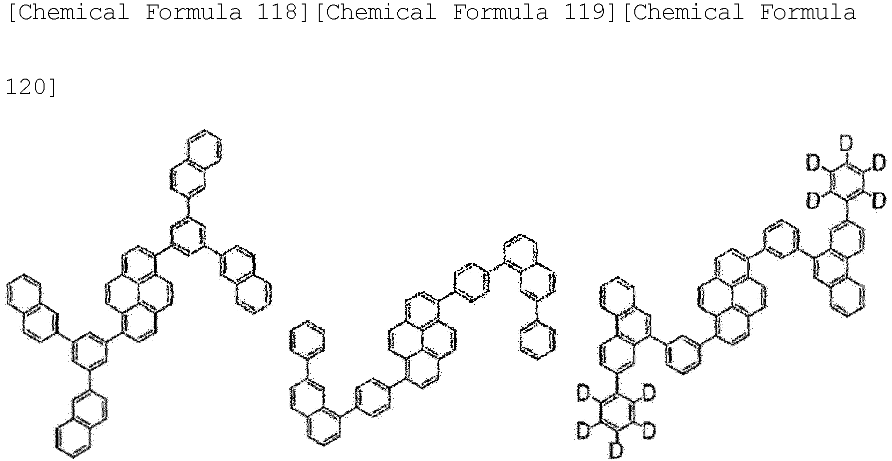

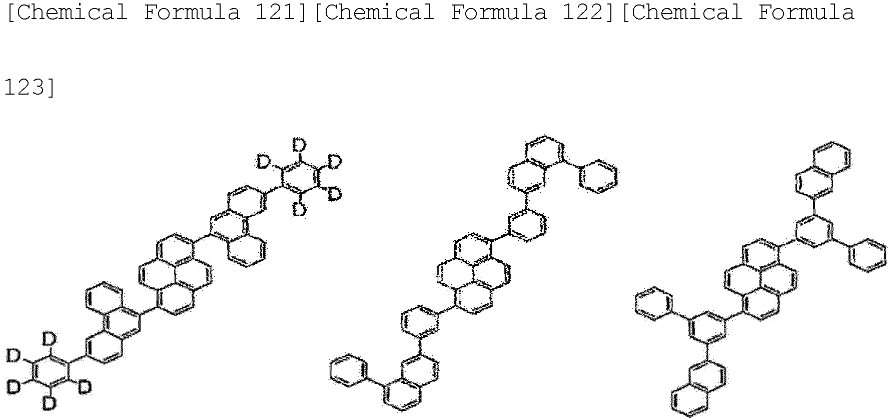

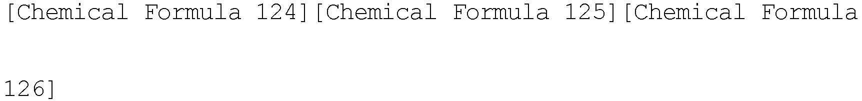

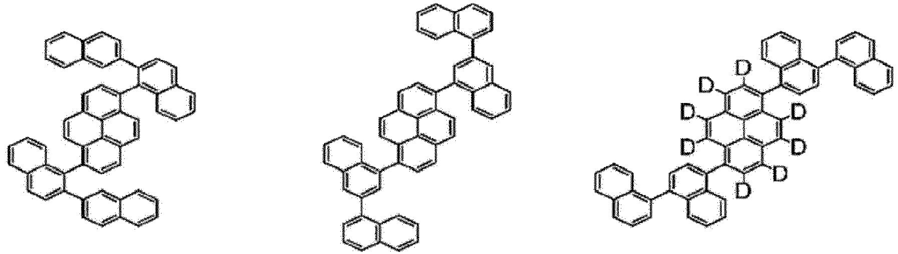

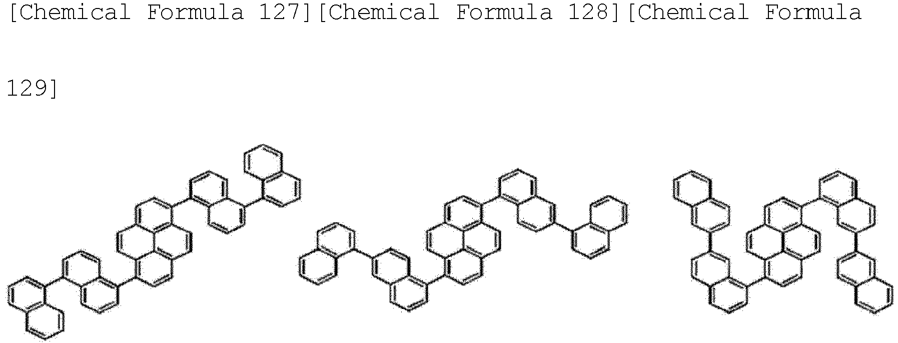

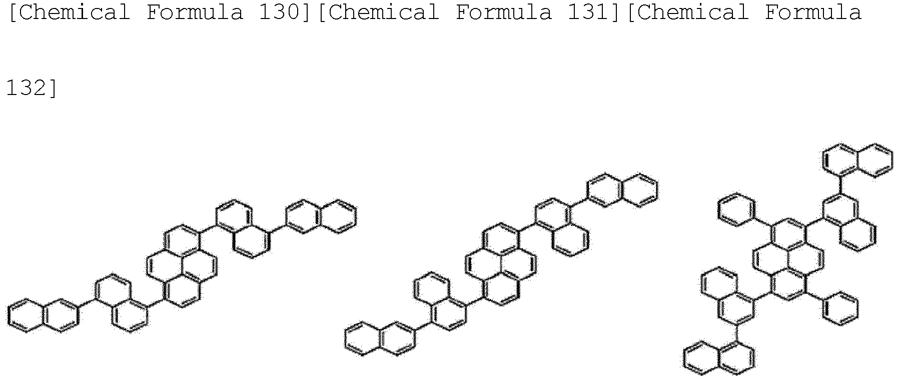

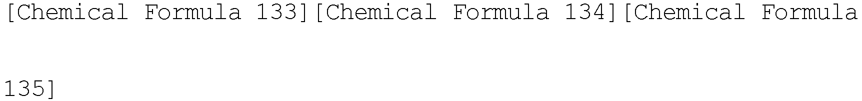

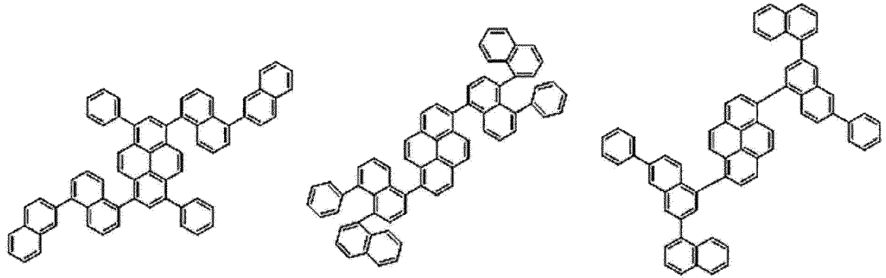

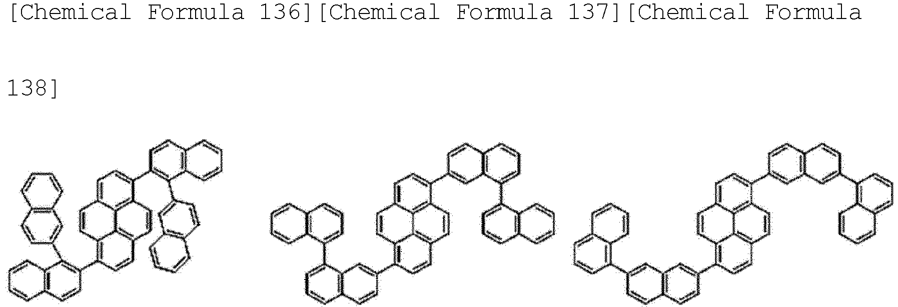

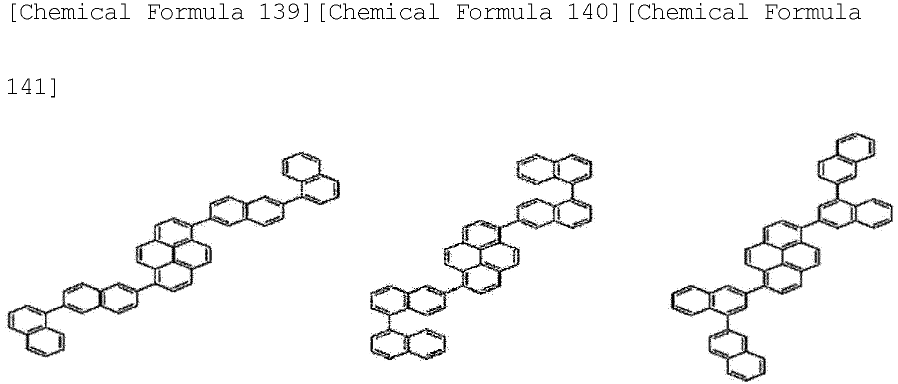

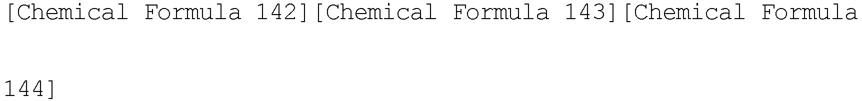

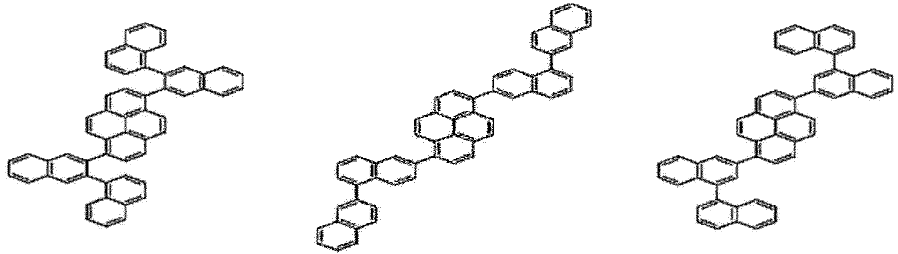

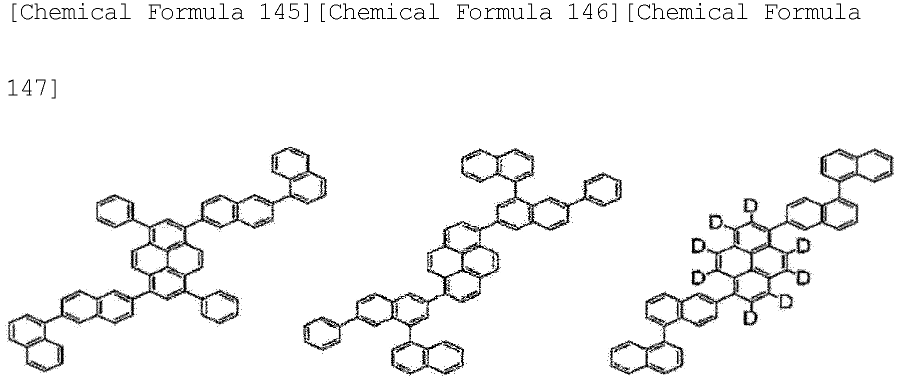

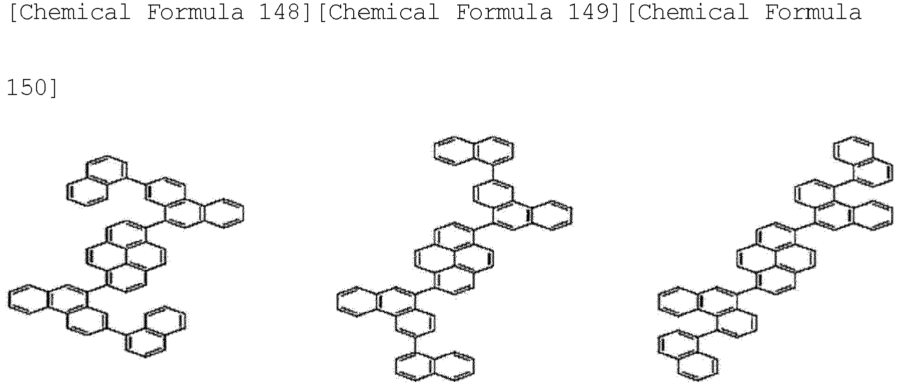

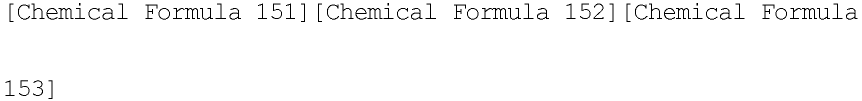

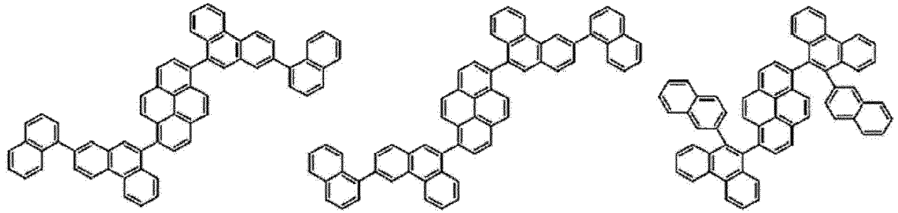

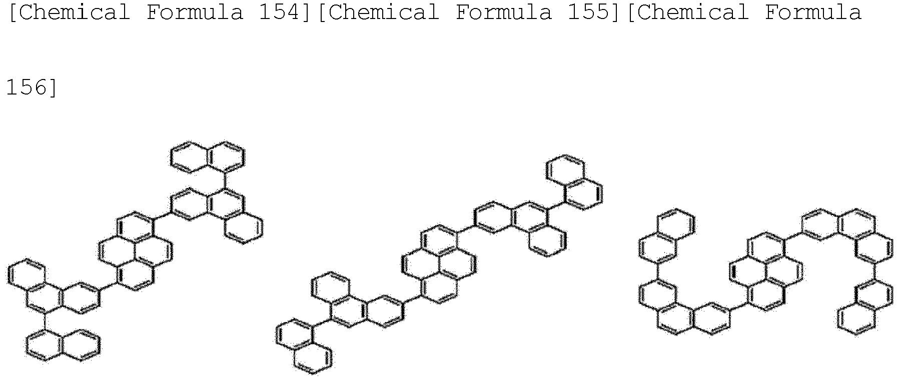

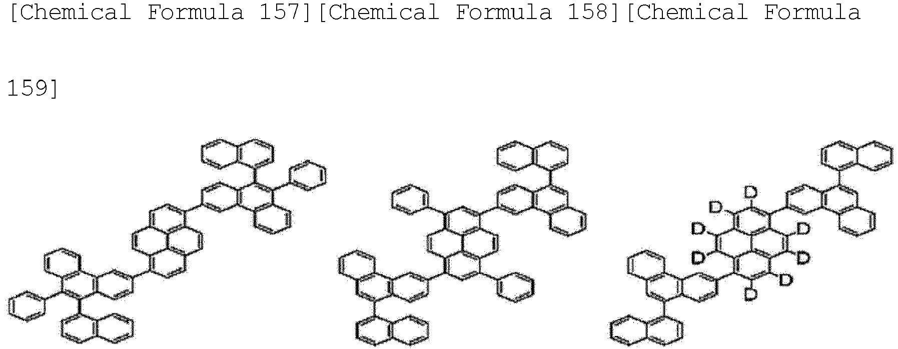

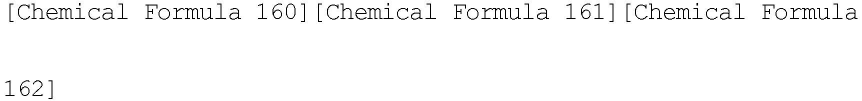

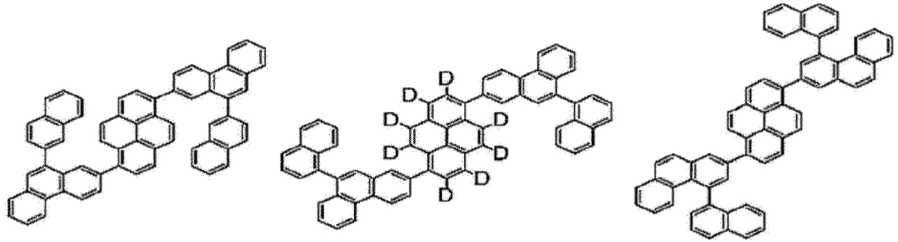

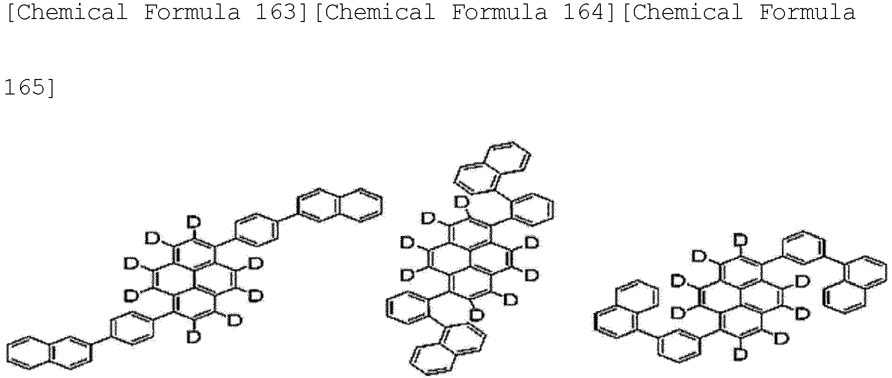

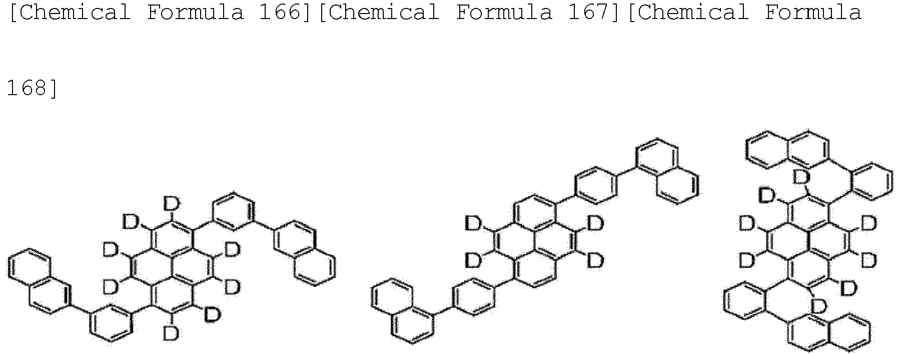

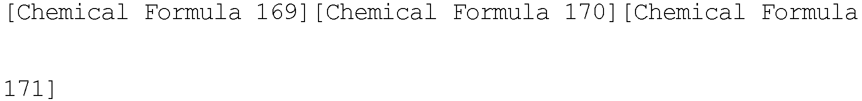

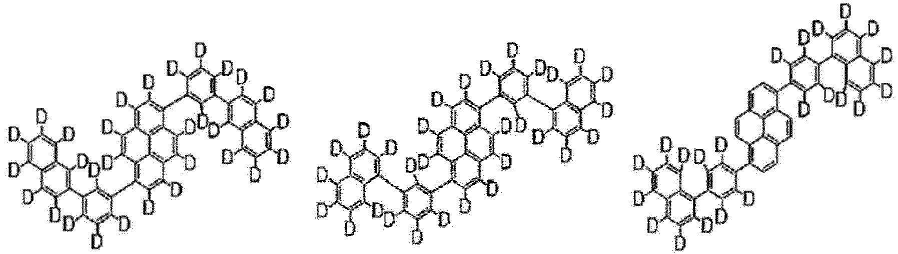

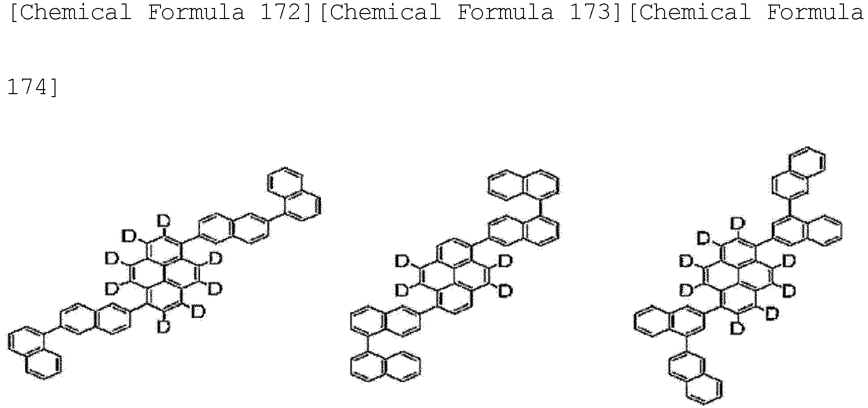

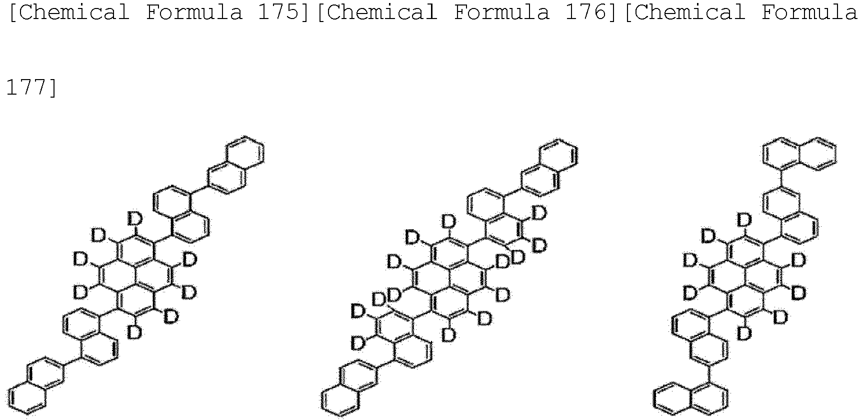

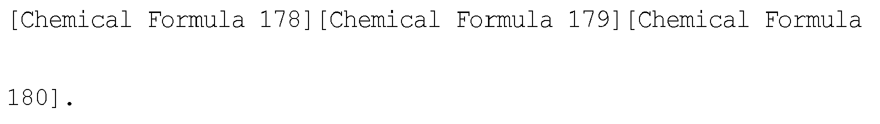

12. The organic light-emitting diode of claim 1, comprising: at least one of a hole transport layer and a hole injection layer between the first electrode and the first light-emitting layer; and at least one of an electron transport layer and an electron injection layer between the first light-emitting layer and the second electrode.

13. The organic light-emitting diode of claim 1, wherein at least one of the first dopant in the first light-emitting layer and the second dopant in the second light-emitting layer is at least one represented by any one selected from the following Chemical Formulas D1 to D10: 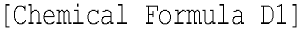 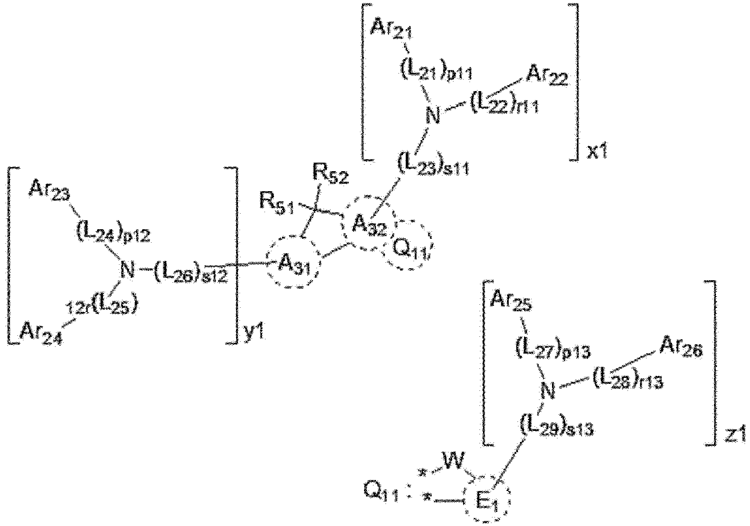 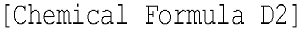 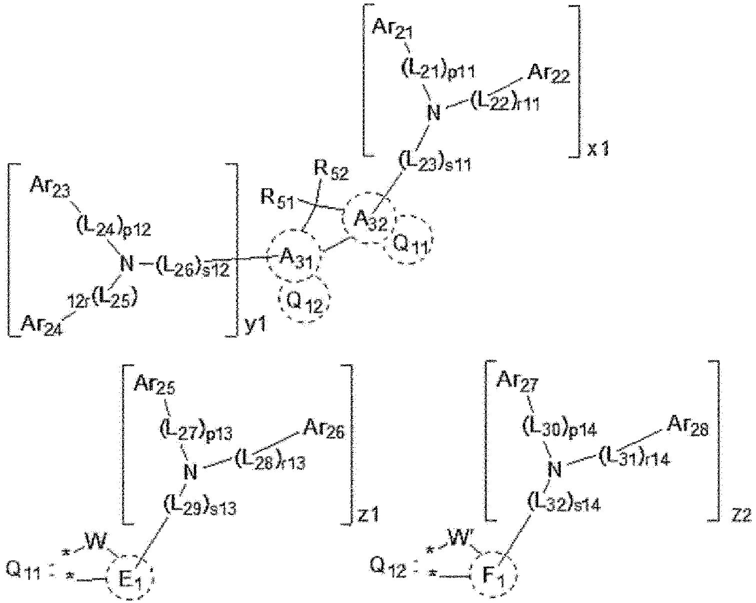 wherein,
A₃₁, A₃₂, E₁, and F₁, which are same or different, are each independently a substituted or unsubstituted aromatic hydrocarbon ring of 6 to 50 carbon atoms, or a substituted or unsubstituted heteroaromatic ring of 2 to 40 carbon atoms,
wherein two adjacent carbon atoms of the aromatic ring A₃₁ and two adjacent carbon atoms of the aromatic ring A₃₂ form a 5-membered fused ring together with a carbon atom to which substituents R₅₁ and R₅₂ are bonded;
linkers L₂₁ to L₃₂, which are same or different, are each independently selected from among a single bond, a substituted or unsubstituted alkylene of 1 to 60 carbon atoms, a substituted or unsubstituted alkenylene of 2 to 60 carbon atoms, a substituted or unsubstituted alkynylene of 2 to 60 carbon atoms, a substituted or unsubstituted cycloalkylene of 3 to 60 carbon atoms, a substituted or unsubstituted heterocycloalkylene of 2 to 60 carbon atoms, a substituted or unsubstituted arylene of 6 to 60 carbon atoms, and a substituted or unsubstituted heteroarylene of 2 to 60 carbon atoms;
W and W', which are same or different, are each independently any one selected from among N-R₅₃, CR₅₄R₅₅, SiR₅₆R₅₇, GeR₅₈R₅₉, O, S, and Se;
R₅₁ to R₅₉, and Ar₂₁ to Ar₂₈, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted alkenyl of 2 to 30 carbon atoms, a substituted or unsubstituted alkynyl of 2 to 20 carbon atoms, a substituted or unsubstituted cycloalkyl of 3 to 30 carbon atoms, a substituted or unsubstituted cycloalkenyl of 5 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a substituted or unsubstituted alkylgermyl of 1 to 30 carbon atoms, a substituted or unsubstituted arylgermyl of 1 to 30 carbon atoms, a cyano, a nitro, and a halogen, wherein R₅₁ and R₅₂ together can form a mono- or polycyclic aliphatic or aromatic ring that can be a heterocyclic ring bearing a heteroatom selected from among N, O, P, Si, S, Ge, Se, and Te as a ring member;
p11 to p14, r11 to r14, and s11 to s14 are each independently an integer of 1 to 3, wherein when any of them is 2 or greater, the corresponding linkers L₂₁ to L₃₂ can be same or different,
x1 is 1, and y1, z1, and z2, which are same or different, are each independently an integer of 0 to 1; and
Ar₂₁ can form a ring with Ar₂₂, Ar₂₃ can form a ring with Ar₂₄, Ar₂₅ can form a ring with Ar₂₆, and Ar₂₇ can form a ring with Ar₂₈,
two adjacent carbon atoms of the A₃₂ ring moiety of Chemical Formula D1 can occupy respective positions of Structural Formula Q₁₁ to form a fused ring, and
two adjacent carbon atoms of the A₃₁ ring moiety of Chemical Formula D2 can occupy respective positions of structural Formula Q₁₂ to form a fused ring, and two adjacent carbon atoms of the A₃₂ ring moiety of Chemical Formula D2 can occupy respective positions of Structural Formula Q₁₁ to form a fused ring, 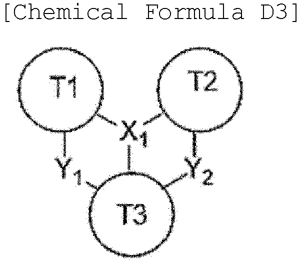 wherein,
X₁ is any one selected from among B, P, and P=O
T1 to T3, which are same or different, are each independently a substituted or unsubstituted aromatic hydrocarbon ring of 6 to 50 carbon atoms, or a substituted or unsubstituted heteroaromatic ring of 2 to 40 carbon atoms;
Y₁ is any one selected from among N-R₆₁, CR₆₂R₆₃, O, S, and SiR₆₄R₆₅;
Y₂ is any one selected from among N-R₆₆, CR₆₃R₆₈, O, S, and SiR₆₉R₇₀;
R₆₁ to R₇₀, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a cyano, and a halogen, and wherein at least one of R₆₁ to R₇₀ can be connected to at least one of T₁ to T₃ to form an additional mono- or polycyclic aliphatic or aromatic ring; 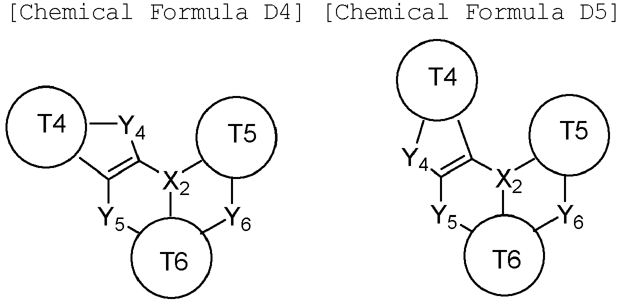 wherein,
X₂ is any one selected from among B, P, and P=O,
T4 to T6 are as defined for T1 to T3 in Chemical Formula D3,
Y₄ is any one selected from among N-R₆₁, CR₆₂R₆₃, O, S, and SiR₆₄R₆₅;
Y₅ is any one selected from among N-R₆₆, CR₆₇R₆₈, O, S, and SiR₆₉R₇₀;
Y₆ is any one selected from among N-R₇₁, CR₇₂R₇₃, O, S, and SiR₇₄R₇₅; and
R₆₁ to R₇₅ being as defined for R₆₁ to R₇₀ in Chemical Formula D3; 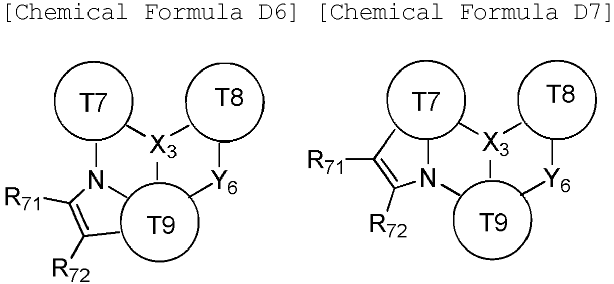
X₃ is any one selected from among B, P, and P=O,
T7 to T9 are defined as for T1 to T3 in Chemical Formula D3,
Y₆ is any one selected from among N-R₆₁, CR₆₂R₆₃, O, S, and SiR₆₄R₆₅;
R₆₁ to R₆₅ and R₇₁ to R₇₂ are each as defined for R₆₁ to R₇₀ in Chemical Formula D3,
wherein at least one of R₇₁ to R₇₂ can be connected to each other to form an additional mono- or polycyclic aliphatic or aromatic ring; 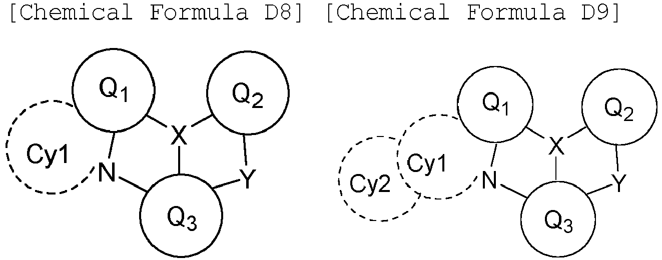 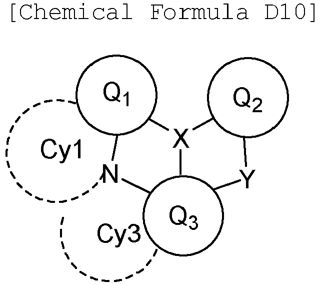 wherein,
X is any one selected from among B, P, and P=O,
Q₁ to Q₃ are each as defined for T1 to T3 in Chemical Formula D3,
Y is any one selected from among N-R₃, CR₄R₅, O, S, and Se,
R₃ to R₅ are each as defined for R₆₁ to R₇₀ in Chemical Formula D3,
R₃ to R₅ can each be connected to the Q₂ or Q₃ ring moiety to form an additional mono- or polycyclic aliphatic or aromatic ring,
R₄ and R₅ can be connected to each other to form an additional mono- or polycyclic aliphatic or aromatic ring,
the ring formed by Cy1 is a substituted or unsubstituted alkylene of 1 to 10 carbon atoms, except for the nitrogen (N) atom, the aromatic carbon atom of Q₁ to which the nitrogen (N) atom is connected, and the aromatic carbon atom of Q₁ to which Cy1 is to bond,
"Cy2" in Chemical Formula D9 forms a saturated hydrocarbon ring added to Cy1 wherein the ring formed by Cy2 is a substituted or unsubstituted alkylene of 1 to 10 carbon atoms, except for the carbon atoms included in Cy1, and
the ring formed by Cy3 in Chemical Formula D10 is a substituted or unsubstituted alkylene of 1 to 10 carbon atoms, except for the aromatic carbon atom of Q₃ to which Cy3 is to bond, the aromatic carbon atom of Q₃ to which the nitrogen (N) atom is connected, the nitrogen (N) atom, and the carbon atom of Cy1 to which the nitrogen (N) atom is connected,
wherein the term "substituted" in the expression "substituted or unsubstituted" used for compounds of Chemical Formulas D1 to D10 means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

14. The organic light-emitting diode of claim 1, wherein the first light-emitting layer comprises at least one of the compounds represented by Chemical Formula A and the second light-emitting layer employs an anthracene derivative represented by the following Chemical Formula E as a host: 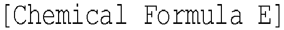 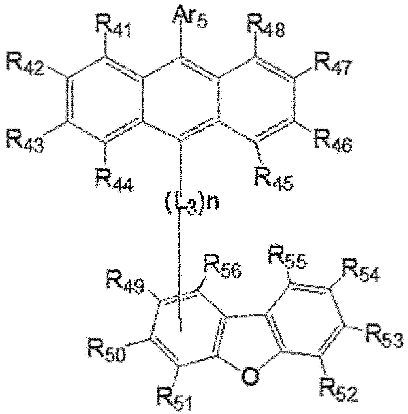 wherein
R₄₁ to R₅₆, which are same or different, are each independently any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted akylsilyl of 1 to 30 carbon atoms, a substituted or unsubstituted arylsilyl of 6 to 30 carbon atoms, a nitro, a cyano, and a halogen;
Ar₅ is a substituted or unsubstituted aryl of 6 to 50 carbon atoms or a substituted or unsubstituted heteroaryl of 2 to 50 carbon atoms;
L₃ is a single bond, a substituted or unsubstituted arylene of 6 to 20 carbon atoms, or a substituted or unsubstituted heteroarylene of 2 to 20 carbon atoms, and
n is an integer of 1 to 2 wherein when n is 2, the L₁'s are same or different,
wherein the team "substituted" in the expression "substituted or unsubstituted" used for compounds of Chemical Formula E means having at least one substituent selected from the group consisting of a deuterium atom, a cyano, a halogen, a hydroxyl, a nitro, an alkyl of 1 to 24 carbon atoms, a halogenated alkyl of 1 to 24 carbon atoms, alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

15. The organic light-emitting diode of claim 14, wherein the anthracene derivative represented by Chemical Formula E is an anthracene derivative represented by Chemical Formula E-1 or E-2: 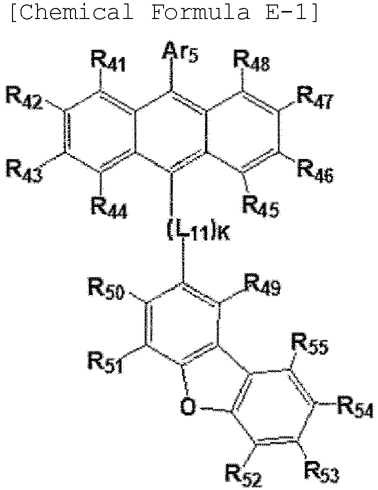 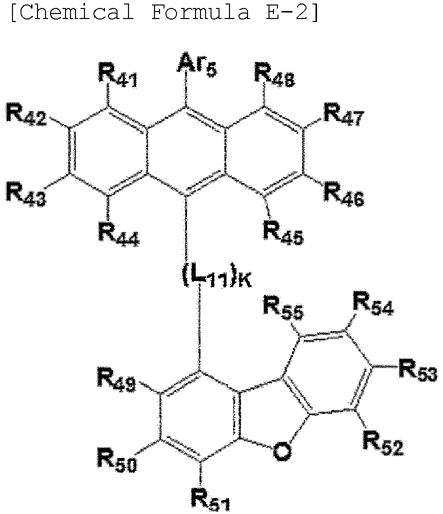 wherein,
R₄₁ to R₄₈, R₄₉ to R₅₅ which are same or different, are each independently any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
Ar₅ is a substituted or unsubstituted aryl of 6 to 50 carbon atoms or a substituted or unsubstituted heteroaryl of 2 to 50 carbon atoms;
L₁₁ is any one selected from among a single bond, a substituted or unsubstituted arylene of 6 to 20 carbon atoms, and a substituted or unsubstituted heteroarylene of 2 to 20 carbon atoms; and
k is an integer of 1 to 2 wherein when k is 2, the L₁₁'s are same or different,
wherein the team "substituted" in the expression "substituted or unsubstituted" used for compounds of Chemical Formulas E-1 and E-2 means having at least one substituent selected from the group consisting of a deuterium atom, a cyano, a halogen, a hydroxyl, a nitro, an alkyl of 1 to 24 carbon atoms, a halogenated alkyl of 1 to 24 carbon atoms, alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

16. The organic light-emitting diode of claim 14, wherein the anthracene derivative, represented by Chemical Formula E, in the second light-emitting layer bears at least one deuterium atom.

17. The organic light-emitting diode of claim 13, wherein the first dopant in the first light-emitting layer and the second dopant in the second light-emitting layer are same or different and are each independently selected from among the compounds represented by Chemical Formulas D1 to D10.
